# EUROPEAN PATENT SPECIFICATION

(11) **EP 1 155 131 B1**
(45) Date of publication and mention of the grant of the patent: **23.07.2008**
(21) Application number: 00908750.3
(22) Date of filing: 22.02.2000
(51) Int. Cl.: C12N 15/67, C12N 15/85, C12N 15/63, C12Q 1/68, C12N 15/10, C12N 15/62, G01N 33/50

(54) **VECTOR COMPRISING A SPLICE ACCEPTOR TRAP AND A POLY A TRAP AND THE CORRESPONDING EUKARYOTIC HOST CELLS**
VEKTOR MIT EINER SPLICE ACCEPTOR TRAP UND EINER POLY A TRAP UND ENTSPRECHENDE EUKARYOTISCHE WIRTSZELLEN
VECTOR COMPRENANT UNE TRAPPE D'ACCEPTEUR D'EPISSAGE ET UNE TRAPPE POLY A ET LES CELLULES EUCARYOTES CORRESPONDANTES

(30) Priority: 19.02.1999 US 253022; 08.03.1999 US 263814; 26.03.1999 US 276820
(43) Date of publication of application: 21.11.2001
(73) Proprietor: ABT Holding Company, Cleveland OH 44115-2634 (US)
(72) Inventor: HARRINGTON, John, J., Mentor, OH 44060 (US); SHERF, Bruce, Spencer, OH 44275 (US); RUNDLETT, Stephen, Chagrin Falls, OH 44022 (US)
(74) Representative: Wright, Simon Mark
(86) International application number: PCT/US2000/004429
(87) International publication number: WO 2000/049162

(56) References cited:
- EP-A- 0 775 747
- EP-A- 0 779 362
- WO-A-00/31236
- WO-A-93/18176
- WO-A-94/12650
- WO-A-95/31560
- WO-A-96/29411
- WO-A-97/24455
- WO-A-97/29202
- WO-A-98/12337
- WO-A-99/05527
- WO-A-99/07389
- WO-A-99/15650
- WO-A-99/50426
- YOSHIDA MICHIO ET AL: "A new strategy of gene trapping in ES cells using 3' RACE." TRANSGENIC RESEARCH, vol. 4, no. 4, 1995, pages 277-287, XP000946967 ISSN: 0962-8819
- M SALMINEN ET AL: "Efficient polyA trap approach allows the capture of genes specifically active in differentiated embryonic stem cells and in mouse embryos" DEVELOPMENTAL DYNAMICS,WILEY-LISS, INC., NEW YORK, NY,US, vol. 212, 1998, pages 326-333, XP002111635 ISSN: 1058-8388
- BUDAVARI, S. ET AL.: "The Merck Index" , MERCK RSEARCH LABORATORIES DIVISION, MERCK & CO. INC. , NEW YORK XP002150255 page 627, paragraph 3729 page 773, paragraph 4558 page 855, paragraph 5011 page 1488, paragraph 8864
- MORISHITA, K. ET AL.: "Retroviral activation of a novel gene encoding a zinc finger protein in IL-3-dependent myeloid leukemia cell lines." CELL, vol. 54, 9 September 1988 (1988-09-09), pages 831-40, XP002142120
- RAINERI, I. ET AL.: "HIV-1 promoter insertion revealed by selective detection of chimeric provirus-host gene transcripts." NUCLEIC ACID RESEARCH, vol. 20, no. 23, 1992, pages 6261-6, XP002142121

## Description

### BACKGROUND OF THE INVENTION

### Field of the Invention

The present invention is in the fields of molecular biology and cellular biology. The invention is directed generally to activation of gene expression or causing over-expression of a gene by recombination methods *in situ.* More specifically, the invention is directed to activation of endogenous genes by non-targeted integration of specialized activation vectors, which are provided by the invention, into the genome of a host cell. The invention also is directed to methods for the identification, activation, and isolation of genes that were heretofore undiscoverable, and to host cells and vectors comprising such isolated genes. Also referred to are isolated genes, gene products, nucleic acid molecules, and compositions comprising such genes, gene products and nucleic acid molecules, that may be used in a variety of therapeutic and diagnostic applications. Thus, by the present invention, endogenous genes, including those associated with human disease and development, may be identified, activated, and isolated without prior knowledge of the sequence, structure, function, or expression profile of the genes.

### Related Art

Identification and over-expression of novel genes associated with human disease is an important step towards developing new therapeutic drugs. Current approaches to creating libraries of cells for protein over-expression are based on the production and cloning of cDNA. Thus, in order to identify a new gene using this approach, the gene must be expressed in the cells that were used to make the library. The gene also must be expressed at sufficient levels to be adequately represented in the library. This is problematic because many genes are expressed only in very low quantities, in a rare population of cells, or during short developmental periods.

Furthermore, because of the large size of some mRNAs, it is difficult or impossible to produce full length cDNA molecules capable of expressing the biologically active protein. Lack of full-length cDNA molecules has also been observed for small mRNAs and is thought to be related to sequences in the message that are difficult to produce by reverse transcription or that are unstable during propagation in bacteria. As a result, even the most complete cDNA libraries express only a fraction of the entire set of possible genes.

Finally, many cDNA libraries are produced in bacterial vectors. Use of these vectors to express biologically active mammalian proteins is severely limited since most mammalian proteins do not fold correctly and/or are improperly glycosylated in bacteria.

Therefore, a method for creating a more representative library for protein expression, capable of facilitating faithful expression of biologically active proteins, would be extremely valuable.

Current methods for over-expressing proteins involve cloning the gene of interest and placing it, in a construct, next to a suitable promoter/enhancer, polyadenylation signal, and splice site, and introducing the construct into an appropriate host cell.

An alternative approach involves the use of homologous recombination to activate gene expression by targeting a strong promoter or other regulatory sequence to a previously identified gene.

WO 90/14092 describes *in situ* modification of genes, in mammalian cells, encoding proteins of interest. This application describes single-stranded oligonucleotides for site-directed modification of genes encoding proteins of interest. A marker may also be included. However, the methods are limited to providing an oligonucleotide sequence substantially homologous to a target site. Thus, the method requires knowledge of the site required for activation by site-directed modification and homologous recombination. Novel genes are not discoverable by such methods.

WO 91/06667 describes methods for expressing a mammalian gene *in situ.* With this method, an amplifiable gene is introduced next to a target gene by homologous recombination. When the cell is then grown in the appropriate medium, both the amplifiable gene and the target gene are amplified and there is enhanced expression of the target gene. As above, methods of introducing the amplifiable gene are limited to homologous recombination, and are not useful for activating novel genes whose sequence (or existence) is unknown.

WO 91/01140 describes the inactivation of endogenous genes by modification of cells by homologous recombination. By these methods, homologous recombination is used to modify and inactivate genes and to produce cells which can serve as donors in gene therapy.

WO 92/20808 describes methods for modifying genomic target sites *in situ.* The modifications are described as being small, for example, changing single bases in DNA. The method relies upon genomic modification using homologous DNA for targeting.

WO 92/19255 describes a method for enhancing the expression of a target gene, achieved by homologous recombination in which a DNA sequence is integrated into the genome or large genomic fragment. This modified sequence can then be transferred to a secondary host for expression. An amplifiable gene can be integrated next to the target gene so that the target region can be amplified for enhanced expression. Homologous recombination is necessary to this targeted approach.

WO 93/09222 describes methods of making proteins by activating an endogenous gene encoding a desired product. A regulatory region is targeted by homologous recombination and replacing or disabling the region normally associated with the gene whose expression is desired. This disabling or replacement causes the gene to be expressed at levels higher than normal.

WO 94/12650 describes a method for activating expression of and amplifying an endogenous gene *in situ* in a cell, which gene is not expressed or is not expressed at desired levels in the cell. The cell is transfected with exogenous DNA sequences which repair, alter, delete, or replace a sequence present in the cell or which are regulatory sequences not normally functionally linked to the endogenous gene in the cell. In order to do this, DNA sequences homologous to genomic DNA sequences at a preselected site are used to target the endogenous gene. In addition, amplifiable DNA encoding a selectable marker can be included. By culturing the homologously recombinant cells under conditions that select for amplification, both the endogenous gene and the amplifiable marker are co-amplified and expression of the gene increased.

WO 95/31560 describes DNA constructs for homologous recombination. The constructs include a targeting sequence, a regulatory sequence, an exon, and an unpaired splice donor site. The targeting is achieved by homologous recombination of the construct with genomic sequences in the cell and allows the production of a protein *in vitro* or *in vivo.*

WO 96/29411 describes methods using an exogenous regulatory sequence, an exogenous exon, either coding or non-coding, and a splice donor site introduced into a preselected site in the genome by homologous recombination. In this application, the introduced DNA is positioned so that the transcripts under control of the exogenous regulatory region include both the exogenous exon and endogenous exons present in either the thrombopoietin, DNase I, or β-interferon genes, resulting in transcripts in which the exogenous and exogenous exons are operably linked. The novel transcription units are produced by homologous recombination.

U.S. Patent No. 5,272,071 describes the transcriptional activation of transcriptionally silent genes in a cell by inserting a DNA regulatory element capable of promoting the expression of a gene normally expressed in that cell. The regulatory element is inserted so that it is operably linked to the normally silent gene. The insertion is accomplished by means of homologous recombination by creating a DNA construct with a segment of the normally silent gene (the target DNA) and the DNA regulatory element used to induce the desired transcription.

U.S. Patent No. 5, 578,461 discusses activating expression of mammalian target genes by homologous recombination. A DNA sequence is integrated into the genome or a large genomic fragment to enhance the expression of the target gene. The modified construct can then be transferred to a secondary host. An amplifiable gene can be integrated adjacent to the target gene so that the target region is amplified for enhanced expression.

WO99/07389 is concerned with targeted gene discovery.

Both of the above approaches (construction of an over-expressing construct by cloning or by homologous recombination *in vivo)* require the gene to be cloned and sequenced before it can be over-expressed. Furthermore, using homologous recombination, the genomic sequence and structure must also be known.

Unfortunately, many genes have not yet been identified and/or sequenced. Thus, a method for over-expressing a gene of interest, whether or not it has been previously cloned, and whether or not its sequence and structure are known, would be useful.

### BRIEF SUMMARY OF THE INVENTION

The invention is, therefore, generally directed to methods for over-expressing an endogenous gene in a cell, comprising introducing a vector containing a transcriptional regulatory sequence into the cell, allowing the vector to integrate into the genome of the cell by non-homologous recombination, and allowing over-expression of the endogenous gene in the cell. The method does not require previous knowledge of the sequence of the endogenous gene or even of the existence of the gene. Hence, the invention is directed to non-targeted gene activation, which as used herein means the activation of endogenous genes by non-targeted or non-homologous (as opposed to targeted or homologous) integration of specialized activation vectors into the genome of a host cell.

Thus, the present invention provides a eukaryotic host cell which is *in vitro,* the cell comprising a vector non-homologously integrated into the genome of the cell, the vector comprising:
(a) a first promoter operably linked to an exon and an unpaired splice donor site; and
(b) a second promoter operably linked to a selectable marker lacking a polyadenylation signal,
where said first and second promoters are present in said vector in the same orientation, both the first and second promoters function in the eukaryotic cell, the vector is non-homologously integrated into the genome of the cell so that the unpaired splice donor site of the vector is paired with a splice acceptor site from an endogenous gene and where the endogenous gene is activated or over-expressed to produce protein.

Also referred to are novel vector constructs for activating gene expression or over-expressing a gene through non-homologous recombination. The novel construct lacks homologous targeting sequences. That is, it does not contain nucleotide sequences that target host cell DNA and promote homologous recombination at the target site, causing over-expressing of a cellular gene via the introduced transcriptional regulatory sequence.

Novel vector constructs include constructs with a transcriptional regulatory sequence operably linked to a translational start codon, a signal secretion sequence, and an unpaired splice donor site; constructs with a transcriptional regulatory sequence, operably linked to a translation start codon, an epitope tag, and an unpaired splice donor site; constructs containing a transcriptional regulatory sequence operably linked to a translational start codon, a signal sequence and an epitope tag, and an unpaired splice donor site; constructs containing a transcriptional regulatory sequence operably linked to a translation start codon, a signal secretion sequence, an epitope tag, and a sequence-specific protease site, and an unpaired splice donor site.

The vector construct contains one or more selectable markers for recombinant host cell selection. Alternatively, selection can be effected by phenotypic selection for a trait provided by the activated endogenous gene product.

These vectors, and indeed any of the vectors disclosed herein, and variants of the vectors that will be readily recognized by one of ordinary skill in the art, can be used in any of the methods described herein to form any of the compositions producible by these methods.

The transcriptional regulatory sequence used in the vector constructs of the invention includes a promoter. In preferred embodiments, the promoter is a viral promoter. In highly preferred embodiments, the viral promoter is the cytomegalovirus immediate early promoter. In alternative embodiments, the promoter is a cellular, non-viral promoter or inducible promoter.

The transcriptional regulatory sequence used in the vector construct of the invention may also include, but is not limited to, an enhancer. In preferred embodiments, the enhancer is a viral enhancer. In highly preferred embodiments, the viral enhancer is the cytomegalovirus immediate early enhancer. In alternative embodiments, the enhancer is a cellular non-viral enhancer.

In preferred embodiments of the methods described herein, the vector construct be, or may contain, linear RNA or DNA.

The cell containing the vector may be screened for expression of the gene.

The cell over-expressing the gene can be cultured *in vitro* under conditions favoring the production, by the cell, of desired amounts of the gene product (also referred to interchangeably herein as the "expression product") of the endogenous gene that has been activated or whose expression has been increased. The expression product can then be isolated and purified to use, for example, in protein therapy or drug discovery.

Alternatively, the cell expressing the desired gene product can be allowed to express the gene product *in vivo*. In certain such aspects, the cell containing a vector construct of the invention integrated into its genome may be introduced into a eukaryote (such as a vertebrate, particularly a mammal, more particularly a human) under conditions favoring the overexpression or activation of the gene by the cell *in vivo* in the eukaryote. In related such aspects, the cell may be isolated and cloned prior to being introduced into the eukaryote.

It is to be understood, however, that any vector used in the methods described herein can include one or more amplifiable markers. Thereby, amplification of both the vector and the DNA of interest (i.e., containing the over-expressed gene) occurs in the cell, and further enhanced expression of the endogenous gene is obtained. Accordingly, methods can include a step in which the endogenous gene is amplified.

The invention is also directed to an *in vitro* method for activating or over-expressing an endogenous gene to produce protein in a cell comprising:
(a) transfecting a genome-containing cell with a vector where the vector comprises: (i) a first promoter operably linked to an exon and an unpaired splice donor site; and (ii) a second promoter operably linked to a selectable marker lacking a polyadenylation signal, where said first and second promoters are present in said vector in the same orientation and both the first and second promoters function in the eukaryotic cell; and
(b) allowing the vector to integrate non-homologously into the genome of the cell so that the unpaired donor site of the vector is paired with a splice acceptor site of the endogenous gene.

The cell containing the vector may be screened for expression of the gene.

The cell over-expressing the gene can be cultured *in vitro* so as to produce desirable amounts of the gene product of the endogenous gene whose expression has been activated or increased. The gene product can then be isolated and purified.

Alternatively, the cell can be allowed to express the desired gene product *in vivo.*

Any of the vector constructs employed in the invention can also include a secretion signal sequence. The secretion signal sequence is arranged in the construct so that it will be operably linked to the activated endogenous protein. Thereby, secretion of the protein of interest occurs in the cell, and purification of that protein is facilitated. Accordingly, methods can include a step in which the protein expression product is secreted from the cell.

The invention also encompasses cells made by any of the above methods. The invention encompasses cells in which the vector constructs have integrated into the cellular genome, and cells which are over-expressing desired gene products from an endogenous gene, over-expression being driven by the introduced transcriptional regulatory sequence.

The cells can be isolated and cloned.

The methods can be carried out in any cell of eukaryotic origin; such as fungal, plant or animal. In preferred embodiments, the methods of the invention may be carried out in vertebrate cells, and particularly mammalian cells including but not limited to rat, mouse, bovine, porcine, sheep, goat and human cells, and more particularly in human cells.

A single cell made by the methods described above can over-express a single gene or more than one gene. More than one gene in a cell can be activated by the integration of a single type of construct into multiple locations in the genome. Similarly, more than one gene in a cell can be activated by the integration of multiple constructs (i.e., more than one type of construct) into multiple locations in the genome. Therefore, a cell can contain only one type of vector construct or different types of constructs, each capable of activating an endogenous gene.

The invention is also directed to methods for making the cells described above by one or more of the following: introducing one or more of the vector constructs of the invention into a cell; allowing the introduced construct(s) to integrate into the genome of the cell by non-homologous recombination; allowing over-expression of one or more endogenous genes in the cell; and isolating and cloning the cell. The invention is also directed to cells produced by such methods, which may be isolated cells.

The invention also encompasses methods for using the cells described above to over-express a gene, such as an endogenous cellular gene, that has been characterized (for example, sequenced), uncharacterized (for example, a gene whose function is known but which has not been cloned or sequenced), or a gene whose existence was, prior to over-expression, unknown. The cells can be used to produce desired amounts of an expression product in *vitro* or *in vivo.* If desired, this expression product can then be isolated and purified, for example by cell lysis or by isolation from the growth medium (as when the vector contains a secretion signal sequence).

The invention also encompasses libraries of cells made by the above described methods. Thus, the invention also provides a library of cells of the invention. A library can encompass all of the clones from a single transfection experiment or a subset of clones from a single transfection experiment. The subset can over-express the same gene or more than one gene, for example, a class of genes. The transfection can have been done with a single construct or with more than one construct.

A library can also be formed by combining all of the recombinant cells from two or more transfection experiments, by combining one or more subsets of cells from a single transfection experiment or by combining subsets of cells from separate transfection experiments. The resulting library can express the same gene, or more than one gene, for example, a class of genes. Again, in each of these individual transfections, a unique construct or more than one construct can be used.

Libraries can be formed from the same cell type or different cell types.

The invention is also directed to methods for making libraries by selecting various subsets of cells from the same or different transfection experiments.

The invention is also directed to methods of using the above-described cells or libraries of cells to over-express or activate endogenous genes, or to obtain the gene expression products of such over-expressed or activated genes. According to this aspect of the invention, the cell or library may be screened for the expression of the gene and cells that express the desired gene product may be selected. The cell can then be used to isolate or purify the gene product for subsequent use. Expression in the cell can occur by culturing the cell *in vitro,* under conditions favoring the production of the expression product of the endogenous gene by the cell, or by allowing the cell to express the gene *in vivo*.

In preferred embodiments of the invention, the methods include a process wherein the expression product is isolated or purified. In highly preferred embodiments, the cells expressing the endogenous gene product are cultured under conditions favoring production of sufficient amounts of gene product for commercial application, and especially for diagnostic, therapeutic and drug discovery uses.

Any of the methods can further comprise introducing double-strand breaks into the genomic DNA in the cell prior to or simultaneously with vector integration.

Also referred to are vector constructs that are useful for activating expression of endogenous genes and for isolating the mRNA and cDNA corresponding to the activated genes.

In another embodiment, The invention may employ a vector construct comprising (a) a first transcriptional regulatory sequence operably linked to a selectable marker lacking a polyadenylation signal; and (b) a second transcriptional regulatory sequence operably linked to an exon-splice donor site complex, wherein the first transcriptional regulatory sequence is in the same orientation in the vector construct as is the second transcriptional regulatory sequence, and wherein the first transcriptional regulatory sequence is upstream of the second transcriptional regulatory sequence in the vector construct.

In additional embodiments, the invention may employ vector constructs comprising a transcriptional regulatory sequence operably linked to a selectable marker lacking a polyadenylation signal, and further comprising an unpaired splice donor site.

The invention may employ vector constructs comprising a first transcriptional regulatory sequence operably linked to a selectable marker lacking a polyadenylation signal, and further comprising a second transcriptional regulatory sequence operably linked to an unpaired splice donor site.

According to the invention, the transcriptional regulatory sequence (or first or second transcriptional regulatory sequence, in vector constructs having more than one transcriptional regulatory sequence) comprises a promoter and is preferably a promoter, including an animal cell promoter, a plant cell promoter, or a fungal cell promoter, most preferably a promoter selected from the group consisting of a CMV immediate early gene promoter, an SV40 T antigen promoter, and a β-actin promoter. Other promoters of animal, plant, or fungal cell origin that may be used in accordance with the invention are known in the art and will be familiar to one of ordinary skill in view of the teachings herein.

The selectable marker used in the vector constructs of the invention may be any marker or marker gene that, upon integration of a vector containing the selectable marker into the host cell genome, permits the selection of a cell containing or expressing the marker gene. Suitable such selectable markers include, but are not limited to, a neomycin gene, a hypoxanthine phosphribosyl transferase gene, a puromycin gene, a dihydrooratase gene, a glutamine synthetase gene, a histidine D gene, a carbamyl phosphate synthase gene, a dihydrofolate reductase gene, a multidrug resistance 1 gene, an aspartate transcarbamylase gene, a xanthine-guanine phosphoribosyl transferase gene, an adenosine deaminase gene, and a thymidine kinase gene.

The invention also is directed to eukaryotic host cells, which may be isolated host cells, comprising one or more of the vector constructs of the invention. Preferred such eukaryotic host cells include, but are not limited to, animal cells (including, but not limited to, mammalian (particularly human) cells, insect cells, avian cells, annelid cells, amphibian cells, reptilian cells, and fish cells), plant cells, and fungal (particularly yeast) cells. In certain such host cells, the vector construct may be integrated into the genome of the host cell.

Also referred to are primer molecules comprising a PCR-amplifiable sequence and a degenerate 3' terminus. Primer molecules according to this aspect preferably have the general structure:

5'-(dT)ₐ-X-N_{b}-TTTATT-3',

wherein a is a whole number from 1 to 100 (preferably from 10 to 30), X is a PCR-amplifiable sequence consisting of a nucleic acid sequence of about 10-20 nucleotides in length, N is any nucleotide, and b is a whole number from 0 to 6.
One preferred such primer has the nucleotide sequence 5'-TTTTTTTT-
TTTTCGTCAGCGGCCGCATCNNNNTTTATT-3' (SEQ ID NO:10).
The primer molecules may be biotinylated.

Also referred to are methods for first strand cDNA synthesis comprising (a) annealing a first primer of the invention (such as the primer described above) to an RNA template molecule to form an first primer-RNA complex, and (b) treating this first primer-RNA complex with reverse transcriptase and one or more deoxynucleoside triphosphate molecules under conditions favoring the reverse transcription of the first primer-RNA complex to synthesize a first strand cDNA.

Also referred to are methods for isolating activated genes, particularly from a host cell genome. These methods exploit the structure of the mRNA molecules produced using the non-targeted gene activation vectors of the invention. One such method comprises, for example, (a) introducing a vector construct comprising a transcriptional regulatory sequence and an unpaired splice donor site into a host cell (preferably one of the eukaryotic host cells described above), (b) allowing the vector construct to integrate into the genome of the host cell by non-homologous recombination, under conditions such that the vector activates an endogenous gene comprising an exon in the genome, (c) isolating RNA from the host cell, (d) synthesizing first strand cDNA according to the method described above, (e) annealing a second primer specific for the vector-encoded exon to the first strand cDNA to create a second primer-first strand cDNA complex, and (f) contacting the second primer-first strand cDNA complex with a DNA polymerase under conditions favoring the production of a second strand cDNA substantially complementary to the first strand cDNA. Methods according to this aspect may comprise one or more additional steps, such as treating the second strand cDNA with a restriction enzyme that cleaves at a restriction site located on the vector downstream of the unpaired splice donor site, or amplifying the second strand cDNA using a third primer specific for the vector-encoded exon and a fourth primer specific for the second primer. Also referred to are isolated genes produced according to these methods, and to vectors (which may be expression vectors) and host cells comprising these isolated genes. The invention also is directed to methods of producing a polypeptide, comprising cultivating a host cell comprising the isolated gene (or a vector, particularly an expression vector, comprising the isolated gene), and culturing the host cell under conditions favoring the expression by the host cell of a polypeptide encoded by the isolated gene. The invention also provides additional methods of producing a polypeptide, comprising introducing into a host cell a vector comprising a transcriptional regulatory sequence operably linked to an exonic region followed by an unpaired splice donor site, and culturing the host cell under conditions favoring the expression by said host cell of a polypeptide encoded by the exonic region, wherein the exon contains a translational start site positioned at any of the open reading frame positions relative to the 5'-most base of the unpaired splice donor site (e.g., the "A" in the ATG start codon may be at position -3 or at an increment of 3 bases upstream therefrom (e.g., -6, -9,-12, -15, -18, etc.), at position -2 or at an increment of 3 bases upstream therefrom (e.g., -5, -8, -11,-14, -17, -20, etc.), or at position -1 or at an increment of 3 bases upstream therefrom (e.g., -4, -7, -10,-13, -16, -19, etc.), relative to the 5'-most base of the splice donor site). In related embodiments, the methods of the invention may further comprise isolating the polypeptide. Also referred to are polypeptides, which may or may not be isolated polypeptides, produced according to these methods.

Other preferred embodiments of the present invention will be apparent to one of ordinary skill in light of the following drawings and description of the invention, and of the claims.

### BRIEF DESCRIPTION OF THE DRAWINGS

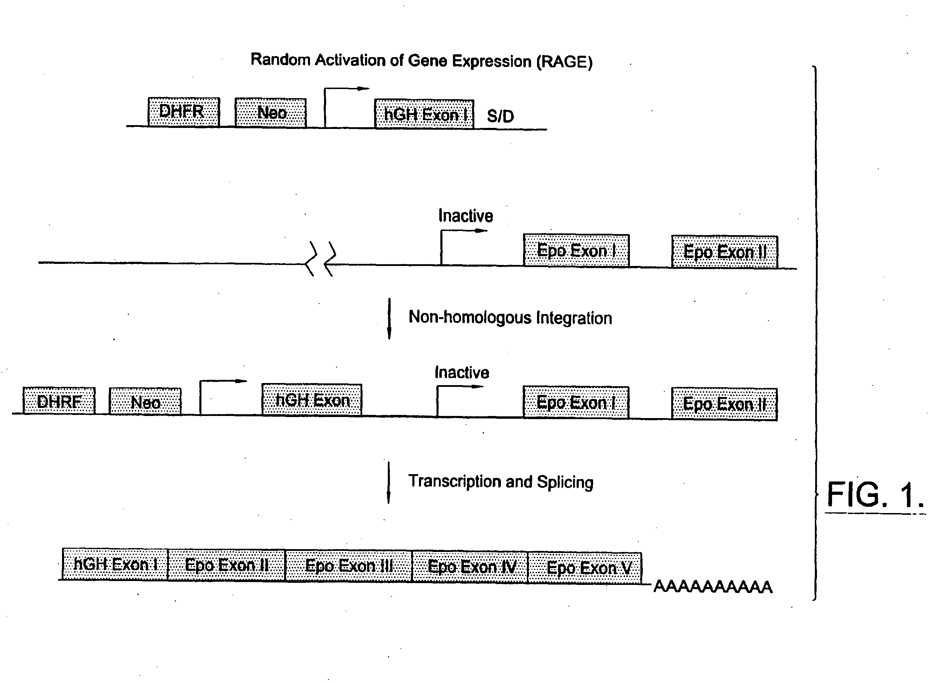

**FIGURE 1****.** Schematic diagram of gene activation events described herein. The activation construct is transfected into cells and allowed to integrate into the host cell chromosomes at DNA breaks. If breakage occurs upstream of a gene of interest (e.g., Epo), and the appropriate activation construct integrates at the break such that its regulatory sequence becomes operably linked to the gene of interest, activation of the gene will occur. Transcription and splicing produce a chimeric RNA molecule containing exonic sequences from the activation construct and from the endogenous gene. Subsequent translation will result in the production of the protein of interest. Following isolation of the recombinant cell, gene expression can be further enhanced via gene amplification.

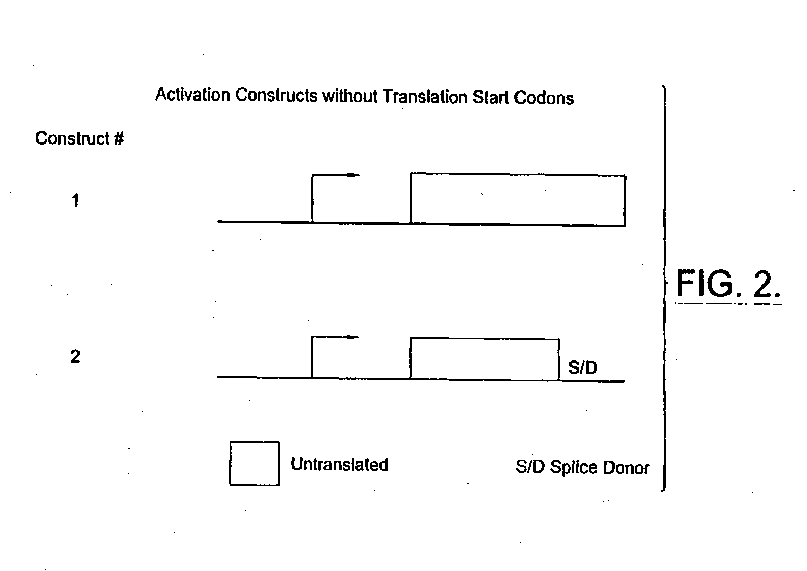

**FIGURE 2****.** Schematic diagram of non-translated activation constructs. The arrows denote promoter sequences. The exonic sequences are shown as open boxes and the splice donor sequence is indicated by S/D. Construct numbers corresponding to the description below are shown on the left. The selectable and amplifiable markers are not shown.

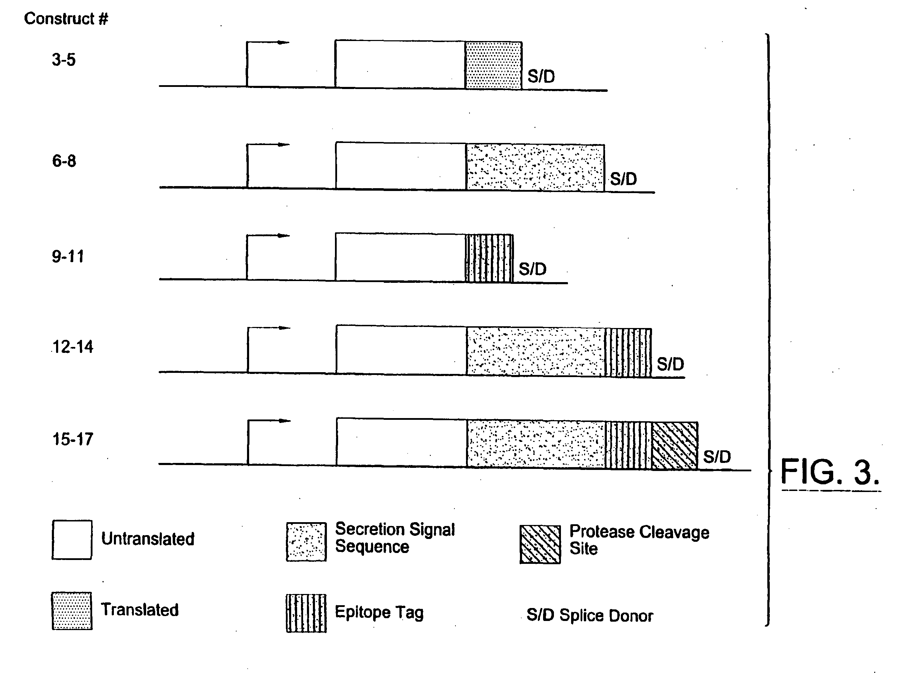

**FIGURE 3****.** Schematic diagram of translated activation constructs. The arrows denote promoter sequences. The exonic sequences are shown as open boxes and the splice donor sequence is indicated by S/D. The translated, signal peptide, epitope tag, and protease cleavage sequences are shown in the legend below the constructs. Construct numbers corresponding to the description below are shown on the left. The selectable and amplifiable markers are not shown.

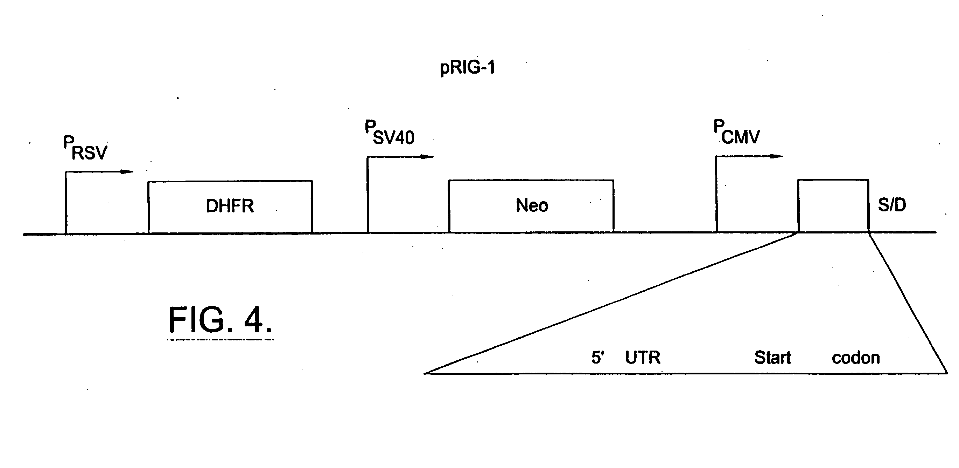

**FIGURE 4****.** Schematic diagram of an activation construct capable of activating endogenous genes.

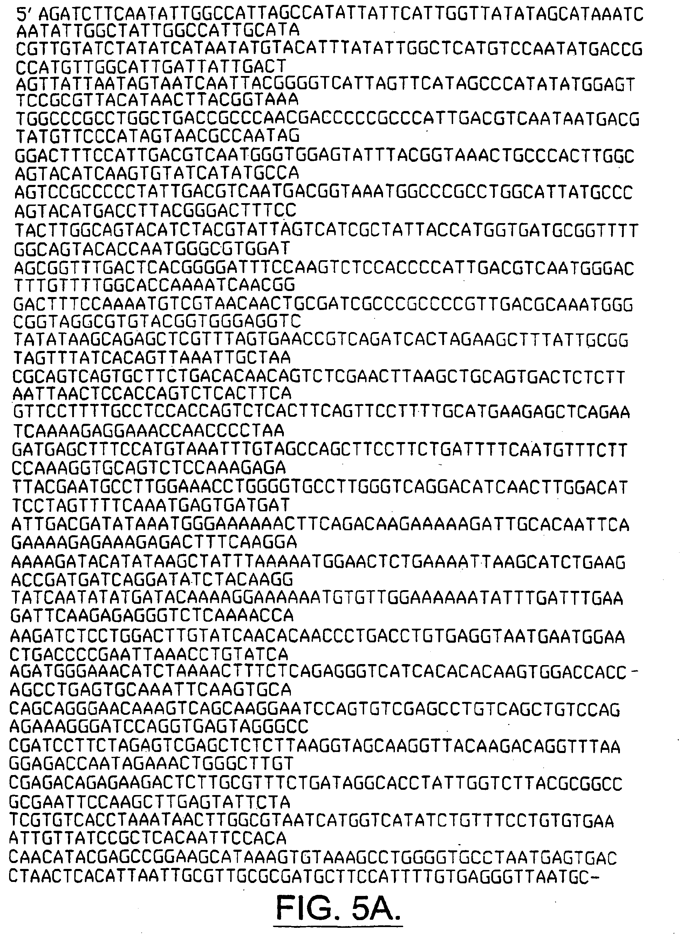

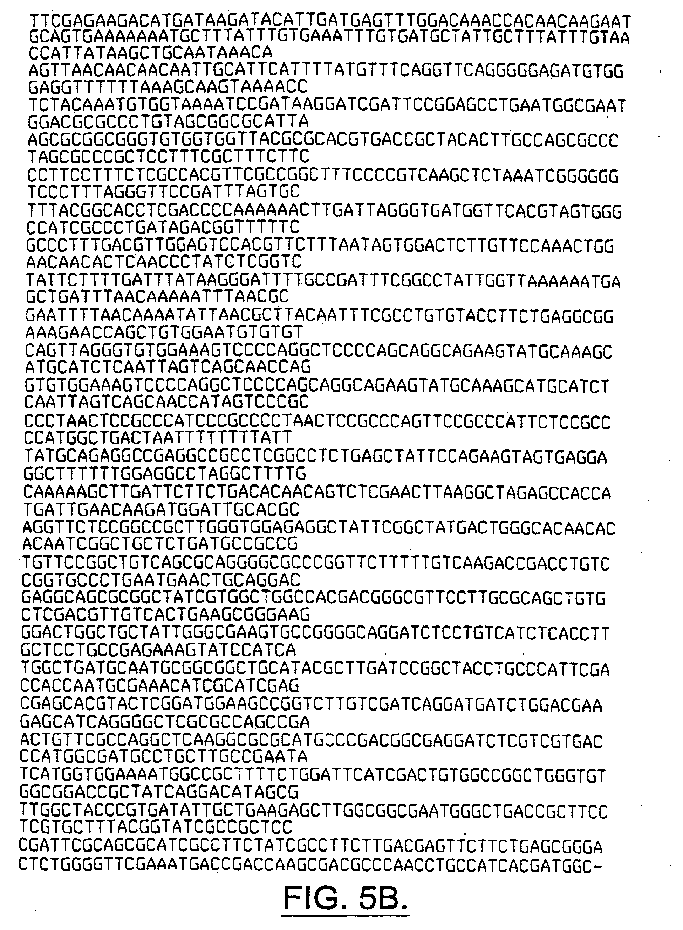

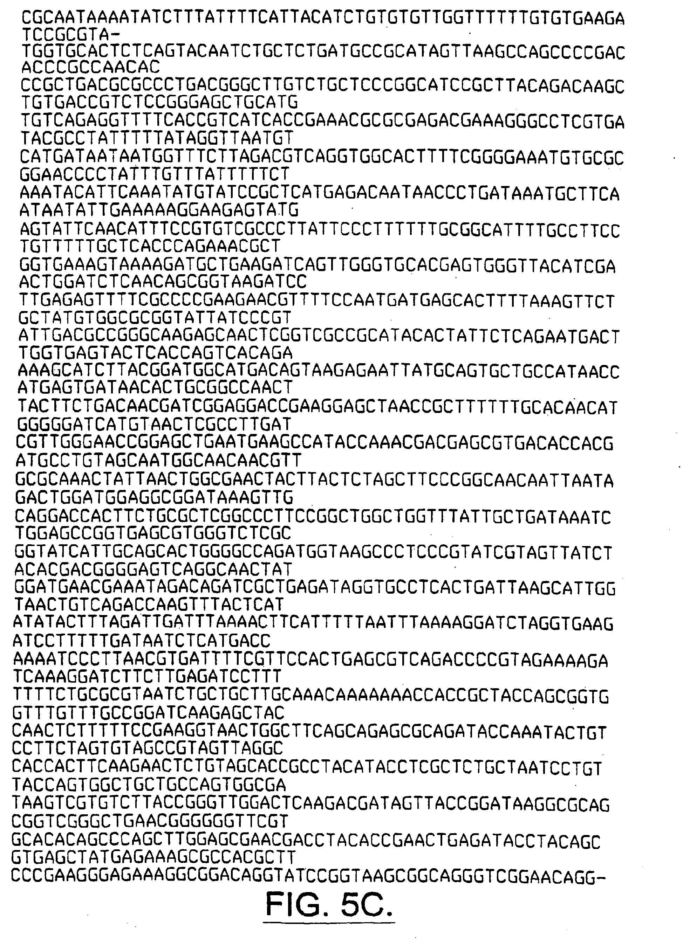

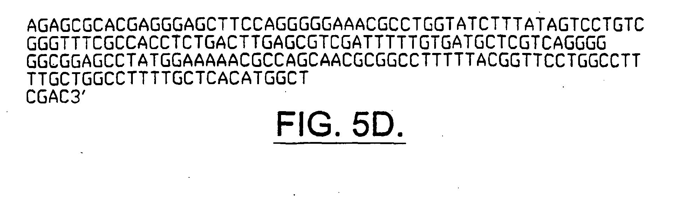

**FIGURE 5A-5D**. Nucleotide sequence of pRIG8R1-CD2 (SEQ ID NO:7).

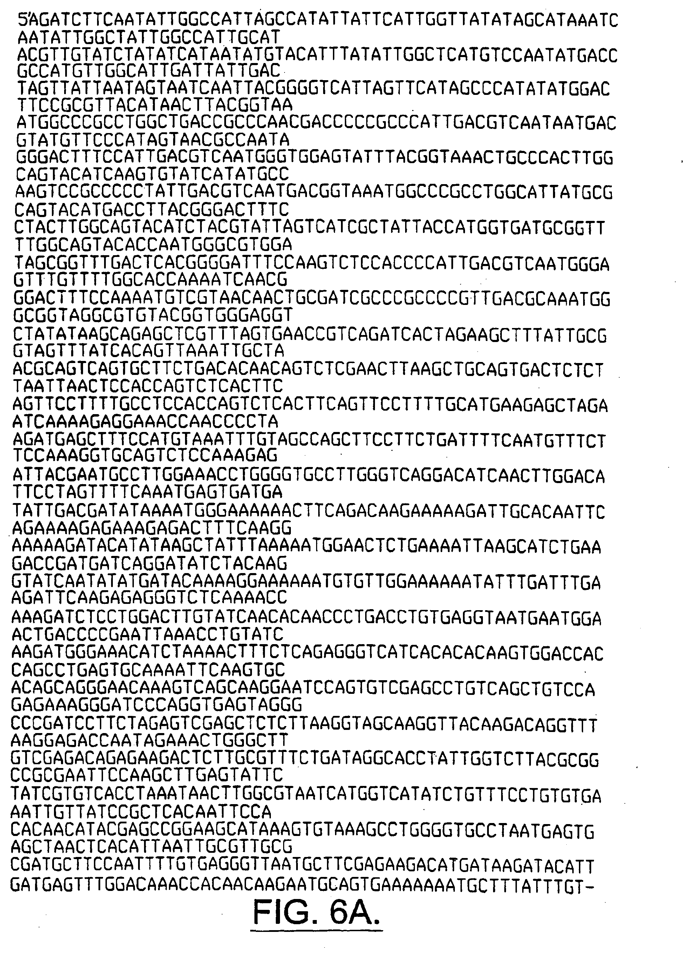

**FIGURES 6A-6C****.** Nucleotide sequence of pRIG8R2-CD2 (SEQ ID NO:8).

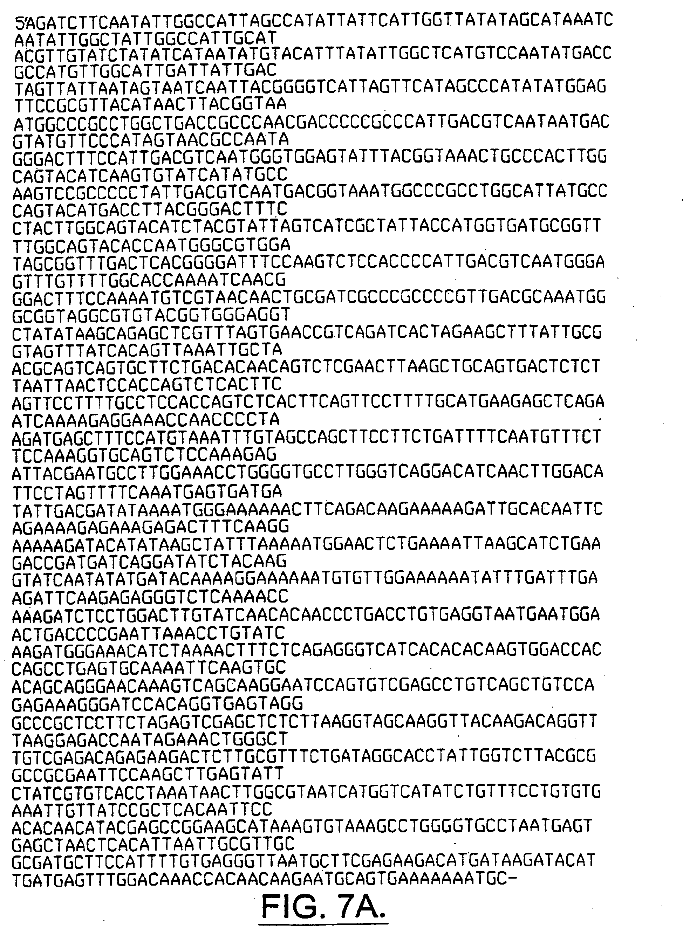

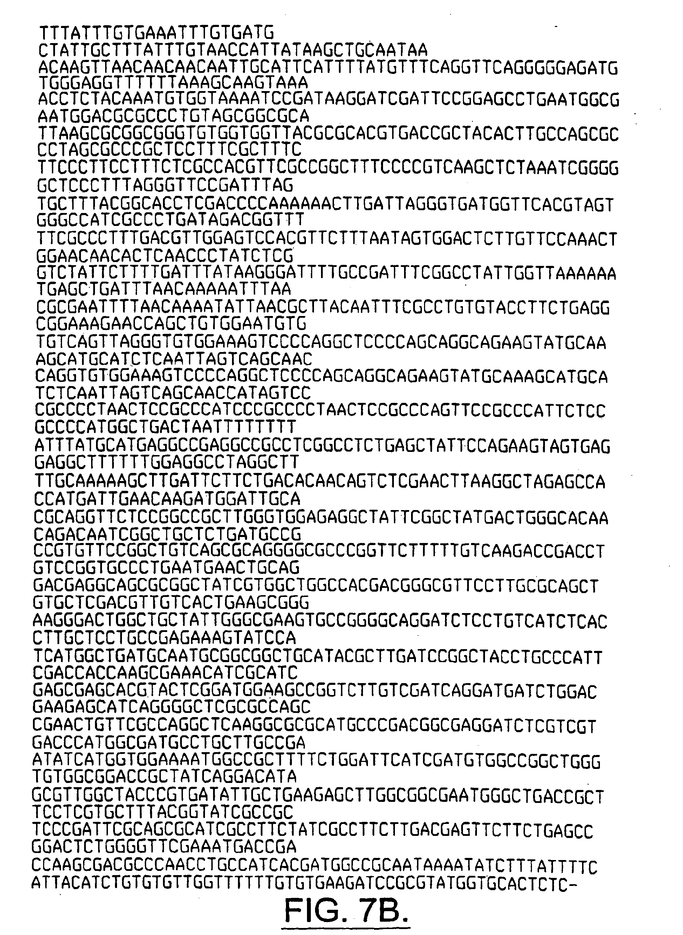

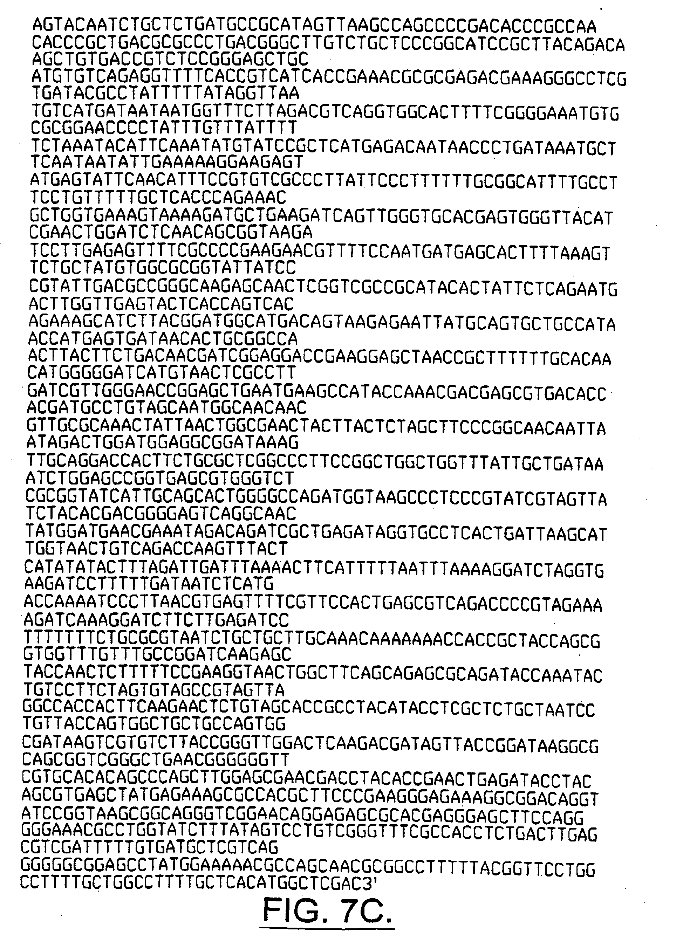

**FIGURES 7A-7C****.** Nucleotide sequence of pRIG8R3-CD2 (SEQ ID NO:9).

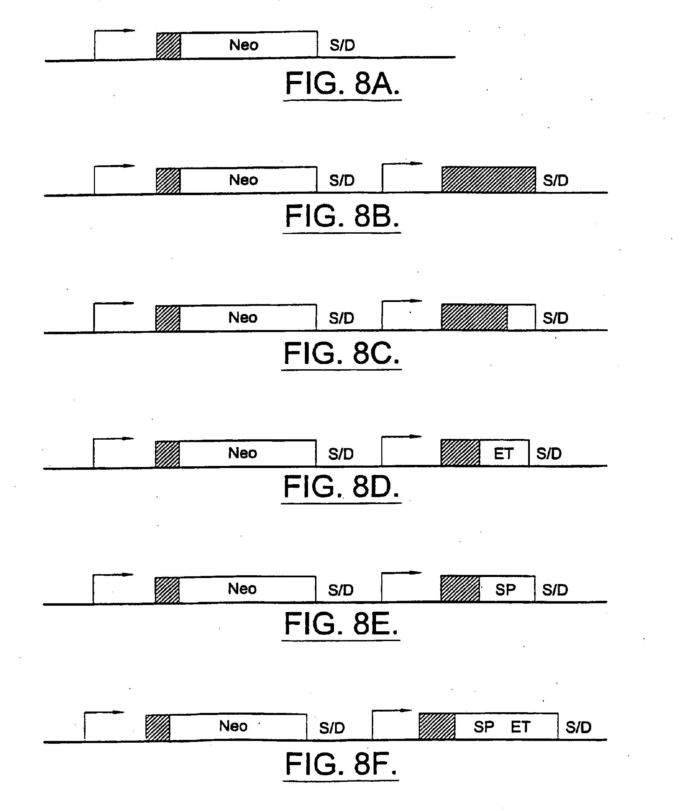

**FIGURES 8A-8F****.** Examples of poly(A) trap vectors. Each vector is illustrated schematically in its linearized form. Each horizontal line represents a DNA molecule. The arrows denote promoter sequences located on the DNA molecule, and face in the direction of transcription. Transcribed regions include all sequences located downstream of a promoter. Untranslated regions are designated by hatched boxes and open reading frames are designated by open boxes. The following designations were used: splice donor site (S/D), signal secretion sequence (SP), epitope tag (ET), neomycin resistance gene (Neo). In the vectors depicted in Figures 8B-8E, it is possible to omit the splice donor site immediately downstream of the Neo gene. In vectors lacking a splice donor site between the neo gene and the downstream promoter, the Neo transcript will utilize the splice donor site located 3' of the downstream promoter. In addition, as shown in the vectors depicted in Figures 8B-8E, a downstream promoter may drive expression of an exon. It is recognized that this exon, when present, may encode codons in any reading frame. Using multiple vectors, codons in each of the 3 possible reading frames can be created.

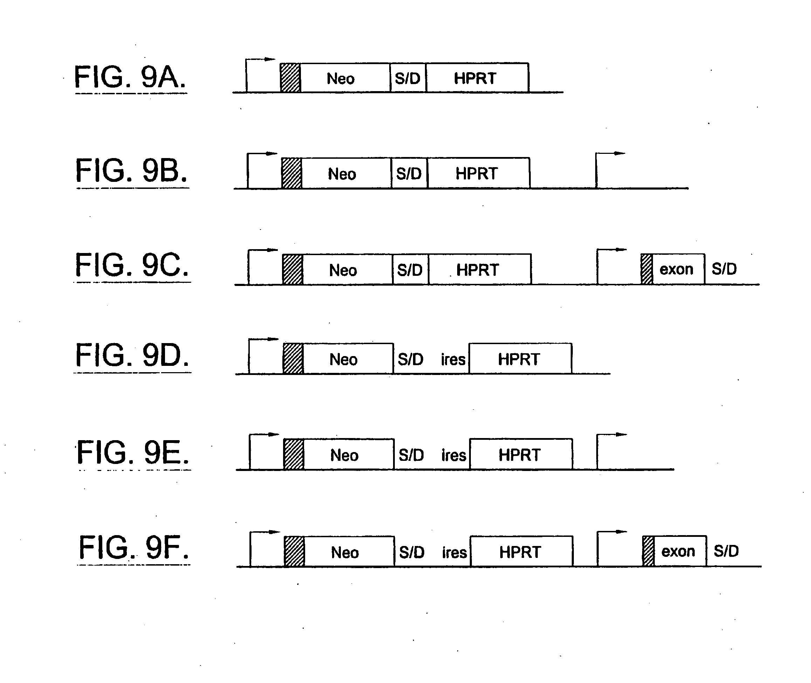

**FIGURES 9A-9F****.** Examples of splice acceptor trap vectors containing a positive and a negative selectable marker driven from a single promoter. Each vector is illustrated schematically in its linearized form. Each horizontal line represents a DNA molecule. The arrows denote promoter sequences located on the DNA molecule, and face in the direction of transcription. Transcribed regions include all sequences located downstream of a promoter. Untranslated regions are designated by hatched boxes. Poly(A) signals are not present in these examples. As described in the specification, however, poly(A) signals may be placed on the vector 3' of either or both selectable markers. The following designations were used: splice donor site (S/D), signal secretion sequence (SP), epitope tag (ET), internal ribosome entry site (ires), hypoxanthine phosphoribosyl transferase (HPRT), and neomycin resistance gene (Neo). In these examples, Neo represents the positive selectable marker and HPRT represents the negative selectable marker. In the vectors shown in Figures 9C and 9F, the region designated exon contains a translation start codon. As described in the Detailed Description, the exon may encode a methionine residue, a partial signal sequence, a full signal secretion sequence, a portion of a protein, or an epitope tag. In addition, the codons may be present in any reading frame relative to the splice donor site. In other vector examples not shown, the region designated exon lacks a translation start codon.

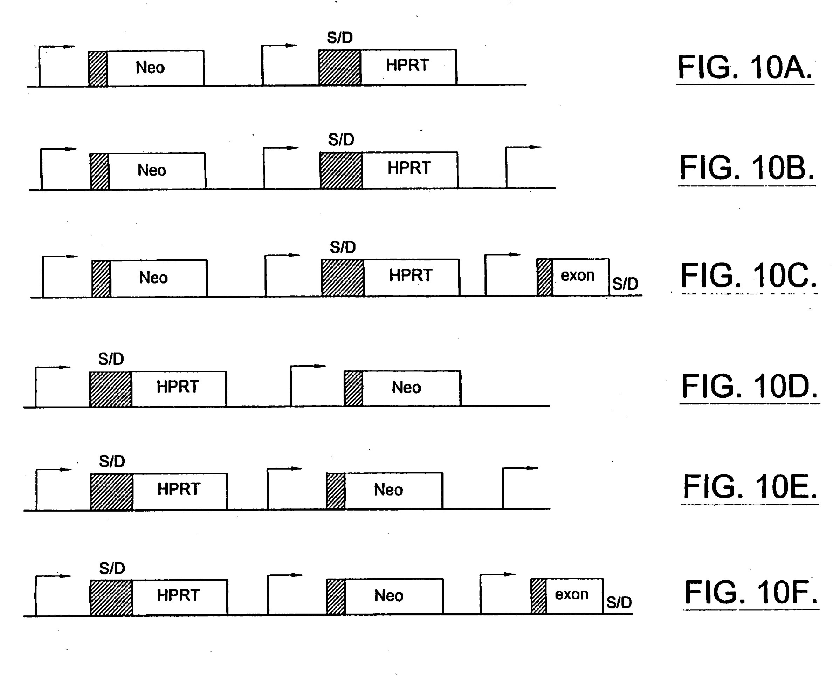

**FIGURES 10A-10F****.** Examples of splice acceptor trap vectors containing a positive and negative selectable marker driven from different promoters. Each vector is illustrated schematically in its linearized form. Each horizontal line represents a DNA molecule. The arrows denote promoter sequences located on the DNA molecule, and face in the direction of transcription. Transcribed regions include all sequences located downstream of a promoter. Untranslated regions are designated by hatched boxes. Poly(A) signals are not present in these examples. As described in the specification, however, poly(A) signals may be placed on the vector 3' of either or both selectable markers. The following designations were used: splice donor site (S/D), internal ribosome entry site (ires), hypoxanthine phosphoribosyl transferase (HPRT), and neomycin resistance gene (Neo). In the vectors shown in Figures 10A-10F, Neo represents the positive selectable marker and HPRT represents the negative selectable marker. As shown, the vectors depicted in Figures 10A-10F do not contain a splice donor site 3' of the Neo gene; however, in other vectors not shown, a splice donor site may be located 3' of the Neo gene to facilitate splicing of the positive selection marker to an endogenous exon. In the vectors shown in Figures 10C and 10F, the region designated exon contains a translation start codon. As described in the Detailed Description, the exon may encode a methionine residue, a partial signal sequence, a full signal secretion sequence, a portion of a protein, or an epitope tag. In addition, the codons may be present in any reading frame relative to the splice donor site. In other vector examples not shown, the region designated exon lacks a translation start codon.

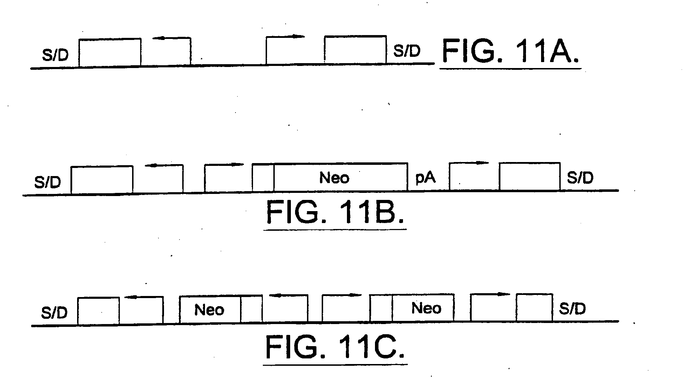

**FIGURES 11A-11C****.** Schematic diagram of bidirectional activation vectors. The arrows denote promoter sequences. The exons are shown as checkered boxes and splice donor sites are indicated by S/D. The hatched boxes indicate exon sequences operably linked to the upstream promoter. It is understood that the exons on these vectors may be untranslated, or may contain a start codon and additional codons as described herein. As illustrated in the vectors depicted in Figures 11B-11C, the vectors may contain a selectable marker. In these vectors, the neomycin resistance (Neo) gene is illustrated. In Figure 11B, a polyadenylation signal (pA) is located downstream of the selectable marker. In Figure 11C, polyadenylation signals are omitted from the vector.

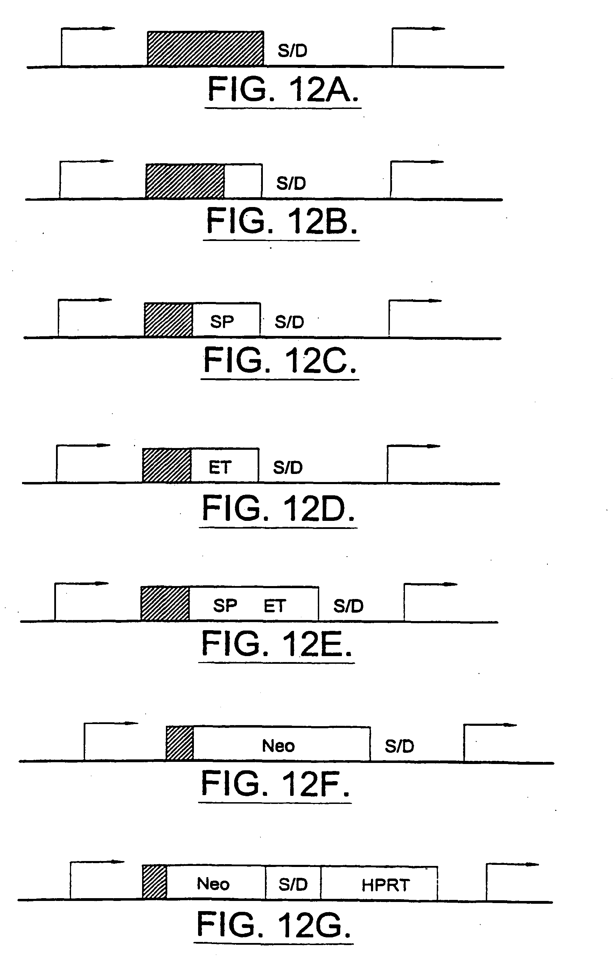

**FIGURES 12A-12G****.** Examples of vectors useful for recovering exon I from activated endogenous genes. Each vector is illustrated schematically in its linearized form. Each horizontal line represents a DNA molecule. The arrows denote promoter sequences located on the DNA molecule, and face in the direction of transcription. Transcribed regions include all sequences located downstream of a promoter. Untranslated regions are designated by hatched boxes. Poly(A) signals are not present in the vectors depicted. As discussed in the Detailed Description, however, poly(A) signals may be placed on the vector 3' of either or both selectable markers. The following designations were used: splice donor site (S/D), internal ribosome entry site (ires), hypoxanthine phosphoribosyl transferase (HPRT), and neomycin resistance gene (Neo). In these examples, Neo represents the positive selectable marker and HPRT represents the negative selectable marker. It is also recognized that in these examples, the region designated exon, when present, lacks a translation start codon. In other examples not shown, the region designated exon contains a translation start codon. Furthermore, when the vector exon contains a translation start codon, the exon may encode a methionine residue, a partial signal sequence, a full signal secretion sequence, a portion of a protein, or an epitope tag. In addition, the codons may be present in each reading frame relative to the splice donor site.

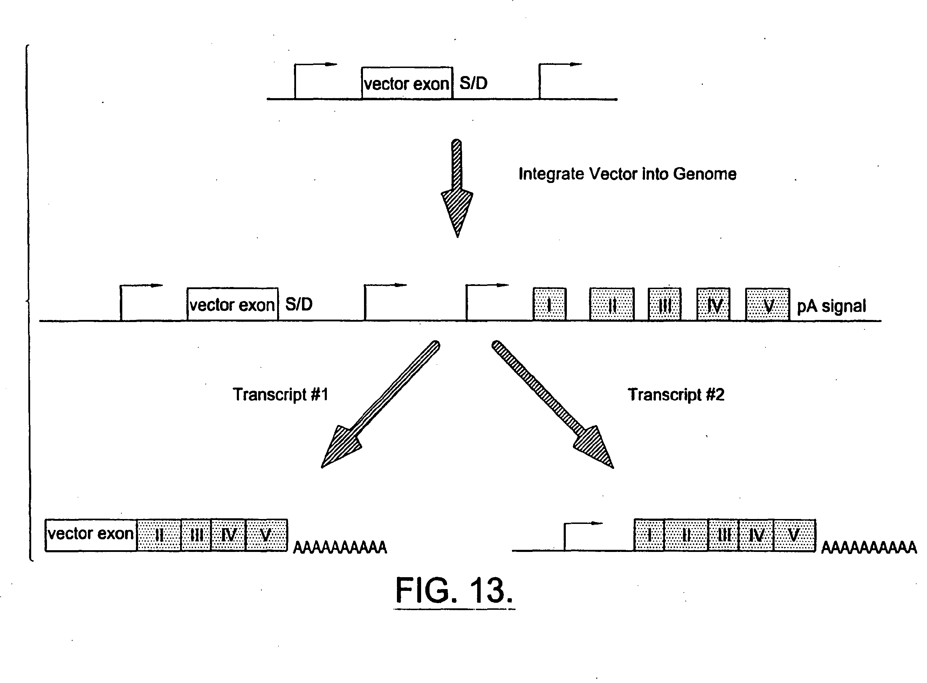

**FIGURE 13****.** Illustration depicting two transcripts produced from the integrated vectors described in Figures 12A-12G. DNA strands are depicted as horizontal lines. Vector DNA is shown as a black line. Endogenous genomic DNA is shown as a grey line. Rectangles depict exons. Vector-encoded exons are shown as open rectangles, while endogenous exons are shown as shaded boxes. S/D denotes a splice donor site. Following integration, the vector encoded promoters activate transcription of the endogenous gene. Transcription resulting from the upstream promoter produces a spliced RIVA molecule containing the vector encoded exon joined to the second and subsequent exons from an endogenous gene. Transcription from the downstream promoter, on the other hand, produces a transcript containing the sequences downstream of the integrated joined to exon I and the subsequent exons from an endogenous gene.

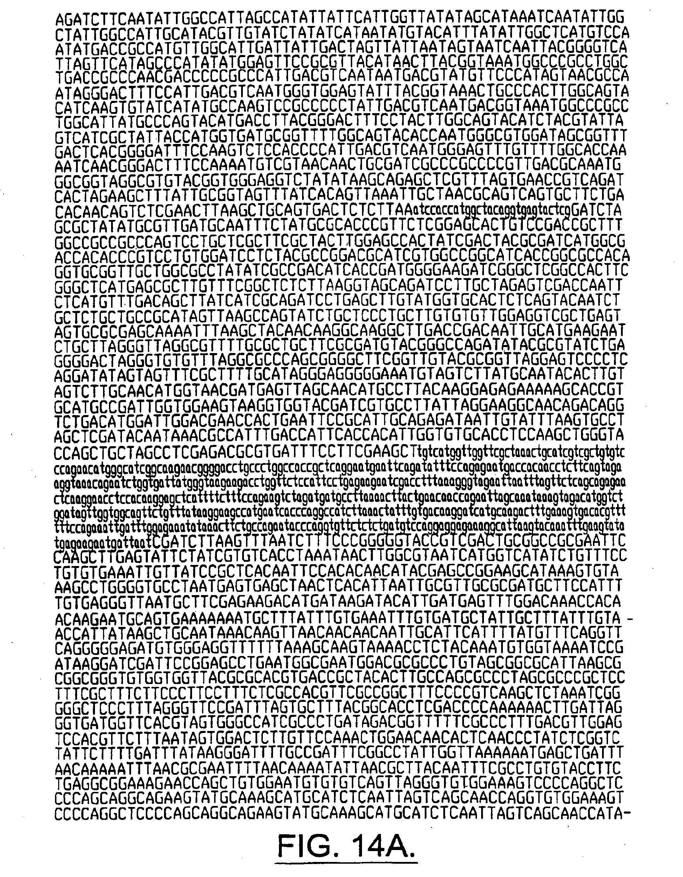

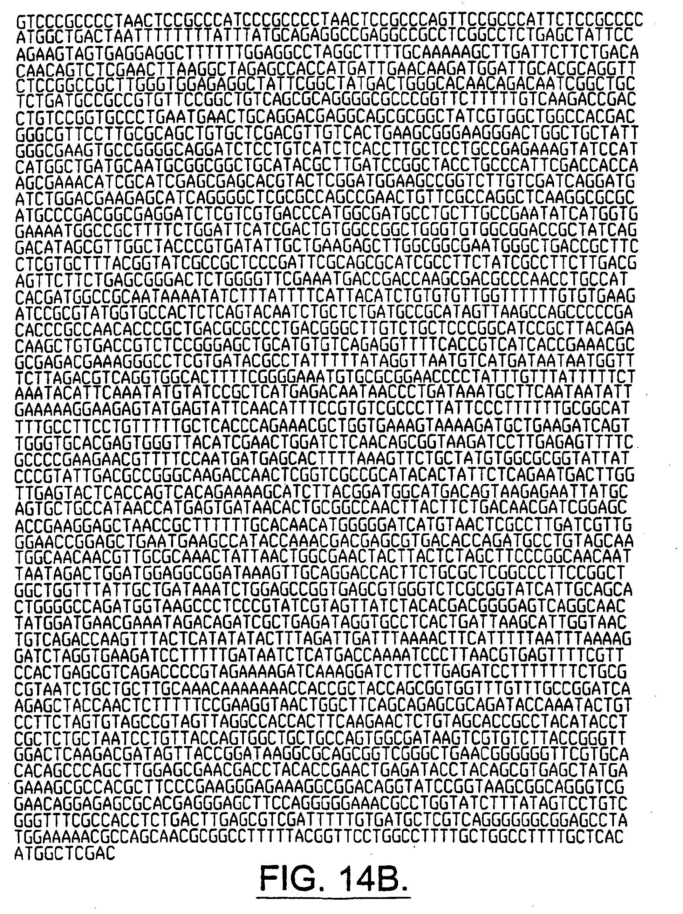

**FIGURES 14A-14B****.** Nucleotide sequence of pRIG1 (SEQ ID NO:18).

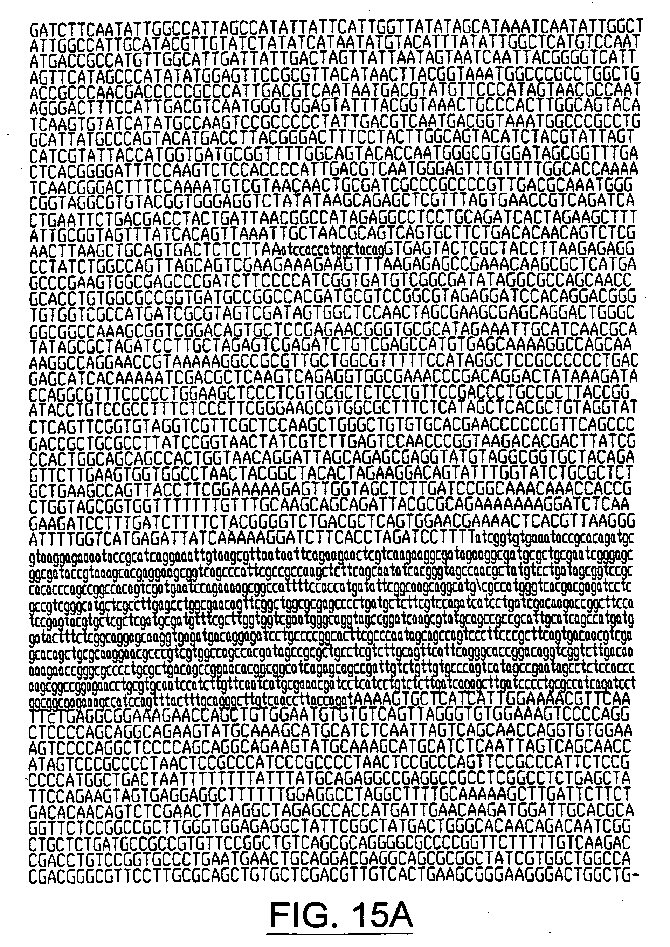

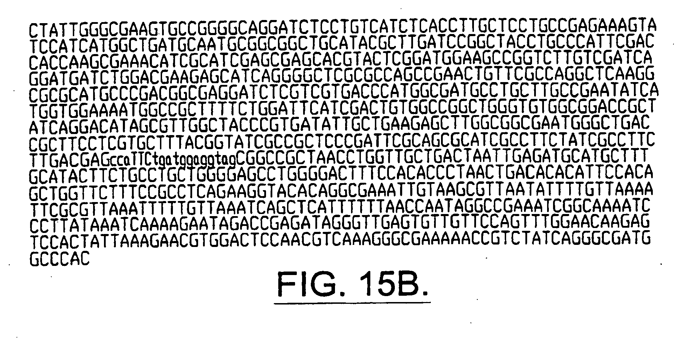

**FIGURES 15A-15B****.** Nucleotide sequence of pRIG21b (SEQ ID NO:19).

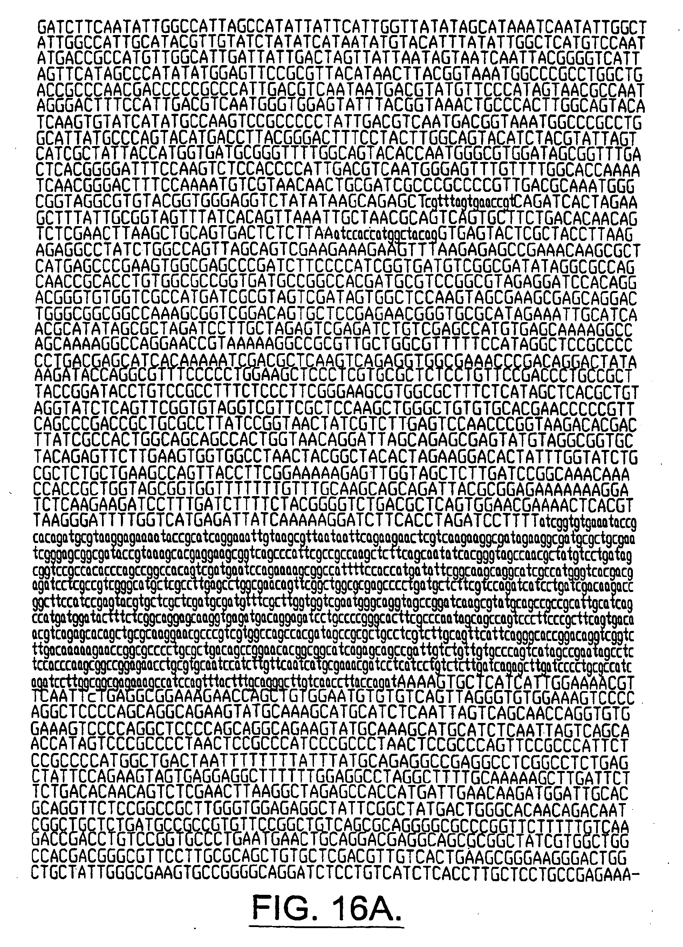

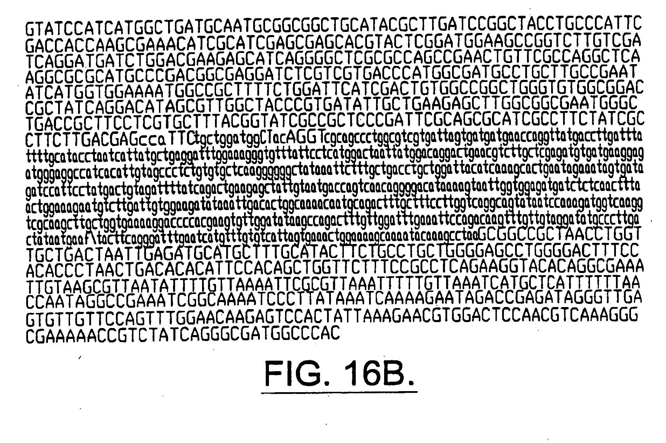

**FIGURES 16A-16B****.** Nucleotide sequence of pRIG22b (SEQ ID NO:20).

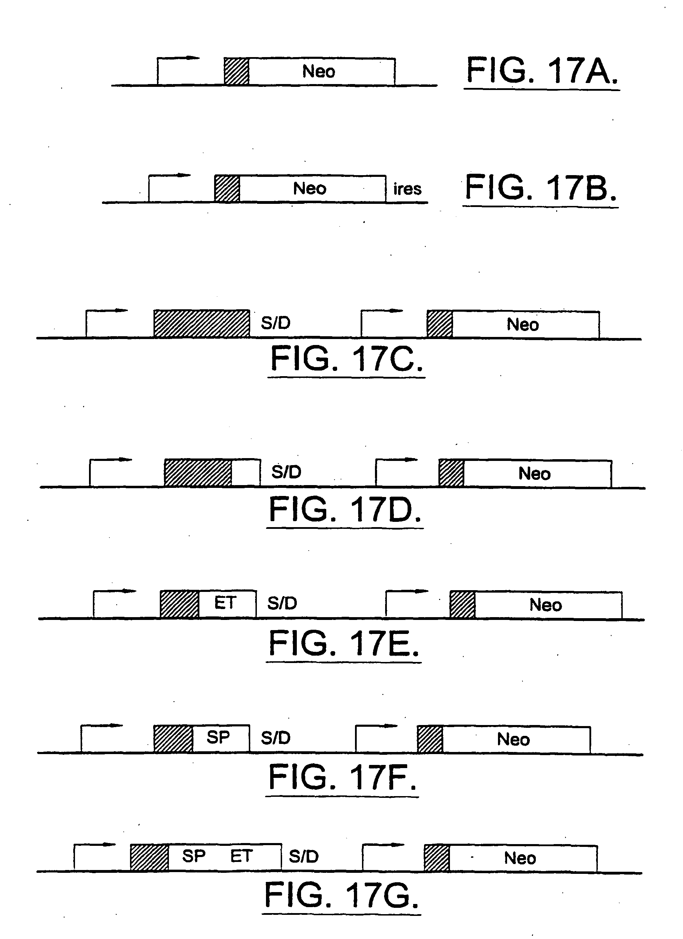

**FIGURES 17A-17G****.** Examples of poly(A) trap vectors. Each vector is illustrated schematically in its linearized form. Each horizontal line represents a DNA molecule. The arrows denote promoter sequences located on the DNA molecule, and face in the direction of transcription. Transcribed regions include all sequences located downstream of a promoter. Boxes indicate exons. Hatched boxes indicate untranslated regions. The following designations were used: splice donor site (S/D), signal secretion sequence (SP), epitope tag (ET), neomycin resistance gene (Neo), vector promoter #1 (VP#1), and vector promoter #2 (VP#2). As shown in the vectors depicted in Figures 17C-17G, a promoter operably linked to an exonand an unpaired splice donor site can be positioned upstream of the selectable marker. It is recognized that this exon, when present, may encode codons a start codon in any reading frame relative to the splice donor site. To activate protein expression from genes with different reading frames, three separate vectors can be used, each with a start codon in a different reading frame relative to the splice donor site.

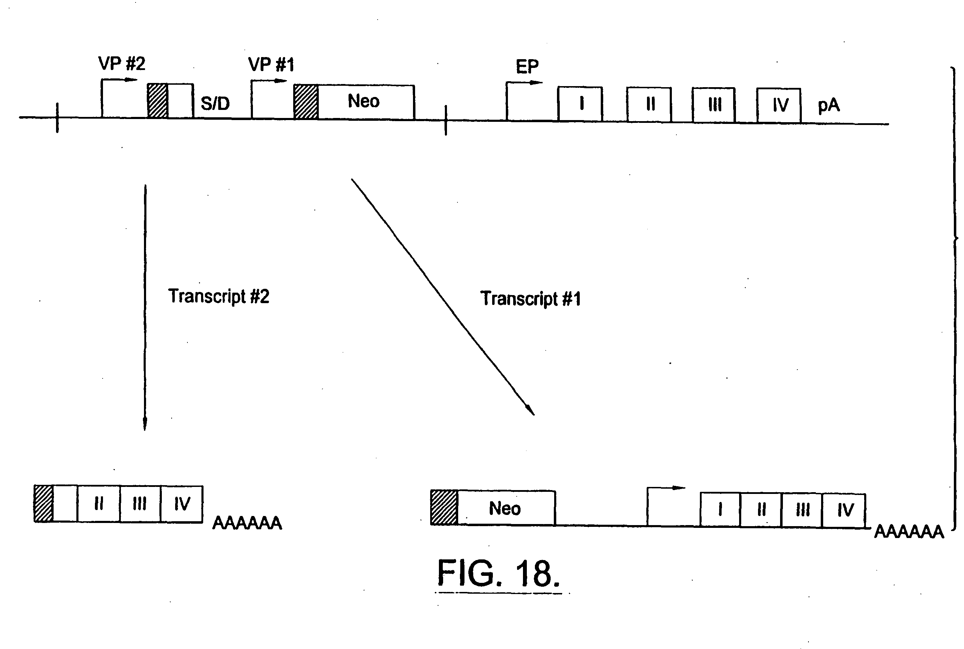

**FIGURE 18****.** Illustration of the transcripts produced by the vector from Figure 17C upon integration into a host cell genome upstream of a multi-exon endogenous gene. Each horizontal line represents a DNA molecule. Vertical lines running through the DNA strand mark the upstream and downstream vector/cellular genome boundaries. The arrows denote promoter sequences located on the DNA molecule, and face in the direction of transcription. Transcribed regions include all sequences located downstream of a promoter. Boxes indicate exons. Hatched boxes indicate untranslated regions. The endogenous exons are numbered using roman numerals. The following designations were used: splice donor site (S/D), neomycin resistance gene (Neo), vector promoter #1 (VP#1), vector promoter #2 (VP#2), endogenous promoter (EP) and polyadenylation signal (pA). Following integration, vector promoter #1 expresses a chimeric transcript containing the Neo gene linked to the genomic sequences downstream of the integration site, including the processed (spliced) exons from the endogenous gene. Since transcript #1 contains a poly (A) signal from the endogenous gene, the Neo gene product will be efficiently produced, thereby conferring drug resistance on the cell. In addition to transcript #1, the integrated vector will generate a second transcript, designated transcript #2, originating from vector promoter#2. The structure of transcript #2 facilitates efficient translation of the protein encoded by the endogenous gene. As exemplified in Figure 17, vectors containing alternative coding information in the vector encoded exon can be used to produce different chimeric proteins, containing, for example, signal sequences and/or epitope tags.

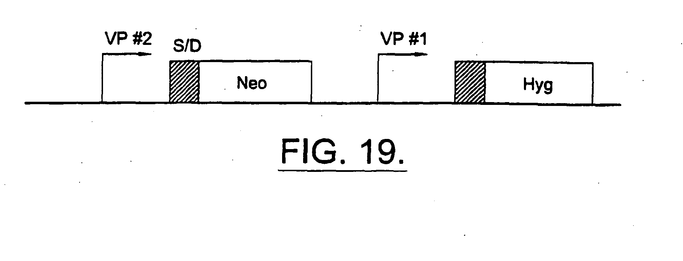

**FIGURE 19****.** Example of dual positive selectable marker vector. The vector is illustrated schematically in its linearized form. The horizontal line represents a DNA molecule. The arrows denote promoter sequences located on the DNA molecule, and face in the direction of transcription. Transcribed regions include all sequences located downstream of a promoter. Boxes indicate exons. Hatched boxes indicate untranslated regions. Poly(A) signals are not present in these examples. The following designations were used: splice donor site (S/D), hygromycin resistance gene (Hyg), neomycin resistance gene (Neo), vector promoter #1, and vector promoter #2.

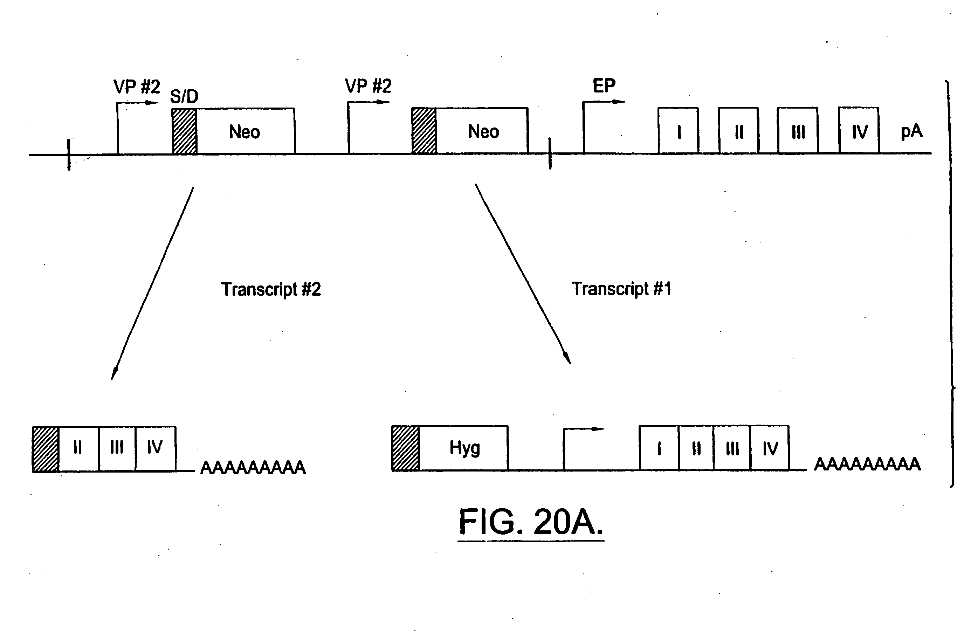

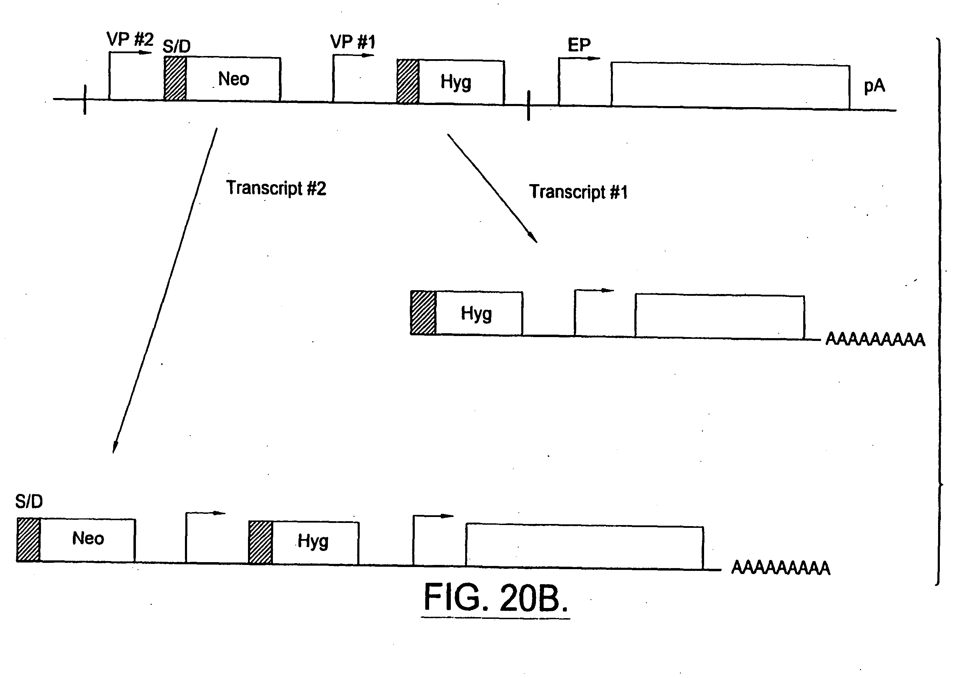

**FIGURES 20A-20B****.** Examples of transcripts produced by a dual positive selectable marker vector integrated into a host cell genome adjacent to an endogenous gene. Figure 20A illustrates the transcripts produced upon vector integration near a multi-exon gene. Figure 20B illustrates the transcripts produced upon vector integration near a single exon gene. Each horizontal line represents a DNA molecule. Vertical lines running through the DNA strand mark the upstream and downstream vector/cellular genome boundaries. The arrows denote promoter sequences located on the DNA molecule, and face in the direction of transcription. Transcribed regions include all sequences located downstream of each promoter Boxes indicate exons. Hatched boxes indicate untranslated regions. The endogenous exons are numbered using roman numerals. The following designations were used: splice donor site (S/D), hygromycin resistance gene (Hyg), neomycin resistance gene (Neo), vector promoter #1 (VP#1), vector promoter #2 (VP#2), endogenous promoter (EP), and polyadenylation signal (pA). Following integration, vector promoter #1 expresses a chimeric transcript containing the Hyg gene linked to the genomic sequences downstream of the integration site, including the processed (spliced) exons from the endogenous gene. Since transcript #1 contains a poly (A) signal from the endogenous gene, the Hyg gene product will be efficiently produced, thereby conferring drug resistance on the cell. In addition to transcript #1, the integrated vector will generate a second transcript, designated transcript #2, originating from vector promoter#2. In Figure 20A, the neo gene is removed from transcript #2 upon splicing from the vector encoded splice donor site, and the first endogenous splice acceptor located downstream of the vector integration site (i.e. exon II in this example). Since multi-exon genes contain splice acceptor sites at the 5' end of each exon (except exon I), the neo gene will be removed from transcript #2 in cells in which the vector has integrated near, and transcriptionally activated, a multi-exon gene. As a result, cells having activated multi-exon genes may be eliminated by selecting with G418 and hygromycin. In Figure 20B, the neo gene is not removed from transcript #2 by splicing, since single exon genes do not contain any splice acceptor sequences. Thus, cells containing a vector integrated near single exon genes will survive double selection with G418 and hygromycin. These cells can be used to efficiently isolate the activated single exon genes using methods described herein.

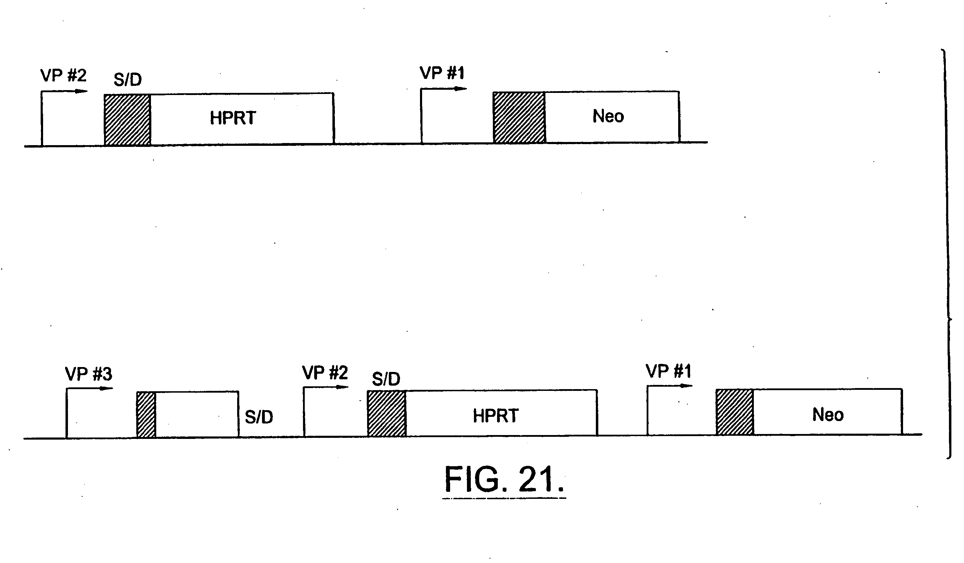

**FIGURES 21A-21B.** Examples of dual trap vectors containing a positive and a negative selectable marker. Each vector is illustrated schematically in its linearized form. Each horizontal line represents a DNA molecule. The arrows denote promoter sequences located on the DNA molecule, and face in the direction of transcription. Transcribed regions include all sequences located downstream of a promoter. Boxes indicate exons. Hatched boxes indicate untranslated regions. The following designations were used: splice donor site (S/D), hypoxanthine phosphoribosyl transferase (HPRT), neomycin resistance gene (Neo), vector promoter #1 (VP #1), vector promoter #2 (VP#2), and vector promoter #3 (VP#3). In the vectors shown in Figures 21A-21B, Neo represents the positive selectable marker and HPRT represents the negative selectable marker. In re 21B a third promoter is located upstream of the selectable markers. This upstream promoter is operably linked to an exon and unpaired splice donor site. The region designated exon contains a translation start codon in this example. As described herein, the exon may encode a methionine residue, a partial signal sequence, a full signal secretion sequence, a portion of a protein, or an epitope tag. In addition, the codons may be present in any reading frame relative to the splice donor site. In other vector examples not shown, the region designated exon lacks a translation start codon.

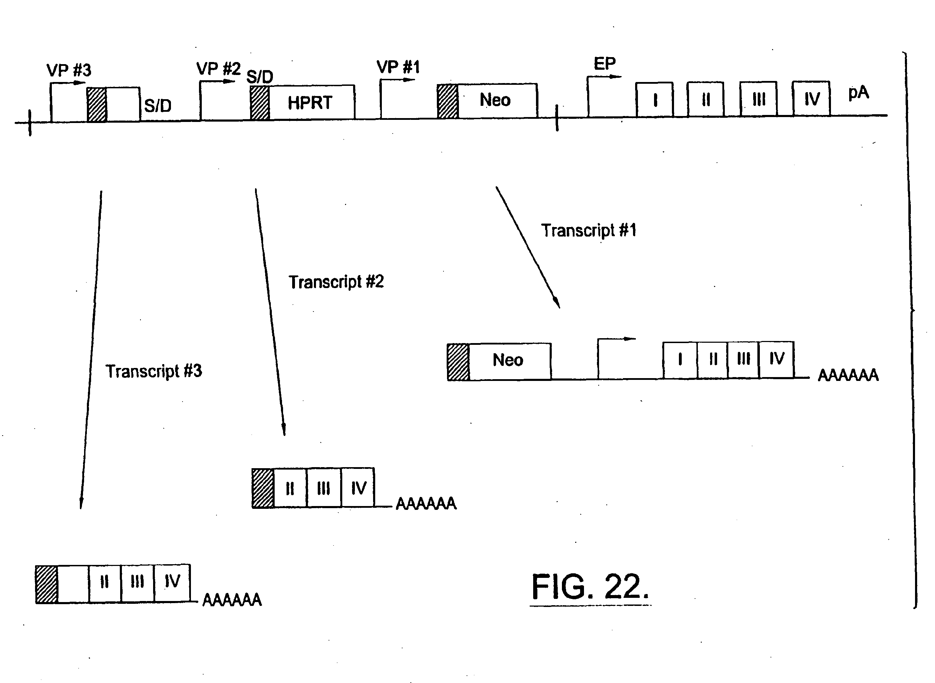

**FIGURE 22****.** Examples of transcripts produced by a dual positive/negative selectable marker vector integrated into a host cell genome upstream of a multi-exon endogenous gene. Each horizontal line represents a DNA molecule. Vertical lines running through the DNA strand mark the upstream and downstream vector/cellular genome boundaries. The arrows denote promoter sequences located on the DNA molecule, and face in the direction of transcription. Transcribed regions include all sequences located downstream of each promoter. Boxes indicate exons. Hatched boxes indicate untranslated regions. The endogenous exons are numbered using roman numerals. The following designations were used: splice donor site (S/D), neomycin resistance gene (Neo), vector promoter #1 (VP#1), vector promoter #2 (VP#2), vector promoter #3 (VP#3), polyadenylation signal (pA), and endogenous promoter (EP). Following integration, vector promoter #1 expresses a chimeric transcript containing the Neo gene linked to the genomic sequences downstream of the integration site, including the processed (spliced) exons from the endogenous gene. Since transcript #1 contains a poly (A) signal from the endogenous gene, the Neo gene product will be efficiently produced, thereby conferring drug resistance on the cell. In addition to transcript #1, the integrated vector will generate a second transcript, designated transcript #2, originating from vector promoter #2. In this example, the vector has integrated upstream of a multi-exon gene. Since multi exon genes contain splice acceptor sites at the 5' end of each exon, the HPRT gene will be removed from transcript #2 in cells in which the vector has integrated near, and transcriptionally activated, a multi-exon gene. As a result, cells containing activated multi-exon genes may be isolated by selecting with G418 and 8-Azaguanine 6-Thioguanine (AgThg). Thus, cells containing a vector integrated near single exon genes will survive double selection with G418 and AgThg. These cells can be used to efficiently isolate the activated multi-exon genes using methods described herein. In addition to transcripts #1 and #2, a third transcript, designated transcript #3 is produced from the integrated vector. Transcript #3, originating from vector promoter #3, contains an exonic sequence suitable for directing protein expression from the endogenous gene. This occurs following splicing from the first splice donor site downstream of promoter #3 to the first downstream splice acceptor site from the endogenous gene. In addition to directing protein expression, transcript #3, and/or transcripts #1 and/or #2, can be isolated for gene discovery purposes using the methods described herein.

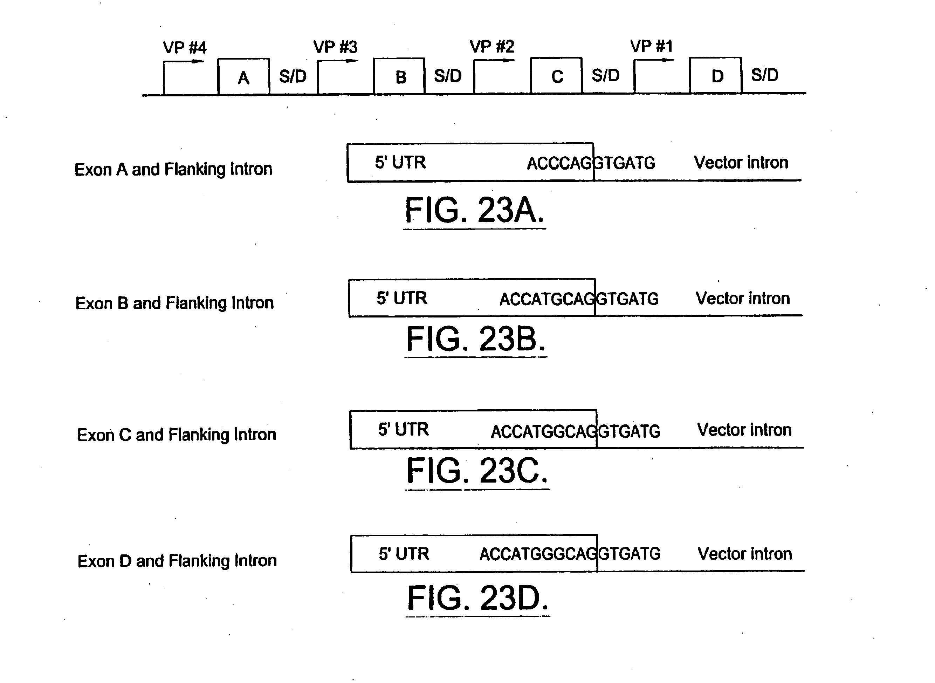

**FIGURES 23A-23D****.** Example of a multi-Promoter/Activation Exon Vector. Each vector is illustrated schematically in its linearized form. Each horizontal line represents a DNA molecule. The arrows denote promoter sequences. Boxes indicate exons. Hatched boxes indicate untranslated regions. It is understood that the exons on these vectors may be untranslated, or may contain a start codon and additional codons as described herein The following designations were used: splice donor site (S/D), vector promoter #1 (VP #1), vector promoter #2 (VP#2), vector promoter #3 (VP #3), and vector promoter #4 (VP#4). Individual vector activation exons are designated A, B, C, and D. Each activation exon may contain a different structure. The structure of each activation exon and its flanking intron are shown below. It is understood, however, that any activation exon described herein, may be used on these vectors, in any combination and/or order, including exons that encode signal sequences, partial signal sequences, epitope tags, proteins, portions of proteins, and protein motifs. Any of the exons may lack a start codon. In addition, while not illustrated in these examples, these vectors may contain a selectable marker and/or an amplifiable marker. The selectable marker may contain a poly (A) signal or a splice donor site. When present, the splice donor site may be located upstream or downstream of the selectable marker. Alternatively, the selectable marker may not be operably linked to a poly (A) signal and/or a splice donor site.

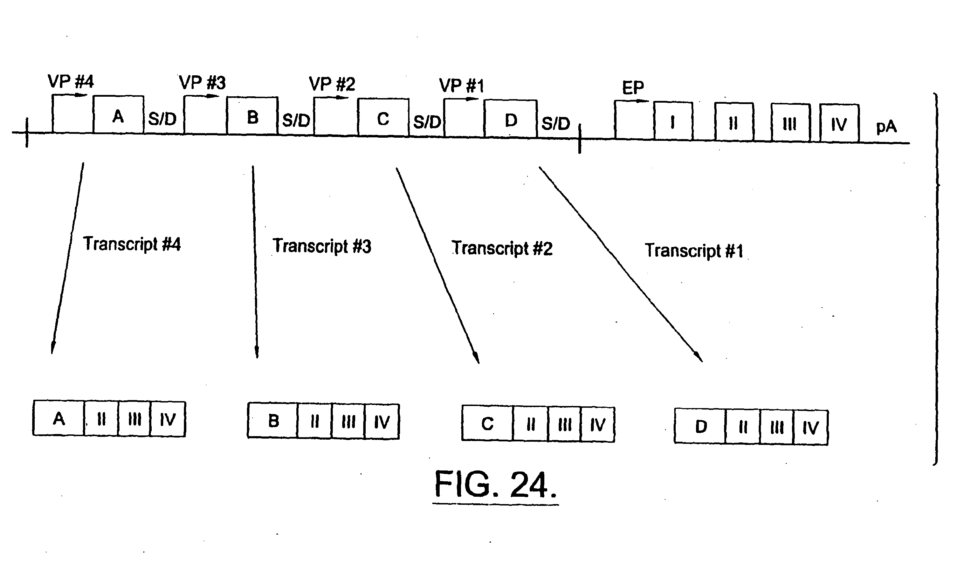

**FIGURE 24****.** Examples of transcripts produced from a multi-Promoter/Activation Exon Vector upon integration into a host cell genome upstream of an endogenous gene. Each horizontal line represents a DNA molecule. Vertical lines running through the DNA strand mark the upstream and downstream vector/cellular genome boundaries. The arrows denote promoter sequences located on the DNA molecule, and face in the direction of transcription. Transcribed regions include all sequences located downstream of each promoter. Boxes indicate exons. Hatched boxes indicate untranslated regions.. The endogenous exons are numbered using roman numerals. The following designations were used: splice donor site (S/D), vector promoter #1 (VP #1), vector promoter #2 (VP#2), vector promoter #3 (VP #3), vector promoter #4 (VP#4), endogenous promoter (EP), and polyadenylation signal (pA). Individual vector activation exons are designated A, B, C, and D. Following integration, each vector encoded promoter is capable of producing a different transcript. Each transcript contains a different activation exon joined to the first downstream splice acceptor site from an endogenous gene (exon II in this example). Individual activation exons are designated by (A), (B), (C), or (D). Endogenous exons are designated by (I), (II), (III), or (IV). Generally, the coding sequence and/or reading frames, if present, are different among the activation exons. While four activation exons are illustrated in this example, any number of activation exons may be present on the integrated vector.

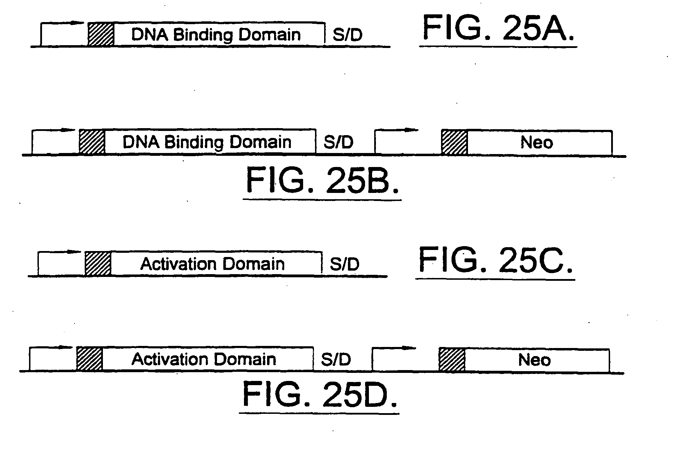

**FIGURES 25A-25D****.** Examples of activation vectors useful for detection of protein-protein interactions. Each vector is illustrated schematically in its linearized form. Each horizontal line represents a DNA molecule. The arrows denote promoter sequences. Boxes indicate exons. Hatched boxes indicate untranslated regions. The following designations were used: splice donor site (S/D), neomycin resistance gene (Neo). It is also recognized that the DNA binding domain and the Activation domain may be encoded in any reading frame (relative to the splice donor site), allowing activation of endogenous genes with different reading frames.

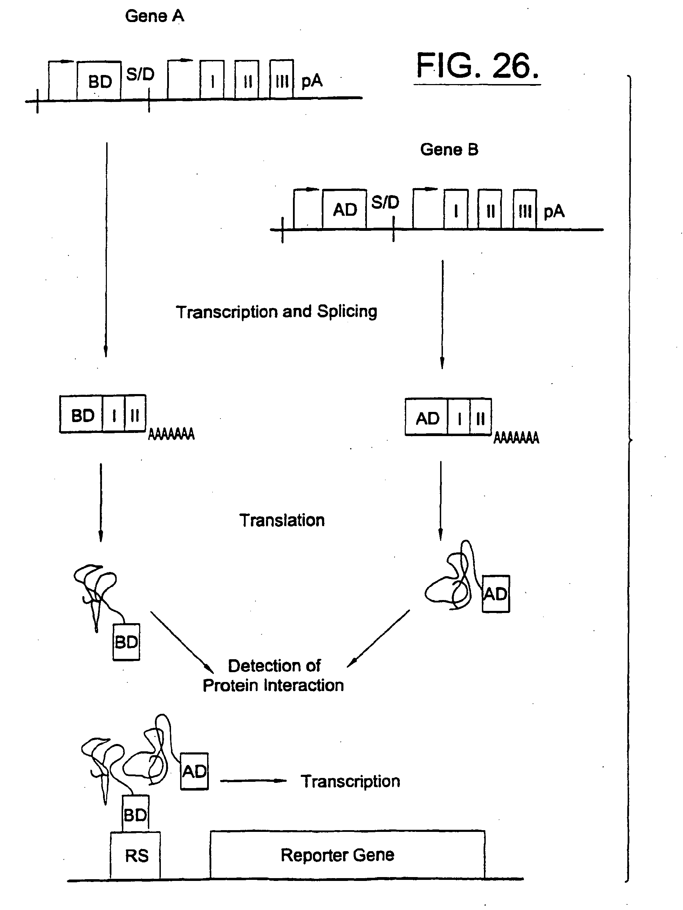

**FIGURE 26****.** Schematic illustration depicting one approach to detecting protein-protein interactions using the vectors shown in Figure 25. Each horizontal line represents a DNA molecule. Vertical lines running through the DNA strand mark the upstream and downstream vector/cellular genome boundaries. The arrows denote promoter sequences located on the DNA molecule, and face in the direction of transcription. Transcribed regions include all sequences located downstream of each promoter. Boxes indicate exons. Hatched boxes indicate untranslated regions. The endogenous exons are numbered using roman numerals. The following designations were used: splice donor site (S/D), binding domain (BD), activation domain (AD), recognition sequence (RS), and polyadenylation signal (pA). The binding domain vector is shown integrated into the genome of a host cell, upstream of an endogenous gene, designated gene A. The activation domain vector is shown integrated into the genome of the same host cell upstream of an endogenous gene, designated gene B. Both vectors are integrated into the genome of the same host cell. Following integration, each vector is capable of producing a fusion protein containing the binding domain (or activation domain, as the case may be) and the protein encoded by the downstream endogenous gene. If the binding domain fusion protein interacts with the activation domain fusion protein, a protein complex will be formed. This complex is capable of increasing expression of a reporter gene present in the cell.

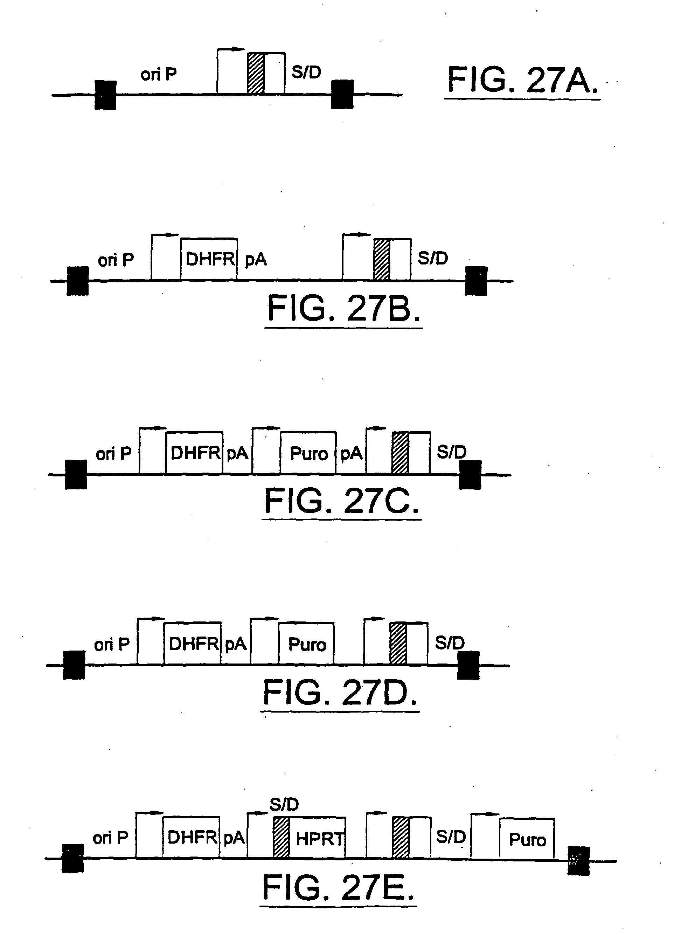

**FIGURE 27****.** Examples of activation vectors useful for in *vitro* and *in vivo* transposition. Each vector is illustrated schematically in its linearized form. Each horizontal line represents a DNA molecule. The arrows denote promoter sequences. Boxes indicate exons. Hatched boxes indicate untranslated regions. The solid boxes indicate the transposon signals. It is recognized that there is directionality to the transposon signals, and that the signals are oriented in the configuration suitable for the type of transposition reaction (integration, inversion, or deletion). The following designations were used: splice donor site (S/D), neomycin resistance gene (Neo), dihydrofolate reductase (DHFR), puromycin resistance gene (Puro), poly (A) signal (pA), and the Epstein Barr Virus origin of replication (ori P). It is also recognized that activation exon may be encode amino acids in any reading frame (relative to the splice donor site), allowing activation of endogenous genes with different reading frames.

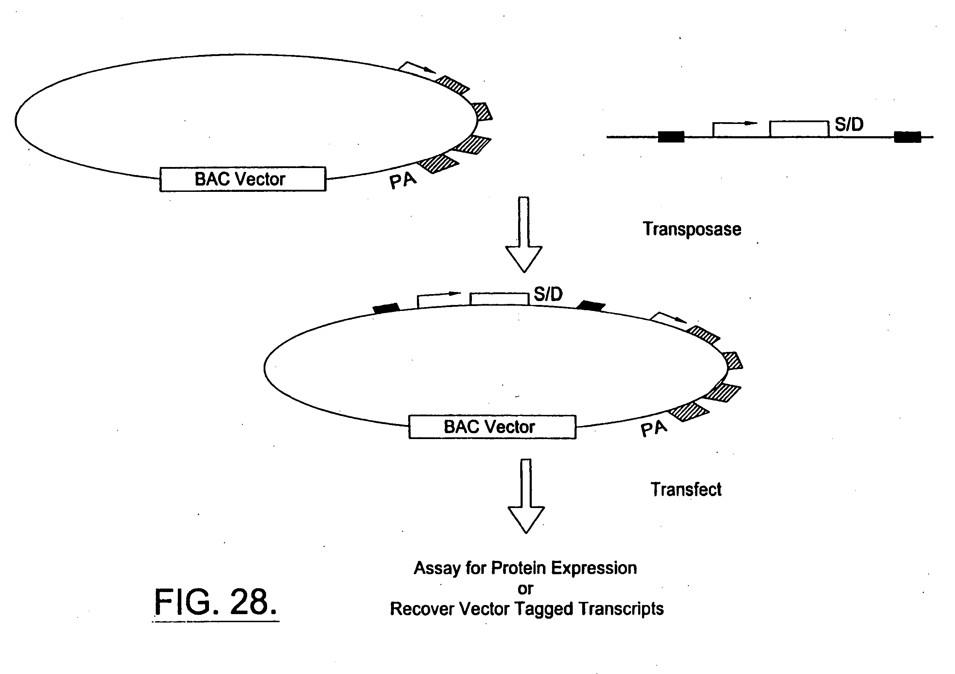

**FIGURE 28****.** Schematic illustration depicting integration of an activation vector into a cloned genomic DNA fragment by *in vitro* transposition. Each horizontal line represents a DNA molecule. The cloned genomic DNA is in a BAC vector. The single line represents the genomic DNA and the rectangle depicts the BAC vector sequences. The arrows denote promoter sequences located on the DNA molecule, and face in the direction of transcription. Transcribed regions include all sequences located downstream of each promoter. The vector activation exon is depicted as an open box. Exons from a gene encoded in the cloned genomic fragment are depicted as hatched boxes. The solid boxes indicate the transposon signals. It is recognized that there is directionality to the transposon signals, and that the signals are oriented in the configuration suitable for the type of transposition reaction (integration, inversion, or deletion). The following designations were used: splice donor site (S/D), and polyadenylation signal (pA). To integrate the vector into the genomic fragment, the activation vector is incubated with the cloned genomic DNA in the presence of transposase. Following integration of the activation vector into the genomic fragment, the plasmid may be transfected directly into an appropriate eukaryotic host cell to express the gene located downstream of the vector integration site. Alternatively, the BAC plasmid may be transformed into *E. coli* to produce larger quantities of
plasmid for transfection into the appropriate eukaryotic host cell.

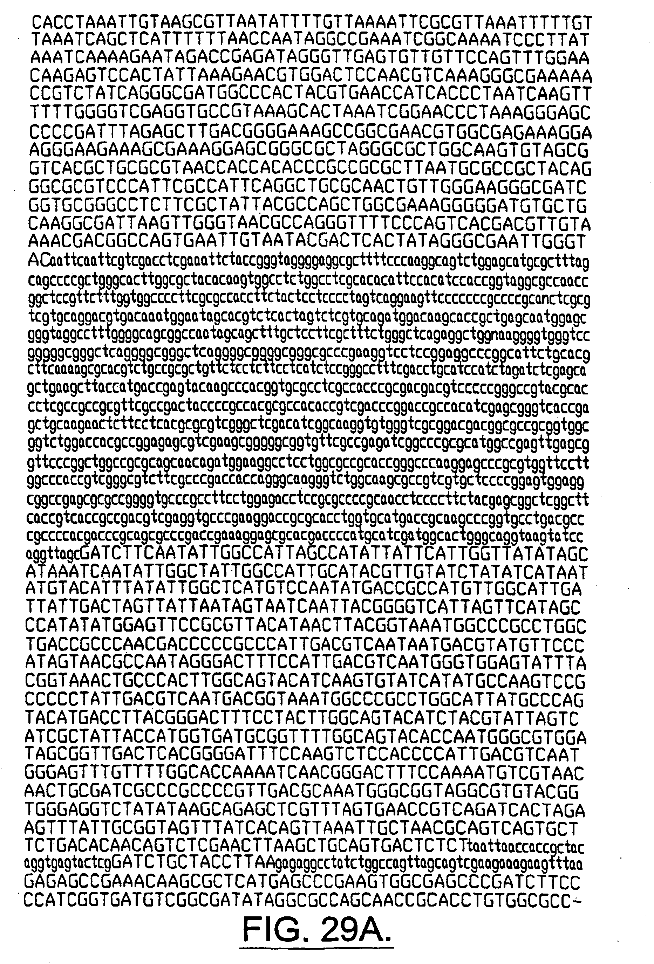

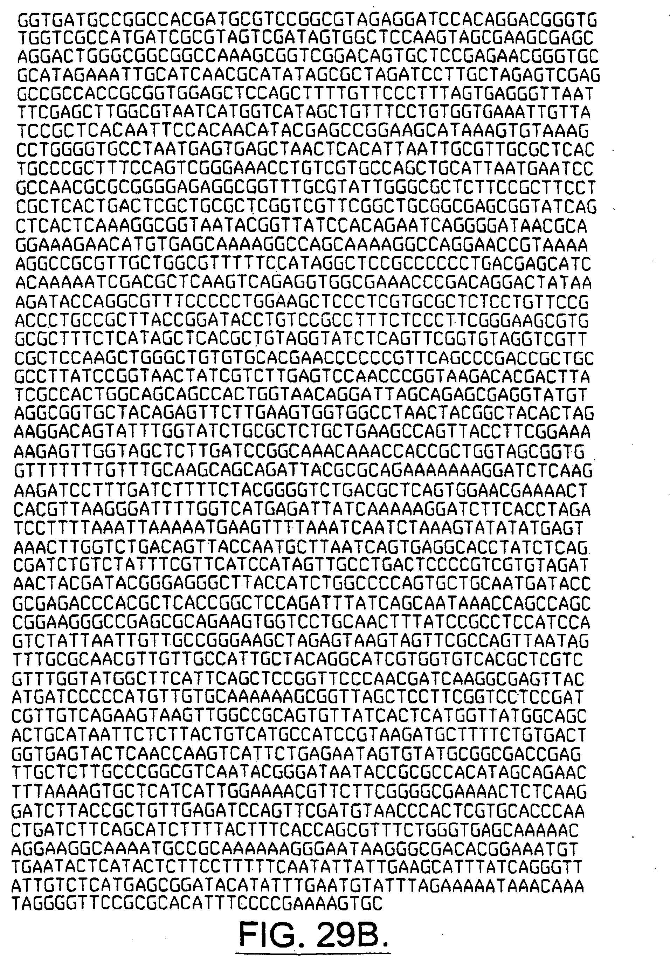

**FIGURES 29A-29B****.** Nucleotide sequence of pRIG14.

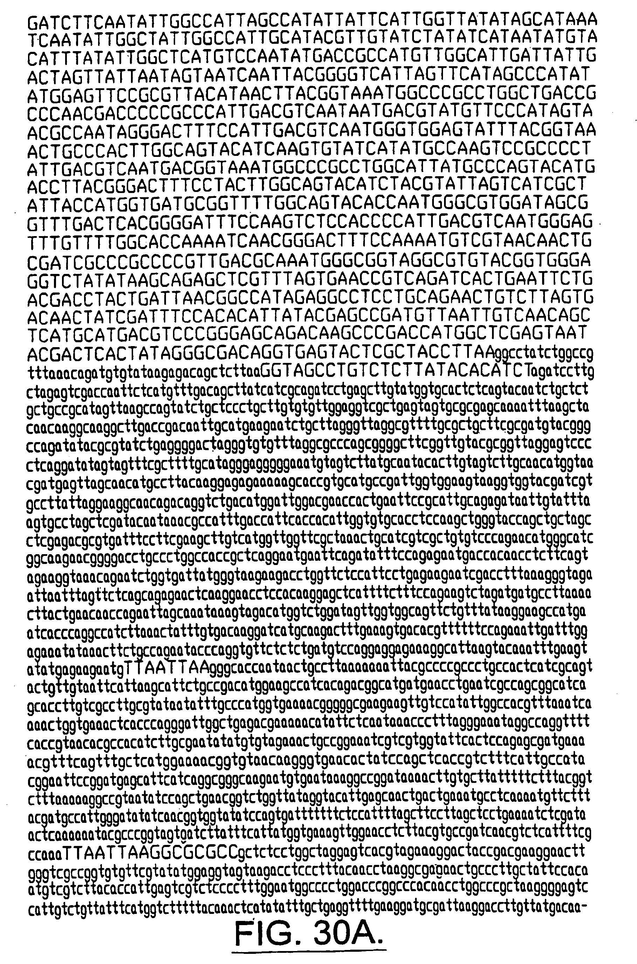

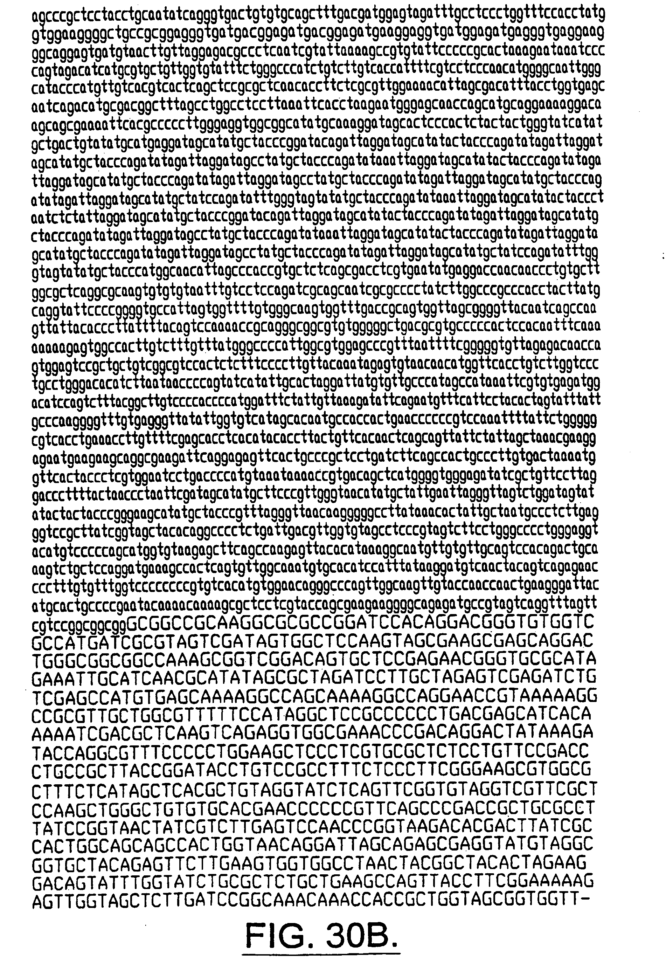

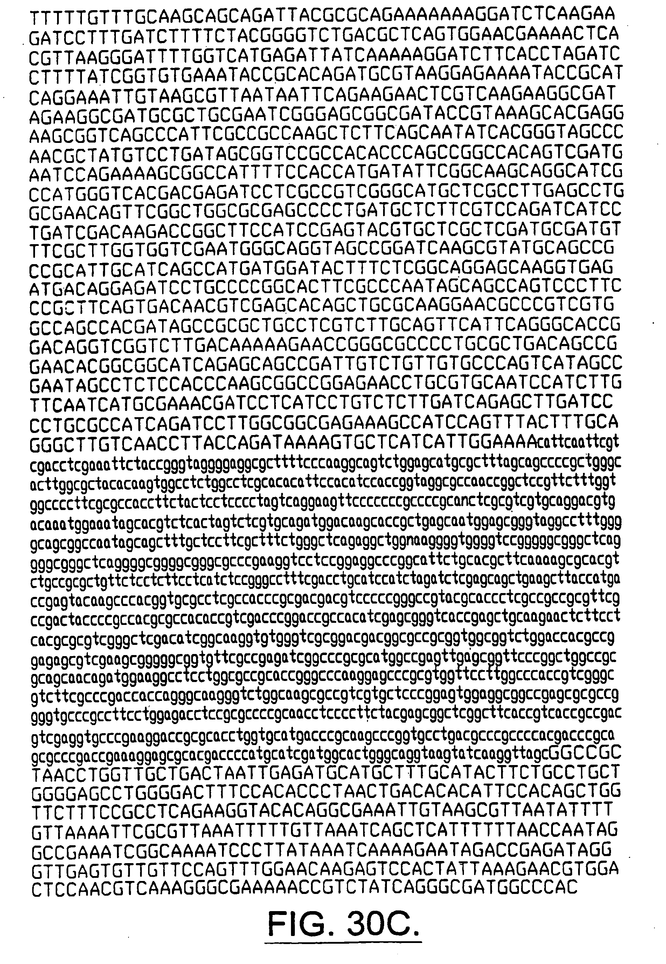

**FIGURES 30A-30C****.** Nucleotide sequence of pRIGl9.

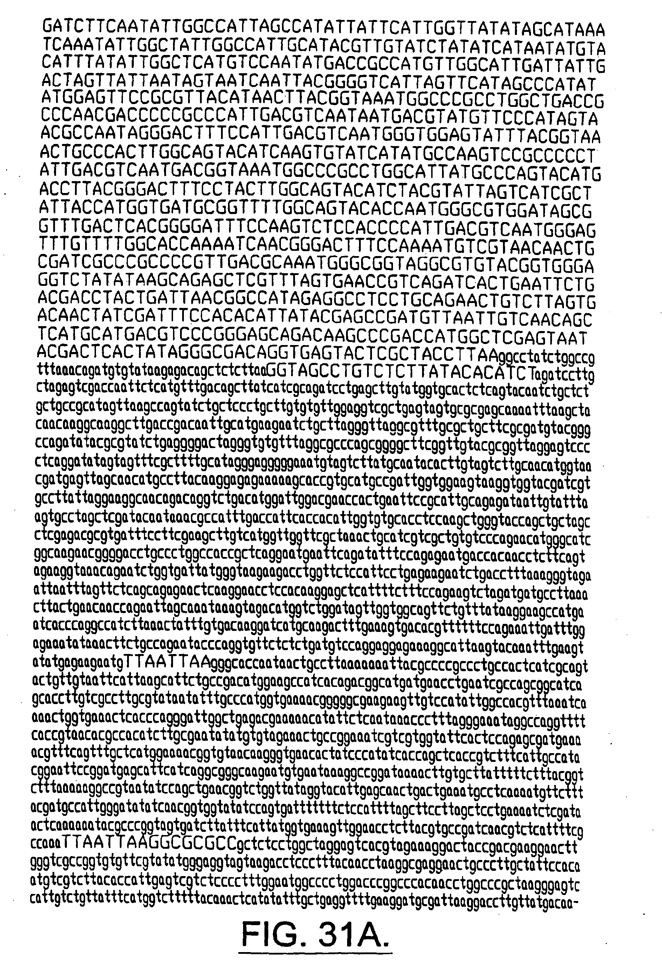

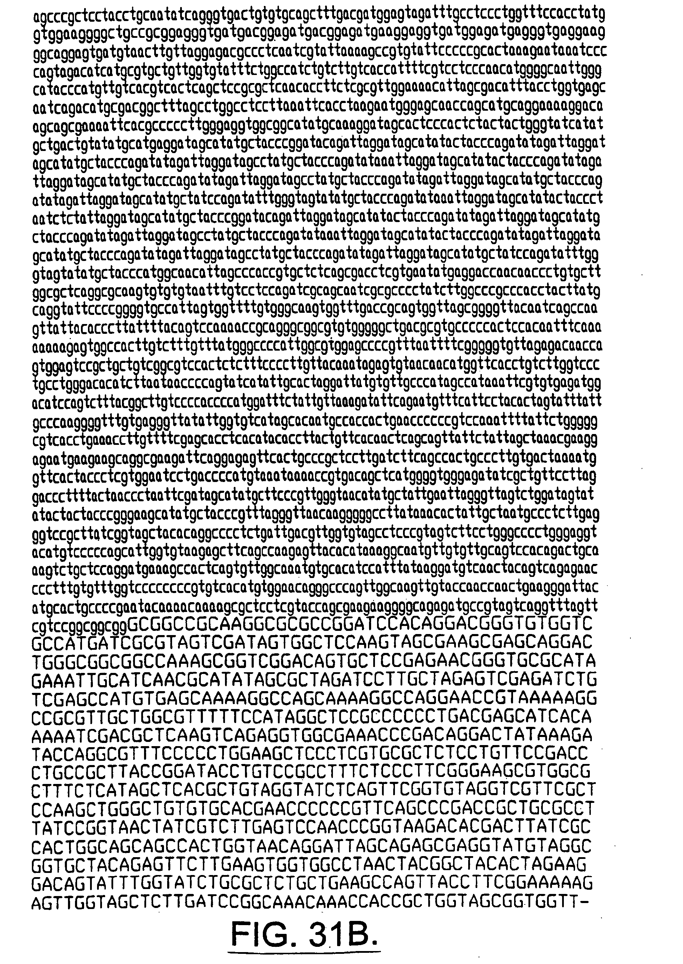

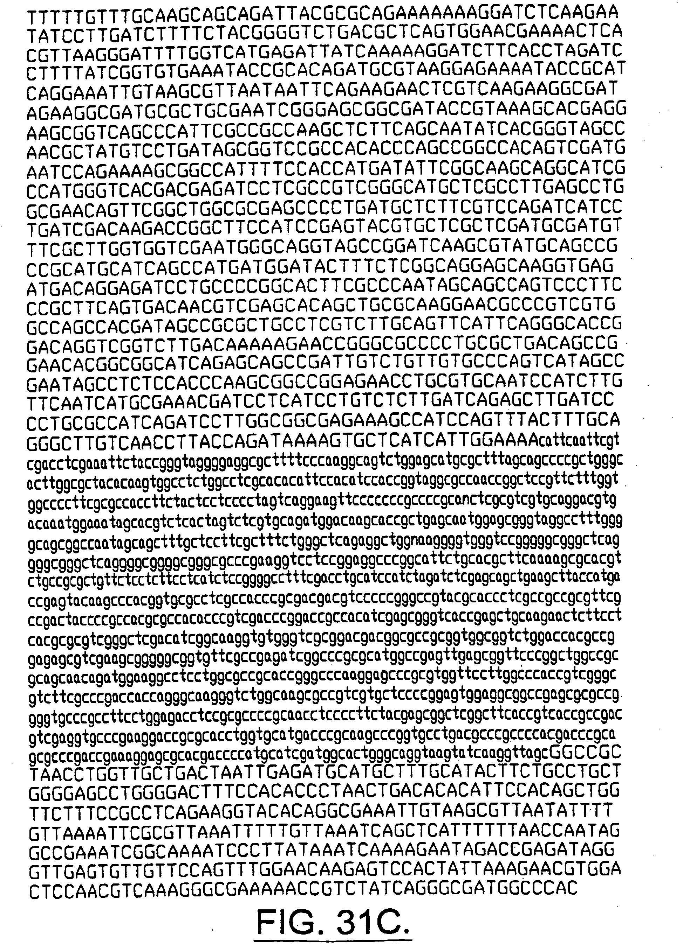

**FIGURES 31A-31C****.** Nucleotide sequence ofpRIG20.

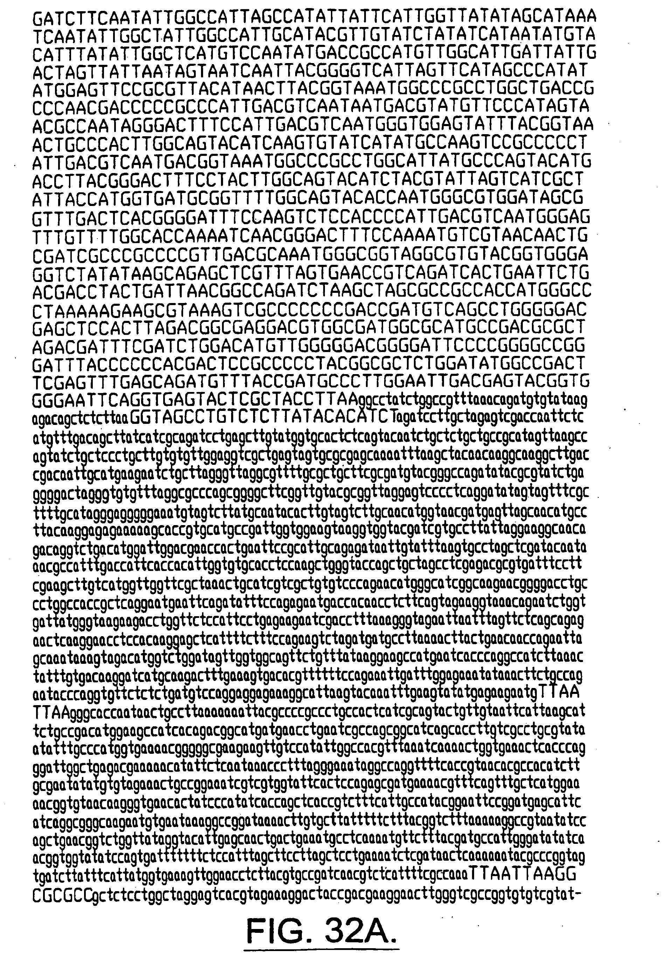

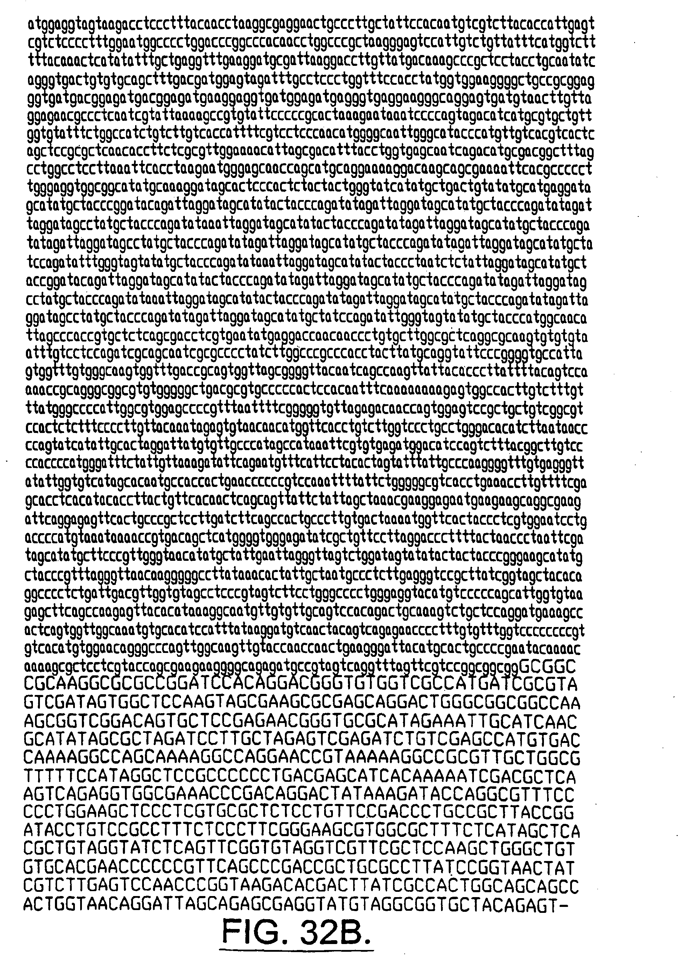

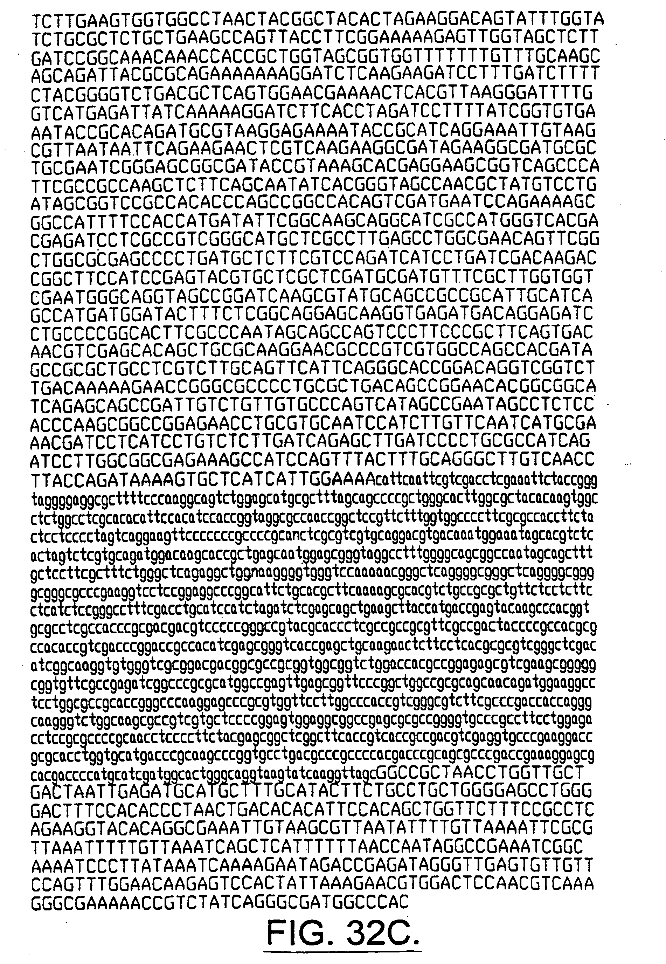

**FIGURES 32A-32C****.** Nucleotide sequence of pRIGad1.

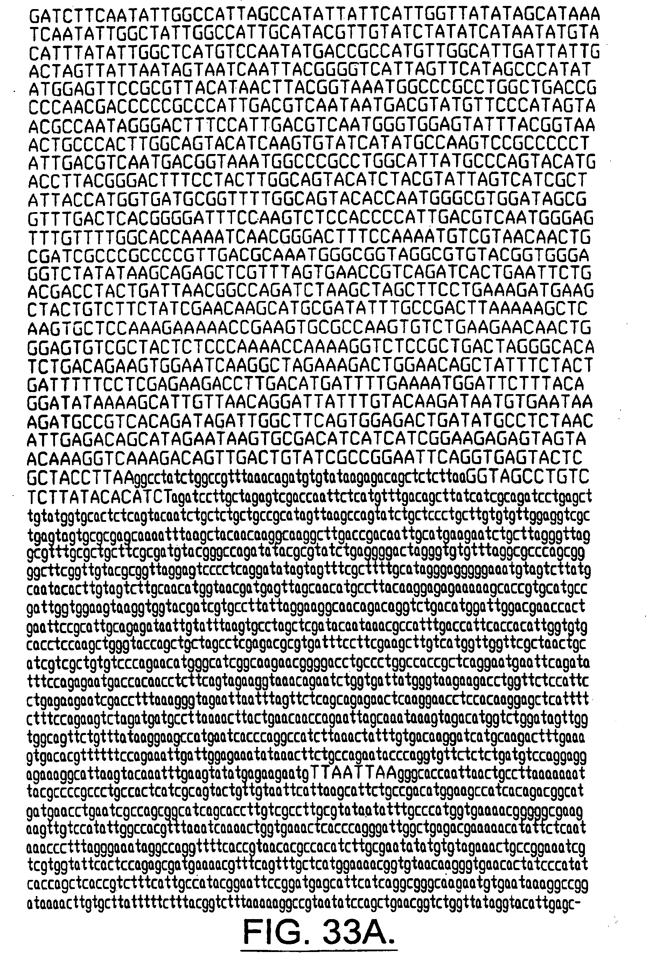

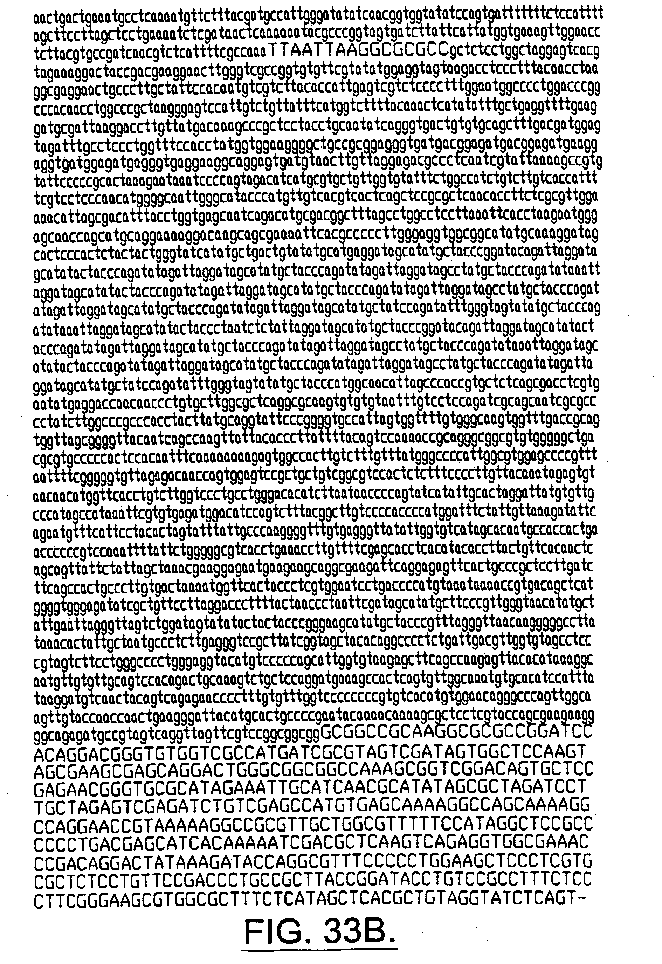

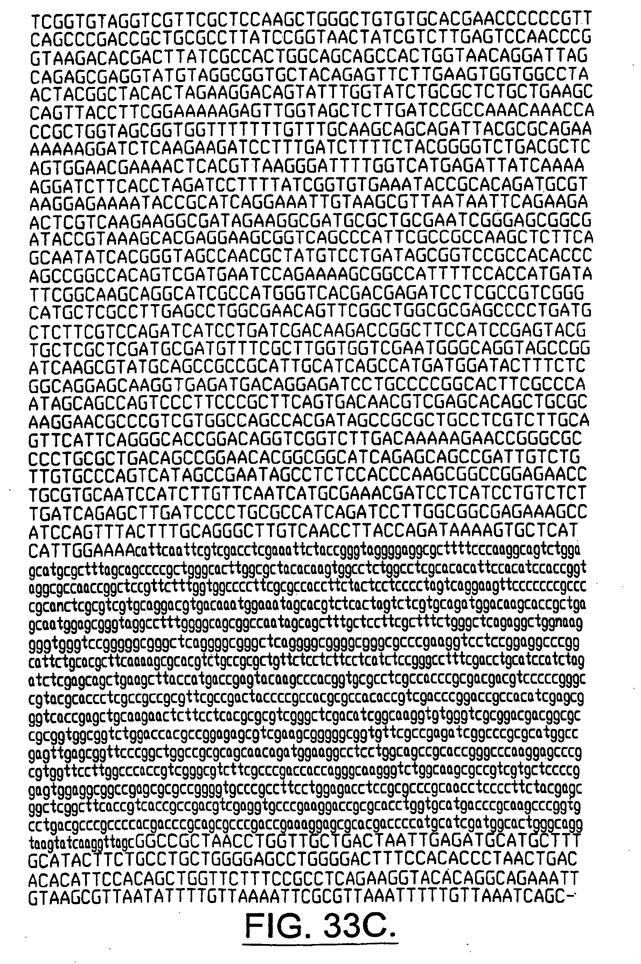

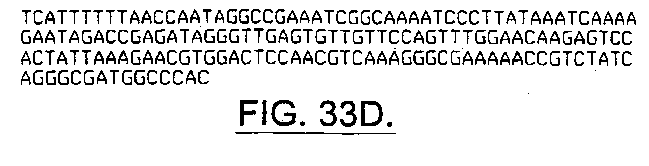

**FIGURES 33A-33D****.** Nucleotide sequence of pRIGbdl.

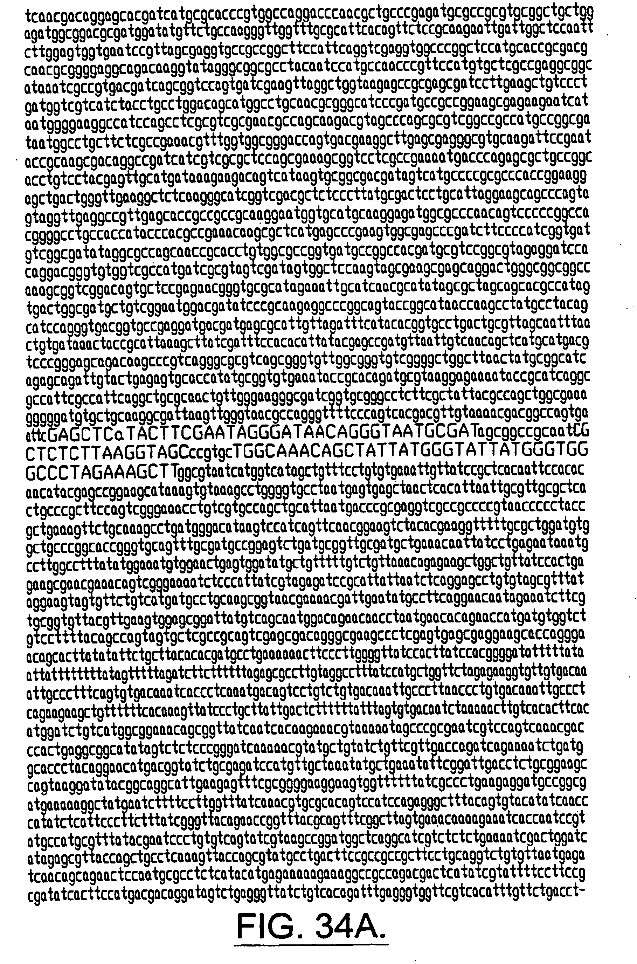

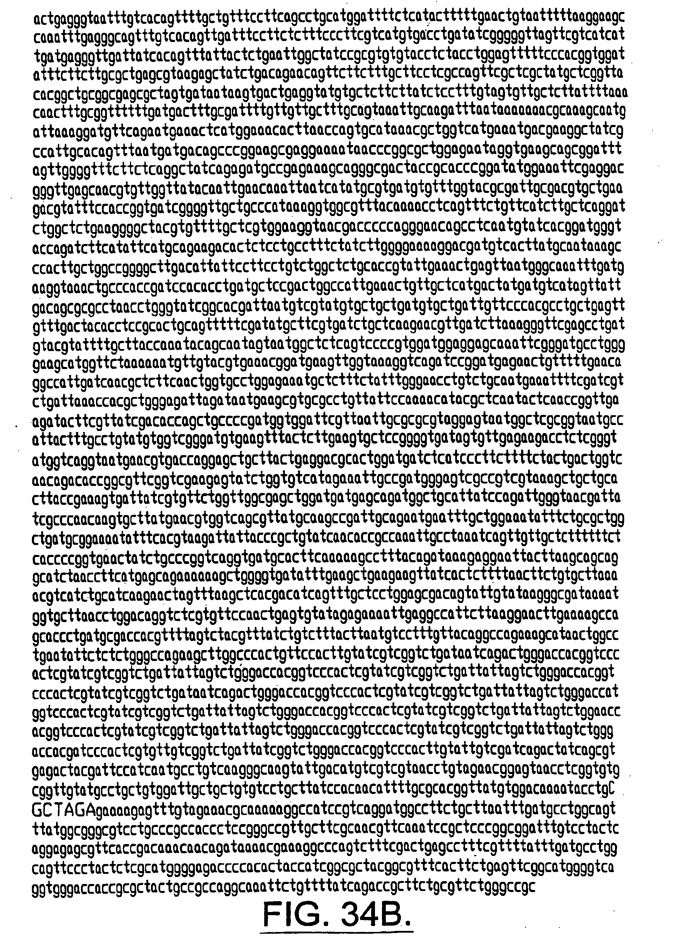

**FIGURES 34A-34B****.** Nucleotide sequence of pUniBAC.

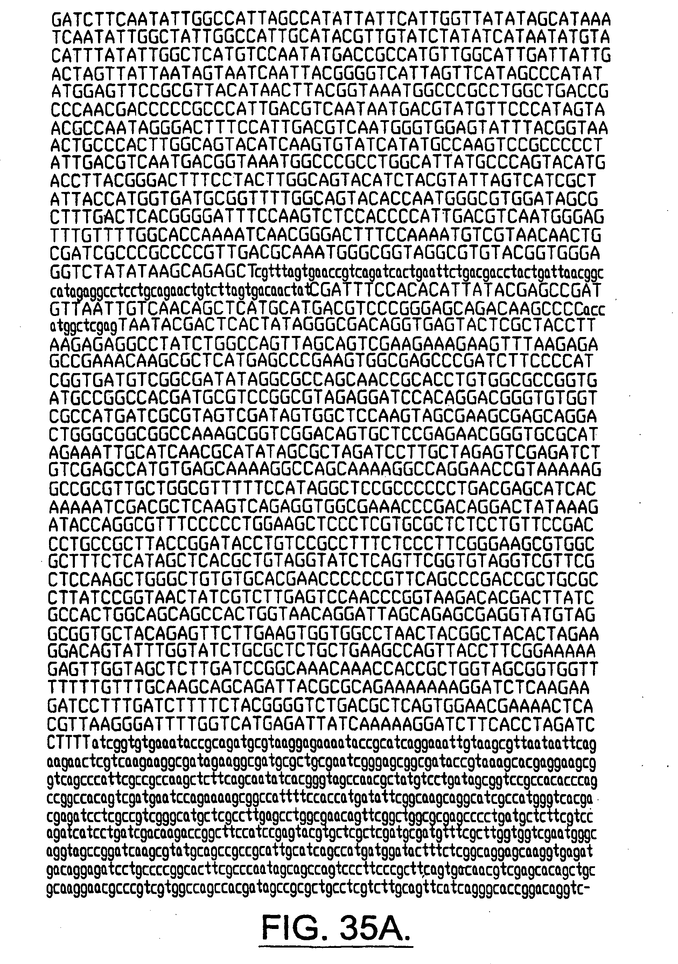

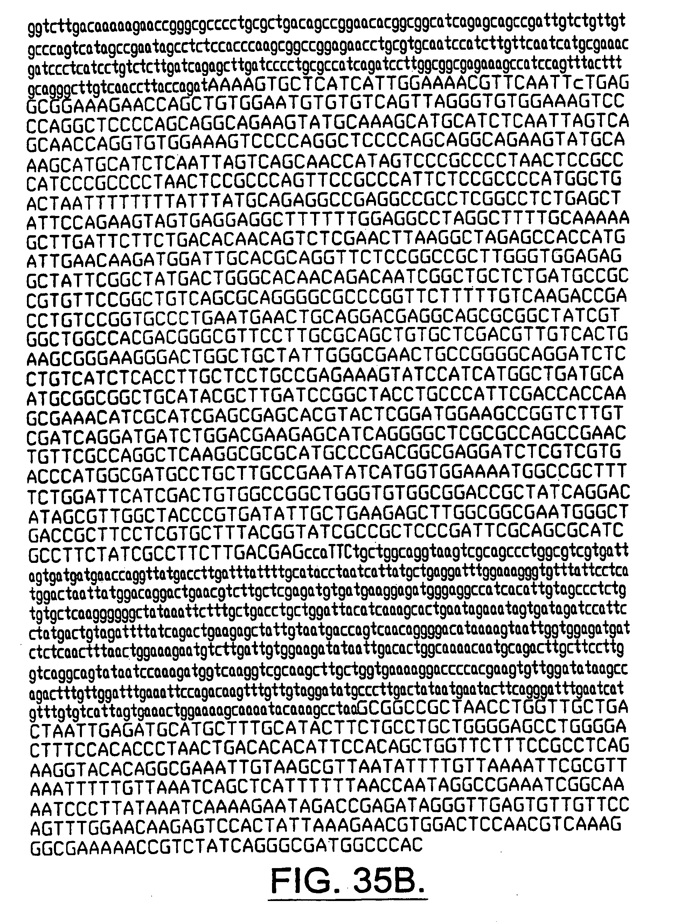

**FIGURES 35A-35B****.** Nucleotide sequence of pRIG22.

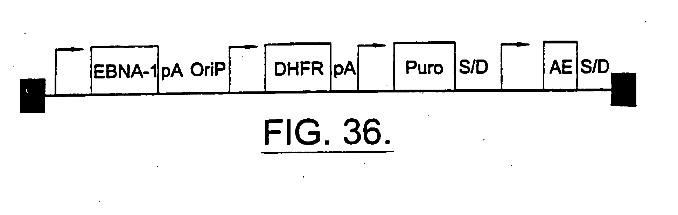

**FIGURE 36****.** Schematic diagram of pRIG-TP. The vector is shown in its linearized form. The horizontal line represents a DNA molecule. The arrows denote promoters. Open boxes indicate exons. Filled boxes represent transposon recombination signals (from Tn5 - compatible with the *in vitro* transposition kit available from Epicentre Technologies). The following designations were used: splice donor site (S/D), puromycin resistance gene (puro), dihydrofolate reductase gene (DHFR), Epstein Barr nuclear antigen - 1 replication protein (EBNA-1), Epstein Barr virus origin of replication (ori P), poly (A) signal (pA), and activation exon (AE). It is understood that the activation exon can contain any sequence capable of directing protein synthesis, including a translation start codon in any reading frame, a partial secretion signal sequence, an entire secretion signal sequence, an epitope tag, a protein, a portion of a protein, or a protein motif. The activation exon may also lack a translation start codon.

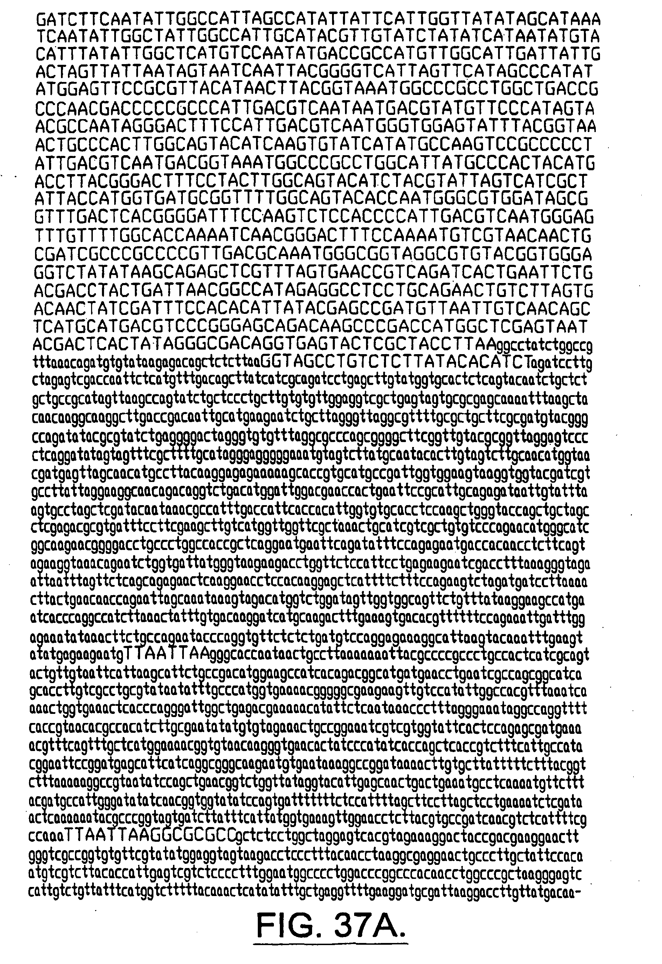

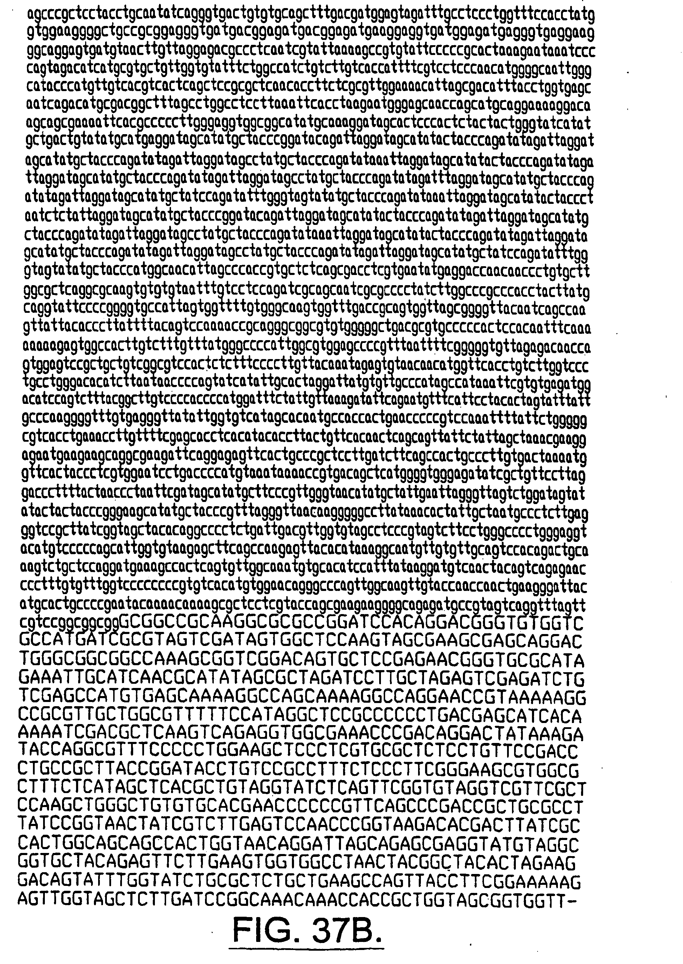

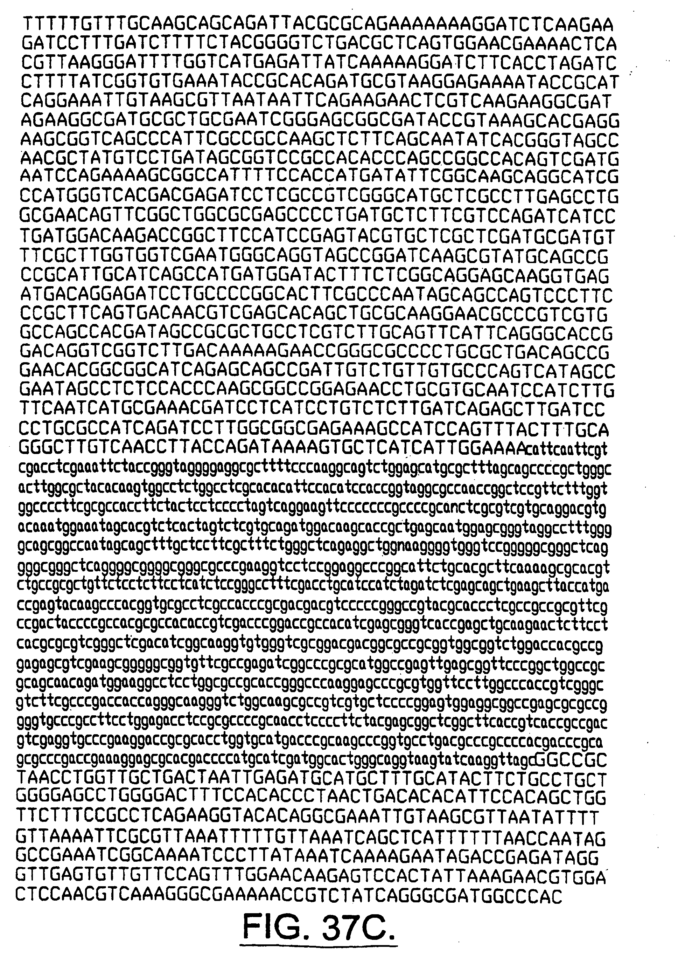

**FIGURES 37A-37C****.** Nucleotide sequence of pRIG-T.

### DETAILED DESCRIPTION OF THE INVENTION

There are great advantages to gene activation by non-homologous recombination over other gene activation procedures. Unlike previous methods of protein over-expression, the methods described herein do not require that the gene of interest be cloned (isolated from the cell). Nor do they require any knowledge of the DNA sequence or structure of the gene to be over-expressed *(i.e.,* the sequence of the ORF, introns, exons, or upstream and downstream regulatory elements) or knowledge of a gene's expression patterns (i.e., tissue specificity, developmental regulation, etc.). Furthermore, the methods do not require any knowledge pertaining to the genomic organization of the gene of interest (i.e., the intron and exon structure).

The methods of the present invention thus involve vector constructs that do not contain target nucleotide sequences for homologous recombination. A target sequence allows homologous recombination of vector DNA with cellular DNA at a predetermined site on the cellular DNA, the site having homology for sequences in the vector, the homologous recombination at the predetermined site resulting in the introduction of the transcriptional regulatory sequence into the genome and the subsequent endogenous gene activation.

The method of the present invention does not involve integration of the vector at predetermined sites. Instead, the present methods involve integration of the vector constructs of the invention into cellular DNA (e.g., the cellular genome) by nonhomologous or "illegitimate" recombination, also called "non-targeted gene activation."

In related embodiments, the present invention also concerns non-targeted gene activation. Non-targeted gene activation has a number of important applications. First, by activating genes that are not normally expressed in a given cell type, it becomes possible to isolate a cDNA copy of genes independent of their normal expression pattern. This facilitates isolation of genes that are normally expressed in rare cells, during short developmental periods, and/or at very low levels. Second, by translationally activating genes, it is possible to produce protein expression libraries without the need for cloning the full-length cDNA. These libraries can be screened for new enzymes and proteins and/or for interesting phenotypes resulting from over-expression of an endogenous gene. Third, cell-lines over-expressing a specific protein can be created and used to produce commercial quantities of protein. Thus, activating endogenous genes provides a powerful approach to discovering and isolating new genes and proteins, and to producing large amounts of specific proteins for commercialization.

The vectors described herein do not contain target sequences. A target sequence is a sequence on the vector that has homology with a sequence or sequences within the gene to be activated or upstream of the gene to be activated, the upstream region being up to and including the first functional splice acceptor site on the same coding strand of the gene of interest, and by means of which homology the transcriptional regulatory sequence that activates the gene of interest is integrated into the genome of the cell containing the gene to be activated. In the case of an enhancer integration vector for activating an endogenous gene, the vector does not contain homology to any sequence in the genome upstream or downstream of the gene of interest (or within the gene of interest) for a distance extending as far as enhancer function is operative.

The present methods, therefore, are capable of identifying new genes that have been or can be missed using conventional and currently available cloning techniques. By using the constructs and methodology described herein, unknown and/or uncharacterized genes can be rapidly identified and over-expressed to produce proteins. The proteins have use as, among other things, human therapeutics and diagnostics and as targets for drug discovery.

The methods are also capable of producing over-expression of known and/or characterized genes for *in vitro* or *in vivo* protein production.

A "known" gene is directed to the level of characterization of a gene. The invention allows expression of genes that have been characterized, as well as expression of genes that have not been characterized. Different levels of characterization are possible. These include detailed characterization, such as cloning, DNA, RNA, and/or protein sequencing, and relating the regulation and function of the gene to the cloned sequence (e.g., recognition of promoter and enhancer sequences, functions of the open reading frames, introns, and the like). Characterization can be less detailed, such as having mapped a gene and related function, or having a partial amino acid or nucleotide sequence, or having purified a protein and ascertained a function. Characterization may be minimal, as when a nucleotide or amino acid sequence is known or a protein has been isolated but the function is unknown. Alternatively, a function may be known but the associated protein or nucleotide sequence is not known or is known but has not been correlated to the function. Finally, there may be no characterization in that both the existence of the gene and its function are not known. The invention allows expression of any gene at any of these or other specific degrees of characterization.

Many different proteins (also referred to herein interchangeably as "gene products" or "expression products") can be activated or over-expressed by a single activation construct and in a single set of transfections. Thus, a single cell or different cells in a set of transfectants (library) can over-express more than one protein following transfection with the same or different constructs. Previous activation methods require a unique construct to be created for each gene to be activated.

Further, many different integration sites adjacent to a single gene can be created and tested simultaneously using a single construct. This allows rapid determination of the optimal genomic location of the activation construct for protein expression.

Using previous methods, the 5' end of the gene of interest had to be extensively characterized with respect to sequence and structure. For each activation construct to be produced, an appropriate targeting sequence had to be isolated. Usually, this must be an isogenic sequence isolated from the same person or laboratory strain of animal as the cells to be activated. In some cases, this DNA may be 50 kb or more from the gene of interest. Thus, production of each targeting construct required an arduous amount of cloning and sequencing of the endogenous gene. However, since sequence and structure information is not required for the methods of the present invention, unknown genes and genes with uncharacterized upstream regions can be activated.

This is made possible using *in situ* gene activation using non- homologous recombination of exogenous DNA sequences with cellular DNA. Methods and compositions (e.g., vector constructs) required to accomplish such *in situ* gene activation using non- homologous recombination are provided by the present invention.

DNA molecules can recombine to redistribute their genetic content by several different and distinct mechanisms, including homologous recombination, site-specific recombination, and non-homologous/illegitimate recombination. Homologous recombination involves recombination between stretches of DNA that are highly similar in sequence. It has been demonstrated that homologous recombination involves pairing between the homologous sequences along their length prior to redistribution of the genetic material. The exact site of crossover can be at any point in the homologous segments. The efficiency of recombination is proportional to the length of homologous targeting sequence (Hope, *Development 113:399 (1991); Reddy et al., J. Virol. 65:1507* (1991)), the degree of sequence identity between the two recombining sequences (von Melchner *et al., Genes Dev. 6*:919 (1992)), and the ratio of homologous to non-homologous DNA present on the construct (Letson, *Genetics 117*:759 (1987)).

Site-specific recombination, on the other hand, involves the exchange of genetic material at a predetermined site, designated by specific DNA sequences. In this reaction, a protein recombinase binds to the recombination signal sequences, creates a strand scission, and facilitates DNA strand exchange. *Cre*/*Lox* recombination is an example of site specific recombination.

Non-homologous/illegitimate recombination, such as that used advantageously by the methods of the present invention, involves the joining (exchange or redistribution) of genetic material that does not share significant sequence homology and does not occur at site-specific recombination sequences. Examples of non-homologous recombination include integration of exogenous DNA into chromosomes at non-homologous sites, chromosomal translocations and deletions, DNA end-joining, double strand break repair of chromosome ends, bridge-breakage fusion, and concatemerization of transfected sequences. In most cases, non-homologous recombination is thought to occur through the joining of "free DNA ends." Free ends are DNA molecules that contain an end capable of being joined to a second DNA end either directly, or following repair or processing. The DNA end may consist of a 5' overhang, 3' overhang, or blunt end.

As used herein, retroviral insertion and other transposition reactions are loosely considered forms of non-homologous recombination. These reactions do not involve the use of homology between the recombining molecules. Furthermore, unlike site-specific recombination, these types of recombination reactions do not occur between discrete sites. Instead, a specific protein/DNA complex is required on only one of the recombination partners (i.e., the retrovirus or transposon), with the second DNA partner *(i.e.,* the cellular genome) usually being relatively non-specific. As a result, these "vectors" do not integrate into the cellular genome in a targeted fashion, and therefore they can be used to deliver the activation construct according to the present invention.

Vector constructs useful for the methods described herein contain a transcriptional regulatory sequence that undergoes non-homologous recombination with genomic sequences in a cell to over-express an endogenous gene in that cell. The vector constructs of the invention also lack homologous targeting sequences. That is, they do not contain DNA sequences that target host cell DNA and promote homologous recombination at the target site. Thus, integration of the vector constructs of the present invention into the cellular genome occurs by non-homologous recombination, and can lead to over-expression of a cellular gene via the introduced transcriptional regulatory sequence contained on the integrated vector construct.

The invention is generally directed to methods for over-expressing an endogenous gene in a cell, comprising introducing a vector containing a transcriptional regulatory sequence into the cell, allowing the vector to integrate into the genome of the cell by non-homologous recombination, and allowing over-expression of the endogenous gene in the cell. The method does not require previous knowledge of the sequence of the endogenous gene or even of the existence of the gene. Where the sequence of the gene to be activated is known, however, the constructs can be engineered to contain the proper configuration of vector elements (e.g., location of the start codon, addition of codons present in the first exon of the endogenous gene, and the proper reading frame) to achieve maximal overexpression and/or the appropriate protein sequence.

In certain embodiments of the invention, the cell containing the vector may be screened for expression of the gene.

The cell over-expressing the gene can be cultured in *vitro* under conditions favoring the production, by the cell, of desired amounts of the gene product of the endogenous gene that has been activated or whose expression has been increased. If desired, the gene product can then be isolated or purified to use, for example, in protein therapy or drug discovery.

Alternatively, the cell expressing the desired gene product can be allowed to express the gene product *in vivo*.

Other activation vectors include constructs with a transcriptional regulatory sequence and an exonic sequence containing a start codon; a transcriptional regulatory sequence and an exonic sequence containing a translational start codon and a secretion signal sequence; constructs with a transcriptional regulatory sequence and an exonic sequence containing a translation start codon, and an epitope tag; constructs containing a transcriptional regulatory sequence and an exonic sequence containing a translational start codon, a signal sequence and an epitope tag; constructs containing a transcriptional regulatory sequence and an exonic sequence with a translation start codon, a signal secretion sequence, an epitope tag, and a sequence-specific protease site. In each of the above constructs, the exon on the construct is located immediately upstream of an unpaired splice donor site.

The constructs can also contain a regulatory sequence, a selectable marker lacking a poly(A) signal, an internal ribosome entry site (ires), and an unpaired splice donor site (Figure 4). A start codon, signal secretion sequence, epitope tag, and/or a protease cleavage site may optionally be included between the ires and the unpaired splice donor sequence. When this construct integrates upstream of a gene, the selectable marker will be efficiently expressed since a poly(A) site will be supplied by the endogenous gene. In addition the downstream gene will also be expressed since the ires will allow protein translation to initiate at the downstream open reading frame (*i.e*. the endogenous gene). Thus, the message produced by this activation construct will be polycistronic. The advantage of this construct is that integration events that do not occur near genes and in the proper orientation will not produce a drug resistant colony. The reason for this is that without a poly(A) tail (supplied by the endogenous gene), the neomycin resistance gene will not express efficiently. By reducing the number of nonproductive integration events, the complexity of the library can be reduced without affecting its coverage (the number of genes activated), and this will facilitate the screening process.

In another embodiment of this construct, *cre-lox* recombination sequences can be included between the regulatory sequence and the *neo* start codon and between the ires and the unpaired splice donor site (between the ires and the start codon, if present). Following isolation of cells that have activated the gene of interest, the *neo* gene and ires can be removed by transfecting the cells with a plasmid encoding the *cre* recombinase. This would eliminate the production of the polycistronic message and allow the endogenous gene to be expressed directly from the regulatory sequence on the integrated activation construct. Use of *Cre* recombination to facilitate deletion of genetic elements from mammalian chromosomes has been described (Gu et al., Science 265:103 (1994); Sauer, Meth. Enzymology 225:890-900 (1993)).

Also referred to are constructs including the following (See also Figures 1-4):
1) Construct with a regulatory sequence and an exon lacking a translation start codon.
2) Construct with a regulatory sequence and an exon lacking a translation start codon followed by a splice donor site.
3) Construct with a regulatory sequence and an exon containing a translation start codon in reading frame 1 (relative to the splice donor site), followed by an unpaired splice donor site.
4) Construct with a regulatory sequence and an exon containing a translation start codon in reading frame 2 (relative to the splice donor site), followed by an unpaired splice donor site.
5) Construct with a regulatory sequence and an exon containing a translation start codon in reading frame 3 (relative to the splice donor site), followed by an unpaired splice donor site.
6) Construct with a regulatory sequence and an exon containing a translation start codon and a signal secretion sequence in reading frame 1 (relative to the splice donor site), followed by an unpaired splice donor site.
7) Construct with a regulatory sequence and an exon containing a translation start codon and a signal secretion sequence in reading frame 2 (relative to the splice donor site), followed by an unpaired splice donor site.
8) Construct with a regulatory sequence and an exon containing a translation start codon and a signal secretion sequence in reading frame 3 (relative to the splice donor site), followed by an unpaired splice donor site.
9) Construct with a regulatory sequence and an exon containing (from 5' to 3') a translation start codon and an epitope tag in reading frame 1 (relative to the splice donor site), followed by an unpaired splice donor site.
10) Construct with a regulatory sequence and an exon containing (from 5' to 3') a translation start codon and an epitope tag in reading frame 2 (relative to the splice donor site), followed by an unpaired splice donor site.
11) Construct with a regulatory sequence and an exon containing (from 5' to 3') a translation start codon and an epitope tag in reading frame 3 (relative to the splice donor site), followed by an unpaired splice donor site.
12) Construct with a regulatory sequence and an exon containing (from 5' to 3') a translation start codon, a signal secretion sequence, and an epitope tag in reading frame 1 (relative to the splice donor site), followed by an unpaired splice donor site.
13) Construct with a regulatory sequence and an exon containing (from 5' to 3) a translation start codon, a signal secretion sequence, and an epitope tag in reading frame 2 (relative to the splice donor site), followed by an unpaired splice donor site.
14) Construct with a regulatory sequence and an exon containing (from 5' to 3') a translation start codon, a signal secretion sequence, and an epitope tag in reading frame 3 (relative to the splice donor site), followed by an unpaired splice donor site.
15) Construct with a regulatory sequence and an exon containing (from 5' to 3') a translation start codon, a signal secretion sequence, an epitope tag, and a sequence specific protease site in reading frame 1 (relative to the splice donor site), followed by an unpaired splice donor site.
16) Construct with a regulatory sequence and an exon containing (from 5' to 3') a translation start codon, a signal secretion sequence, an epitope tag, and a sequence specific protease site in reading frame 2 (relative to the splice donor site), followed by an unpaired splice donor site.
17) Construct with a regulatory sequence and an exon containing (from 5' to 3') a translation start codon, a signal secretion sequence, an epitope tag, and a sequence specific protease site in reading frame 3 (relative to the splice donor site), followed by an unpaired splice donor site.
18) Construct with a regulatory sequence linked to a selectable marker, followed by an internal ribosome entry site, and an unpaired splice donor site.
19) Construct 18 in which a cre/lox recombination signal is located between a) the regulatory sequence and the open reading frame of the selectable marker and b) between the ires and the unpaired splice donor site.
20) Construct with a regulatory sequence operably linked to an exon containing green fluorescent protein lacking a stop codon, followed by an unpaired splice donor site.

It is to be understood, however, that any vector used in the methods described herein can include one or more *(i.e.,* one, two, three, four, five, or more, and most preferably one or two) amplifiable markers. Accordingly, methods can include a step in which the endogenous gene is amplified. Placement of one or more amplifiable markers on the activation construct results in the juxtaposition of the gene of interest and the one or more amplifiable markers in the activated cell. Once the activated cell has been isolated, expression can be further increased by selecting for cells containing an increased copy number of the locus containing both the gene of interest and the activation construct. This can be accomplished by selection methods known in the art, for example by culturing cells in selective culture media containing one or more selection agents that are specific for the one or more amplifiable markers contained on the genetic construct or vector.

Following activation of an endogenous gene by nonhomologous integration of any of the vectors described above, the expression of the endogenous gene may be further increased by selecting for increased copies of the amplifiable marker(s) located on the integrated vector. While such an approach may be accomplished using one amplifiable marker on the integrated vector, in an alternative embodiment the invention provides such methods wherein two or more (i.e., two, three, four, five, or more, and most preferably two) amplifiable markers may be included on the vector to facilitate more efficient selection of cells that have amplified the vector and flanking gene of interest. This approach is particularly useful in cells that have a functional endogenous copy of one or more of the amplifiable marker(s) that are contained on the vector, since the selection procedure can result in isolation of cells that have incorrectly amplified the endogenous amplifiable marker(s) rather than the vector-encoded amplifiable marker(s). This approach is also useful to select against cells that develop resistance to the selective agent by mechanisms that do not involve gene amplification. The approach using two or more amplifiable markers is advantageous in these situations because the probability of a cell developing resistance to two or more selective agents (resistance to which is encoded by two or more amplifiable markers) without amplifying the integrated vector and flanking gene of interest is significantly lower than the probability of the cell developing resistance to any single selective agent. Thus, by selecting for two or more vector encoded amplifiable markers, either simultaneously or sequentially, a greater percentage of cells that are ultimately isolated will contain the amplified vector and gene of interest.

Thus, in another embodiment, the vectors of the invention may contain two or more (*i.e.,* two, three, four, five, or more, and most preferably two) amplifiable markers. This approach allows more efficient amplification of the vector sequences and adjacent gene of interest following activation of expression.

Examples of amplifiable markers that may be used constructing the present vectors include, but are not limited to, dihydrofolate reductase, adenosine deaminase, aspartate transcarbamylase, dihydro-orotase, and carbamyl phosphate synthase.

It is also understood that any of the constructs described herein may contain a eukaryotic viral origin of replication, either in place of, or in conjunction with an amplifiable marker. The presence of the viral origin of replication allows the integrated vector and adjacent endogenous gene to be isolated as an episome and/or amplified to high copy number upon introduction of the appropriate viral replication protein. Examples of useful viral origins include, but are not limited to, SV40 ori and EBV ori P.

The invention also encompasses embodiments in which the constructs disclosed herein consist essentially of the components specifically described for these constructs. It is also understood that the above constructs are examples of constructs useful in the methods described herein, but that the invention encompasses functional equivalents of such constructs.

The term "vector" is understood to generally refer to the vehicle by which the nucleotide sequence is introduced into the cell. It is not intended to be limited to any specific sequence. The vector could itself be the nucleotide sequence that activates the endogenous gene or could contain the sequence that activates the endogenous gene. Thus, the vector could be simply a linear or circular polynucleotide containing essentially only those sequences necessary for activation, or could be these sequences in a larger polynucleotide or other construct such as a DNA or RNA viral genome, a whole virion, or other biological construct used to introduce the critical nucleotide sequences into a cell. It is also understood that the phrase "vector construct" or the term "construct" may be used interchangeably with the term "vector" herein.

The vector can contain DNA sequences that exist in nature or that have been created by genetic engineering or synthetic processes.

The construct, upon nonhomologous integration into the genome of a cell, can activate expression of an endogenous gene. Expression of the endogenous gene may result in production of full length protein, or in production of a truncated biologically active form of the endogenous protein, depending on the integration site (e.g., upstream region versus intron 2). The activated gene may be a known gene (e.g., previously cloned or characterized) or unknown gene (previously not cloned or characterized). The function of the gene may be known or unknown.

Examples of proteins with known activities include, but are not limited to, cytokines, growth factors, neurotransmitters, enzymes, structural proteins, cell surface receptors, intracellular receptors, hormones, antibodies, and transcription factors. Specific examples of known proteins that can be produced by this method include, but are not limited to, erythropoietin, insulin, growth hormone, glucocerebrosidase, tissue plasminogen activator, granulocyte-colony stimulating factor (G-CSF), granulocyte/macrophage colony stimulating factor (GM-CSF), macrophage colony-stimulating factor (M-CSF) interferon α, interferon β, interferon γ, interleukin-2, interleukin-3, interleukin-4, interleukin-6, interleukin-8, interleukin-10, interleukin-11, interleukin-12, interleukin-13, interleukin-14, TGF-β, blood clotting factor V, blood clotting factor VII, blood clotting factor VIII, blood clotting factor IX, blood clotting factor X, TSH-β, bone growth factor-2, bone growth factor-7, tumor necrosis factor, alpha-1 antitrypsin, anti-thrombin III, leukemia inhibitory factor, glucagon, Protein C, protein kinase C, stem cell factor, follicle stimulating hormone β, urokinase, nerve growth factors, insulin-like growth factors, insulinotropin, parathyroid hormone, lactoferrin, complement inhibitors, platelet derived growth factor, keratinocyte growth factor, hepatocyte growth factor, endothelial cell growth factor, neurotropin-3, thrombopoietin, chorionic gonadotropin, thrombomodulin, alpha glucosidase, epidermal growth factor, and fibroblast growth factor. The invention also allows the activation of a variety of genes expressing transmembrane proteins, and production and isolation of such proteins, including but not limited to cell surface receptors for growth factors, hormones, neurotransmitters and cytokines such as those described above, transmembrane ion channels, cholesterol receptors, receptors for lipoproteins (including LDLs and HDLs) and other lipid moieties, integrins and other extracellular matrix receptors, cytoskeletal anchoring proteins, immunoglobulin receptors, CD antigens (including CD2, CD3, CD4, CD8, and CD34 antigens), and other cell surface transmembrane structural and functional proteins that are known in the art. As one of ordinary skill will appreciate, other cellular proteins and receptors that are known in the art may also be produced by the methods of the invention.

One of the advantages of the method described herein is that virtually any gene can be activated. However, since genes have different genomic structures, including different intron/exon boundaries and locations of start codons, a variety of activation constructs is provided to activate the maximum number of different genes within a population of cells.

These constructs can be transfected separately into cells to produce libraries. Each library contains cells with a unique set of activated genes. Some genes will be activated by several different activation constructs. In addition, portions of a gene can be activated to produce truncated, biologically active proteins. Truncated proteins can be produced, for example, by integration of an activation construct into introns or exons in the middle of an endogenous gene rather than upstream of the second exon.

Use of different constructs also allows the activated gene to be modified to contain new sequences. For example, a secretion signal sequence can be included on the activation construct to facilitate the secretion of the activated gene. In some cases, depending on the intron/exon structure or the gene of interest, the secretion signal sequence can replace all or part of the signal sequence of the endogenous gene. In other cases, the signal sequence will allow a protein which is normally located intracellularly to be secreted.

The regulatory sequence on the vector can be a constitutive promoter. Alternatively, the promoter may be inducible. Use of inducible promoters will allow low basal levels of activated protein to be produced by the cell during routine culturing and expansion. The cells may then be induced to produce large amounts of the desired proteins, for example, during manufacturing or screening. Examples of inducible promoters include, but are not limited to, the tetracycline inducible promoter and the metallothionein promoter.

The regulatory sequence on the vectors of the invention comprises a promoter which may be tissue specific.

The regulatory sequence on the vector can be isolated from cellular or viral genomes. Examples of cellular regulatory sequences include, but are not limited to, regulatory elements from the actin gene, metallothionein I gene, immunoglobulin genes, casein I gene, serum albumin gene, collagen gene, globin genes, laminin gene, spectrin gene, ankyrin gene, sodium/potassium ATPase gene, and tubulin gene. Examples of viral regulatory sequences include, but are not limited to, regulatory elements from *Cytomegalovirus* (CMV) immediate early gene, adenovirus late genes, SV40 genes, retroviral LTRs, and *Herpesvirus* genes. Typically, regulatory sequences contain binding sites for transcription factors such as NF-kB, SP-1, TATA binding protein, AP-1, and CAAT binding protein. Functionally, the regulatory sequence is defined by its ability to promote, enhance, or otherwise alter transcription of an endogenous gene.

In certain preferred embodiments, the regulatory sequence is a viral promoter. In particularly preferred embodiments, the promoter is the CMV immediate early gene promoter. In alternative embodiments, the regulatory element is a cellular, non-viral promoter.

In alternative preferred embodiments, the regulatory element may contain an enhancer. In particularly preferred such embodiments, the enhancer is the cytomegalovirus immediate early gene enhancer. In alternative embodiments, the enhancer is a cellular, non-viral enhancer.

In alternative preferred embodiments, the regulatory element may contain a repressor. In particularly preferred such embodiments, the repressor may be a viral repressor or a cellular, non-viral repressor.

The transcriptional regulatory sequence can also comprise one or more scaffold-attachment regions or matrix attachment sites, negative regulatory elements, and transcription factor binding sites. Regulatory sequences can also include locus control regions.

The invention also encompasses the use of retrovirus transcriptional regulatory sequences, e.g., long terminal repeats. Where these are used, however, they are not necessarily linked to any retrovirus sequence that materially affects the function of the transcriptional regulatory sequence as a promoter or enhancer of transcription of the endogenous gene to be activated (*i.e*., the cellular gene with which the transcriptional regulatory sequence recombines to activate).

The first regulatory element is operably linked to an exon and an unpaired splice donor site. The exon may be a naturally occurring sequence or may be non-naturally occurring (e.g., produced synthetically). To activate endogenous genes lacking a start codon in their first exon (e.g., follicle stimulating honnone-β), a start codon is preferably omitted from the exon on the vector. To activate endogenous genes containing a start codon in the first exon (e.g., erythropoietin and growth hormone), the exon on the vector preferably contains a start codon, usually ATG and preferably an efficient translation initiation site (Kozak, J. Mol Biol. 196: 947 (1987)). The exon may contain additional codons following the start codon. These codons may be derived from a naturally occurring gene or may be non-naturally occurring (e.g., synthetic). The codons may be the same as the codons present in the first exon of the endogenous gene to be activated. Alternatively, the codons may be different than the codons present in the first exon of the endogenous gene. For example, the codons may encode an epitope tag, signal secretion sequence, transmembrane domain, selectable marker, or screenable marker. An unpaired splice donor site may be present immediately 3' of the exonic sequence. When the structure of the gene to be activated is known, the splice donor site should be placed adjacent to the vector exon in a location such that the codons in the vector will be in frame with the codons of the second exon of the endogenous gene following splicing. When the structure of the endogenous gene to be activated is not known, separate constructs, each containing a different reading frame, are used.

Operably linked is defined as a configuration that allows transcription through the designated sequence(s). For example, a regulatory sequence that is operably linked to an exonic sequence indicates that the exonic sequence is transcribed. When a start codon is present on the vector, operably linked also indicates that the open reading frame from the vector exon is in frame with the open reading frame of the endogenous gene. Following nonhomologous integration, the regulatory sequence (e.g., a promoter) on the vector becomes operably linked to an endogenous gene and facilitates transcription initiation, at a site generally referred to as a CAP site. Transcription proceeds through the exonic elements on the vector (and, if present, through the start codon, open reading frame, and/or unpaired splice donor site), and through the endogenous gene. The primary transcript produced by this operable linkage is spliced to create a chimeric transcript containing exonic sequences from both the vector and the endogenous gene. This transcript is capable of producing the endogenous protein when translated.

An exon or "exonic sequence" is defined as any transcribed sequence that is present in the mature RNA molecule. The exon on the vector may contain untranslated sequences, for example, a 5' untranslated region. Alternatively, or in conjunction with the untranslated sequences, the exon may contain coding sequences such as a start codon and open reading frame. The open reading frame can encode naturally occurring amino acid sequences or non-naturally occurring amino acid sequences (e.g., synthetic codons). The open reading frame may also encode a signal secretion sequence, epitope tag, exon, selectable marker, screenable marker, or nucleotides that function to allow the open reading frame to be preserved when spliced to an endogenous gene.

Splicing of primary transcripts, the process by which introns are removed, is directed by a splice donor site and a splice acceptor site, located at the 5' and 3' ends of introns, respectively. The consensus sequence for splice donor sites is (A/C)AG GURAGU (where R represents a purine nucleotide) with nucleotides in positions 1-3 located in the exon and nucleotides GURAGU located in the intron.

An unpaired splice donor site is defined herein as a splice donor site present on the activation construct without a downstream splice acceptor site. When the vector is integrated by nonhomologous recombination into a host cell's genome, the unpaired splice donor site becomes paired with a splice acceptor site from an endogenous gene. The splice donor site from the vector, in conjunction with the splice acceptor site from the endogenous gene, will then direct the excision of all of the sequences between the vector splice donor site and the endogenous splice acceptor site. Excision of these intervening sequences removes sequences that interfere with translation of the endogenous protein.

The terms upstream and downstream, as used herein, are intended to mean in the 5' or in the 3' direction, respectively, relative to the coding strand. The term "upstream region" of a gene is defined as the nucleic acid sequence 5' of its second exon (relative to the coding strand) up to and including the last exon of the first adjacent gene having the same coding strand. Functionally, the upstream region is any site 5' of the second exon of an endogenous gene capable of allowing a nonhomologously integrated vector to become operably linked to the endogenous gene.

The vector construct can contain a selectable marker to facilitate the identification and isolation of cells containing a nonhomologously integrated activation construct. Examples of selectable markers include genes encoding neomycin resistance (neo), hypoxanthine phosphoribosyl transferase (HPRT), puromycin (pac), dihydro-orotase glutamine synthetase (GS), histidine D (his D), carbamyl phosphate synthase (CAD), dihyrofolate reductase (DHFR), multidrug resistance 1 (mdr1), aspartate transcarbamylase, xanthine-guanine phosphoribosyl transferase (gpt), and adenosine deaminase (ada).

Alternatively, the vector can contain a screenable marker, in place of or in addition to, the selectable marker. A screenable marker allows the cells containing the vector to be isolated without placing them under drug or other selective pressures. Examples of screenable markers include genes encoding cell surface proteins, fluorescent proteins, and enzymes. The vector containing cells may be isolated, for example, by FACS using fluorescently-tagged antibodies to the cell surface protein or substrates that can be converted to fluorescent products by a vector encoded enzyme.

Alternatively, selection can be effected by phenotypic selection for a trait provided by the endogenous gene product. In this embodiment, activated cells can be selected based on a phenotype conferred by the activated gene. Examples of selectable phenotypes include cellular proliferation, growth factor independent growth, colony formation, cellular differentiation (e.g., differentiation into a neuronal cell, muscle cell, epithelial cell, etc.), anchorage independent growth, activation of cellular factors (e.g., kinases, transcription factors, nucleases, etc.), expression of cell surface receptors/proteins, gain or loss of cell-cell adhesion, migration, and cellular activation (e.g., resting versus activated T cells).

The vector may contain one or more (*i.e*., one, two, three, four, five, or more, and most preferably one or two) amplifiable markers to allow for selection of cells containing increased copies of the integrated vector and the adjacent activated endogenous gene. Examples of amplifiable markers include but are not limited to dihydrofolate reductase (DHFR), adenosine deaminase (ada), dihydro-orotase glutamine synthetase (GS), and carbamyl phosphate synthase (CAD).

The vector may contain eukaryotic viral origins ofreplication useful for gene amplification. These origins may be present in place of, or in conjunction with, an amplifiable marker.

The vector may also contain genetic elements useful for the propagation of the construct in micro-organisms. Examples of useful genetic elements include microbial origins of replication and antibiotic resistance markers.

These vectors, and any of the vectors disclosed herein, and obvious variants recognized by one of ordinary skill in the art, can be used in any of the methods described herein to form any of the compositions producible by those methods.

Nonhomologous integration of the construct into the genome of a cell results in the operable linkage between the regulatory elements from the vector and the exons from an endogenous gene. In preferred embodiments, the insertion of the vector regulatory sequences is used to upregulate expression of the endogenous gene. Upregulation of gene expression includes converting a transcriptionally silent gene to a transcriptionally active gene. It also includes enhancement of gene expression for genes that are already transcriptionally active, but produce protein at levels lower than desired. In other embodiments, expression of the endogenous gene may be affected in other ways such as downregulation of expression, creation of an inducible phenotype, or changing the tissue specificity of expression.

According to the invention, *in vitro* methods of production of a gene expression product may comprise, for example, (a) introducing a vector of the invention into a cell; (b) allowing the vector to integrate into the genome of the cell by non-homologous recombination; (c) allowing over-expression of an endogenous gene in the cell by upregulation of the gene by the transcriptional regulatory sequence contained on the vector; (d) screening the cell for over-expression of the endogenous gene; and (e) culturing the cell under conditions favoring the production of the expression product of the endogenous gene by the cell. Such in *vitro* methods of the invention may further comprise isolating the expression product to produce an isolated gene expression product. In such methods, any art-known method of protein isolation may be advantageously used, including but not limited to chromatography (e.g., HPLC, FPLC, LC, ion exchange, affinity, size exclusion, and the like), precipitation (e.g., ammonium sulfate precipitation, immunoprecipitation, and the like), electrophoresis, and other methods of protein isolation and purification that will be familiar to one of ordinary skill in the art.

Analogously, *in vivo* methods of production of a gene expression product may comprise, for example, (a) introducing a vector of the invention into a cell; (b) allowing the vector to integrate into the genome of the cell by non-homologous recombination; (c) allowing over-expression of an endogenous gene in the cell by upregulation of the gene by the transcriptional regulatory sequence contained on the vector; (d) screening the cell for over-expression of the endogenous gene; and (e) introducing the isolated and cloned cell into a eukaryote under conditions favoring the overexpression of the endogenous gene by the cell *in vivo* in the eukaryote. According to this aspect of the invention, any eukaryote may be advantageously used, including fungi (particularly yeasts), plants, and animals, more preferably animals, still more preferably vertebrates, and most preferably mammals, particularly humans. In certain related embodiments, the invention provides such methods which further comprise isolating and cloning the cell prior to introducing it into the eukaryote.

As used herein the phrases "conditions favoring the production" of an expression product, "conditions favoring the overexpression" of a gene, and "conditions favoring the activation" of a gene, in a cell or by a cell *in vitro* refer to any and all suitable environmental, physical, nutritional or biochemical parameters that allow, facilitate, or promote production of an expression product, or overexpression or activation of a gene, by a cell *in vitro.* Such conditions may, of course, include the use of culture media, incubation, lighting, humidity, etc., that are optimal or that allow, facilitate, or promote production of an expression product, or overexpression or activation of a gene, by a cell *in vitro.* Analogously, as used herein the phrases "conditions favoring the production" of an expression product, "conditions favoring the overexpression" of a gene, and "conditions favoring the activation" of a gene, in a cell or by a cell *in vivo* refer to any and all suitable environmental, physical, nutritional, biochemical, behavioral, genetic, and emotional parameters under which an animal containing a cell is maintained, that allow, facilitate, or promote production of an expression product, or overexpression or activation of a gene, by a cell in a eukaryote *in vivo.* Whether a given set of conditions are favorable for gene expression, activation, or overexpression, *in vitro* or *in vivo,* may be determined by one of ordinary skill using the screening methods described and exemplified below, or other methods for measuring gene expression, activation, or overexpression that are routine in the art.

As used herein, the phrase "activating an endogenous gene" means inducing the production of a transcript encoding the endogenous gene at levels higher than those normally found in the cell containing the endogenous gene. In some applications, "activating an endogenous gene" may also mean producing the protein, or a portion of the protein, encoded by the endogenous gene at levels higher than those normally found in the cell containing the endogenous gene.

The invention also encompasses cells made by any of the above methods. The invention encompasses cells in which the vector constructs have integrated, and cells which are over-expressing desired gene products from an endogenous gene, over-expression being driven by the introduced transcriptional regulatory sequence.

Cells used in this invention can be derived from any eukaryotic species and can be primary, secondary, or immortalized. Furthermore, the cells can be derived from any tissue in the organism. Examples of useful tissues from which cells can be isolated and activated include, but are not limited to, liver, kidney, spleen, bone marrow, thymus, heart, muscle, lung, brain, testes, ovary, islet, intestinal, bone marrow, skin, bone, gall bladder, prostate, bladder, embryos, and the immune and hematopoietic systems. Cell types include fibroblast, epithelial, neuronal; stem, and follicular. However, any cell or cell type can be used to activate gene expression using this invention.

The methods can be carried out in any cell of eukaryotic origin, such as fungal, plant or animal. Preferred embodiments include vertebrates and particularly mammals, and more particularly, humans.

The construct can be integrated into primary, secondary, or immortalized cells. Primary cells are cells that have been isolated from a vertebrate and have not been passaged. Secondary cells are primary cells that have been passaged, but are not immortalized. Immortalized cells are cell lines that can be passaged, apparently indefinitely.

In preferred embodiments, the cells are immortalized cell lines. Examples of immortalized cell lines include, but are not limited to, HT1080, HeLa, Jurkat, 293 cells, KB carcinoma, T84 colonic epithelial cell line, Raji, Hep G2 or Hep 3B hepatoma cell lines, A2058 melanoma, U937 lymphoma, and W138 fibroblast cell line, somatic cell hybrids, and hybridomas.

Cells used in this invention can be derived from any eukaryotic species, including but not limited to mammalian cells (such as rat, mouse, bovine, porcine, sheep, goat, and human), avian cells, fish cells, amphibian cells, reptilian cells, plant cells, and yeast cells. Preferably, overexpression of an endogenous gene or gene product from a particular species is accomplished by activating gene expression in a cell from that species. For example, to overexpress endogenous human proteins, human cells are used. Similarly, to overexpress endogenous bovine proteins, for example bovine growth hormone, bovine cells are used.

The cells can be derived from any tissue in the eukaryotic organism. Examples of useful vertebrate tissues from which cells can be isolated and activated include, but are not limited to, liver, kidney, spleen, bone marrow, thymus, heart, muscle, lung, brain, immune system (including lymphatic), testes, ovary, islet, intestinal, stomach, bone marrow, skin, bone, gall bladder, prostate, bladder, zygotes, embryos, and hematopoietic tissue. Useful vertebrate cell types include, but are not limited to, fibroblasts, epithelial cells, neuronal cells, germ cells (*i.e.,* spermatocytes/sperinatozoa and oocytes), stem cells, and follicular cells. Examples of plant tissues from which cells can be isolated and activated include, but are not limited to, leaf tissue, ovary tissue, stamen tissue, pistil tissue, root tissue, tubers, gametes, seeds, embryos, and the like. One of ordinary skill will appreciate, however, that any eukaryotic cell or cell type can be used to activate gene expression using the present invention.

Any of the cells produced by any of the methods described are useful for screening for expression of a desired gene product and for providing desired amounts of a gene product that is over-expressed in the cell. The cells can be isolated and cloned.

Cells produced by this method can be used to produce protein *in vitro* (e.g., for use as a protein therapeutic) or *in vivo* (e.g., for use in the manufacture of medicaments for cell therapy).

Commercial growth and production conditions often vary from the conditions used to grow and prepare cells for analytical use (e.g., cloning, protein or nucleic acid sequencing, raising antibodies, X-ray crystallography analysis, enzymatic analysis, and the like). Scale up of cells for growth in roller bottles involves increase in the surface area on which cells can attach. Microcarrier beads are, therefore, often added to increase the surface area for commercial growth. Scale up of cells in spinner culture may involve large increases in volume. Five liters or greater can be required for both microcarrier and spinner growth. Depending on the inherent potency (specific activity) of the protein of interest, the volume can be as low as 1-10 liters. 10-15 liters is more common. However, up to 50-100 liters may be necessary and volume can be as high as 10,000-15,000 liters. In some cases, higher volumes may be required. Cells can also be grown in large numbers of T flasks, for example 50-100.

Despite growth conditions, protein purification on a commercial scale can also vary considerably from purification for analytic purposes. Protein purification in a commercial practical context can be initially the mass equivalent of 10 liters of cells at approximately 10⁴ cells/ml. Cell mass equivalent to begin protein purification can also be as high as 10 liters of cells at up to 10⁶ or 10⁷ cells/ml. As one of ordinary skill will appreciate, however, a higher or lower initial cell mass equivalent may also be advantageously used in the present methods.

Another commercial growth condition, especially when the ultimate product is used clinically, is cell growth in serum-free medium, by which is intended medium containing no serum or not in amounts that are required for cell growth. This obviously avoids the undesired co-purification of toxic contaminants (e.g., viruses) or other types of contaminants, for example, proteins that would complicate purification. Serum-free media for growth of cells, commercial sources for such media, and methods for cultivation of cells in serum-free media, are well-known to those of ordinary skill in the art.

A single cell made by the methods described above can over-express a single gene or more than one gene. More than one gene can be activated by the integration of a single construct or by the integration of multiple constructs in the same cell (i.e., more than one type of construct). Therefore, a cell can contain only one type of vector construct or different types of constructs, each capable of activating an endogenous gene.

The invention is also directed to methods for making the cells described above by one or more of the following: introducing one or more of the vector constructs; allowing the introduced construct(s) to integrate into the genome of the cell by non-homologous recombination; allowing over-expression of one or more endogenous genes in the cell; and isolating and cloning the cell.

The term "transfection" has been used herein for convenience when discussing introducing a polynucleotide into a cell. However, it is to be understood that the specific use of this term has been applied to generally refer to the *introduction* of the polynucleotide into a cell and is also intended to refer to the introduction by other methods described herein such as electroporation, liposome-mediated introduction, retrovirus-mediated introduction, and the like (as well as according to its own specific meaning).

The vector can be introduced into the cell by a number of methods known in the art. These include, but are not limited to, electroporation, calcium phosphate precipitation, DEAE dextran, lipofection, and receptor mediated endocytosis, polybrene, particle bombardment, and microinjection. Alternatively, the vector can be delivered to the cell as a viral particle (either replication competent or deficient). Examples of viruses useful for the delivery of nucleic acid include, but are not limited to, adenoviruses, adeno-associated viruses, retroviruses, Herpesviruseses, and vaccinia viruses. Other viruses suitable for delivery of nucleic acid molecules into cells that are known to one of ordinary skill may be equivalently used in the present methods.

Following transfection, the cells are cultured under conditions, as known in the art, suitable for nonhomologous integration between the vector and the host cell's genome. Cells containing the nonhomologously integrated vector can be further cultured under conditions, as known in the art, allowing expression of activated endogenous genes.

The vector construct can be introduced into cells on a single DNA construct or on separate constructs and allowed to concatemerize.

Whereas in preferred embodiments, the vector construct is a double-stranded DNA vector construct, vector constructs also include single-stranded DNA, combinations of single- and double-stranded DNA, single-stranded RNA, double-stranded RNA, and combinations of single- and double-stranded RNA. Thus, for example, the vector construct could be single-stranded RNA which is converted to cDNA by reverse transcriptase, the cDNA converted to double-stranded DNA, and the double-stranded DNA ultimately recombining with the host cell genome.

In preferred embodiments, the constructs are linearized prior to introduction into the cell. Linearization of the activation construct creates free DNA ends capable of reacting with chromosomal ends during the integration process. In general, the construct is linearized downstream of the regulatory element (and exonic and splice donor sequences, if present). Linearization can be facilitated by, for example, placing a unique restriction site downstream of the regulatory sequences and treating the construct with the corresponding restriction enzyme prior to transfection. While not required, it is advantageous to place a "spacer" sequence between the linearization site and the proximal most functional element (e.g., the unpaired splice donor site) on the construct. When present, the spacer sequence protects the important functional elements on the vector from exonucleolytic degradation during the transfection process. The spacer can be composed of any nucleotide sequence that does not change the essential functions of the vector as described herein.

Circular constructs can also be used to activate endogenous gene expression. It is known in the art that circular plasmids, upon transfection into cells, can integrate into the host cell genome. Presumably, DNA breaks occur in the circular plasmid during the transfection process, thereby generating free DNA ends capable of joining to chromosome ends. Some of these breaks in the construct will occur in a location that does not destroy essential vector functions (e.g., the break will occur downstream of the regulatory sequence), and therefore, will allow the construct to be integrated into a chromosome in a configuration capable of activating an endogenous gene. As described above, spacer sequences may be placed on the construct (e.g., downstream of the regulatory sequences). During transfection, breaks that occur in the spacer region will create free ends at a site in the construct suitable for activation of an endogenous gene following integration into the host cell genome.

The invention also encompasses libraries of cells made by the above described methods. A library can encompass all of the clones from a single transfection experiment or a subset of clones from a single transfection experiment. The subset can over-express the same gene or more than one gene, for example, a class of genes. The transfection can have been done with a single type of construct or with more than one type of construct.

A library can also be formed by combining all of the recombinant cells from two or more transfection experiments, by combining one or more subsets of cells from a single transfection experiment or by combining subsets of cells from separate transfection experiments. The resulting library can express the same gene, or more than one gene, for example, a class of genes. Again, in each of these individual transfections, a unique construct or more than one construct can be used.

Libraries can be formed from the same cell type or different cell types.

The library can be composed of a single type of cell containing a single type of activation construct which has been integrated into chromosomes at spontaneous DNA breaks or at breaks generated by radiation, restriction enzymes, and/or DNA breaking agents, applied either together (to the same cells) or separately (applied to individual groups of cells and then combining the cells together to produce the library). The library can be composed of multiple types of cells containing a single or multiple constructs which were integrated into the genome of a cell treated with radiation, restriction enzymes, and/or DNA breaking agents, applied either together (to the same cells) or separately (applied to individual groups of cells and then combining the cells together to produce the library).

The invention is also directed to methods for making libraries by selecting various subsets of cells from the same or different transfection experiments. For example, all of the cells expressing nuclear factors (as determined by the presence of nuclear green fluorescent protein in cells transfected with construct 20) can be pooled to create a library of cells with activated nuclear factors. Similarly, cells expressing membrane or secreted proteins can be pooled. Cells can also be grouped by phenotype, for example, growth factor independent growth, growth factor independent proliferation, colony formation, cellular differentiation (e.g., differentiation into a neuronal cell, muscle cell, epithelial cell, etc.), anchorage independent growth, activation of cellular factors (e.g., kinases, transcription factors, nucleases, etc.), gain or loss of cell-cell adhesion, migration, or cellular activation (e.g., resting versus activated T cells).

The invention is also directed to methods of using libraries of cells to over-express an endogenous gene. The library is screened for the expression of the gene and cells are selected that express the desired gene product. The cell can then be used to purify the gene product for subsequent use. Expression of the cell can occur by culturing the cell *in vitro* or by allowing the cell to express the gene *in vivo.*

The invention is also directed to methods of using libraries to identify novel gene and gene products.

The invention is also directed to methods for increasing the efficiency of gene activation by treating the cells with agents that stimulate or effect the patterns of non-homologous integration. It has been demonstrated that gene expression patterns, chromatin structure, and methylation patterns can differ dramatically from cell type to cell type. Even different cell lines from the same cell type can have significant differences. These differences can impact the patterns of non-homologous integration by affecting both the DNA breakage pattern and the repair process. For example, chromatinized stretches of DNA (characteristics likely associated with inactive genes) may be more resistant to breakage by restriction enzymes and chemical agents, whereas they may be susceptible to breakage by radiation.

Furthermore, inactive genes can be methylated. In this case, restriction enzymes that are blocked by CpG methylation will be unable to cleave methylated sites near the inactive gene, making it more difficult to activate that gene using methylation-sensitive enzymes. These problems can be circumvented by creating activation libraries in several cell lines using a variety of DNA breakage agents. By doing this, a more complete integration pattern can be created and the probability of activating a given gene maximized.

The methods of the invention can include introducing double strand breaks into the DNA of the cell containing the endogenous gene to be over-expressed. These methods introduce double-strand breaks into the genomic DNA in the cell prior to or simultaneously with vector integration. The mechanism of DNA breakage can have a significant effect on the pattern of DNA breaks in the genome. As a result, DNA breaks produced spontaneously or artificially with radiation, restriction enzymes, bleomycin, or other breaking agents, can occur in different locations.

In order to increase integration efficiency and to improve the random distribution of integration sites, cells can be treated with low, intermediate, or high doses of radiation prior to or following transfection. By artificially inducing double strand breaks, the transfected DNA can now integrate into the host cell chromosome as part of the DNA repair process. Normally, creation of double strand breaks to serve as the site of integration is the rate limiting step. Thus, by increasing chromosome breaks using radiation (or other DNA damaging agents), a larger number of integrants can be obtained in a given transfection. Furthermore, the mechanism of DNA breakage by radiation is different than by spontaneous breakage.

Radiation can induce DNA breaks directly when a high energy photon hits the DNA molecule. Alternatively, radiation can activate compounds in the cell which in turn, react with and break the DNA strand. Spontaneous breaks, on the other hand, are thought to occur by the interaction between reactive compounds produced in the cell (such as superoxides and peroxides) and the DNA molecule. However, DNA in the cell is not present as a naked, deproteinized polymer, but instead is bound to chromatin and present in a condensed state. As a result, some regions are not accessible to agents in the cell that cause double strand breaks. The photons produced by radiation have wave lengths short enough to hit highly condensed regions of DNA, thereby inducing breaks in DNA regions that are under represented in spontaneous breaks. Thus, radiation is capable of creating different DNA breakage patterns, which in turn, should lead to different integration patterns.

As a result, libraries produced using the same activation construct in cells with and without radiation treatment will potentially contain different sets of activated genes. Finally, radiation treatment increases efficiency of nonhomologous integration by up to 5-10 fold, allowing complete libraries to be created using fewer cells. Thus, radiation treatment increases the efficiency of gene activation and generates new integration and activation patterns in transfected cells. Useful types of radiation include α, β, γ, x-ray, and ultraviolet radiation. Useful doses of radiation vary for different cell types, but in general, dose ranges resulting in cell viabilities of 0.1% to >99% are useful. For HT1080 cells, this corresponds to radiation doses from a ¹³⁷Cs source of approximately 0.1 rads to 1000 rads. Other doses may also be useful as long as the dose either increases the integration frequency or changes the pattern of integration sites.

In addition to radiation, restriction enzymes can be used to artificially induce chromosome breaks in transfected cells. As with radiation, DNA restriction enzymes can create chromosome breaks which, in turn, serve as integration sites for the transfected DNA. This larger number of DNA breaks increases the overall efficiency of integration of the activation construct. Furthermore, the mechanism of breakage by restriction enzymes differs from that by radiation, the pattern of chromosome breaks is also likely to be different.

Restriction enzymes are relatively large molecules compared to photons and small metabolites capable of damaging DNA. As a result, restriction enzymes will tend to break regions that are less condensed then the genome as a whole. If the gene of interest lies within an accessible region of the genome, then treatment of the cells with a restriction enzyme can increase the probability of integrating the activation construct upstream of the gene of interest. Since restriction enzymes recognize specific sequences, and since a given restriction site may not lie upstream of the gene of interest, a variety of restriction enzymes can be used. It may also be important to use a variety of restriction enzymes since each enzyme has different properties (e.g., size, stability, ability to cleave methylated sites, and optimal reaction conditions) that affect which sites in the host chromosome will be cleaved. Each enzyme, due to the different distribution of cleavable restriction sites, will create a different integration pattern.

Therefore, introduction of restriction enzymes (or plasmids capable of expressing restriction enzymes) before, during, or after introduction of the activation construct will result in the activation of different sets of genes. Finally, restriction enzyme-induced breaks increase the integration efficiency by up to 5-10 fold (Yorifuji et al., Mut. Res. 243:121 (1990)), allowing fewer cells to be transfected to produce a complete library. Thus, restriction enzymes can be used to create new integration patterns, allowing activation of genes which failed to be activated in libraries produced by non-homologous recombination at spontaneous breaks or at other artificially induced breaks.

Restriction enzymes can also be used to bias integration of the activation construct to a desired site in the genome. For example, several rare restriction enzymes have been described which cleave eukaryotic DNA every 50-1000 kilobases, on average. If a rare restriction recognition sequence happens to be located upstream of a gene of interest, by introducing the restriction enzyme at the time of transfection along with the activation construct, DNA breaks can be preferentially upstream of the gene of interest. These breaks can then serve as sites for integration of the activation construct. Any enzyme can be that cleaves in an appropriate location in or near the gene of interest and its site is under-represented in the rest of the genome or its site is over-represented near genes (e.g., restriction sites containing CpG). For genes that have not been previously identified, restriction enzymes with 8 bp recognition sites (e.g., *Not*I, *Sfi*I*, Pme*I, *Swa*I, *Sse*I, *Srf*I, *Sgr*Al, *Pac*I, *Asc*I*, Sgf*I, and *Sse83871*), enzymes recognizing CpG containing sites (e.g., *Eag*I, *Bsi-WI, Mlu*I, and *Bss*HII) and other rare cutting enzymes can be used.

In this way, "biased" libraries can be created which are enriched for certain types of activated genes. In this respect, restriction enzyme sites containing CpG dinucleotides are particularly useful since these sites are under-represented in the genome at large, but over-represented in the form of CpG islands at the 5' end of many genes, the very location that is useful for gene activation. Enzymes recognizing these sites, therefore, will preferentially cleave at the 5' end of genic sequences.

Restriction enzymes can be introduced into the host cell by several methods. First, restriction enzymes can be introduced into the cell by electroporation (Yorifuji et al., Mut. Res. 243:121 (1990); Winegar et al., Mut. Res. 225:49 (1989)). In general, the amount of restriction enzyme introduced into the cell is proportional to its concentration in the electroporation media. The pulse conditions must be optimized for each cell line by adjusting the voltage, capacitance, and resistance. Second, the restriction enzyme can be expressed transiently from a plasmid encoding the enzyme under the control of eukaryotic regulatory elements. The level of enzyme produced can be controlled by using inducible promoters, and varying the strength of induction. In some cases, it may be desirable to limit the amount of restriction enzyme produced (due to its toxicity). In these cases, weak or mutant promoters, splice sites, translation start codons, and poly(A) tails can be utilized to lower the amount of restriction enzyme produced. Third, restriction enzymes can be introduced by agents that fuse with or permeabilize the cell membrane. Liposomes and streptolysin O (Pimplikar et al., J. Cell Biol. 125:1025 (1994)) are examples of this type of agent. Finally, mechanical perforation (Beckers et al., Cell 50:523-534 (1987)) and microinjection can also be used to introduce nucleases and other proteins into cells. However, any method capable of delivering active enzymes to a living cell is suitable.

DNA breaks induced by bleomycin and other DNA damaging agents can also produce DNA breakage patterns that are different. Thus, any agent or incubation condition capable of generating double strand breaks in cells is useful for increasing the efficiency and/or altering the sites of non-homologous recombination. Examples of classes of chemical DNA breaking agents include, but are not limited to, peroxides and other free radical generating compounds, alkylating agents, topoisomerase inhibitors, anti-neoplastic drugs, acids, substituted nucleotides, and enediyne antibiotics.

Specific chemical DNA breaking agents include, but are not limited to, bleomycin, hydrogen peroxide, cumene hydroperoxide, tert-butyl hydroperoxide, hypochlorous acid (reacted with aniline, 1-naphthylamine or 1-naphthol), nitric acid, phosphoric acid, doxorubicin, 9-deoxydoxorubicin, demethyl-6-deoxyrubicin, 5-iminodaunorubicin, adriamycin, 4'-(9- acridinylamino)methanesulfon-m-anisidide, neocarzinostatin, 8-methoxycaffeine, etoposide, ellipticine, iododeoxyuridine, and bromodeoxyuridine.

It has been shown that DNA repair machinery in the cell can be induced by pre-exposing the cell to low doses of a DNA breaking agent such as radiation or bleomycin. By pretreating cells with these agents approximately 24 hours prior to transfection, the cell will be more efficient at repairing DNA breaks and integrating DNA following transfection. In addition, higher doses of radiation or other DNA breaking agents can be used since the LD50 (the dose that results in lethality in 50% of the exposed cells) is higher following pretreatment. This allows random activation libraries to be created at multiple doses and results in a different distribution of integration sites within the host cell's chromosomes.

### Screening

Once an activation library (or libraries) is created, it can be screened using a number of assays. Depending on the characteristics of the protein(s) of interest (e.g., secreted versus intracellular proteins) and the nature of the activation construct used to create the library, any or all of the assays described below can be utilized. Other assay formats can also be used.

***ELISA**.* Activated proteins can be detected using the enzyme-linked immunosorbent assay (ELISA). If the activated gene product is secreted, culture supernatants from pools of activation library cells are incubated in wells containing bound antibody specific for the protein of interest. If a cell or group of cells has activated the gene of interest, then the protein will be secreted into the culture media. By screening pools of library clones (the pools can be from 1 to greater than 100,000 library members), pools containing a cell(s) that has activated the gene of interest can be identified. The cell of interest can then be purified away from the other library members by sib selection, limiting dilution, or other techniques known in the art. In addition to secreted proteins, ELISA can be used to screen for cells expressing intracellular and membrane-bound proteins. In these cases, instead of screening culture supernatants, a small number of cells is removed from the library pool (each cell is represented at least 100-1000 times in each pool), lysed, clarified, and added to the antibody-coated wells.

***ELISA Spot Assay**.* ELISA spot are coated with antibodies specific for the protein of interest. Following coating, the wells are blocked with 1% BSA/PBS for 1 hour at 37°C. Following blocking, 100,000 to 500,000 cells from the random activation library are applied to each well (representing ~10% of the total pool). In general, one pool is applied to each well. If the frequency of a cell expressing the protein of interest is 1 in 10,000 *(i.e.,* the pool consists of 10,000 individual clones, one of which expresses the protein of interest), then plating 500,000 cells per well will yield 50 specific cells. Cells are incubated in the wells at 37°C for 24 to 48 hours without being moved or disturbed. At the end of the incubation, the cells are removed and the plate is washed 3 times with PBS/0.05% Tween 20 and 3 times with PBS/1%BSA. Secondary antibodies are applied to the wells at the appropriate concentration and incubated for 2 hours at room temperature or 16 hours at 4°C. These antibodies can be biotinylated or labeled directly with horseradish peroxidase (HRP). The secondary antibodies are removed and the plate is washed with PBS/1% BSA. The tertiary antibody or streptavidin labeled with HRP is added and incubated for 1 hour at room temperature.

***FACS assay**.* The fluorescence-activated cell sorter (FACS) can be used to screen the random activation library in a number of ways. If the gene of interest encodes a cell surface protein, then fluorescently-labeled antibodies are incubated with cells from the activation library. If the gene of interest encodes a secreted protein, then cells can be biotinylated and incubated with streptavidin conjugated to an antibody specific to the protein of interest (Manz et al., Proc. Natl. Acad Sci. (USA) 92:1921 (1995)). Following incubation, the cells are placed in a high concentration of gelatin (or other polymer such as agarose or methylcellulose) to limit diffusion of the secreted protein. As protein is secreted by the cell, it is captured by the antibody bound to the cell surface. The presence of the protein of interest is then detected by a second antibody which is fluorescently labeled. For both secreted and membrane bound proteins, the cells can then be sorted according to their fluorescence signal. Fluorescent cells can then be isolated, expanded, and further enriched by FACS, limiting dilution, or other cell purification techniques known in the art.

***Magnetic Bead Separation.*** The principle of this technique is similar to FACS. Membrane bound proteins and captured secreted proteins (as described above) are detected by incubating the activation library with an antibody-conjugated magnetic beads that are specific for the protein of interest. If the protein is present on the surface of a cell, the magnetic beads will bind to that cell. Using a magnet, the cells expressing the protein of interest can be purified away from the other cells in the library. The cells are then released from the beads, expanded, analyzed, and further purified if necessary.

***RT-PCR.*** A small number of cells (equivalent to at least the number of individual clones in the pool) is harvested and lysed to allow purification of the RNA. Following isolation, the RNA is reversed-transcribed using reverse transcriptase. PCR is then carried out using primers specific for the cDNA of the gene of interest.

Alternatively, primers can be used that span the synthetic exon in the activation construct and the exon of the endogenous gene. This primer will not hybridize to and amplify the endogenously expressed gene of interest. Conversely, if the activation construct has integrated upstream of the gene of interest and activated gene expression, then this primer, in conjunction with a second primer specific for the gene will amplify the activated gene by virtue of the presence of the synthetic exon spliced onto the exon from the endogenous gene. Thus, this method can be used to detect activated genes in cells that normally express the gene of interest at lower than desired levels.

***Phenotypic Section.*** In this embodiment, cells can be selected based on a phenotype conferred by the activated gene. Examples of phenotypes that can be selected for include proliferation, growth factor independent growth, colony formation, cellular differentiation (e.g., differentiation into a neuronal cell, muscle cell, epithelial cell, etc.), anchorage independent growth, activation of cellular factors (e.g., kinases, transcription factors, nucleases, etc.), gain or loss of cell-cell adhesion, migration, and cellular activation (e.g., resting versus activated T cells). Isolation of activated cells demonstrating a phenotype, such as those described above, is important because the activation of an endogenous gene by the integrated construct is presumably responsible for the observed cellular phenotype. Thus, the activated gene may be an important therapeutic drug or drug target for treating or inducing the observed phenotype.

The sensitivity of each of the above assays can be effectively increased by transiently upregulating gene expression in the library cells. This can be accomplished for NF-κB site-containing promoters (on the activation construct) by adding PMA and tumor necrosis factor-α, e.g., to the library. Separately, or in conjunction with PMA and TNF-α, sodium butyrate can be added to further enhance gene expression. Addition of these reagents can increase expression of the protein of interest, thereby allowing a lower sensitivity assay to be used to identify the gene activated cell of interest.

Since large activation libraries are created to maximize activation of many genes, it is advantageous to organize the library clones in pools. Each pool can consist of 1 to greater than 100,000 individual clones. Thus, in a given pool, many activated proteins are produced, often in dilute concentrations (due to the overall size of the pool and the limited number of cells within the pool that produce a given activated protein). Thus, concentration of the proteins prior to screening effectively increases the ability to detect the activated proteins in the screening assay. One particularly useful method of concentration is ultrafiltration; however, other methods can also be used. For example, proteins can be concentrated non-specifically, or semi-specifically by adsorption onto ion exchange, hydrophobic, dye, hydroxyapatite, lectin, and other suitable resins under conditions that bind most or all proteins present. The bound proteins can then be removed in a small volume prior to screening. It is advantageous to grow the cells in serum free media to facilitate the concentration of proteins.

In another embodiment, a useful sequence that can be included on the activation construct is an epitope tag. The epitope tag can consist of an amino acid sequence that allows affinity purification of the activated protein (e.g., on immunoaffinity or chelating matrices). Thus, by including an epitope tag on the activation construct, all of the activated proteins from an activation library can be purified. By purifying the activated proteins away from other cellular and media proteins, screening for novel proteins and enzyme activities can be facilitated. In some instances, it may be desirable to remove the epitope tag following purification of the activated protein. This can be accomplished by including a protease recognition sequence (e.g., Factor IIa or enterokinase cleavage site) downstream from the epitope tag on the activation construct. Incubation of the purified, activated protein(s) with the appropriate protease will release the epitope tag from the proteins(s).

In libraries in which an epitope tag sequence is located on the activation construct, all of the activated proteins can be purified away from all other cellular and media proteins using affinity purification. This not only concentrates the activated proteins, but also purifies them away from other activities that can interfere with the assay used to screen the library.

Once a pool of clones containing cells over-expressing the gene of interest is identified, steps can be taken to isolate the activated cell. Isolation of the activated cell can be accomplished by a variety of methods known in the art. Examples of cell purification methods include limiting dilution, fluorescence activated cell sorting, magnetic bead separation, sib selection, and single colony purification using cloning rings.

In preferred embodiments of the invention, the methods include a process wherein the expression product is purified. In highly preferred embodiments, the cells expressing the endogenous gene product are cultured so as to produce amounts of gene product feasible for commercial application, and especially diagnostic and therapeutic and drug discovery uses.

Any vector used in the methods described herein can include an amplifiable marker. Thereby, amplification of both the vector and the DNA of interest (i.e., containing the over-expressed gene) occurs in the cell, and further enhanced expression of the endogenous gene is obtained. Accordingly, methods can include a step in which the endogenous gene is amplified.

Once the activated cell has been isolated, expression can be further increased by amplifying the locus containing both the gene of interest and the activation construct. This can be accomplished by each of the methods described below, either separately or in combination.

Amplifiable markers are genes that can be selected for higher copy number. Examples of amplifiable markers include dihydrofolate reductase, adenosine deaminase, aspartate transcarbamylase, dihydro-orotase, and carbamyl phosphate synthase. For these examples, the elevated copy number of the amplifiable marker and flanking sequences (including the gene of interest) can be selected for using a drug or toxic metabolite which is acted upon by the amplifiable marker. In general, as the drug or toxic metabolite concentration increases, cells containing fewer copies of the amplifiable marker die, whereas cells containing increased copies of the marker survive and form colonies. These colonies can be isolated, expanded, and analyzed for increased levels of production of the gene of interest.

Placement of an amplifiable marker on the activation construct results in the juxtaposition of the gene of interest and the amplifiable marker in the activated cell. Selection for activated cells containing increased copy number of the amplifiable marker and gene of interest can be achieved by growing the cells in the presence of increasing amounts of selective agent (usually a drug or metabolite). For example, amplification of dihydrofolate reductase (DHFR) can be selected using methotrexate.

As drug-resistant colonies arise at each increasing drug concentration, individual colonies can be selected and characterized for copy number of the amplifiable marker and gene of interest, and analyzed for expression of the gene of interest. Individual colonies with the highest levels of activated gene expression can be selected for further amplification in higher drug concentrations. At the highest drug concentrations, the clones will express greatly increased amounts of the protein of interest.

When amplifying DHFR, it is convenient to plate approximately 1 x 10⁷ cells at several different concentrations of methotrexate. Useful initial concentrations of methotrexate range from approximately 5 nM to 100 nM. However, the optimal concentration of methotrexate must be determined empirically for each cell line and integration site. Following growth in methotrexate containing media, colonies from the highest concentration of methotrexate are picked and analyzed for increased expression of the gene of interest. The clone(s) with the highest concentration of methotrexate are then grown in higher concentrations of methotrexate to select for further amplification of DHFR and the gene of interest. Methotrexate concentrations in the micromolar and millimolar range can be used for clones containing the highest degree of gene amplification.

Placement of a viral origin of replication(s) (e.g., ori P or SV40 in human cells, and polyoma ori in mouse cells) on the activation construct will result in the juxtaposition of the gene of interest and the viral origin of replication in the activated cell. The origin and flanking sequences can then be amplified by introducing the viral replication protein(s) in trans. For example, when ori P (the origin of replication on Epstein-Barr virus) is utilized, EBNA-I can be expressed transiently or stably. EBNA-1 will initiate replication from the integrated ori P locus. The replication will extend from the origin bi-directionally. As each replication product is created, it too can initiate replication. As a result, many copies of the viral origin and flanking genomic sequences including the gene of interest are created. This higher copy number allows the cells to produce larger amounts of the gene of interest.

At some frequency, the replication product will recombine to form a circular molecule containing flanking genomic sequences, including the gene of interest. Cells that contain circular molecules with the gene of interest can be isolated by single cell cloning and analysis by Hirt extraction and Southern blotting. Once purified, the cell containing the episomal genomic locus at elevated copy number (typically 10-50 copies) can be propagated in culture. To achieve higher amplification, the episome can be further boosted by including a second origin adjacent to the first in the original construct. For example, T antigen can be used to boost the copy number of ori P/SV40 episomes to a copy number of ~1000 (Heinzel et al., J. Virol. 62:3738 (1988)). This substantial increase in copy number can dramatically increase protein expression.

The invention encompasses over-expression of endogenous genes both *in vivo* and *in vitro.* Therefore, the cells could be used *in vitro* to produce desired amounts of a gene product or could be used *in vivo* to provide that gene product in the intact animal.

The invention also provides the proteins produced by the methods described herein. The proteins can be produced from either known, or previously unknown genes. Examples of known proteins that can be produced by this method include, but are not limited to, erythropoietin, insulin, growth hormone, glucocerebrosidase, tissue plasminogen activator, granulocyte-colony stimulating factor, granulocyte/macrophage colony stimulating factor, interferon α, interferon β, interferon γ, interleukin-2, interleukin-6, interleukin-11, interleukin-12, TGF β, blood clotting factor V, blood clotting factor VII, blood clotting factor VIII, blood clotting factor IX, blood clotting factor X, TSH-β, bone growth factor 2, bone growth factor-7, tumor necrosis factor, alpha-1 antitrypsin, anti-thrombin III, leukemia inhibitory factor, glucagon, Protein C, protein kinase C, macrophage colony stimulating factor, stem cell factor, follicle stimulating hormone β, urokinase, nerve growth factors, insulin-like growth factors, insulinotropin, parathyroid hormone, lactoferrin, complement inhibitors, platelet derived growth factor, keratinocyte growth factor, neurotropin-3, thrombopoietin, chorionic gonadotropin, thrombomodulin, alpha glucosidase, epidermal growth factor, FGF, macrophage-colony stimulating factor, and cell surface receptors for each of the above-described proteins.

Where the protein product from the activated cell is purified, any method of protein purification known in the art may be employed.

### Isolation of Cells Containing Activated Membrane Protein-Encoding Genes

Genes that encode membrane associated proteins are particularly interesting from a drug development standpoint. These genes and the proteins they encode can be used, for example, to develop small molecule drugs using combinatorial chemistry libraries and high through-put screening assays. Alternatively, the proteins or soluble forms of the proteins (e.g., truncated proteins lacking the transmembrane region) can be used as therapeutically active agents in humans or animals. Identification of membrane proteins can also be used to identify new ligands (e.g., cytokines, growth factors, and other effector molecules) using two hybrid approaches or affinity capture techniques. Many other uses of membrane proteins are also possible.

Current approaches to identifying genes that encode integral membrane proteins involve isolation and sequencing of genes from cDNA libraries. Integral membrane proteins are then identified by ORF analysis using hydrophobicity plots capable of identifying the transmembrane region of the protein. Unfortunately, using this approach a gene encoding an integral membrane protein can not be identified unless the gene is expressed in the cells used to produce the cDNA library. Furthermore, many genes are only expressed in very rare cells, during short developmental windows, and/or at very low levels. As a result, these genes can not be efficiently identified using the currently available approaches.

The present invention allows endogenous genes to be activated without any knowledge of the sequence, structure, function, or expression profile of the genes. Using the disclosed methods, genes may be activated at the transcription level only, or at both the transcription and translation levels. As a result, proteins encoded by the activated endogenous gene can be produced in cells containing the integrated vector. Furthermore, using specific vectors disclosed herein, the protein produced from the activated endogenous gene can be modified, for example, to include an epitope tag. Other vectors (e.g., vectors 12-17 described above) may encode a signal peptide followed by an epitope tag. This vector can be used to isolate cells that have activated expression of an integral membrane protein (see Example 5 below). This vector can also be used to direct secretion of proteins that are not normally secreted.

Thus, the invention also is directed to methods for identifying an endogenous gene encoding a cellular integral membrane protein or a transmembrane protein. Such methods of the invention may comprise one or more steps. For example, one such method of the invention may comprise (a) introducing one or more vectors of the invention into a cell; (b) allowing the vector to integrate into the genome of the cell by non-homologous recombination; (c) allowing over-expression of an endogenous gene in the cell by upregulation of the gene by a transcriptional regulatory sequence contained on the integrated vector construct; (d) screening the cell for over-expression of the endogenous gene; and (e) characterizing the activated gene to determine its identity as a gene encoding a cellular integral membrane protein. In related embodiments, the invention provides such methods further comprising isolating the activated gene from the cell prior to characterizing the activated gene.

To identify genes that encode integral membrane proteins, vectors integrated into the genome of cells will comprise a regulatory sequence linked to an exonic sequence containing a start codon, a signal sequence, and an epitope tag, followed by an unpaired splice donor site. Upon integration and activation of an endogenous gene, a chimeric protein is produced containing the signal peptide and epitope tag from the vector fused to the protein encoded by the downstream exons of the endogenous gene. This chimeric protein, by virtue of the presence of the vector encoded signal peptide, is directed to the secretory pathway where translation of the protein is completed and the protein is secreted. If, however, the activated endogenous gene encodes an integral membrane protein, and the transmembrane region of that gene is encoded by exons located 3' of the vector integration site, then the chimeric protein will go to the cell surface, and the epitope tag will be displayed on the cell surface. Using known methods of cell isolation (for example flow cytometric sorting, magnetic bead cell sorting, immunoadsorption, or other methods that will be familiar to one of ordinary skill in the art), antibodies to the epitope tag can then be used to isolate the cells from the population that display the epitope tag and have activated an integral membrane encoding gene. These cells can then be used to study the function of the membrane protein. Alternatively, the activated gene may then be isolated from these cells using any art-known method, e.g., through hybridization with a DNA probe specific to the vector-encoded exon to screen a cDNA library produced from these cells, or using the genetic constructs described herein.

The epitope tag encoded by the vector exon may be a short peptide capable of binding to an antibody, a short peptide capable of binding to a substance (e.g., poly histidine/ divalent metal ion supports, maltose binding protein/maltose supports, glutathione S-transferase/glutathione support), or an extracellular domain (lacking a transmembrane domain) from an integral membrane protein for which an antibody or ligand exists. It will be understood, however, that other types of epitope tags that are familiar to one of ordinary skill in the art may be used equivalently in accordance with the invention.

### Vectors for Non-targeted Activation of Endogenous Genes

As noted above, non-targeted gene activation has a number of important applications, including activating endogenous genes in host cells which provides a powerful approach to discovering and isolating new genes and proteins, and to producing large amounts of specific proteins for commercialization. For some applications of non-targeted gene activation, it is desirable to create libraries of cells in which each member of the library contains an activation vector integrated into a unique location in the host cell genome, and in which each member of the library has activated a different endogenous gene. Furthermore, it would be desirable to remove cells from the library that contain an integrated vector, but fail to activate an endogenous gene. Since eukaryotic genomes often contain large regions that lack genes, integration of an activation vector into a region devoid of genes can occur frequently. These integrated vectors, however, fail to activate an endogenous gene, and yet are capable of conferring drug resistance on the host cells when a selectable marker (driven by a suitable promoter and followed by a polyadenylation signal) is included on the activation vector. Even more problematic for gene discovery applications, a transcript containing vector sequences is produced in these cells regardless of whether or not a gene has been activated. In cases where a gene has not been activated, these vector sequence-containing transcripts contain non-genic genomic DNA sequences. As a result, when isolating activated genes, one cannot isolate all RNA (or cDNA) molecules that are derived from the integrated vector (i.e. transcripts containing vector sequences), since many of these transcripts do not encode an endogenous gene. To overcome these difficulties, the present invention provides highly specific vectors and methods that facilitate isolation of vector-activated genes.

These vectors of the invention are useful for activating expression of endogenous genes and for isolating the mRNA and cDNA corresponding to the activated genes. One such vector reduces the number of cells in which the vector integrated into the genome but failed to activate expression from (or transcription through) an endogenous gene. By removing these cells, fewer library members can be created and screened to isolate a given number of activated genes. Furthermore, vector-containing cells that fail to activate gene expression produce an RNA molecule that can interfere with isolation of bona fide activated genes. Thus, the vectors disclosed herein are particularly useful for producing cells suitable for protein over-expression and/or for isolating cDNA molecules corresponding to activated genes. The second type of vector of the invention is useful for isolating exon I from activated endogenous genes. As a result, these vectors can be used to obtain full-length genes from activated RNA transcripts. Each of the functional vector components described herein may be used separately, or in combination with each other.

### Poly(A) Trap Activation Vectors

To facilitate isolation of activated genes, the present invention employs novel gene activation vectors that are capable of producing a drug resistant colony, preferentially upon activation of an endogenous gene. Such vectors are referred to herein as "poly(A) trap vectors." Examples ofpoly(A) trap vectors are shown in Figures 8A-8F. The nucleotide sequence of one such dual poly(A) trap vector, designated pRIG21b, is shown in Figures 15A-15B (SEQ ID NO:19). These vectors contain a transcriptional regulatory sequence (which may be any transcriptional regulatory sequence comprising a promoter, including but not limited to regulatory sequences comprising the promoters, enhancers, and repressors described herein, and which is most preferably a promoter such as a CMV immediate early gene promoter, an SV40 T antigen promoter, a tetracycline-inducible promoter, or a β-actin promoter) operably linked to a selectable marker gene lacking a poly(A) signal. Since the selectable marker gene lacks a polyadenylation signal, its message will not be stable, and the marker gene product will not be efficiently produced. However, if the activation vector integrates upstream of an endogenous gene, the selectable marker can utilize the polyadenylation signal of the endogenous gene, thereby allowing production of the selectable marker protein in sufficient amounts to confer drug resistance. Thus, cells that integrate this activation vector generally form a drug resistant colony only if an endogenous gene has been activated.

The vectors employed in the invention also comprise a splice acceptor trap (SAT).

The poly(A) trap activation vectors can include any selectable or screenable marker. Furthermore, the selectable marker can be expressed from any promoter that is functional in the cells used to create the integration library. Thus, the selectable marker can be expressed by viral or non-viral promoters. Optionally, an unpaired splice donor site may be included in the construct, preferably 3' of the selectable marker to allow the exon encoding the selectable marker to be spliced directly to the exons of the endogenous gene. When a downstream transcriptional regulatory sequence and a splice donor site is included on the vector, the inclusion of a splice donor site adjacent to the selectable marker results in the removal of these downstream elements from the messenger RNA.

In a related embodiment, a second transcriptional regulatory sequence (which may be any transcriptional regulatory sequence comprising a promoter, including but not limited to the promoters described herein, and which preferably is a promoter) may be located downstream of, and in the same orientation as, the selectable marker. An unpaired splice donor site is linked to the downstream transcriptional regulatory sequence. In this configuration, the poly(A) trap vector is capable of producing a message containing the downstream vector-encoded exon spliced to endogenous exons. As described below, these chimeric transcripts can be translated into native or modified protein, depending on the nature of the vector-encoded exon.

As used herein, a "vector-encoded exon" means a region of a vector downstream of the transcriptional regulatory sequence and between the transcription start site and the unpaired splice donor site found on the vector. The vector-encoded exon is present at the 5' end of the transcript containing the endogenous gene in the fully processed message. Analogously, as used herein, a "vector-encoded intron" is the region of the vector located downstream of the unpaired splice donor site. When a linearization site is present on the vector, the vector-encoded intron is the region of the vector that is downstream of the vector-encoded exon between the unpaired splice donor site and the linearization site. The vector-encoded intron is removed from the activated gene transcript during RNA processing.

### Splice Acceptor Trap (SAT)

As an approach for removing cells that fail to activate an endogenous gene, the invention employs vectors designated herein as "Splice Acceptor Trap" (SAT) vectors. These vectors are designed to splice from a vector encoded splice donor site to an endogenous splice acceptor. Furthermore, the vectors may be designed to produce a product that is toxic to the host cells (or a product that can be selected against) if splicing does not occur. Thus, these vectors facilitate elimination of cells in which the vector-encoded exon failed to splice to an endogenous exon. The vectors employed in the invention also comprise a poly (A) trap.

Splice acceptor trap vectors can contain both a positive selectable marker and a negative selectable marker gene oriented in the same direction on the vector. As used herein, a positive selectable marker is a gene that, upon expression, produces a protein capable of facilitating the isolation of cells expressing the marker. Analogously, as used herein, a negative selectable marker is a gene that, upon expression, produces a protein capable of facilitating removal of cells expressing the marker.

The positive selectable marker and the negative selectable marker are preferably separated in the vector construct by an unpaired splice donor site. In other embodiments, however, the positive selectable marker may be fused to the negative selectable marker gene. In this configuration, an unpaired splice donor site is located between the positive and negative selectable marker, such that the reading frame of the negative selectable marker is preserved. The unpaired splice donor site is preferably located at the junction of the positive and negative selectable markers. However, the unpaired splice donor site may be located anywhere in the fusion gene such that upon splicing to an endogenous splice acceptor site, the positive selectable marker will be expressed in an active form and the negative selectable marker will be expressed in an inactive form, or not at all. In this configuration, the positive selectable marker is located upstream of the negative selectable marker.

It will also be apparent to one of ordinary skill in view of the description contained herein that the positive and negative selectable markers on the SAT vector need not be expressed as a fusion protein. In one embodiment, an internal ribosomal entry site (ires) is inserted between the positive selectable marker and the negative selectable marker. In this configuration, the unpaired splice donor site can be positioned between the two markers, or in the open reading frame of either marker gene such that, upon splicing, the positive selectable marker will be expressed in an active form and the negative selectable marker will be expressed in an inactive form, or not at all. In another embodiment, the positive selectable marker may be driven from a different transcriptional regulatory sequence than the negative selectable marker. In this configuration, the unpaired splice donor site is located in the 5' untranslated region of the negative selectable marker or anywhere in the open reading frame of the negative selectable marker such that, upon splicing, the negative selectable marker will be produced in an inactive form or not at all. Furthermore, when the positive and negative markers are driven from different transcriptional regulatory sequences, the positive selectable marker may be located upstream or downstream of the negative selectable marker, and the positive selectable marker may contain or lack a splice donor site at its 3' end.

The vectors described herein may contain any positive selectable marker. Examples of positive selectable markers useful in this invention include genes encoding neomycin (neo), hypoxanthine phosphoriosyl transferase (HPRT), puromycin (pac), dihydro-oratase, glutamine synthetase (GS), histidine D (his D), carbamyl phosphate synthase (CAD), dihydrofolate reductase (DHFR), multidrug resistance 1 (mdr1), aspartate transcarbamylase, xanthine-guanine phosphoribosyl transferase (gpt), and adenosine deaminase (ada). Alternatively, the vectors may contain a screenable marker in place of the positive selectable marker. Screenable markers include any protein capable of producing a recognizable phenotype in the host cell. Examples of screenable markers included cell surface epitopes (such as CD2) and enzymes (such as β-galactosidase).

The vectors described herein may also, or alternatively, contain any negative selectable marker that can be selected against. Examples of negative selectable markers include hypoxanthine phosphoribosyl transferase (HPRT), thymidine kinase (TK), and diptheria toxin. The negative selectable marker can also be a screenable marker, such as a cell surface protein or an enzyme. Cells expressing the negative screenable marker may be removed by, for example, Fluorescence Activated Cell Sorting (FACS) or magnetic bead cell sorting.

To isolate cells that have activated expression of an endogenous gene, the cells containing the integrated vector can be placed under the appropriate drug selection. Selection for the positive selectable marker and against the negative selectable marker can occur simultaneously. In another embodiment, selection can occur sequentially. When selection occurs sequentially, selection for the positive selectable marker can occur first, followed by selection against the negative selectable marker. Alternatively, selection against the negative selectable marker can occur first, followed by selection for the positive selectable marker.

The positive and negative markers are expressed by a transcriptional regulatory element located upstream of the translation start site of each gene. When a positive/negative marker fusion gene or an ires sequence is used, a single transcriptional regulatory element drives expression of both markers. A poly(A) signal may be placed 3' of each selectable marker. If a positive/negative fusion gene is used a single poly(A) signal is positioned 3' of the markers. Alternatively, a poly(A) signal may be excluded from the vector to provide additional specificity for a gene activation event (see dual poly(A)/splice acceptor trap below).

### Dual Poly(A)/Splice Acceptor Trap Vectors

The vectors employed in the invention are dual poly (A)/splice acceptor trap vectors. To further reduce the number of cells that lack a gene activation event, vectors that confer host cell survival only if the vector-encoded exon has spliced to an exon from an endogenous gene and has acquired a poly(A) signal may be employed. These vectors are designated herein as "dual poly(A)/splice acceptor trap vectors" or as "dual poly(A)/SAT vectors." By requiring both splicing and polyadenylation to occur for cell survival, cells that fail to activate an endogenous gene are more efficiently eliminated from the activation library.

The dual poly(A)/splice acceptor trap vectors may contain a positive selectable marker and a negative selectable marker configured as described for the SAT vectors; however, neither gene contains a functional poly(A) signal. Thus, the positive selectable marker will not be expressed at high levels unless splicing occurs to capture an endogenous poly(A) signal. Aside from the lack of a poly(A) signal, all other features and embodiments of this type of vector are the same as those of the SAT vectors as described herein. Examples of dual poly(A)/SAT vectors are shown in Figures 9C and 9F and 10C and 10F. The nucleotide sequence of one such dual poly(A)/SAT vector, designated pRIG22b, is shown in Figures 16A-16B (SEQ ID NO:20).

### Vectors for Activating Protein Expression from Endogenous Genes

In many applications of non-targeted gene activation, it is desirable to produce protein from the activated endogenous gene. To accomplish this, a second transcriptional regulatory sequence (which may be any transcriptional regulatory sequence comprising a promoter, including but not limited to the promoters described herein, and which is preferably a promoter can be placed downstream of the selectable marker(s) on any of the vectors described herein. The downstream transcriptional regulatory sequence is positioned to drive expression in the same direction as the upstream selectable marker(s).

To increase the frequency of protein expression using non-targeted gene activation, the downstream transcriptional regulatory sequence on the vector is operably linked to an exonic sequence followed by a splice donor site. In a preferred embodiment, the vector exon lacks a start codon. This vector is particularly useful for activating protein expression from genes that do not encode the translation start codon in exon I. In an alternative preferred embodiment, the vector exon contains a start codon. Additional codons can be located between the translational start codon and the splice donor site. For example, a partial signal secretion sequence can be encoded on the vector exon. The partial signal sequence can be any amino acid sequence capable of complementing a partial signal sequence from an endogenous gene to produce a functional signal sequence. The partial sequence may encode between one and one hundred amino acids, and may be derived from existing genes, or may consist of novel sequences. Thus, this vector is useful for producing and secreting protein from genes that encode part of the endogenous signal sequence in exon I, and the remainder in subsequent exons. In another example of a vector useful for activating a particular type of endogenous gene, a functional signal sequence can be encoded on the vector exon. This vector allows protein to be produced and secreted from genes that encode a signal sequence in exon I. It can also be used to produce secreted forms of proteins that are not normally secreted.

In cases where a start codon is included on the vector exon, it can be advantageous to produce a vector in each reading frame. This is achieved by varying the number of nucleotides between the start codon and the splice donor junction site. Together, the preferred vector configurations are capable of producing protein from endogenous genes, regardless of the exon/intron structure, location of the translation start codon, or reading frame.

### Vectors for Isolating Exon I from Activated Endogenous Genes

The non-targeted gene activation vectors described above are useful for activating and isolating endogenous genes and for producing protein from endogenous genes. Upon integration upstream of an endogenous gene, however each of these vectors produces a transcript that lacks exon I from the endogenous gene. Since the vectors are designed to produce a transcript containing the vector encoded exon spliced to the first splice acceptor site downstream of the vector integration site, and since the first exon of eukaryotic genes does not contain a splice acceptor site, normally, the first exon of endogenous genes will not be recovered on mRNA molecules derived from non-targeted gene activation. For some genes, such as genes that contain coding information in the first exon, there is a need to efficiently recover the first exon of the activated endogenous gene.

To recover the first exon of activated endogenous genes, a transcriptional regulatory sequence (which may be any transcriptional regulatory sequence, including but not limited to the promoters, enhancers, and repressors described herein, and which is preferably a promoter or an enhancer, and most preferably a promoter) is included on the activation vector downstream of a second transcriptional regulatory sequence (which may also be any transcriptional regulatory sequence, including but not limited to the promoters, enhancers, and repressors described herein, and which is preferably a promoter or an enhancer, and most preferably a promoter) which drives expression of a vector encoded exon. Thus, the upstream transcriptional regulatory sequence is linked to an unpaired splice donor site and the downstream transcriptional regulatory sequence is not linked to a splice donor site. Both transcriptional regulatory sequences are oriented to drive expression in the same direction. Examples of such exon I recovery vectors are shown in Figures 12A-12G. The integration of this type of vector will create at least two different types of RNA transcripts (Figure 13). The first transcript is derived from the upstream transcriptional regulatory sequence and contains the vector exon spliced to exon II of an endogenous gene. The second transcript is derived from the downstream transcriptional regulatory sequence and contains, from 5' to 3', the region between the vector and the transcription start site of the gene, exon I, exon II, and all downstream exons. Using methods described herein, both transcripts can be recovered and analyzed, allowing the characterization of exon I from genes isolated by non-targeted gene activation.

The exon located on the activation vector can encode a selectable marker, a protein, a portion of a protein, secretion signal sequences, a portion of a signal sequence, an epitope, or nothing. When a protein is encoded by the exon, a poly(A) signal may be included downstream of the vector encoded gene. Alternatively, a poly(A) signal may be omitted. In another embodiment, a positive and negative selectable marker may be operably linked to the upstream transcriptional regulatory sequence(s). In this embodiment, the position of the unpaired splice donor site relative to the selectable markers is described above for the SAT vectors and the dual poly(A)/SAT vectors.

### Gene Activation Vectors for Single-Exon and Multi-Exon Gene Trapping

As noted above, in one embodiment the poly(A) trap vectors of the invention may contain a promoter operably linked to a selectable marker followed by an unpaired splice donor site. Such vectors, when integrated into or near a gene, produce transcripts containing the selectable marker spliced onto an endogenous gene. Since the endogenous gene encodes a poly(A) signal, the resulting mRNA is polyadenylated, thereby allowing the transcript to be translated at levels sufficient to confer drug resistance on the cell containing the integrated vector.

While the vectors described above are capable of "trapping" endogenous genes, the splice donor site downstream of a selectable marker cannot be used in, and in some cases can interfere with, several potential applications for such vectors. First, these vectors cannot be used to selectively trap single exon genes, since these genes do not contain a splice acceptor site. Second, these vectors often "trap" cryptic genes, since drug resistance relies solely on vector integration upstream of a poly (A) signal. Unfortunately, cryptic poly (A) signals exist in the genome, leading to formation of drug resistant cells and creation of non-genic transcripts containing the selectable marker. These cells and transcripts can interfere with gene discovery applications using these vectors. Third, without novel modifications such as those described herein (see above), these vectors are not capable of efficiently producing protein from the activated endogenous gene. Furthermore, protein expression from an endogenous gene can be poor even when an internal ribosome entry site (ires) is included between the selectable marker and the splice donor site, since translation from an ires is generally less efficient than translation from the first start codon at the 5' end of a transcript. Thus, there is a need for vectors that are capable of more specifically trapping endogenous genes, including single exon genes, and that are capable of efficiently expressing protein from the activated endogenous genes.

Thus, in additional embodiments, the present invention provides such vectors. In one such embodiment, the vector may contain a promoter operably linked to one or more (i.e., one, two, three, four, five, or more) selectable markers, wherein the selectable marker is not followed by a splice donor site or a poly(A) signal (see Figures 17A-17G). In general, upon integration into a host cell genome, this vector will fail to produce sufficient quantities of selectable marker since the marker transcript will not be polyadenylated. However, if the vector integrates in close proximity to, or into, a gene, including a single exon gene, the selectable marker will acquire a poly(A) signal from the endogenous gene, thereby stabilizing the marker transcript and conferring a drug resistant phenotype on the cell. In addition to selecting for vector integration into or near genes, vectors according to this aspect of the invention can also be used to recover exon I from the activated gene, as described in the section of this application entitled "Vectors for Isolating Exon I from Activated Endogenous Genes."

Any of the vectors of the invention can contain an internal ribosome entry site (ires) 3' of the downstream selectable marker. The ires allows translation of the endogenous gene upon vector integration into an endogenous gene. Optionally, a translation start codon may be included between the selectable marker and the ires sequence. When a start codon is present, additional codons may be present on the exon. The start codon, and if present additional codons, may be present in any, and collectively all, reading frames relative to the splice donor site. Furthermore, the codons downstream of the translation start codon, if present, may encode, for example, a signal secretion signal, a partial signal sequence, a protein (including a

Any of the vectors described herein may contain, upstream of the selectable marker(s), a second transcriptional regulatory sequence (most preferably a promoter) operably linked to a exonic region, followed by an unpaired splice donor site. This upstream exon is particularly useful for expressing protein from activated endogenous genes. The exon may lack a translation start codon. Alternatively, the exon may contain a translation start codon. When a start codon is present, additional codons may be present on the exon. The start codon, and if present additional codons, may be present in any, and collectively all, reading frames relative to the splice donor site. Furthermore, the codons downstream of the translation start codon, if present, may encode, for example, a signal secretion sequence, a partial signal sequence, a protein (including a full-length protein, a portion of a protein, a protein motif, an epitope tag, etc.), or a spacer region.

### Activation Vectors Useful for Detecting Protein-protein Interactions

Genetic approaches for detecting protein-protein interactions have previously been described (*see,* e.g., U.S. Patent Nos. 5,283,173; 5,468,614; and 5,667,973). This approach relies on cloning a first cDNA molecule next to, and in frame with, a gene fragment encoding a DNA binding domain; and cloning a second cDNA molecule next to, and in frame with, a gene fragment encoding a transcription transactivation domain. Each chimeric gene is expressed from a promoter region located upstream of the chimeric gene. To detect expression, both chimeric genes are transfected into a reporter cell. If the first chimeric protein interacts with the second chimeric protein (via the proteins encoded by the cloned cDNA's fused to the DNA binding and transcription activation domains), then the DNA binding domain and the transcription activation domain will be joined within a single protein complex. As a result, the protein-protein interaction complex can bind to the regulatory region of the reporter gene and activate its expression.

A limitation of this previous approach is that it is only capable of detecting protein-protein interactions between genes that have been cloned as cDNA. As described herein, many genes are expressed at very low levels, in rare cell types, or during short developmental windows; and therefore, these genes are typically absent from cDNA libraries. Furthermore, many genes are too large to be isolated efficiently as full-length clones, thereby making it difficult to use these previous approaches.

The present invention is capable of activating protein expression from endogenous genes or from transfected genomic DNA. Unlike previous approaches, virtually any gene can be efficiently expressed, regardless of its normal expression pattern. Furthermore, since the present invention is also capable of modifying the protein expressed from the endogenous gene (or from the transfected genomic DNA), it is also possible to produce chimeric proteins for use in protein-protein interaction assays.

To detect protein-protein interactions by the present invention, two vectors are used. The first vector, generally referred to as BD/SD (binding domain/splice donor), contains a promoter operably linked to a polynucleotide encoding a DNA binding domain and an unpaired splice donor site. The second vector, generally referred to as AD/SD (activation domain/splice donor), contains a promoter operably linked to a polynucleotide encoding a transcription activation domain and an unpaired splice donor site. To accommodate genes that have different reading frames, the binding domain and activation domain can be encoded in each of the three possible reading frames relative to the unpaired splice donor site. In addition, BD/SD and AD/SD vectors can have other functional elements, as described herein for other vectors, including selectable markers and amplifiable markers. The vectors also contain selectable markers oriented in a configuration that permits selection for cells in which the vector has activated a gene. Multi-promoter/activation exon vectors are also useful. Several examples of BD/SD and AD/SD vectors are illustrated in Figure 25. An example illustrating detection of a protein-protein interaction using these vectors is depicted in Figure 26.

The DNA binding domain of the BD/SD vector may encode any protein domain capable of binding to a specific nucleotide sequence. When a transcription activation protein is used to supply the DNA binding domain, the transcription activation domain is omitted from the BD/SD vector. Examples of genes encoding proteins with DNA binding domains include, but are not limited to, the yeast GAL4 gene, the yeast GCN4 gene, and the yeast ADR1 gene. Other genes from prokaryotic and eukaryotic sources may also be used to supply DNA binding domains.

The transcription activation domain of the AD/SD vector encodes a protein domain capable of enhancing transcription of a reporter gene when positioned near the promoter region of the reporter gene. When a transcription activation protein is used to supply the transcription activation domain, the DNA binding domain is omitted from the AD/SD vector. Examples of genes encoding proteins with transcription activation domains include, but are not limited to, the yeast GAL4 gene, the yeast GCN4 gene, and the yeast ADR1 gene. Other genes from prokaryotic and eukaryotic sources may also be used to supply transcription activation domains.

In the present invention, protein-protein interactions are detected using the BD/SD and AD/SD vectors, described above, to activate expression of genes located in stretches of genomic DNA.

In one embodiment, the BD/SD vector is integrated randomly into the genome of a reporter cell line. As with other vectors described herein, the BD/SD vectors are capable of activating protein expression from genes located downstream of the vector integration site. Since the activation exon on the BD/SD vector encodes a DNA binding domain, the activated endogenous protein will be produced as a fusion protein containing the DNA binding domain at its N-terminus. Thus, by integrating the BD/SD vector into the genome of a host cell, a library of fusion proteins can be created, wherein each protein will contain a DNA binding domain at its N-tenninus.

It is also recognized that the AD/SD vector can be integrated into the genome of a reporter cell line to produce a library of cells, wherein each member of the library is expressed as a different endogenous gene fused to a transcription activation domain.

Once created, the BD/SD library may be transfected with a vector expressing a specific gene (referred to below as gene X) fused to a transcription activation domain. This allows virtually any gene encoded in the genome to be tested for an interaction to gene X. Likewise, the AD/SD library may be transfected with a vector expressing a specific gene (e.g. gene X) fused to a DNA binding domain. This allows virtually any gene encoded in the genome to be tested for an interaction to gene X. It is also recognized that the specific gene may be stably expressed in the host cell prior to construction of the BD/SD or AD/SD libraries.

In an alternative embodiment, genomic DNA is cloned into the BD/SD and/or AD/SD vector(s) downstream of the DNA binding domain and activation domain, respectively. If a gene is present and correctly oriented in the genomic DNA, then the BD/SD vector (or the AD/SD vector) will be capable of expressing the gene as a fusion protein useful for detecting protein-protein interactions. Like integration of BD/SD (or AD/SD) vectors *in situ,* any gene can be tested regardless of whether it has been previously isolated as a cDNA molecule.

In another embodiment, a second library is created in the cells of the first library. For example, the AD/SD vector can be integrated into cells comprising the BD/SD library. Conversely, the BD/SD vector can be integrated into cells comprising the AD/SD library. This allows all proteins expressed as binding domain fusion proteins to be tested against all activation domain fusion protein. Since the present invention is capable of expressing substantially all of the proteins (as fusions with the binding and activation domains) in a eukaryotic organism, this approach, for the first time, allows all combinations of protein-protein interactions to be tested in a single library. To survey all protein-protein interactions in an organism, the library within a library must be substantially comprehensive. For example, to detect ~50% of protein-protein interactions in an organism containing 100,000 genes, the first library must contain at least 100,000 cells, each expressing an activated gene. Within each clone of the first library, the second vector would then be used to create a library of at least 100,000 clones, each containing an activated gene. Thus, the total library would contain 100,000 clones x 100,000 clones, or 10¹⁰ total clones. This assumes all genes are activated at equal frequencies, and that each gene activation event results in production of a fusion protein in frame with the activated endogenous gene. To produce libraries with greater than 50% coverage of protein-protein interactions, and/or to ensure that proteins that are activated at lower frequencies are represented, larger libraries can be created.

It is also recognized that library vs. library screens can be created in several ways. First, both libraries are produced, simultaneously or sequentially, by integrating BD/SD. and AD/SD vectors into the genome of the same reporter cells. Second, a first library is created by integrating a BD/SD vector into the genome of a reporter cell, and a second library is produced by transfecting the AD/SD vector containing cloned genomic DNA. It is recognized that in this approach, the AD/SD library may be created first, followed by introduction of a BD/SD vector containing cloned genomic DNA. It is also recognized that the first library can be created by transfecting the BD/SD vector (or AD/SD vector) containing cloned genomic DNA, followed by integrating the second vector into the reporter cell genome. Third, both libraries are created, simultaneously or sequentially, by transfecting cells with a BD/SD and AD/SD vectors, wherein each vector contains a cloned fragment of genomic DNA. Fourth, it is recognized that when cloned genomic fragments are used in either the BD/SD vector or the AD/SD vector, a cDNA library may be created in the other vector and introduced into cells. This allows all of the genes present in the cDNA library to be tested for interaction with all other genes in the genome.

Since library/library screens involve the creation of large libraries of cells, it is important to maximize the frequency of gene activation and in frame fusion protein production among the members of the library. This can be accomplished in at least two ways. First, the BD/SD and AD/SD vectors can contain selectable markers in a configuration that "traps" genes. Examples of selection trap vectors are shown in Figures 8, 9, 10, 17, 19, 21, and 25. These vectors select for cells in which the activation vector has transcriptionally activated a gene. Second, multiple promoter/activation exon units can be included on the BD/SD and AD/SD vectors. Each promoter/activation exon unit encodes the binding domain (or activation domain) in a different reading frame relative to the unpaired splice donor site. An example of a multi-promoter/exon vector is illustrated infigure 23. This type of vector ensures that any gene activated at the transcription level will be produced as an in frame fusion protein from on of the promoter/activation exon units on the vector. Third, the vectors can be introduced into the reporter cells using efficient transfection procedures. In this respect, insertion of BD/SD and AD/SD vectors by retroviral integration is advantageous.

Reporter cells useful in the present invention include any cell that is capable of properly splicing the transcripts produced by the BD/SD and AD/SD vectors. The reporter cells contain a reporter gene that is expressed at higher levels in the presence of a protein-protein interaction between proteins expressed from BD/SD and AD/SD vectors. The reporter gene may be a selectable marker, such as any of the markers described herein. Alternatively, the reporter gene may be a screenable marker. Examples of useful selectable markers and screenable markers are described herein.

In the reporter cells, a minimal promoter is operably linked to the reporter gene. To allow increased expression of the reporter gene in the presence of a protein-protein interaction, a DNA binding site is positioned in or near the minimal promoter, such that the DNA binding site is recognized by the protein encoded by the DNA binding domain region of the BD/SD vector. In the absence of a protein-protein interaction, the DNA binding domain fusion protein produced from BD/SD lacks a transcription activation domain, and therefore, can not activate transcription from the minimal promoter of the reporter gene. If, however, the DNA binding domain fusion protein produced from BD/SD interacts with the activation domain fusion protein produced from the AD/SD vector, then the protein complex can activate expression of the reporter gene. Increased reporter gene expression can be detected using an assay for the screenable marker, or using drug selection for a selectable marker.

It is also recognized that other reporter systems can be used in conjunction with the present invention to detect protein-protein interactions. Specifically, any protein that contains two separable domains, each required to be in close proximity with the other to produce a biochemical or structural activity, can be used in conjunction with the present invention.

### Multi-Promoter/Activation Exon Vectors

In applications of nontargeted gene activation in which the goal is to activate protein expression from an unknown gene, a collection of vectors typically must be used. Thus, in an additional embodiment, the invention provides vectors containing one or more promoter/activation exon units (see Figures 20A-20E).

To accommodate the variety of gene structures that exist in the genomes of eukaryotic cells, vectors according to this aspect of the invention preferably contain a transcriptional regulatory sequence (e.g., a promoter) operably linked to an activation exon with a different structure. Collectively, these activation exons are capable of activating protein expression from substantially all endogenous genes. For example, to activate protein expression from genes that encode a translation start codon in exon II (or exons downstream of exon II), one vector can contain a transcriptional regulatory sequence (e.g., a promoter) operably linked to an activation exon lacking a translation start codon. To activate protein expression from all types of genes that encode a translation start codon in exon I, three separate vectors must be used, each containing a transcriptional regulatory sequence (e.g., a promoter) operably linked to a different activation exon. Each activation exon encodes a start codon in a different reading frame. Additional activation exon configurations are also useful. For example, to activate protein expression and secretion from genes that encode a portion of their signal secretion sequence in exon I, three separate vectors must be used, each containing a transcriptional regulatory sequence (e.g., a promoter) operably linked to a different activation exon. Each activation exon encodes a partial signal sequence in a different reading frame. To activate protein expression and secretion from genes that encode their entire signal sequence in exon I, three vectors must be used, each containing a transcriptional regulatory sequence (e.g., a promoter) operably linked to a different activation exon. Each activation exon contains an entire signal secretion sequence in a different reading frame. In addition to activating expression of genes that encode secreted proteins, promoter/activation exons encoding entire signal sequences will also activate expression and secretion of proteins that are not normally secreted. This, for example, can facilitate protein purification of proteins that are normally intracellularly localized.

Other useful coding sequences can be included on the activation exon of vectors according to this aspect of the invention, including but not limited to sequences encoding proteins (including full length proteins, portions of proteins, protein motifs, and/or epitope tags). As described herein, vectors according to this aspect of the invention can be integrated, individually or collectively, into the genome of a host cell to produce a library of cells. Each member of the library will potentially overexpress a different endogenous protein. Thus, these collections of vectors make it possible to activate all or substantially all of the endogenous genes in a eukaryotic host cell.

When integrating a collection of vectors into host cells, as described above, activation of protein expression can be achieved from substantially any gene. Unfortunately, to produce protein from all endogenous genes, a large number of library members must be generated. In part, this is due to the large number of genes encoded by the host cell. In addition, using this approach, many cells will contain a vector integrated into or near an endogenous gene; however, the integrated vector will contain an activation exon with a structure that is incompatible with activating protein expression from the endogenous gene. For example, the vector exon may encode a start codon in reading frame 1 (relative to the splice junction), whereas the protein encoded by the first exon downstream of the integrated vector may be in reading frame 2 (relative to the splice junction). Thus, many library members will contain an integrated vector that has activated transcription of an endogenous gene, but that failed to produce the protein encoded by the endogenous gene.

To decrease the number of cells that fail to activate protein expression following vector integration into or near an endogenous gene, a vector containing multiple promoter/activation exons can be used. On this vector, each promoter/activation exon unit can be capable of activating protein expression from an endogenous gene with a different structure. Since a single vector comprising multiple activation exons is capable of producing multiple transcripts, each containing a different activation exon, a single vector integrated into or near a gene can be capable of activating protein expression, regardless of the structure of the endogenous gene (see Figure 21).

Multi-promoter/activation exon vectors can contain two or more promoter/activation exons. Each promoter/activation exon unit may be followed by an unpaired splice donor site. In one such embodiment, two promoter/activation exons are included on the vector, wherein each promoter/activation exon is capable of activating protein expression from a different type of endogenous gene. In a preferred embodiment, the vector may contain three promoter/activation exons, wherein each exon encodes a translation start codon in a different reading frame. In another preferred embodiment, the vector may contain three promoter/activation exons, wherein each exon encodes a partial signal secretion sequence in a different reading frame. In yet another preferred embodiment, the vector may contain three promoter/activation exons, wherein each exon encodes an entire signal secretion sequence in a different reading frame. Additional embodiments include each of the vectors above containing a fourth promoter/activation exon, wherein the fourth activation exon does not encode a translation start codon.

Any number (e.g., one or more, two or more, three or more, four or more, five or more, etc.) of promoter/activation exon units may be included on the vector. When multiple promoter/activation exons are present on a single vector, they are preferably oriented in the same direction relative to one another (*i.e.,* the promoters drive expression in the same direction).

The promoters that drive transcription of different activation exons may be the same as one another or one or more promoters may be different. The promoters may be viral, cellular, or synthetic. The promoters may be constitutive or inducible. Other types of promoters and regulatory sequences, recognizable to one skilled in the art or as described herein, may also be used in preparing the vectors according to this aspect of the invention.

Any of the vectors containing multiple promoter/activation exon units may optionally include one or more amplifiable marker(s). The vectors contain a selectable marker. The amplifiable markers may contain a poly(A) signal. Alternatively, the markers may lack a poly(A) signal. The selectable marker may be a positive or negative selectable marker. The selectable marker may contain an unpaired splice donor site upstream, within, or downstream of the marker. Alternatively, the selectable marker may lack an unpaired splice donor site. The selectable marker(s) and/or amplifiable marker(s), when present, may be located upstream, among, or downstream of the promoter/activation exon units. The selectable and/or amplifiable marker(s) may be located on the vector in any orientation relative to the promoter/activation exon units. When the purpose of the selectable marker is to trap endogenous genes, the selectable marker is preferably oriented in the same direction as the promoter/activation exons.

### Amplifiable Markers

Any of the vectors described herein may also optionally comprise one or more (e.g., two, three, four, five, or more) amplifiable markers. Examples of amplifiable markers include those described in detail hereinabove. Preferably, the amplifiable marker(s) are located upstream of the positive/negative selectable marker(s). It may be advantageous to omit a polyadenylation signal from the amplifiable marker(s) to eliminate the possibility of capturing a vector-encoded poly(A) signal derived from vector concatemerization prior to integration.

When present, the amplifiable marker(s) may be located upstream of the activation transcriptional regulatory sequence (i.e. the promoter responsible for directing transcription from the vector through the endogenous gene). The amplifiable marker(s) may be present on the vector in any orientation (i.e. the open reading frame may be present on either DNA strand).

It is also understood that the amplifiable marker(s) can also be the same gene as the positive selectable marker. Examples of genes that can be used both as positive selectable markers and amplifiable markers include dihydrofolate reductase, adenosine deaminase (ada), dihydro-orotase, glutamine synthase (GS), and carbamyl phosphate synthase (CAD).

In some embodiments and for certain applications, it may be desirable to place multiple amplifiable markers on the vector. Use of more than one amplifiable marker allows dual selection, or alternatively sequential selection, for each amplifiable marker. This facilitates the isolation of cells that have amplified the vector and flanking genomic locus, including the gene of interest.

### Promoters

It is understood that any promoter and regulatory element may be used on these activation vectors to drive expression of the selectable marker, amplifiable marker (if present), and/or the endogenous gene. In additional preferred embodiments, the promoter driving expression of the endogenous gene is a strong promoter. The CMV immediate early gene promoter, SV40 T antigen promoter, and β-actin promoter are examples of this type of promoter. In another preferred embodiment, an inducible promoter is used to drive expression of the endogenous genes. This allows endogenous proteins to be expressed in a more controlled fashion. The Tetracycline inducible promoter, heat shock promoter, ectdysone promoter, and metallothionein promoter are examples of this type of promoter. In yet another embodiment, a tissue specific promoter is used to drive expression of endogenous genes. Examples of tissue specific promoters include, but are not limited to, immunoglobulin promoters, casein promoter, and growth hormone promoter.

### Restriction Sites

The vectors of the invention can contain one or more restriction sites located downstream of the unpaired splice donor site in the vector. These restriction sites can be used to linearize plasmid vectors prior to transfection. In the linear configuration, the activation vector contains, from 5' to 3' relative to the transcribed strand, a promoter, a splice donor site, and a linearization site.

A restriction site(s) may also be included in the vector intron to facilitate removal of vector intron-containing cDNA molecules. In this embodiment, the vector contains, from 5' to 3' relative to the transcribed strand, a promoter, a splice donor site, a restriction site, and a linearization site. By including a restriction site between the unpaired splice donor site and the linearization site, unspliced transcripts can be removed by digestion of cDNA with the appropriate restriction enzyme. cDNA molecules derived from gene activation have removed the vector intron containing the restriction site, and therefore, will not be digested. This allows gene activated transcripts to be preferentially enriched during ' amplification/cloning, and greatly facilitates identification and analysis of endogenous genes.

A restriction site(s) may also be included in the vector exon to facilitate cloning of activated genes. Following gene activation, mRNA is recovered from cells and synthesized into cDNA. By digesting the cDNA with a restriction enzyme that cuts in the vector exon, gene activated cDNA molecules will contain an appropriate overhang at the 5' end for subsequent cloning into a suitable vector. This facilitates isolation of gene activated cDNA molecules.

In one embodiment, the restriction site located in the vector exon is different than the restriction site(s) located in the vector intron. This facilitates removal of cDNA molecules that contain a vector intron since the digested cDNA fragments from vector intron containing transcripts can be designed to have an overhang that is incompatible with the cloning vector (see below). Alternatively, degenerate restriction sites recognized by the same enzyme may be located in the vector exon and intron. Enzymes that cleave these sites are capable of cleaving multiple sites, sites with an odd number of bases in the recognition sequence, sites with interrupted palindromes, nonpalindromic sequences, or sites containing one or more degenerate bases. In other words, restriction sites recognized by the same restriction endonuclease may be used if the enzyme produces an overhang in the vector exon that is different from the overhang produced in the vector intron. Since different overhangs are produced, a cloning vector containing a site that is compatible with the vector exon overhang, and incompatible with the vector intron overhang may be used to preferentially clone vector exon containing and vector intron lacking cDNA molecules. Examples of useful degenerate restriction sites include DNA sequences recognized by Sfi I, Acci, Af1 III, SapI, Ple I, Tsp45 I, ScrF I, Tse I, PpuM I, Rsr II, and SgrA I.

The restriction site(s) located in the vector intron and/or exon can be a rare restriction site (e.g. an 8 bp restriction site) or an ultra-rare site (e.g. a site recognized by intron encoded nucleases). Examples of restriction enzymes with 8 bp recognitions sites include *Not*I, *Sfi*I, *Pac*I, *Asc*I, *Fse*I, *Pme*I, *Sgf*I, *Srf*I, *Sbf*I, Sse 8387 I, and *Swa*I. Examples of intron encoded restriction enzymes include I-*Ppo*I, I-*Sce*I, I-*Ceu*I, PI-*Psp*I, and PI-*Tli*I. Alternatively, restriction sites smaller than 8 bp can be placed on the vector. For example, restriction sites composed of 7 bp, 6 bp, 5 bp, or 4 bp can be used. In general, the use of smaller the restriction recognition sites will lead to the cloning of less than full-length genes. In some cases, such as creation of hybridization probes, isolation of smaller cDNA clones may be advantageous.

### Bidirectional Activation Vectors

The activation vectors described herein can also be bidirectional. When a single activation transcriptional regulatory sequence is present on the vector, gene activation occurs only when the vector integrates into an appropriate location (e.g. upstream of the gene) and in the correct orientation. That is, in order to activate an endogenous gene, the promoter on the activation construct must face the endogenous gene allowing transcription of the coding strand. As a result of this directionality requirement, only half of the integration events into a locus may result in the transcriptional activation of an endogenous gene. The other half of integration events result in the vector transcribing away from a gene of interest. Therefore, to increase the gene activation frequency by a factor of two, the present invention provides bidirectional vectors that may be used to activate an endogenous gene regardless of the orientation in which the vector integrates into the host cell genome.

The bidirectional vectors according to this aspect of the invention may include those selectable markers and amplifiable markers described in detail herein. The bidirectional vectors are configured as dual poly(A)/splice acceptor trap vectors, as described above. Other vector configurations described for unidirectional vectors may also be incorporated into bidirectional vectors.

### Co-transfection of Genomic Dna with Non-targeted Activation Vectors

It is recognized that any of the vectors described herein can be integrated into, or otherwise combined with, genomic DNA prior to transfection into a eukaryotic host cell. This permits high level expression from virtually any gene in the genome, regardless of the normal expression characteristics of the gene. Thus, the vectors of the invention can be used to activate expression from genes encoded by isolated genomic DNA fragments. To accomplish this, the vector is integrated into, or otherwise combined with, genomic DNA containing at least one gene, or portion of a gene. Typically, the activation vector must be positioned within or upstream of a gene in order to activate gene expression. Once inserted (or joined), the downstream gene may be expressed (as a transcript or a protein) by introducing the vector/genomic DNA into an appropriate eukaryotic host cell. Following introduction into the host cell, the vector encoded promoter drives expression through the gene encoded in the isolated DNA, and following splicing, produces a mature mRNA molecule. Using appropriate activation vectors, this process allows protein to be expressed from any gene encoded by the transfected genomic DNA. In addition, using the methods described herein, cDNA molecules, corresponding to genes encoded by the transfected genomic DNA, can be generated and isolated.

To achieve stable expression of the activated gene, the transfected activation vector/genomic DNA can be integrated into the host cell genome. Alternatively, the transfected activation vector/genomic DNA can be maintained as a stable episome (e.g. using a viral origin of replication and/or nuclear retention function - see below). In yet another embodiment, the activated gene may be expressed transiently, for example, from a plasmid.

As used herein, the term "genomic DNA" refers to the unspliced genetic material from a cell. Splicing refers to the process of removing introns from genes following transcription. Thus, genomic DNA, in contrast to mRNA and cDNA, contains exons and introns in an unspliced form. In the present invention, genomic DNA derived from eukaryotic cells is particularly useful since most eukaryotic genes contain exons and introns, and since many of the vectors of the present invention are designed to activate genes encoded in the genomic DNA by splicing to the first downstream exon, and removing intervening introns.

Genomic DNA useful in the present invention may be isolated using any method known in the art. A number of methods for isolating high molecular weight genomic DNA and ultra-high molecular weight genomic DNA (intact and encased in agarose plugs) have been described (Sambrook et al., Molecular Cloning, Cold Spring Harbor Laboratory Press, (1989)). In addition, commercial kits for isolating genomic DNA of various sizes are also available (Gibco/BRL, Stratagene, Clontech, etc.).

The genomic DNA used in the invention may encompass the entire genome of an organism. Alternatively, the genomic DNA may include only a portion of the entire genome from an organism. For example, the genomic DNA may contain multiple chromosomes, a single chromosome, a portion of a chromosome, a genetic locus, a single gene, or a portion of a gene.

Genomic DNA useful in the invention may be substantially intact (i.e. unfragmented) prior to introduction into a host cell. Alternatively, the genomic DNA may be fragmented prior to introduction into a host cell. This can be accomplished by, for example, mechanical shearing, nuclease treatment, chemical treament, irradiation, or other methods known in the art. When the genomic DNA is fragmented, the fragmentation conditions may be adjusted to produce DNA fragments of any desirable size. Typically, DNA fragments should be large enough to contain at least one gene, or a portion of a gene (e.g. at least one exon). The genomic DNA may be introduced directly into an appropriate eukaryotic host cell without prior cloning. Alternatively, the genomic DNA (or genomic DNA fragments) may be cloned into a vector prior to transfection. Useful vectors include, but are not limited to, high and intermediate copy number plasmids (e.g. pUC, pBluescript, pACYC184, pBR322, etc.), cosmids, bacterial artificial chromosomes (BACs), yeast artificial chromosomes (YACs), P1 artificial chromosomes (PACs), and phage (e.g. lambda, M13, etc.). Other cloning vectors known in the art may also be used. When genomic DNA has been cloned into a cloning vector, specific cloned DNA fragments may be isolated and used in the present invention. For example, YAC, BAC, PAC, or cosmid libraries can be screened by hybridization to identify clones that map to specific chromosomal regions. Optionally, once isolated, these clones can be ordered to produce a contig through the chromosomal region of interest. To rapidly isolate cDNA copies of the genes present in this contig, these genomic clones may be transfected, separately or en masse, with the activation vector into a host cell. cDNA containing a vector encoded exon, and lacking a vector encoded intron, can then be isolated and analyzed. Thus, since all genes present in a contig can be rapidly isolated as cDNA clones, this approach greatly enhances the speed of positional cloning approaches.

Any activation vector described herein, including derivatives recognized by those skilled in the art, may be co-transfected with genomic DNA, and therefore, are useful in the present invention. Examples of other useful vectors include, but are not limited to, poly A trap vectors (e.g. vectors illustrated in Figures 8, 9, 11C, 12F, and 17), dual poly (A)/Splice acceptor trap vectors (e.g. vectors illustrated in Figures 9, 10, 12G, 19, and 21), bi-directional vectors (e.g. vectors illustrated in Figure 11), single exon trap vectors (e.g. the vector illustrated in Figure 19), multi-promoter/activation exon vectors (e.g. the vector illustrated in Figure 23), vectors for isolating cDNA's corresponding to activated genes, and vectors for activating protein expression from activated genes (e.g. vectors illustrated in Figures 2, 3, 4, 8B-F, 9B-C, 9E-F, 10B-C, 10E-F, 11, 12, 17B-G, and 23).

The activation vector may also contain a viral origin of replication. The presence of a viral origin of replication allows vectors containing genomic fragments to be propagated as an episome in the host cell. Examples of useful viral origins of replication include ori P (Epstein Barr Virus), SV40 ori, BPV ori, and vaccinia ori. To facilitate replication from these origins, the appropriate viral replication proteins may be expressed from the vector. For example, EBV ori P and SV40 ori containing vectors may also encode and express EBNA-1 or T antigen, respectively. Alternatively, the vectors may be introduced into cells that are already expressing the viral replication protein (e.g. EBNA-1 or T antigen). Examples of cells expressing EBNA-1 and T antigen include human 293 cells transfected with an EBNA-1 expression unit (Clontech) and COS-7 cells (American Type Culture Collection; ATCC No. CRL-1651), respectively.

The activation vector may also contain an amplifiable marker. This enables cells containing increased copies of the vector and flanking genomic DNA, either episomal or integrated in the host cell genome, to be isolated. Cells containing increased copies of the vector and flanking genomic DNA express the activated gene at higher levels, facilitating gene isolation and protein production.

The activation vector and genomic DNA may be introduced into any host cell capable of splicing from the vector-encoded splice donor site to a splice acceptor site encoded by the genomic DNA. In a preferred embodiment, the genomic DNA/activation vector are transfected into a host cell from the same species as the cell from which the genomic DNA was isolated. In some instances, however, it is advantageous to transfect the genomic DNA into a host cell from a species that is different from the cell from which the genomic DNA was isolated. For example, transfection of genomic DNA from one species into a host cell of a second species can facilitate analysis of the genes activated in the transfected genomic DNA using hybridization techniques. Under high stringency hybridization, activated genes that were encoded by the transfected DNA can be distinguished from genes derived from the host cell. Transfection of genomic DNA from one species into a host cell from another species can also be used to produce protein in a heterologous cell. This may allow protein to be produced in heterologous cells that provide growth, protein modification, or manufacturing advantages.

The activation vector may be co-transfected into a host cell along with genomic DNA, wherein the vector is not attached to the genomic DNA prior to introduction into the cell. In this embodiment, the genomic DNA will become fragmented during the transfection process, thereby creating free DNA ends. These DNA ends can become joined to the co-transfected activation vector by the cell's DNA repair machinery. Following joining to the activation vector, the genomic DNA and activation vector can be integrated into the host cell genome by the process of non-homologous recombination. If, during this process, a vector becomes joined to a gene encoded by the transfected genomic DNA, the vector will activate its expression.

Alternatively, the non-targeted activation vector may be physically linked to the genomic DNA prior to transfection. In a preferred embodiment, genomic DNA fragments are ligated to the vector prior to transfection. This is advantageous because it maximizes the probability of the vector becoming operably linked to a gene encoded by the genomic DNA, and minimizes the probability of the vector integrating into the host cell genome without the heterologous genomic DNA.

In a related embodiment, the genomic DNA may be cloned into the activation vector, downstream of the activation exon. In this embodiment, cloning of large genomic fragments can be facilitated in vectors capable of accommodating large genomic fragments. Thus, the activation vector may be constructed in BACs, YACs, PACs, cosmids, or similar vectors capable of propagating large fragments of genomic DNA.

Another method for joining the activation vector to genomic DNA involves transposition. In this embodiment, the activation vector is integrated into the genomic DNA by transposition or retroviral integration reactions prior to transfection into a cell. Accordingly, activation vectors can contain cis sequences necessary for facilitating transposition and/or retroviral integration. Examples of vectors containing transposon signals are illustrated in Figure 27; however, it is recognized that any vector described herein may contain transposon signals.

Any transposition system capable of inserting foreign sequences into genomic DNA can be used in the present invention. In addition, transposons capable of facilitating inversions and deletions can also be used to practice the invention. While deletion and inversion systems do not integrate the activation vector into genomic DNA, they do allow the activation vector to change positions relative to cloned genomic DNA when the genomic DNA has been cloned into the activation vector. Thus, multiple genes within a given genomic fragment can be activated by shuffling the activation vector (by integration, inversion, or deletion) into multiple positions within, or outside of, the genomic fragment. Examples of transposition systems useful for the present invention include, but are not limited to δγ, Tn 3, Tn5, Tn7,Tn9, Tn10, Ty, retroviral integration and retro-transposons (Berg et al., Mobile DNA, ASM Press, Washington DC, pp. 879-925 (1989); Strathman et al., Proc. Natl. Acad Sci. USA 88:1247 (1991); Berg et al., Gene 113:9 (1992); Liu et al., NucL Acids Res. 15:9461 (1987), Martin et al., Proc. Natl. Acad Sci. USA 92:8398 (1995); Phadnis et al., Proc. MatL Acad Sci. USA 86:5908 (1989); Tomcsanyi et al., J. Bacteriol. 172:6348 (1990); Way et al., Gene 32:369 (1984); Bainton et al., Cell 65:805 (1991); Ahmed et al., J. Mol. Biol. 178:941 (1984); Benjamin et al., Cell 59:373 (1989); Brown et al., Cell 49:347 (1987); Eichinger et al., Cell 54:955 (1988); Eichinger et al., Genes Dev. 4:324 (1990); Braiterman et al., Mol. Cell. Biol. 14:5719 (1994); Braiterman et al., Mol. Cell. Biol. 14:5731 (1994); York et al., Nucl. Acids Res. 26:1927 (1998); Devine et al., Nucl. Acids Res. 18:3765 (1994); Goryshin et al., J. Biol. Chem. 273:7367 (1998).

Using transposition, an activation vector may be integrated into any form of genomic DNA. For example, the activation vector may be integrated into either intact or fragmented genomic DNA. Alternatively, the activation vector may be integrated into a cloned fragment of genomic DNA (Figure 28). In this embodiment, the genomic DNA may reside in any cloning vector, including high and intermediate copy number plasmids (e.g. pUC, pBluescript, pACYC184, pBR322, etc.), cosmids, bacterial artificial chromosomes (BACs), yeast artificial chromosomes (YACs), P1 artificial chromosomes (PACs), and phage (e.g. lambda, M13, etc.). Other cloning vectors known in the art may also be used. As described above, genomic fragments from specific genetic loci may be isolated an used as a substrate for activation vector integration.

Following integration of the activation vector, the genomic DNA may be introduced directly into a suitable host cell for expression of the activated gene. Alternatively, the genomic DNA may be introduced into and propagated in an intermediate host cell. For example, following integration of an activation vector into a BAC genomic library, the BAC library can be transformed into *E. coli.* This allows plasmids containing the transposon to be enriched by selecting for an antibiotic resistance marker residing on the activation vector. As a result, BAC plasmids lacking an integrated activation vector will be removed by antibiotic selection.

The transposition mediated activation vector integration may occur *in vitro* using purified enzymes. Alternatively, the transposition reaction may occur *in vivo.* For example, transposition may be carried out in bacteria, using a donor strain carrying the transposon either on a vector or as integrated copies in the genome. A target of interest is introduced into the transposer host where it receives integrations. Targets bearing insertions are then recovered from the host by genetic selection. Similarly, eukaryotic host cells, such as yeast, plant, insect, or mammalian cells, can be used to carry out the transposon mediated integration of an activation vector into a fragment of genomic DNA.

### Isolation of mRNA and cDNA Produced from Activated Endogenous Genes

In additional embodiments, the present invention is directed to methods for isolating genes, particularly genes contained within the genome of a eukaryotic cell, that are activated using the vectors of the invention. These methods exploit the structure of the mRNA molecules produced using the non-targeted gene activation vectors of the invention. The methods of the invention described herein allow virtually any activated gene to be isolated, regardless of whether it has been previously isolated and characterized, and regardless of whether it has a known biological activity. This is made possible by the nature of the chimeric transcripts produced from the integrated vectors of of the present invention. Using methods described herein, activation vectors can be integrated into the genome of a cell. Typically, the activation vectors, however, are integrated into the genome of many cells to produce a library of unique integration events. Each member of the library contains the vector located at a unique integration site(s), and potentially contains an activated endogenous gene. Gene activation occurs when the activation vector integrates upstream of the 3'-most exon of an endogenous gene and in an orientation capable of allowing transcription from the vector to proceed through the endogenous gene. The integration site may be in an intron or exon of the endogenous gene, or may be upstream of the transcription start site of the gene. Following integration, the activation constructs are designed to produce a transcript capable of splicing from an exon encoded by the activation vector to an exon encoded by the endogenous gene. As a result, a chimeric message is produced that contains the vector exon linked to the exons from an endogenous gene, wherein the endogenous exons are derived from the region located downstream of the vector integration site. The structure of this chimeric transcript can be exploited for gene discovery purposes. For example, the chimeric transcripts can be rapidly isolated to use as probes (to isolate the full length cDNA or genomic copy of the gene or to characterize the gene) or for direct sequencing and/or characterization.

To isolate the chimeric transcripts activated by vector insertion, cDNA is produced from a library member containing the activation event. It is also possible to isolate chimeric transcripts from pools of library members in order to increase the through-put of the procedure. cDNA can then be produced from the mRNA harvested from the activated cells. Alternatively, total RNA may be used to produce cDNA. In either case, first strand synthesis can be carried out using an oligo dT primer, an oligo dT/poly(A) signal primer, or a random primer. To facilitate cloning of the cDNA product, a poly dT based primer can be used with the structure: 5'-Primer X(dT)₁₋₁₀₀-3'. The oligo dT/poly(A) signal primer can have the structure 5'-(dT)₁₀₋₃₀-Primer X-N₀₋₆-TTTATT -3'. The random primer can have the structure: 5'-(Primer X)NNNNNN -3'. In each primer, Primer X is any sequence that can be used to subsequently PCR amplify target nucleic acid molecules. Where the activated gene amplification product is to be cloned, it is useful to include one or more restriction sites within the primer X sequence to facilitate subsequent cloning. Other primers recognized by those skilled in the art can be used to create first strand cDNA products, including primers that lack a Primer X region.

In accordance with the invention, the primers may be conjugated with one or more hapten molecules to facilitate subsequent isolation of nucleic acid molecules (e.g., first and/or second strand cDNA products) comprising such primers. After the primer becomes associated with the nucleic acid molecule (via incorporation during cDNA synthesis), selective isolation of the molecule containing the haptenylated primer may be accomplished using a corresponding ligand which specifically interacts with and binds to the hapten via ligand-hapten interactions. In preferred such aspects, the ligand may be bound to, for example, a solid support. Once bound to the solid support, the molecules of interest (haptenylated primer-containing nucleic acid molecules) can be separated from contaminating nucleic acids and other materials by washing the support matrix with a solution, preferably a buffer or water. Cleavage of one or more of the cleavage sites within the primer, or by treatment of the solid support containing the nucleic acid molecule with a high ionic strength elution buffer, then allows for removal of the nucleic acid molecule of interest from the solid support.

Preferred solid supports for use in this aspect of the invention include, but are not limited to, nitrocellulose, diazocellulose, glass, polystyrene, polyvinylchloride, polypropylene, polyethylene, dextran, Sepharose, agar, starch, nylon, latex beads, magnetic beads, paramagnetic beads, superparamagnetic beads or microtitre plates and most preferably a magnetic bead, a paramagnetic bead or a superparamagnetic bead, that comprises one or more ligand molecules specifically recognizing and binding to the hapten molecule on the primer.

Particularly preferred hapten molecules for use on the primer molecules of the invention, include without limitation: (i) biotin; (ii) an antibody; (iii) an enzyme; (iv) lipopolysaccharide; (v) apotransferrin; (vi) ferrotransferrin; (vii) insulin; (viii) cytokines (growth factors, interleukins or colony-stimulating factors); (ix) gp120; (x) β-actin; (xi) LFA-1; (xii) Mac-1; (xiii) glycophorin; (xiv) laminin; (xv) collagen; (xvi) fibronectin; (xvii) vitronectin; (xviii) integrins αᵥβ₁ and αᵥβ₃; (xix) integrins α₃β₁, α₄β₁, α₄β₇, α₅β₁, αᵥβ₁, α_{11b}β₃, αᵥβ₃ and αᵥβ₆; (xx) integrins α₁β₁, α₂β₁, α₃β₁ and αᵥβ₃; (xxi) integrins α₁β₁, α₂β₁, α₃β₁, α₆β₁,α₇β₁ and α₆β₅; (xxii) ankyrin; (xxiii) C3bi, fibrinogen or Factor X; (xxiv) ICAM-1 or ICAM-2; (xxv) spectrin or fodrin; (xxvi) CD4; (xxvii) a cytokine (e.g., growth factor, interleukin or colony-stimulating factor) receptor; (xxviii) an insulin receptor; (xxix) a transferrin receptor; (xxx) Fe⁺⁺⁺; (xxxi) polymyxin B or endotoxin-neutralizing protein (ENP); (xxxii) an enzyme-specific substrate; (xxxiii) protein A, protein G, a cell-surface Fc receptor or an antibody-specific antigen; and (xxxiv) avidin and streptavidin. Particularly preferred is biotin.

Particularly preferred ligand molecules according to this aspect of the invention, which correspond in order to the above-described hapten molecules, include without limitation: (i) avidin and streptavidin; (ii) protein A, protein G, a cell-surface Fc receptor or an antibody- specific antigen; (iii) an enzyme-specific substrate; (iv) polymyxin B or endotoxin-neutralizing protein (ENP); (v) Fe⁺⁺⁺; (vi) a transferrin receptor; (vii) an insulin receptor; (viii) a cytokine (e.g., growth factor, interleukin or colony-stimulating factor) receptor; (ix) CD4; (x) spectrin or fodrin; (xi) ICAM-1 or ICAM-2; (xii) C3bi, fibrinogen or Factor X; (xiii) ankyrin; (xiv) integrins α₁β₁, α₂β₁, α₃β₁, α₆β₁, α₇β₁ and α₆β₅ ; (xv) integrins α₁β₁, α₂β₁, α₃β₁ and αᵥβ₃; (xvi) integrins α₃β₁, α₄β₁, α₄β₇, α₅β₁, αᵥβ₁, α_{IIb}β₃, αᵥβ₃ and αᵥβ₆; (xvii) integrins αᵥβ₁ and αᵥβ₃; (xviii) vitronectin; (xix) fibronectin; (xx) collagen; (xxi) laminin; (xxii) glycophorin; (xxiii) Mac-1; (xxiv) LFA-1; (xxv) β-actin; (xxvi) gp120; (xxvii) cytokines (growth factors, interleukins or colony-stimulating factors); (xxviii) insulin; (xxix) ferrotransferrin; (xxx) apotransferrin; (xxxi) lipopolysaccharide; (xxxii) an enzyme; (xxxiii) an antibody; and (xxxiv) biotin. Particularly preferred, for use with biotinylated primers of the invention, are avidin and streptavidin.

Following first strand synthesis, second strand cDNA synthesis may be carried out using a primer specific for the vector encoded exon. This creates double stranded cDNA from all transcripts that were derived from the vector encoded promoter. All cellular mRNA (and cDNA) produced from endogenous promoters remains single stranded since the transcript lacks a vector exon at it 5' end. Once second strand synthesis is carried out, the cDNA may be digested with a restriction enzyme, cloned into a vector, and propagated.

To facilitate cloning, cDNA molecules containing the vector exon are amplified by PCR using a primer specific for the vector exon and a primer specific for the first strand cDNA primer (e.g. Primer X). PCR amplification results in the production of variable length DNA fragments representing different locations of priming during first strand synthesis and/or amplification of multiple chimeric transcripts from different genes. These amplification products can be cloned into plasmids for characterization, or can be labeled and used as a probe.

Other amplification techniques, such as linear amplification using RNA polymerase (Van Gelder, Proc. Natl. Acad. Sci. USA 87:1663-1667 (1990); Eberwine, Methods 10:283-288 (1996)), can be used. For example, when linear amplification by RNA polymerase is used, a promoter (e.g. T7 promoter) can be placed on the vector exon. As a result, gene activated transcripts will contain the promoter sequence at the 5' end of the transcript. Alternatively, a promoter can be ligated onto the cDNA molecule following first strand and second strand synthesis. Using either strategy, RNA polymerase is then incubated with cDNA in the presence of ribonucleotide triphosphates to create RNA transcripts from the cDNA. These transcripts are then reverse transcribed to produce cDNA. Since RNA polymerase can create several thousand transcripts from a single cDNA molecule, and since each of these transcripts can be reverse transcribed into cDNA, a large amplification can be achieved. As with PCR, amplification with RNA polymerase can facilitate cloning of activated genes. Other types of amplification strategies are also possible.

In another embodiment, the vector exon containing cDNA molecules are isolated without amplification. This may be useful in instances where biases occur during amplification (for example, when one DNA fragment amplifies more efficiently than another). To produce cDNA enriched for tagged messages, RNA is isolated from the activation library. A primer (e.g. a random hexamer, oligo(dT), or hybrid primers containing a primer linked to poly(dT) or a random nucleotides) is annealed to the RNA and used to direct first strand synthesis. The first strand cDNA molecules are then hybridized to a primer specific for the vector encoded exon. This primer directs second strand synthesis. Following second strand synthesis, the cDNA may be digested with restriction enzymes that cut in the vector exon and in the first strand primer (e.g. in Primer X - see above). The second strand products may then be cloned into a useful vector to allow them to be propagated.

It will be apparent to one of ordinary skill in view of the description contained herein that the cDNA products made according to the methods of the invention may also be cloned into a cloning vector suitable for transfection or transformation of a variety of prokaryotic (bacterial) or eukaryotic (yeast, plant or animal including human and other mammalian) cells. Such cloning vectors, which may be expression vectors, include but are not limited to chromosomal-, episomal- and virus-derived vectors, e.g., vectors derived from bacterial plasmids or bacteriophages, and vectors derived from combinations thereof, such as cosmids and phagemids, BACs, MACs, YACs, and the like. Other vectors suitable for use in accordance with this aspect of the invention, and methods for insertion of DNA fragments therein and transformation of host cells with such cloning vectors, will be familiar to those of ordinary skill in the art.

### Removal of Unspliced Transcription Products

In some instances, the activation vector will integrate into the genome in a region lacking genes. Alternatively, it may integrate into a region containing a gene(s), but be oriented in a manner that results in the transcription of the non-coding strand. In each of these instances, gene activated transcripts are produced that contain normally untranscribed DNA sequences next to the vector encoded exon. These sequences would complicate identification and analysis of novel genes. Therefore, it would be advantageous to selectively remove these genomic molecules.

To remove cDNA molecules that contain a vector encoded intron, the double strand cDNA is treated with a restriction enzyme that recognizes a sequence located in the vector encoded intron. Preferably, the restriction enzyme creates an overhang that is different from the overhang produced by cleavage of the vector exon. This ensures the cloning of only activated genes by preventing the cleavage products from ligating into the cloning vector.

### Recovery of Exon I from activated endogenous genes

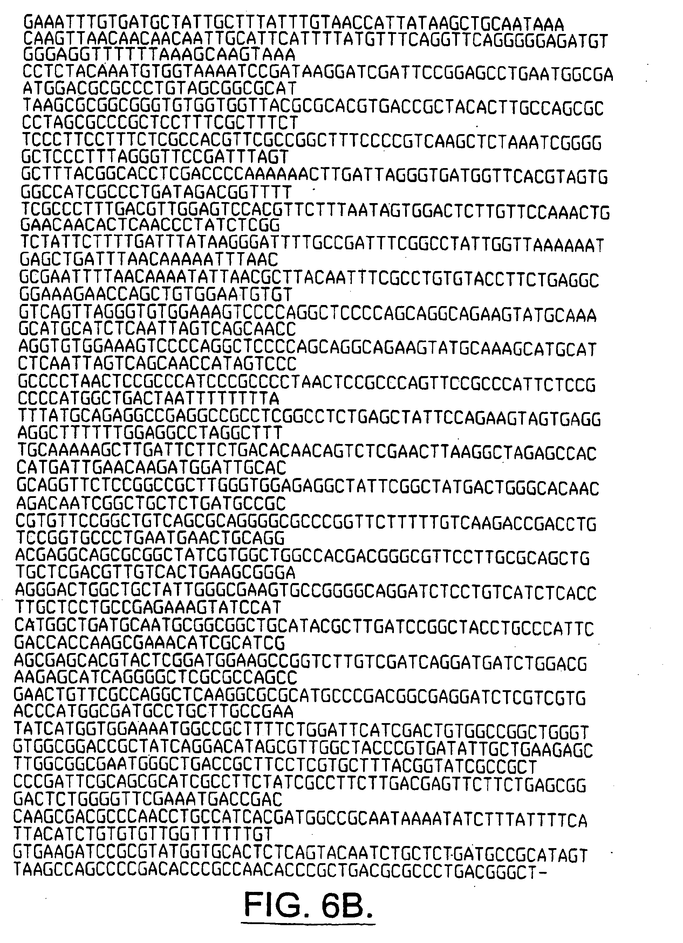

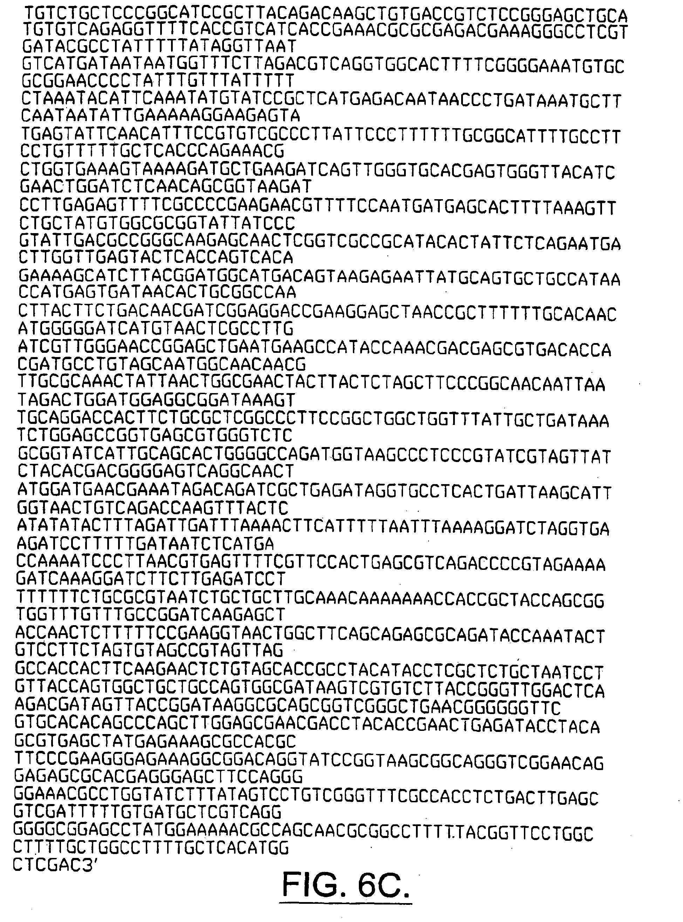

To recover exon I from activated genes, specialized vectors can be used to create non-targeted gene activation libraries. In its simplest form, this vector contains, from 5' to 3', a promoter, an unpaired splice donor site, and a second promoter. The downstream promoter is oriented in the same direction as the upstream promoter. Upon integration upstream of an endogenous gene, this type of vector produces two types of transcripts. The first transcript contains the vector exon joined to exon II of the endogenous gene. Methods for isolating this transcript are described above. The second transcript contains the upstream region of the endogenous gene followed by exon I joined to exon II and other downstream exons from the endogenous gene (Figure 6).

Using a two step process, exon I can be recovered from cells containing the integrated vector. First, vector exon containing transcripts (i.e. Transcript type #1, Figure 13) are isolated using the methods described above. Once isolated, the 5' end of the transcript including exon II can be sequenced to determine the sequence of the flanking endogenous exons. Second, once the sequence of the flanking endogenous exons is known, PCR primers capable of annealing to exon II (or a downstream exon) of the activated gene can be developed. These primers can be used to amplify exon I from Transcript #2 (Figure 13) using a modified form of inverse PCR (Zeiner, M., Biotechniques 17(6):1051-1053 (1994)). Briefly, amplification of exon I from the endogenous gene is achieved by carrying out first strand cDNA synthesis with a gene specific primer, based on the sequence information determined above. Second strand synthesis can be carried out using *E*. *coli* DNA polymerase I under conditions well known to those skilled in the art. The double strand cDNA is then digested with a restriction enzyme that cleaves at least once in the endogenous gene upstream of the first strand cDNA primer, and that does not cleave in the vector exon. Following digestion, the cDNA is self ligated to produce circular molecules. Using inverted PCR primers that anneal in the endogenous gene upstream of the restriction/circularization site, amplification by PCR produces a DNA product containing exon I sequences from the endogenous gene.

### Method for Selecting Cells Containing Higher Levels of Gene Activated Transcripts/Protein

In several embodiments of the disclosed invention, the activation vector contains an amplifiable marker (e.g. DHFR) and a viral origin of replication (e.g. EBV ori P). In other embodiments, an amplifiable marker and viral origin of replication are present on a cloning vector containing a cloned fragment of genomic DNA: In yet another embodiment, the activation vector contains one element (e.g. DHFR) and a cloning vector carrying a genomic insert contains the other element (e.g. Ori P). Regardless of the initial location of the amplifiable marker and viral origin, the elements are combined on the same DNA molecule prior to or during introduction into a host cell.

In addition to the cis-acting elements, a trans-acting viral protein is generally required for efficient replication of the episomes. Examples of trans-acting viral proteins include EBNA-1 and SV40 T antigen. To promote efficient replication of episomes, the trans-acting viral protein can be expressed from the episome. Thus, the viral trans-acting protein may be expressed from the transposing activation vector, or may be positioned on the backbone of the cloning vector. Alternatively, the trans-acting viral protein may be expressed by the eukaryotic host cells into which the episome is introduced.

Once the amplifiable marker and viral origin of replication are on the same molecule and present in a host cell expressing the appropriate viral replication protein(s), the copy number of the episome can be increased. To increase the copy number of the episome, the cells can be placed under the appropriate selection. For example, if DHFR is present on the episome, methotrexate may be added to the culture. The selective agent may be applied at relatively high concentrations to isolate cells in the population that already have a high episome copy number. Alternatively, the selective agent may be applied at lower concentrations, and periodically increased in concentration. Two-fold increases in drug concentration will result in step-wise increases in copy number.

To reduce the frequency of non-specific drug resistance (i.e. drug resistance that is not associated with increased copy number of the episome), more than one amplifiable marker can be placed on the vector. Inclusion of multiple amplifiable markers on the episome allows cells to be selected with multiple drugs (either simultaneously or sequentially). Since non-specific drug resistance is a relatively rare event, the probability of a cell developing non-specific drug resistance to multiple drugs is exceedingly rare. Thus, the presence of multiple amplifiable markers on the episome facilitates isolation of cells that have a high episome copy number.

Amplification of episome copy number increases the number of transcripts derived from the vector activated gene. This, in turn, facilitates isolation of cDNA molecules derived from the activated gene. Furthermore, amplification of episome copy number can dramatically increase protein expression from the activated gene. Higher levels of protein production facilitate generation of proteins for bioassay screening, cell assay screening, and manufacturing purposes.

As a result of the highly desirable characteristics described above, vectors containing a viral origin of replication and an amplifiable marker, and the use of these vectors to rapidly amplify the copy number of episomal vectors, represent a break through that extends beyond the scope of activating expression of genes present in genomic DNA. For example, these vectors can be used to over-express cDNA encoded genes to produce high levels of protein expression without the need to integrate the gene into a host cell genome with an amplifiable marker. Furthermore, like amplification of chromosomal sequences, cell possessing several hundred to several thousand episomal copies of the vector can be isolated and maintained in culture. Thus, the vectors described herein, and their uses, allow high levels of cloned genomic DNA to be propagated in mammalian cells, facilitate isolation of cDNA copies of genes present on the vector as genomic inserts, and maximize protein production from cloned cDNA and genomic copies of eukaryotic genes.

Other suitable modifications and adaptations to the methods and applications described herein will be readily apparent to one of ordinary skill in the relevant arts and may be made without departing from the scope of the invention or any embodiment thereof. Having now described the present invention in detail, the same will be more clearly understood by reference to the following examples, which are included herewith for purposes of illustration only and are not intended to be limiting of the invention.

### EXAMPLES

### Example 1: Transfection of Cells for Activation of Endogenous Gene Expression

### Method: Construction of pRIG-1

Human DHFR was amplified by PCR from cDNA produced from HT1080 cells by PCR using the primers DHFR-F 1

(5' TCCTTCGAAGCTTGTCATGGTTGGTTCGCTAAACTGCAT 3') (SEQ ID NO:1) and DHFR-R1 (5' AAACTTAAGATCGATTAATCATTC-TTCTCATATACTTCAA 3') (SEQ ID NO:2), and cloned into the T site in pTARGET™ (Promega) to create pTARGET:DHFR. The RSV promoter was isolated from PREP9 by digestion with *NheI* and *XbaI* and inserted into the *NheI* site of pTARGET:DHFR to create pTgT:RSV+DHFR. Oligonucleotides JH169 (5' ATCCACCATGGCTACAGGTGAGTACTCG 3') (SEQ ID NO:3) and JHl70 (5' GATCCGAGTACTCACCTGTAGCCATGGTGGATTTAA 3') (SEQ ID NO:4) were annealed and inserted into the I-Ppo-I and *NheI* sites of pTgT:RSV+DHFR to create pTgT:RSV+DHFR+Exl. A 279 bp region corresponding to nucleotides 230-508 of pBR322 was PCR amplified using primers Tet F1 (5' GGCGAGATCTAGCGCTATATGCGTTGATGCAAT 3') (SEQ ID NO:5) and Tet F2 (5' GGCCAGATCTGCTACCTTAAGAGAGCCG-AAACAAGCGCTCATGAGCCCGAA 3') (SEQ ID NO:6). Amplification products were digested with *BglII* and cloned into the *BamHI* site of pTgT:RSV+RSV+DHFR+Exl to create pRIG-1.

### Transfection -- Creation ofpRlG-1 Gene Activation Library in HT1080 Cells

To activate gene expression, a suitable activation construct is selected from the group of constructs described above. The selected activation construct is then introduced into cells by any transfection method known in the art. Examples of transfection methods include electroporation, lipofection, calcium phosphate precipitation, DEAE dextran, and receptor mediated endocytosis. Following introduction into the cells, the DNA is allowed to integrate into the host cell's genome via non-homologous recombination. Integration can occur at spontaneous chromosome breaks or at artificially induced chromosomal breaks.

### Method:

Transfection of human cells with pRIG1. 2x10⁹ HH1 cells, an HPRT⁻ subclone of HT1080 cells, was grown in 150 mm tissue culture plates to 90% confluency. Media was removed from the cells and saved as conditioned media (see below). Cells were removed from the plate by brief incubation with trypsin, added to media/10% fetal bovine serum to neutralize the trypsin, and pelleted at 1000 rpm in a Jouan centrifuge for 5 minutes. Cells were washed in 1X PBS, counted, and repelleted as above. The cell pellet was resuspended at 2.5 x 10⁷ cells/ml final in 1X PBS (Gibco BRL Cat #14200-075). Cells were then exposed to 50 rads ofy irradiation from a¹³⁷Cs source. pRIG1 (Figures 14A-14B; SEQ ID NO:18) was linearized with *Bam*HI*,* purified with phenol/chloroform, precipitated with ethanol, and resuspended in PBS. Purified and linearized activation construct was added to the cell suspension to produce a final concentration of 40 µg/ml. The DNA/irradiated cell mixture was then mixed and 400 µl was placed into each 0.4 cm electroporation cuvettes (Biorad). The cuvettes were pulsed at 250 Volts, 600 µFarads, 50 Ohms using an electroporation apparatus (Biorad). Following the electric pulse, the cells were incubated at room temperature for 10 minutes, and then placed into αMEM/10%FBS containing penicillin/streptomycin (GibcoBRL). The cells were then plated at approximately 7 x 10⁶ cells/150 mm plate containing 35 ml αMEM/10% FBS/penstrep (33% conditioned media/67% fresh media). Following a 24 hour incubation at 37°C, G418 (Gibco/BRL) was added to each plate to a final concentration of 500 µg/ml from a 60 mg/ml stock. After 4 days of selection, the media was replaced with fresh αMEM/10% FBS/penstrep/500 µg/ml G418. The cells were then incubated for another 7-10 days and the culture supernatant assayed for the presence of new protein factors or stored at -80°C for later analysis. The drug resistant clones can be stored in liquid nitrogen for later analysis.

### Example 2: Use of Ionizing Irradiation to Increase the Frequency and Randomness of DNA Integration

### Method:

HH1 cells were harvested at 90% confluency, washed in 1x PBS, and resuspended at a cell concentration of 7.5 x 10⁶ cells/ml in 1X PBS. 15 µg linearized DNA (pRIG-1) was added to the cells and mixed. 400 µl was added to each electroporation cuvette and pulsed at 250 Volts, 600 µFarads, 50 Ohms using an electroporation apparatus (Biorad). Following the electric pulse, the cells were incubated at room temperature for 10 minutes, and then placed into 2.5 ml αMEM/10%FBS/1X penstrep. 300 µl of cells from each shock were irradiated at 0, 50, 500, and 5000 rads immediately prior to or at either 1 hour or 4 hours post transfection. Immediately following irradiation, the cells were plated onto tissue culture plates in complete medium. At 24 hours post plating, G418 was added to the culture to a final concentration of 500 µg/ml. At 7 days post-selection, the culture medium was replaced with fresh complete medium containing 500 µg/ml G418. At 10 days post selection, medium was removed from the plate, the colonies were stained with Coomassie Blue/90% methanol/10% acetic acid and colonies with greater than 50 cells were counted.

### Example 3: Use of Restriction Enzymes to Generate Random, Semi-random, or Targeted Breaks in the Genome

### Method:

HHI cells were harvested at 90% confluence, washed in 1x PBS, and resuspended at a cell concentration of 7.5 x 10⁶ cells/ml in 1X PBS. To test the efficiency of integration, 15 µg linearized DNA (PGK-βgeo) was added to each 400 µl aliquot of cells and mixed. To several aliquots of cells, restriction enzymes *Xba*I, *Not*I, *Hind*III*, Ippo*I (10-500 units) were then added to separate cell/DNA mixture. 400 µl was added to each electroporation cuvette and pulsed at 250 Volts, 600 µFarads, 50 Ohms using an electroporation apparatus (BioRad). Following the electric pulse, the cells were incubated at room temperature for 10 minutes, and then placed into 2.5 ml αMEM/10%FBS/1X penstrep. 300 µl of 2.5 ml total cells from each shock were plated onto tissue culture plates in complete media. At 24 hours post plating, G418 was added to the culture to a final concentration of 600 µg/ml. At 7 days post-selection, the media was replaced with fresh complete media containing 600 µg/ml G418. At 10 days post selection, media was removed from the plate, the colonies were stained with Coomassie Blue/90% methanol/10% acetic acid and colonies with greater than 50 cells were counted.

### Example 4: Amplification by Selecting for Two Amplifiable Markers Located on the Integrated Vector

Following integration of the vector into the genome of a host cell, the genetic locus may be amplified in copy number by simultaneous or sequential selection for one or more amplifiable markers located on the integrated vector. For example, a vector comprising two amplifiable markers may be integrated into the genome, and expression of a given gene (*i.e.,* a gene located at the site of vector integration) can be increased by selecting for both amplifiable markers located on the vector. This approach greatly facilitates the isolation of clones of cells that have amplified the correct locus (i.e., the locus containing the integrated vector).

Once the vector has been integrated into the genome by nonhomologous recombination, individual clones of cells containing the vector integrated in a unique location may be isolated from other cells containing the vector integrated at other locations in the genome. Alternatively, mixed populations of cells may be selected for amplification.

Cells containing the integrated vector are then cultured in the presence of a first selective agent that is specific for the first amplifiable marker. This agent selects for cells that have amplified the amplifiable marker either on the vector or on the endogenous chromosome. These cells are then selected for amplification of the second selectable marker by culturing the cells in the presence of a second selective agent that is specific for the second amplifiable marker. Cells that amplified the vector and flanking genomic DNA will survive this second selective step, whereas cells that amplified the endogenous first amplifiable marker or that developed non-specific resistance will not survive. Additional selections may be performed in similar fashion when vectors containing more than two (e.g., three, four, five, or more) amplifiable markers are integrated into the cell genome, by sequential culturing of the cells in the presence of selective agents that are specific for the additional amplifiable markers contained on the integrated vector. Following selection, surviving cells are assayed for level of expression of a desired gene, and the cells expressing the highest levels are chosen for further amplification. Alternatively, pools of cells resistant to both (if two amplifiable markers are used) or all (if more than two amplifiable markers are used) of the selective agents may be further cultured without isolation of individual clones. These cells are then expanded and cultured in the presence of higher concentrations of the first selective agent (usually twofold higher). The process is repeated until the desired expression level is obtained.

Alternatively, cells containing the integrated vector may be selected simultaneously for both (if two are used) or all (if more than two are used) of the amplifiable markers. Simultaneous selection is accomplished by incorporating both selection agents (if two markers are used) or all of the selection agents (if more than two markers are used) into the selection medium in which the transfected cells are cultured. The majority of surviving cells will have amplified the integrated vector. These clones can then be screened individually to identify the cells with the highest expression level, or they can be carried as a pool. A higher concentration of each selective agent (usually twofold higher) is then applied to the cells. Surviving cells are then assayed for expression levels. This process is repeated until the desired expression levels are obtained.

By either selection strategy (*i.e*., simultaneous or sequential selection), the initial concentration of selective agent is determined independently by titrating the agent from low concentrations with no cytotoxicity to high concentrations that result in cell death in the majority of cells. In general, a concentration that gives rise to discrete colonies (e.g., several hundred colonies per 100,000 cells plated) is chosen as the initial concentration.

### Example 5: Isolation of cDNAs Encoding Transmembrane Proteins

pRIG8R1-CD2 (Figures 5A-5D; SEQ ID NO:7), pRIG8R2-CD2 (Figures 6A-6C; SEQ ID NO:8), and pRIG8R3-CD2 (Figures 7A-7C; SEQ ID NO:9) vectors contain the CMV immediate early gene promoter operably linked to an exon followed by an unpaired splice donor site. The exon on the vector encodes a signal peptide linked to the extra-cellular domain of CD2 (lacking an in frame stop codon). Each vector encodes CD2 in a different reading frame relative to the splice donor site.

To create a library of activated genes, 2 x 10⁷ cells were irradiated with 50 rads from a ¹³⁷Cs source and electroporated with 15 µg of linearized pRIG8R1-CD2 (SEQ ID NO:7). Separately, this was repeated with pRIG8R2-CD2 (SEQ ID NO:8), and again with pRIG8R3-CD2 (SEQ ID NO:9). Following transfection, the three groups of cells were combined and plated into 150 mm dishes at 5 x 10⁶ transfected cells per dish to create library #1. At 24 hours post transfection, library #1 was placed under 500 µg/ml G418 selection for 14 days. Drug resistant clones containing the vector integrated into the host cell genome were combined, aliquoted, and frozen for analysis. Library #2 was created as described above, except that 3 x 10⁷ cells, 3 x 10⁷ cells and 1 x 10⁷ cells were transfected with pRIG8R1-CD2, pRIG8R2-CD2, and pRIG8R3-CD2, respectively.

To isolate cells containing activated genes encoding integral membrane proteins, 3 x 10⁶ cells from each library were cultured and treated as follows:
- Cells were trypsinized using 4 mls of Trypsin- EDTA.
- After the cells had released, the trypsin was neutralized by addition of 8 ml of alpha MEM1/10% FBS.
- The cells were washed once with sterile PBS and collected by centrifugation at 800 x g for 7 minutes.
- The cell pellet was resuspended in 2ml of alpha MEM/10% FBS. 1 ml was used for sorting while the other 1 ml was replated in alpha MEM/10% FBS containing 500 µg/ml G-418, expanded and saved.
- The cells used for sorting were washed once with sterile alpha MEM/10% FBS and collected by centrifugation at 800 x g for 7 minutes.
- The supernatant was removed and the pellet resuspended in 1 ml of alpha MEM/10% FBS. 100 µl of these cells was removed for staining with the isotype control.
- 200 µl of Anti-CD2 FITC (Pharmingen catalog # 30054X) was added to the 900 µl of cells while 20 µl of the Mouse IgG₁ isotype control (Pharmingen catalog # 33814X) was added to the 100 µl of cells. The cells were incubated, on ice, for 20 minutes.
- To the tube that contained the cells stained with the Anti-Human CD2 FITC, 5 ml of PBS/1% FBS were added. To the isotope control, 900 µl of PBS/1% FBS were added. The cells were collected by centrifugation at 600 x g for 6 minutes.
- The supernatant from the tubes was removed. The cells that had been stained with the isotype control were resuspended in 500 µl of alpha MEM/10% FBS, and the cells that had been stained with anti-CD2- FITC were resuspended in 1.5 ml alpha MEM/10% FBS.

Cells were sorted through five sequential sorts on a FACS Vantage Flow Cytometer (Becton Dickinson Immunocytometry Systems; Mountain View, CA). In each sort, the indicated percentage of total cells, representing the most strongly fluorescent cells (see below) were collected, expanded, and resorted. HT1080 cells were sorted as a negative control. The following populations were sorted and collected in each sort:

| | **Library #1** | **Library #2** | **Library #3** |
|---|---|---|---|
| **Sort #1** | 500,000 cells collected (top 10%) | 100,000 cells collected (top 10%) | 40,000 cells collected (top 10%) |
| **Sort #2** | 300,000 cells collected (top 5%) | 220,000 cells collected (top 11%) | 14,000 cells collected (top 5%) |
| **Sort #3** | 90,000 cells collected (top 5%) | 40,000 cells collected (top 10%) | 120,000 cells collected (top 10%) |
| **Sort #4** | 600,000 cells collected (top 40%) | (a) 6,000 cells collected (top 5%); (b) 10,000 cells collected (next 5%) | 280,000 cells collected (top 13%) |
| **Sort #5** | (a) 260,000 cells collected (top 10%); (b) 530,000 cells collected (next 25%) | (a) from group (a) of sort #4, 100,000 cells collected (top 10%), and 350,000 cells collected (next 35%); (b) from group (b) of sort #4, 120,000 cells collected (top 10%) | (Not done) |

Cells from each of the final sorts for each library were expanded and stored in liquid nitrogen.

### Isolation of activated genes from FACS-sorted cells

Once cells had been sorted as described above, activated endogenous genes from the sorted cells were isolated by PCR-based cloning. One of ordinary skill will appreciate, however, that any art-known method of cloning of genes may be equivalently used to isolate activated genes from FACS-sorted cells.

Genes were isolated by the following protocol:
(1) Using PolyATract System 1000 mRNA isolation kit (Promega), mRNA was isolated from 3x10⁷ CD2+ cells (sorted 5 rounds by FACS, as described above) from libraries #1 and #2.
(2) After mRNA isolation, the concentration of mRNA was determined by diluting 0.5 µl of isolated mRNA into 99.5 µl water and measuring OD²⁶⁰. 21 µg of mRNA were recovered from the CD2+ cells.
(3) First strand cDNA synthesis was then carried out as follows:
(a) While the PCR machine was holding at 4° C, first strand reaction mixtures were set up by sequential addition of the following components:
   41 µl DEPC-treated ddH₂O
   4 µl 10mM each dNTP
   8 µl 0.1 MDTT
   16 µl 5x MMLV first strand buffer (Gibco-BRL)
   5 µl (10pmol/µl) of the consensus polyadenylation site primer GD.R1 (SEQ ID NO:10)*
   1 µl RNAsin (Promega)
   3 µl (1.25 µg/µl) mRNA.
   *Note: GD.R1, 5'TITTTTTTTTTTCGTCAGCGGCCGCATCNNNNTTT-ATT 3' (SEQ ID NO:10), is a "Gene Discovery" primer for first strand cDNA synthesis of mRNA; this primer is designed to anneal to the poly-adenylation signal AATAAA and downstream poly-A region. This primer will introduce a *Not*I site into the first strand.
   Once samples had been made up, they were incubated as follows:
(b) 70° for 1 min.
(c) 42° hold.
   2 µl of 400 U/µl SuperScript II (Gibco-BRL; Rockville, MD) was then added to each sample, to give a final total volume of 82 µl. After approximately three minutes, samples were incubated as follows:
(d) 37° for 30 min.
(e) 94° for 2 min.
(f) 4° for 5 min.
2 µl of 20 U/µl RNace-IT (Stratagene) was then added to each sample, and samples were incubated at 37° for 10 min.
(4) Following first strand synthesis, cDNA was purified using a PCR cleanup kit (Qiagen) as follows:
(a) 80 µl of the first strand reaction were transferred to a 1.7 ml siliconized eppendorf tube and adding 400 µl of PB.
(b) Samples were then transferred to a PCR clean-up column and centrifuged for two minutes at 14,000 RPM.
(c) Columns were then disassembled, flowthrough decanted, 750 of µl PE were added to pellets, and tubes were centrifuged for two minutes at 14,000 RPM.
(d) Columns were disassembled and flowthrough decanted, and tubes then centrifuged for two minutes at 14,000 RPM to dry resin.
(e) cDNA was then eluted using 50 µl of EB through transferring column to a new siliconized eppendorf tube which was then centrifuged for two minutes at 14,000 RPM.

(5) Second strand cDNA synthesis was then carried out as follows:
(a) Second strand reaction mixtures were set up at RT, through the sequential addition of the following components:

| | |
|---|---|
| ddH₂0 | 55 µl |
| 10 x PCR buffer | 10 µl |
| 50 mM MgCl₂ | 5 µl |
| 10 mM dNTPs | 2 µl |
| 25 pmol/µl RIG.751-Bio* | 4 µl |
| 25 pmol/µl GD.R2** | 4 µl |
| First strand product | 20 µl |

| | |
|---|---|
| *Note: RIG.F751-Bio, 5' Biotin-CAGATCACTAGAAGCTTTATTGCGG 3' (SEQ ID NO:11), anneals at the cap-site of the transcript expressed from pRIG vectors. **Note: GD.R2, 5' TTTTCGTCAGCGGCCGCATC 3' (SEQ ID NO:12), is a primer used to PCR amplify cDNAs generated using primer GD.R1 (SEQ ID NO:10). GD.R2 is a sub-sequence of GD.R1 with matching sequence up to the degenerate bases preceding the poly signal sequence. | |

(b) Start second strand synthesis:
94° C for 1 min;
add 1 µl *Taq* (5U/µl, Gibco-BRL);
add 1 µl Vent DNA pol (0.1U/µl, New England Biolabs).

(c) Incubate at 63° C for 2 min.
(d) Incubate at 72° C for 3 min.
(e) Repeat step (b) four times.
(f) Incubate at 72° C for 6 min.
(g) Incubate at 4° C (hold)
(h) END
(6) 200 µl of 1 mg/ml Streptavidin-Paramagnetic Particles (SA-PMP) were then prepared by washing three times with STE.
(7) The products of the second strand reaction were added directly to the SA-PMPs and incubated at RT for 30 minutes.
(8) After binding, SA-PMPs were collected through the use of the magnet, and flowthrough material recovered.
(9) Beads were washed three times with 500 µl STE.
(10) Beads were resuspended in 50 µl of STE and collected at the bottom of the tube using the magnet. STE supernatant was then carefully pipetted off.
(11) Beads were resuspended in 50 µl of ddH₂0 and placed into a 100° C water bath for two minutes, to release purified cDNA from PMPs.
(12) Purified cDNA was recovered by collecting PMPs on the magnet and carefully removing the supernatant containing the cDNA.
(13) Purified products were transferred to a clean tube and centrifuged at 14,000
RPM for two minutes to remove all of the residual PMPs.
(14) A PCR reaction was then carried out to specifically amplify RIG activated cDNAs, as follows:
(a) PCR reaction mixtures were set up at RT, through the sequential addition of the following components:

| | |
|---|---|
| H₂O | 59 µl |
| 10 x PCR buffer | 10 µl |
| 50mMMgCl₂ | 5 µl |
| 10 mM dNTPs | 2 µl |
| 25 pmol/µl RIG.F781* | 2 µl |
| 25 pmol/µl GD.R2 | 2 µl |
| second strand product | 20 µl |

| | |
|---|---|
| *Note: RIG.F781, 5' ACTCATAGGCCATAGAGGCCTATCACAG-TTAAATTGCTAACGCAG 3' (SEQ ID NO:13), anneasl downstream of GD.F1 GD.F3, GD.F5-Bio, and RIG.F751-Bio, and adds an *Sfi*I site for 5' cloning of cDNAs. This primer is used in nested PCR amplification of RIG Exonlspecific second strand cDNAs. | |

(b) Start thermal cycler:
94° C for 3 min;
add 1 µl of *Taq* (5U/µl; Gibco-BRL);
add 1 µl of 0.1U/µl Vent DNA polymerase (New England Biolabs)
PCR was then carried out by 10 cycles of steps (c) to (e):

(c) 94° C for 30 sec.
(d) 60° C for 40 sec.
(e) 72° C for 3 min.
PCR was then completed by carrying out the following steps:
(f) 94° C for 30 sec.
(g) 60° C for 40 sec.
(h) 72° C for 3 min.
(i) 72° C + 20 sec each cycle for 10 cycles
(j) 72° C for 5 min
(k) 4° C hold.
(15) After elution of library material with 50 µl EB, samples were digested by adding 10 µl of NEB Buffer 2,40 µl of dH₂O and 2 µl of *Sfi*I and digesting for 1 hour at 50° C, to cut the 5' end of the cDNA at the *Sfi*I site encoded by the forward primer (RIG.F781; SEQ ID NO:13).
(16) Following *Sfi*I digestion, 5 µl of 1M NaCl and 2 µl of *Not*I were added to each sample, and samples digested for one hour at 37° C, to cut the 3' end of the cDNA at the *NotI* site encoded by the first strand primer (GD.Rl; SEQ ID NO: 10).
(17) The digested cDNA was then separated on a 1% low melt agarose gel. cDNAs ranging in size from 1.2Kb to 8Kb were excised from the gel.
(18) cDNA was recovered from the excised agarose gel using Qiaex II Gel Extraction (Qiagen). 2 µl of cDNA (approximately 30mg) was ligated to 7µl (35ng) of pBS-HSB (linearized with *Sfi*I/*Not*I) in a total volume of 10 µl of 1X T4 ligase buffer (NEB), using 400 units of T4 DNA ligase (NEB).
(19) 0.5 µl of the ligation reaction mixture from step (18) was transformed into *E*. *coli* DH10B.
(20) 103 colonies/0.5 µl ligated DNA were recovered.
(21) These colonies were screened for exons using the primers M13F20 and JH182 (RIG Exon1 specific) through PCR in 12.5 µl volumes as follows:
(a) 100 µl of LB (with selective antibiotic) were dispensed into the appropriate number of 96-well plates.
(b) Single colonies were picked and inoculated into individual wells of the 96-well plate, and the plate placed into a 37° C incubator for 2-3 hours without shaking.
(c) A PCR reaction "master mix" was prepared on ice, as follows:

| *# of 96-Well Plates: Total # of 12.5 µl PCR rxns:* | *1 Plate 96* | *2 Plates 192* | *3 Plates 288* | *4 Plates 384* |
|---|---|---|---|---|
| *dH₂O* | *755 µl* | *1.47 ml* | *2.20 ml* | *2.94 ml* |
| *5X PCR Premix-4* | *250 µl* | *500 µl* | *750 µl* | *1.0 ml* |
| *F Primers premix (25 pmol*/*µl)* | *10 µl* | *20 µl* | *30 µl* | *40 µl* |
| *R Primers premix (25 pmol*/*µl)* | *10 µl* | *20 µl* | *30 µl* | *40 µl* |
| *Rnace-It Cocktail* | *3.2 µl* | *6.3 µl* | *9.6 µl* | *12.8 µl* |
| *Taq Polymerase (5 U*/*µl)* | *3.2 µl* | *6.3 µl* | *9. 6 µl* | *12.8 µl* |
| *Total Volume (ml)* | *1.01* | *2.02* | *3.03* | *4.04* |

(d) 10 µl of the master mix were dispensed into each well of the PCR reaction plate.
(e) 2.5 µl from each 100 µl *E. coli* culture were transferred into the corresponding wells of the PCR reaction plate.
(f) PCR was performed, using typical PCR cycle conditions of:
(i) 94° C/2min. (Bacterial lysis and plasmid denaturation)
(ii) 30 cycles of 92° C denaturation for 15 sec; 60° C primer annealing for 20 sec; and 72° C primer extension for 40 sec
(iii) 72° C final extension for 5 min.
(iv) 4° C hold.

(g) Bromophenol blue was then added to the PCR reaction; samples were mixed, centrifuged, and then the entire reaction mix was loaded onto an agarose gel.
(23) Of 200 clones screened, 78% were positive for the vector exon. 96 of these clones were grown as minipreps and purified using a Qiagen 96-well turbo-prep following the Qiagen Miniprep Handbook (April 1997).
(24) Many duplicate clones were eliminated though simultaneous digestion of 2 µl of DNA with NotI, Bam HI, XhoI, XbaI, HindIII, EcoRI in NEB Buffer 3, in a total volume of 22 µl, followed by electrophoresis on a 1% agarose gel.

### Results:

Two different cDNA libraries were screened using this protocol. In the first library (TMT#1), eight of the isolated activated genes were sequenced. Of these eight genes, four genes encoded known integral membrane proteins and six were novel genes. In the second library (TMT#2), 11 isolated activated genes were sequenced. Of these 11 genes, one gene encoded a known integral membrane protein, one gene encoded a partially sequenced gene homologous to an integral membrane protein, and nine were novel genes. In all cases where the isolated gene correspond to a characterized known gene, that gene was an integral membrane protein.

Exemplary significant alignments (obtained from GenBank) for genes isolated from each library are shown below:

### TMT#1 Significant Alignments:

179761|gb|M76559|HUMCACNLB Human neuronal DHP-sensitive
voltage-dependent, calcium channel alpha-2b subunit mRNA
complete CDs.
Length = 3600

>gi|3183974|emb|YI0183|HSMEMD H.sapiens mRNA for MEMD protein
Length = 4235

### TMT#2 Significant Alignments:

>gi|476590|gb|U06715|HSU06715 Human cytochrome B561, HCYTO B561, mRNA,
partial CDs.
Length = 2463

>gi|2184843|gb|AA459959|AA459959 zx66c01.sl Soares total fetus
Nb2HF8 9w Homo sapiens cDNA clone 796414 3' similar to
gb:J03171 INTERFERON-ALPHA RECEPTOR PRECURSOR (HUMAN);
Length = 431

### Example 6: Activation of Endogenous Genes using a Poly(A) Trap Vector

HT1080 cells (1 x 10⁷ cells) were irradiated with 50 rads using a ¹³⁷Cs source and electroporated with 15 µg linearized pRIG14 (Figures 29A-29B). Following transfection, the cells were plated into a 150 mm dish at 5 x 106 cells/dish. At 24 hours, puromycin was added to 3 µg/ml. The cells were incubated at 37° C for 12 days in the presence of 3 µg/ml puromycin. The media was replaced every 5 days. At 12 days, the number of colonies was counted, and the cells were trypsinized and replated onto a new dish. The cells were grown to 90% confluency and harvested for frozen storage and gene isolation. Typically, 1000-3000 colonies were produced per 1 x 10⁷ cells transfected.

### Example 7: Activation of Endogenous Genes Using a Dual Poly(A) Trap/SAT Vector

1x 10⁷ HH1 cells (HPRT-minus HT1080 cells) were irradiated with 50 rads using a ¹³⁷Cs source and electroporated with 15 µg linearized pRIG-22. Following transfection, the cells were plated into a 150 mm dish at 5 x 10⁶ cells/dish. At 24 hours, neomycin was added to 500 µg/ml G481. The cells were incubated at 37° C for 4 days in the presence of 500 µg/ml G418. The media was replaced with fresh media containing 500 µg/ml G418 and AgThg and grown in the presence of both drugs for an additional 7 days. Alternatively, as a control for HPRT activity, the media was replaced with fresh media containing 500 µg/ml G418 and HAT (available from Life Technologies, Inc., Rockville, MD, and used at manufacturer's recommended concentration) and grown in the presence of both drugs for an additional 7 days. At 12 days post transfection, the number of colonies was counted, and the cells were trypsinized and replated onto a new dish. The cells were grown to 90% confluency and harvested for frozen storage and gene isolation. Typically, cells subjected to G418/AgThg selection produced 1000-3000 colonies per 1 x 10¹ cells transfected. In contrast, cells subjected to G418/HAT selection produced approximated 100 colonies per 1 x 10⁷ cells transfected.

### Example 8: Isolation of activated genes

Non-targeted gene activation vectors are integrated into the genome of a eukaryotic cells using the methods of the invention. By integrating the vector into multiple cells, a library is created in which cells are expressing different vector activated genes. RNA is isolated from these cells using a commercial RNA isolation kit. In this example, RNA is isolated from cells using Poly(A) Tract 1000 (Promega). The RNA is converted into cDNA, amplified, size fractionated, and cloned into a plasmid for analysis and sequencing. A brief description of this process is presented.
(1) Place 4 ml GTC Extraction buffer (Poly(A) tract 1000 Kit- Promega) in a 15 ml polycarbonate screw cap tube and add 168 µl 2-mercaptoethanol and place in a 70° C water bath.
(2) Place 8 ml dilution buffer in a 15 ml polycarbonate screw cap tube for every pellet processed and add 168 µl 2-mercaptoethanol and place in a 70° C water bath.
(3) Remove from -80° C storage cell pellets (1 x 10⁷ - 1 x 10⁸ cells) containing non-targeted gene activation vector integrated into their genome. Pipette 4ml GTC Extraction buffer immediately onto cell pellet. Pipette up-and-down several times until the pellet is resuspended and transfer into a 15 ml snap cap polypropylene tube.
(4) Add the 8 ml dilution buffer and mix by inversion.
(5) Add 10 µl (500 pmol) of the biotinlylated oligo dT primer and mix.
(6) Let sit at 70° C for 5 minutes inverting every couple of minutes to ensure even heating.
(7) Centrifuge in a Sorvall HB-6 rotor at 7800 rpm (10k x g) at 25° C for 10 minutes. During this period of time wash 6 ml Strepavidin-Paramagnetic particles (SA-PMPs) 3x with 6 ml 0.5x SSC through use of the Poly(A) Tract system 1000 magnet.
(8) After 3 washes resuspend the SA-PMPs in 6 ml 0.5 x SSC.
(9) Pipette to remove the supernatant from the RNA prep and add to the resuspended SA-PMPs (Be careful when removing supernatant so that you do not disrupt the pellet).
(10) Let the SA-PMP/RNA mix and incubate for 2 minutes at room temperature.
(11) Capture the magnetic beads through use of the Poly(A) Tract system 1000 magnet. Note that it takes some time for all of the beads to pellet due to the high viscosity of the liquid.
(12) Pour off the supernatant and resuspend the beads in 1.7 ml of 0.5 x SSC using a 2 ml pipette and transfer to a 2 ml screw cap tube.
(13) Capture the SA-PMPs using the magnet and remove the supernatant by pipetting with a P1000.
(14) Add 1.7 ml 0.5x SSC and invert the tube several times to mix.
(15) Repeat steps 14 and 15 two more times.
(16) Resuspend the SA-PMPs in 1 ml of nuclease free water and invert several times to mix.
(17) Capture the SA-PMPs and pipette off the mRNA.
(18) Place 0.5 ml of the mRNA into each of two siliconized eppendorf tubes and add 50 µl of DEPC-treated 3M NaOAc solution and 0.55 ml of isopropanol. Invert several times to mix and place at -20° C for at least 4 hours.
(19) Centrifuge the mRNA for 10 minutes at max RPM (14 k).
(20) Carefully pipette off the supernatants and wash pellets with 200 µl 80% ethanol through re-centrifugation for 2 minutes at 14K RPM. Note that the pellets are often brown or tan in color. This color results from residual SA- PMPs.
(21) Remove wash and let pellets air dry for not more than 10 minutes at room temperature.
(22) Resuspend pellets in 5 µl each and combine into a single tube.
(23) Centrifuge at 14K RPM for 2 minutes to remove the residual SA-PMPs and carefully remove the mRNA.
(24) Determine the concentration of mRNA by diluting 0.5 µl into 99.5 µl water and measuring OD 260. Note that I OD 260 = 40 µg RNA.
(25) Set up first strand reaction for both the test sample and the negative control (HT1080) through the sequential addition of the following components while the PCR machine is holding at 4° C:

| | |
|---|---|
| Step 1: | |
| | 42 µl DEPC-treated ddH2O |
| | 4 µl 10mM each dNTP |
| | 8 µl 0.1 M DTT |
| | 16 µl 5x MMLV 1 st strand buffer |
| | 5 µl (10pmol/µl) GDR1 |
| | 1 µl RNAsin (Promega) |
| | 4 µl (1.25 µg/µl) mRNA. |
| | |
| Step 2: | 70° /1 min |
| Step 3: | 42° /hold |
| | |
| | |
| Step 4: | After 1 minute add 2 µl SUPERSCRIPT II® (Life Technologies, Inc.; Rockville, MD) and incubate at 37° C for 30 min |
| Step 5: | 94° /2 min |
| Step 6: | 4°/∞ |
| Step 7: | Add 2 µl RNase and incubate at 37° C for 10 min |
| Step 8: | 4° /∞ |

(26) Analyze 8 µl of cDNA on a 1% agarose gel to check for cDNA synthesis and purify remaining cDNA using the PCR cleanup kit from Qiagen by transferring the 70 µl first strand reaction to a 1.5 ml siliconized eppendorf tube and adding 400 µl PB.
(27) Transfer to a PCR clean-up column and centrifuge 2 minutes at max RPM.
(28) Disassemble column and pour out Flow through. Add 750 µl PE and centrifuge 2 minutes at max RPM.
(29) Disassemble column and pour out Flow throught then centrifuge 2 minutes at max RPM to dry resin.
(30) Elute using 50 µl of EB through transferring column to a new siliconized eppendorf tube and centrifuging for 2 minutes at max RPM.
(31) Second Strand cDNA synthesis set up at RT:

| | |
|---|---|
| H₂O | 8.5 µl |
| 10 X PCR buffer | 5 µl |
| 50 mM MgCl₂ | 2.5 µl |
| 10 mM dNTPs | 1 µl |
| 25 pmol/µl GDF5Bio | 10 µl |
| 25 pmol/µl GDR2 | 10 µl |
| First strand product | 15 µl |

| | |
|---|---|
| *Step 9:* | 94° C/1 min. |
| *Step 10:* | 60° C/10 min. |
| Add 0.25 µl *Taq* polymerase | |
| *Step 11:* | 60° C/2 min. |
| *Step 12:* | 72° C/10 min. |
| *Step 13:* | 94° C/1 min. |
| *Step 14:* | min go to "Step 11" four more times |
| *Step 15:* | 60° C/2 min |
| *Step 16:* | 72° C/10 min |
| *Step 17:* | END |

(32) Prepare 100 µl of SA-PMPs by washing 3 x with STE and collection using a magnet. After the final wash, resuspend the beads in 150 µl STE.
(33) Purify the products of the second strand reaction using the PCR cleanup kit from Qiagen. Elute in 50 µl EB and add he products of the second strand reaction to 150 µl of the PMPs.
(34) Mix gently at RT for 30 minutes.
(35) After binding collect SA-PMPs through use of a magnet and recover flow through material (SAVE THIS MATERIAL!)
(36) Wash the beads 3 x with 500 µl STE and 1x with NEB 2 (1x).
(37) Resuspend the beads in 100 µl NEB 2 (1x).
(38) Add 2 µl *Sfi*I and digest at 50° C for 30 minutes with gentle mixing every 10 minutes.
(39) Recover purified cDNA through use of a magnet and carefully removing the supernatant.
(40) Transfer the products to a new tube and centrifuge at maximum RPM for 2 minutes to remove all of the beads.
(41) Set up a PCR reaction to specifically amplify RAGE activated cDNAs:

| | |
|---|---|
| H₂O | 37 µl |
| 10 X PCR buffer | 10 µl |
| 10 mM dNTPs | 2 µl |
| 25 pmol/µl GDF 781 | 10 µl |
| 25 pmol/µl GDR2 | 10 µl |
| Second strand product | 25 µl |

| | |
|---|---|
| *Step 1:* | 94° C/2 min. |
| *Step 2:* | 94° C/45 sec. |
| *Step 3:* | 60° C/10 min. |
| Add 0.5 µl *Taq* Polymerase | |
| *Step 4:* | 72° C/10 min. |
| *Step 5:* | 94° C/45 sec. |
| *Step 6:* | 60° C/2 min. |
| *Step 7:* | 72° C/10 min. |
| *Step 8:* | Cycle to step 5, 8 more times |
| *Step 9:* | 94° C/45 sec. |
| *Step 10:* | 60° C/2 min. |
| *Step 11:* | 72° C/10 min. + 20 sec each cycle |
| *Stepl2:* | Cycle to step 9, 14 more times |
| *Step 13:* | 72° C/ 5 min. |
| *Step 14:* | 4° C hold |

(42) Check specificity of PCR amplification of HT1080 versus library material through analysis on a 1% agarose gel. If there is a high specificity of cDNA amplification, then use Qiagen PCR clean up kit to purify PCR products.
(43) After elution of library material with 50 µl EB add 10 µl NEB2, 40 µl dH₂O and 2 µl *Sfi*I and digest for 1 hour at 50° C.
(44) Add 5 µl of 1 M NaCl and 2 µl of *Not*I and digest for 1 hour at 37° C.
(45) Prepare and run a 1% L.M. agarose gel and run library material on gel. After visualization of material, cut out fragments ranging in size from 500bp to 10 Kb.
(46) Recover the library DNA from agarose using Qiaex II Gel Extraction Protocol (Qiagen) and elute DNA in 10 µl EB. Ligate 5 µl of this material to 4 µl pBS-HSB (*Sfi*I/*Not*I) or pBS.SNS in a total volume of 10 µl.
(47) Transform *E. coli* with 0.5 µl ligated DNA per 40 µl cells.
(48) Pick colonies, grow overnight in LB, isolate plasmids.
(49) Analyze gene activated cDNA inserts by restriction digest and DNA sequencing.

### Example 9: Isolation of Activated Genes from Subtracted cDNA Pools

Purified mRNAs from non-transfected HT1080 cells was prepared using the Poly-A Tract 1000 system (Promega), as described in Example 8 steps 1-24, and were biotinylated using EZ-LinkTM Biotin LC-ASA reagent (Pierce), as follows:
1.) 25 µl DEPC-treated dH₂O and 15 µl containing 10 µg of HT1080 mRNA was added into a siliconized microfuge tube and held on ice.
2.) Working under subdued light, 40 µl of prepared LC-ASA stock reagent (1 mg/ml in 100% ethanol) was added into the reaction tube.
3.) A UV light (365 nm wavelength) was positioned 5 cm above the microfuge tube and used to irradiate the reaction mix for 15 minutes.
4.) Unlinked biotin reagent was removed from the labeled HT1080 mRNA by passing the reaction mix through an RNase-free MicroSpin P-30 column (BioRad), as prescribed by the manufacturer.

HT1080 cells were transfected with a poly(A) trap pRIG activation vector and grown under selective media to produce a population of drug resistant colonies, as described in Example 1. Purified mRNAs were prepared from the pooled colonies using the Promega Poly-A Tract 1000 system, as described in Example 8. First strand cDNA was prepared from 5 µg of this mRNA using oligo GD.R1 (TTTTTTTTTTTTCGTCAGCGGCCGCATCNNNNTTTATT) (SEQ ID NO:10), as described in Example 8, Step 25. The reaction mix was passed through a Qiagen PCR Quick Clean-up column and the purified 1st strand cDNA was recovered in 100 µl EB.

The subtractive hybridization of biotinylated HT1080 mRNAs (subtractor population) and 1st strand cDNAs prepared from the superpool of pRIG-transfected colonies (target population) was performed as follows:
1.) 9 µg of biotinylated mRNA was added into a 0.5 ml microfuge tube containing 0.5 µg 1st strand cDNA.
2.) 1/100x volume of 10 mg/ml glycogen, 1/10x volume of 3 M sodium acetate, pH 5.5, and 2.6x volume of 100% ethanol were added into the tube and mixed.
3.) The tube was placed at -80° C for 1 hr, then spun in a refrigerated microfuge for 20 minutes.
4.) The pellet of precipitated nucleic acids was drained, washed once with 70% ethanol, then air-dried.
5.) The pellet was solvated in 5 µl HBS (50 mM HEPES, pH 7.6; 2 mM EDTA; 0.2% SDS; 500 mM NaCl) and overlayered with 5 µl light mineral oil, then heated to 95° C for 2 minutes followed by 68° C for 24 hours.
6.) The reaction mix was diluted with 100 µl HB (HBS without SDS) and extracted once with 100 µl chloroform to remove the oil.
7.) The diluted hybridization mix was added to 300 µl streptavidin-coated paramagnetic particles (Promega) which had been pre-washed 3x in 300 µl HB.
8.) The mix was incubated 10 minutes at room temperature and the SA-PMP's and bound Biotin-mRNA:DNA hybrids were removed from solution by magnetic capture.
9.) Steps 7 and 8 were repeated once.
10.) The cleared solution was subjected to one additional round of subtractive hybridization and magnetic removal of captured hybrids (Steps 1-9), with the following exceptions:
   Step 6: the hybridization reaction was diluted with 2x PCR Buffer (40 mM Tris-HCl, pH 8.4; 100 mM KCI).
   Step 7: PMPs were pre-washed in 1X PCR Buffer

The twice-subtracted 1st strand cDNA was used to generate 2nd strand cDNA by combining 45 µl of 1st strand cDNA with 7 µl dH₂O, 5 µl 50 mM MgCl₂, 2 µl premix of 10 mM each dNTP, 1 µl 10x PCR Buffer, 20 µl of 12.5 pmol/µl GD19F1-Bio (5' Biotin-CTCGTTTAGTGCGGCCGCTCAGATCACTGAATTCTGACGACCT) (SEQ ID NO:14), 20 µl of 12.5 pmol/µl GD.R2 (TTTTCGTCAGCGGCCGCATC) (SEQ ID NO:12), and 0.5 µl Taq Polymerase, with thermocycling as described in Example 8, Step 31. The second strand cDNA product was amplified and further processed for the production of an *E*. *coli*-based cDNA library, as described in Example 8, steps 32-49.

### Example 10: Selective Capture of RIG-activated Transcripts

HT1080 cells were transfected with pRIG19 activation vector (Figures 30A-30C) and cultured for 2 weeks in selective media, as described in Example 6. Total RNA was prepared from a pellet comprised of 10⁸ cells using TRIzol® Reagent (Life Technologies, Inc.; Rockville, MD) following the manufacturer's protocol, and was dissolved in 720 µl of DEPC-treated dH₂0 (dH₂O^{DEPC}). Contaminating genomic DNA was eliminated from the RNA preparation by mixing 80 µl NEB 10x Buffer 2, 8 µl Promega RNasin, and 20 µl RQ1 Promega RNase-free DNase, incubating at 37° C for 30 minutes, extracting sequentially with equal volumes of phenol:chlorofom (1:1) and chloroform, mixing with 1/10x volume sodium acetate (pH 5.5), precipitating the RNA with 2x volume of 100% ethanol, and solvating the dried RNA pellet in dH₂O^{DEPC} to a final concentration of 4.8 µg/µl.

mRNA transcripts derived from pRIG19-activated genes were selectively captured from the pool of total cellular RNAs by mixing in a 2 ml RNase-free microfuge tube 150 µl total RNA, 150 µl HBDEPC (50 mM HEPES, pH 7.6; 2 mM EDTA; 500 mM NaCl), 3 µl Promega RNasin, and 2.5 µl (25 pmol/µl) oligo GD 19.R1-Bio (see Table 1), then incubating at 70° C for 5 minutes followed by 50° C for 15 minutes. One ml of Promega streptavidin coated paramagnetic particles (SA-PMPs) was magnetically captured and washed 3x each with 1.5 ml of 0.5x SSC, and the SA-PMPs were left without being resuspended. The warm oligo:RNA hybridization reaction was added directly into the tube containing the semi-dry SA-PMPs. After incubating for 10 minutes at room temperature the SA-PMPs were washed 3x with 1ml 0.5x SSC.

**Table 1: Primer and Oligonucleotide Sequences**

| | Primer/Oligo Name | Sequence | SEQ ID NO: |
|---|---|---|---|
| Forward PCR Primers | GD19.F1-Bio | | 14 |
| | GD19.F2-Bio | | 15 |
| | GD19.F2 | | 16 |
| Reverse PCR Primers | GD.R1 | | 10 |
| | GD.R2 | TTTTCGTCAGCGGCCGCATC | 12 |
| mRNA Capture Oligo | GD19.R1-Bio | | 17 |

After the final magnetic capture, the SA-PMP's were suspended in 190 µl dH₂ODEPCand incubated at 68° C for 15 minutes. PMPs were immobilized by exposure to a magnetic and the cleared solution containing RIG-activated transcripts was transferred to a microfuge tube. 63 µl of captured RIG-activated transcript were transferred to a PCR tube where first and second strand cDNA synthesis was performed using PCR program "1+2CDNA", as follows:
- *Step1:*: 4° C/∞: Add into the PCR tube containing the RIG-activated transcripts 20 µl 5x GibcoBRL RT Buffer, 1 µl Promega RNasin, 10 µl 100 mM DTT, 5 µl dNTP premix at 10 mM each, 1 µl oligo GD.R1 (see Table 1) at 25 pmol/µl.
- *Step 2*:: 70° C/3 minutes
- *Step 3*:: 42° C/10 minutes
- *Step 4:*: Add 2.5 µl SUPERSCRIPT II® (Life Technologies, Inc.), then incubate at 37° C/1 hour
- *Step 5:*: 94° C/2 minutes
- *Step 6:*: 4° C/∞.

To the 1st strand cDNA mix, 2 µl of Stratagene RNase-It was added and the mixture was incubated at 37° C for 15 minutes. 600 µl of Qiagen PB reagent was added to the reaction, then transferred to a Qiagen PCR clean-up column and processed according to the manufacturer's protocol. cDNA was eluted from the column in 50 µl EB and transferred to a PCR tube. The second strand cDNA reaction was performed using oligos GD19.F2-Bio (Table 1) and GD.R2 (Table 1) as described in Example 9. The second strand product was captured on Promega SA-PMPs as described in Example 9, with the exception that the final suspension of SA-PMPs was in 1x NEB 4 Buffer and the captured cDNAs were cleaved from the particles using restriction endonuclease Asc I. Amplification of the second strand cDNA products using oligos GD19.F2 and GD.R2, digestion of the amplified cDNAs using endonucleases *Sfi*I and *Not*I*,* and size selection of cDNAs prior to cloning were all performed as described in Example 9. The final cDNA cleanup was achieved by eluting the cDNA pool off a Qiagen PCR Cleanup column in 30 µl EB. 11 µl of cDNA was mixed with 4 µl 5x GibcoBRL Ligase Buffer, 4 µl pGD5 vector DNA previously prepared by digestion with *Sfi*I*, Not*I*,* and CIP. 1 µl T4 DNA Ligase was added, and the reaction mix was incubated at 16° C overnight. 1 µl of ligation reaction was used to transform electro-competent *E. coli* DH10B cells, which were subsequently plated on LB agar plates containing 12.5 µg/ml chloramphenicol. Typically, 60 to 80 bacterial colonies were recovered per µl of ligation mix transformed.

### Example 11: Selective Capture of RIG-activated Transcripts

HT1080 cells were transfected with pRIG19 activation vector and cultured for 2 weeks in selective media, as described in Example 6. Total RNA was prepared from a pellet comprised of 10⁸ cells using TRIzol® Reagent (Life Technologies, Inc.) following the manufacturer's protocol, and was dissolved in 720 µl of DEPC treated dH₂0 (dH₂O^{DEPC}). Contaminating genomic DNA was eliminated from the RNA preparation by mixing 80 µl NEB 10x Buffer 2, 8 µl Promega RNasin, and 20 µl RQ1 Promega RNase-free DNase, incubating at 37° C for 30 minutes, extracting sequentially with equal volumes of phenol:chlorofom (1:1) and chloroform, mixing with 1/10x volume sodium acetate (pH 5.5), precipitating the RNA with 2x volume of 100% ethanol, and solvating the dried RNA pellet in dH2ODEPC to a final concentration of 4.8 µg/µl.

mRNA transcripts derived from pRIGl9-activated genes were selectively captured from the pool of total cellular RNAs by mixing in a 2 ml RNase-free microfuge tube 150 µl total RNA, 150 µl HBDEPC (50 mM HEPES, pH 7.6; 2 mM EDTA; 500 mM NaCl), 3 µl Promega RNasin, and 2.5 µl (25 pmol/µl) oligo GD19.R1-Bio (see Table 1), then incubating at 70° C for 5 minutes followed by 50° C for 15 minutes. One ml of Promega streptavidin coated paramagnetic particles (SA-PMPs) was magnetically captured and washed 3x each with 1.5 ml of 0.5x SSC, and the SA-PMPs were left without being resuspended. The warm oligo:RNA hybridization reaction was added directly into the tube containing the semi-dry SA-PMPs. After incubating for 10 minutes at room temperature the SA-PMPs were washed 3x with 1ml 0.5x SSC. After the final magnetic capture the SA-PMP's were suspended in 190 µl dH₂O^{DEPC} and incubated at 68° C for 15 minutes. PMPs were immobilized by exposure to a magnetic and the cleared solution containing RIG-activated transcripts was transferred to a microfuge tube. 63 µl of captured RIG-activated transcript were transferred to a PCR tube where first and second strand cDNA synthesis was performed using PCR program "1+2CDNA", as follows:
- *Step1:*: 4° C/∞: Add into the PCR tube containing the RIG-activated transcripts 20 µl 5x GibcoBRL RT Buffer, 1 µl Promega RNasin, 10 µl 100 mM DTT, 5 µl dNTP premix at 10 mM each, 1 µl oligo GD.R1 (see Table 1) at 25pmol/µl.
- *Step 2*:: 70° C/3 minutes
- *Step 3:*: 42° C/10 minutes
- *Step 4*:: Add 2.5 µl SUPERSCRIPT II® (Life Technologies, Inc.), then incubate at 37° C/1 hour
- *Step 5:*: 94° C/2 minutes
- *Step 6:*: 60° C/∞; while holding temperature, the following were added: 2 µl 50 mM MgCl₂, 1 µl oligo GD19.F1-Bio (Table 1) at 25 pmol/µl, and 2 µl Stratagene RNace-It. After 10 minutes, 0.5 µl *Taq* DNA Polymerase (Life Technologies, Inc.) was added and the cycling was continued:
- *Step 7*:: 72° C/10 minutes
- *Step 8*:: 4° C/∞.

The 100 µl volume cDNA reaction mix was transferred to a 1.5 ml siliconized microfuge tube and extracted sequentially with equal volumes of phenol:chloroform (1:1) and chloroform, and the aqueous phase was transferred to a new tube and place in speed-vac for 5 minutes at 37° C. Restriction digestion of the cDNA was performed by adding 74 µl dH₂O, 20 µl NEB 10x Buffer 2, 2 µl mg/ml BSA, 4 µl *SfiI* and incubating at 50° C for 1 hour, then adding 10 µl 1 M NaCl, 4 µl *Not*I and incubating an additional 37° C for 1 hour. The reaction mix was extracted sequentially with equal volumes of phenol:chloroform (1:1) and chloroform, then cDNAs were precipitated by adding 1/100x volume 10 mg/ml glycogen, 1/30x volume 3 M sodium acetate (pH 7.5), 2x volume 100% absolute ethanol, and freezing at -80° C for 1 hour. The cDNA pellet was washed once with 70% ethanol and air dried for 15 minutes, then solvated in 5 µl dH₂O, 1 µl 10X NEB Ligase Buffer, 4 µl pGD5 vector DNA previously prepared by digestion with *Sfi*I*, Not*I*,* and CIP. 0.5 µl T4 DNA Ligase was added, and the reaction mix was incubated at 16° C overnight. 10 µl dH₂O was added to the ligation reaction and 0.5 µl was used to transform electro-competent *E. coli* DH10B cells. Typically, 6 to 10 colonies per µl of transformed ligation mix were observed.

### Example 12: Ligation of Activation Vectors to Genomic DNA and Transfection into Human Cells

Genomic DNA was harvested from a human cell line, HT1080 (10⁸ cells), according to published procedures (Sambrook et al., Molecular Cloning, Cold Spring Harbor Laboratory Press, (1989)). The isolated genomic DNA was digested with *Bam*HI under conditions that resulted in incomplete digestion. This was accomplished by titrating the amount of *Bam*HI in the reaction. Each reaction contained 10 µg genomic DNA and *Bam*HI at a concentration of either 0.01, 0.02, 0.04, 0.08, 0.16, 0.32, 0.64, 1.28, 2.56, 5.62, or 11.24 units. After a one hour incubation at 37° C, the reactions were stopped by phenol extraction, followed by ethanol precipition. The digested DNA from each reaction was separated by agarose gel electrophoresis. Reactions containing DNA predominantly in the range of 10 kb to 400 kb were combined for ligation to the activation vector. The pooled, digested genomic DNA was then added to *Bam*HI linearized activation vector in 1X ligation buffer. Ligase (Life Technologies, Inc., 40 units) was added and the ligation reaction was incubated at 16° C for 24 hours. Following ligation, the genomic DNA/activation vector was transfected into HT1080 cells using LIPOFECTIN® (Life Technologies, Inc.) according to the manufacturer's procedures. Optionally, the HT1080 cells were irradiated prior to or after transfection. When cells were irradiated, doses in the range of 0.1 rads to 200 rads were found to be particularly useful. Following transfection, cells were grown in complete media. At 36 hours post-transfection, G418 (300 µg/ml) were added to the media. At 10-14 days post selection, the drug resistant clones were pooled, expanded, and harvested. Total RNA or mRNA was collected from the harvested cells. cDNA derived from vector activated genes was then synthesized and isolated using the methods described herein (*see,* e.g., Example 8 *supra*).

### Example 13: Co-transfections of BAC Contig Clones with the Activation Vector

Genomic libraries were created in pUniBAC (Figures 34A-34B) according to published procedures (Shizuya et al., Proc. Natl. Acad. Sci. USA 89:8794 (1992)). Typically, the size of genomic fragments can be between 1 kb and 500 kb, and preferably between 50 kb and 500 kb. The BAC library was propagated in *E. coli.* To prepare plasmids for transfection, the library was plated onto LB agar plates containing 12.5 µg/ml chloramphenicol. Approximately 1000 clones were present on each 150 mm plate. Following growth and selection, the colonies from each plate were eluted from the agar plate through the addition of LB and pooled. Each pool (~10,000 clones) was grown in 1 liter LB/12.5 µg/ml chloramphenicol overnight. BAC plasmids were then isolated from each pool using a commercial kit (Qiagen).

Purified BAC clones were digested with I-Ppo-I which cleaves a unique site in the BAC vector flanking the cloning site. Since I-Ppo-I is an ultra-rare cutter, it will not digest the vast majority of genomic DNA inserts. Following digestion, the linearized genomic library clones were cotransfected into HT1080 cells using LIPOFECTIN® (Life Technologies, Inc.) according to the manufacturer's directions. Briefly, 10 µg of BAC genomic DNA was combined with 1 µg of linearized pRIG20 (Figures 31A-31C) in α-MEM (no serum). 5 µg of LIPOFECTIN® was added to the DNA and the mixture was incubated at room temperature for 15 minutes. The DNA/LIPOFECTIN® mixture was then added to 10⁵ HT1080 cells in a 6 well dish. The cells were incubated with the DNA/LIPOFECTIN® in serum free α-MEM for 12 hours, washed, and placed in α-MEM/10%FBS for 36 hours. To select for cells that had integrated the vector and genomic DNA, the transfected cells were replated into a 10 cm dish and incubated in the presence of 300 µg/ml G418 for 10 days. Drug resistant clones were expanded and harvested to allow isolation of the activated cDNA molecules as described herein in Example 8.

### Example 14: In vitro Integration of Activation Vector into Purified Genomic DNA and Transfection of the Integration Products into Host Cells

Genomic DNA was isolated and cloned into the Bacterial Artificial Chromosome, pUniBAC (Figures 34A-34B), using published procedures (Sambrook et al., Molecular Cloning, Cold Spring Harbor Laboratory Press, (1989); Shizuya et al., Proc. Natl. Acad. Sci. USA 89:8794 (1992)). Following ligation of the genomic inserts into pUniBAC, the plasmids were transformed into the *E*. *coli* strain DH10B (Life Technologies, Inc.) and selected on tetracycline. Individual bacterial clones were combined into pools containing approximately 1000 members. Each pool was grown to saturation in 1 liter LB/tetracycline. pUniBAC plasmids containing genomic DNA inserts were isolated from the bacteria using a commercial kit (Qiagen).

For each pool of UniBAC clones, 2 µg of the library were incubated with 50 ng of the activation vector pRIG-T and 1 unit of mutant Tn5 transposase for 2 hours at 37° C (transposase available from Epicentre Technologies). Following incubation, the pUniBAC clones were transformed into DH10B cells and selected on chloramphenicol. All colonies from each pool were combined and grown in 1 liter LB/chloramphenicol. Plasmids were harvested using Qiagen Tip-500 columns according to the manufacturer's instructions.

For each pool, 20 µg of the library was transfected into 2x10⁶ HT1080 cells with 30 µg Ex-gen 500 (MBI Fermentas) according to the manufacturer's instructions. At 48 hours post-transfection, the cells were placed into media containing 3 µg/ml puromycin. After 10 days of growth in the presence of puromycin, drug resistant clones were pooled, expanded and harvested for gene discovery. To isolate vector activated genes, mRNA from each pool of cells was isolated, converted to cDNA, and cloned into plasmids as described in Example 8. Individual cDNA clones were analyzed by restriction digestion and sequencing.

### Example 15: Creation of Protein Expression Libraries from Cloned Genomic DNA

A genomic library containing genomic DNA inserts (100 kb avg. size) was created in pUniBAC as described in Examples 13 and 14. (Note: In some embodiments of the invention, the genomic fragments are cloned into the linearization site of an activation vector, wherein the activation vector is preferably a YAC, BAC, PAC, or Cosmid based vector.) In this example, the activation vector, pRIG-TP, was integrated into the BAC genomic library using *in vitro* transposition as described in Example 14. pRIG-TP is shown in Figure 36. Following integration, the library plasmids were transformed into *E. coli* and BAC vectors containing an integrated pRIG-TP vector were selected for on chloramphenicol plates. Colonies were pooled and grown to saturation in LB/Tetracycline. BAC plasmids were harvested using a commercial kit (Qiagen).

For each transfection, 20 ug of the BAC library was transfected into 2x 10⁶ HT1080 cells using 30 ug Ex-gen 500 (MBI Fermentas) according to the manufacturer's instructions. At 48 hours post transfection, the cells were placed into mdia containing 3 ug/ml puromycin. After 10 days of selection, drug resistant clones were pooled and expanded. The expaned pools of drug resistant clones were divided into separate groups for freezing, protein production, and episome amplification.

To isolate and test activated secreted proteins, culture supernatants were harvested and saved at -80° C until used in specific assays. Activated intracellular proteins were harvested from cell lysates (prepared by any method known in the art) and used in *in vitro* assays.

To amplify the copy number of the BAC episomes, the cells were selected with increasing concentrations of methotrexate. In these experiments, the initial methotrexate concentration was 20 nM. Methotrexate concentrations were doubled every 7 days until cells resistant to 5 µM were obtained. At each methotrexate concentration, a portion of cells were removed for storage and protein production. Activated secreted and intracellular proteins were harvested from these cells as described for the non-methotrexate selected cells.

Having now fully described the present invention in some detail by way of illustration and example for purposes of clarity of understanding, it will be obvious to one of ordinary skill in the art that the same can be performed by modifying or changing the invention within a wide and equivalent range of conditions, formulations and other parameters without affecting the scope of the invention or any specific embodiment thereof, and that such modifications or changes are intended to be encompassed within the scope of the appended claims.

All publications, patents and patent applications mentioned in this specification are indicative of the level of skill of those skilled in the art to which this invention pertains.

### SEQUENCE LISTING

<110> Harrington, John J.
   Sherf, Bruce
   Rundlett, Stephen
<120> Compositions and Methods for
   Non-targeted Activation of Endogenous Genes
<130> 5817-7-1 PC
<140> PCT/US00/04429
   <141> 2000-02-22
<150> 09/253,022
   <151> 1999-02-19
<150> 09/263,814
   <151> 1999-03-08
<150> 09/276,820
   <151> 1999-03-26
<160> 30
<170> FastSEQ for Windows Version 3.0
<210> 1
   <211> 39
   <212> DNA
   <213> Artificial Sequence
<220>
   <223> oligonucleotide primer
<400> 1
   tccttcgaag cttgtcatgg ttggttcgct aaactgcat 39
<210> 2
   <211> 40
   <212> DNA
   <213> Artificial Sequence
<220>
   <223> oligonucleotide primer
<400> 2
   aaacttaaga tcgattaatc attcttctca tatacttcaa 40
<210> 3
   <211> 28
   <212> DNA
   <213> Artificial Sequence
<220>
   <223> oligonucleotide primer
<400> 3
   atccaccatg gctacaggtg agtactcg 28
<210> 4
   <211> 36
   <212> DNA
   <213> Artificial Sequence
<220>
   <223> oligonucleotide primer
<400> 4
   gatccgagta ctcacctgta gccatggtgg atttaa 36
<210> 5
   <211> 33
   <212> DNA
   <213> Artificial Sequence
<220>
   <223> oligonucleotide primer
<400> 5
   ggcgagatct agcgctatat gcgttgatgc aat 33
<210> 6
   <211> 51
   <212> DNA
   <213> Artificial Sequence
<220>
   <223> oligonucleotide primer
<400> 6
   ggccagatct gctaccttaa gagagccgaa acaagcgctc atgagcccga a 51
<210> 7
   <211> 6084
   <212> DNA
   <213> Artificial Sequence
<220>
   <223> pRIG8Rl-CD2 plasmid
<400> 7
   agatcttcaa tattggccat tagccatatt attcattggt tatatagcat aaatcaatat 60
   tggctattgg ccattgcata cgttgtatct atatcataat atgtacattt atattggctc 120
   atgtccaata tgaccgccat gttggcattg attattgact agttattaat agtaatcaat 180
   tacggggtca ttagttcata gcccatatat ggagttccgc gttacataac ttacggtaaa 240
   tggcccgcct ggctgaccgc ccaacgaccc ccgcccattg acgtcaataa tgacgtatgt 300
   tcccatagta acgccaatag ggactttcca ttgacgtcaa tgggtggagt atttacggta 360
   aactgcccac ttggcagtac atcaagtgta tcatatgcca agtccgcccc ctattgacgt 420
   caatgacggt aaatggcccg cctggcatta tgcccagtac atgaccttac gggactttcc 480
   tacttggcag tacatctacg tattagtcat cgctattacc atggtgatgc ggttttggca 540
   gtacaccaat gggcgtggat agcggtttga ctcacgggga tttccaagtc tccaccccat 600
   tgacgtcaat gggagtttgt tttggcacca aaatcaacgg gactttccaa aatgtcgtaa 660
   caactgcgat cgcccgcccc gttgacgcaa atgggcggta ggcgtgtacg gtgggaggtc 720
   tatataagca gagctcgttt agtgaaccgt cagatcacta gaagctttat tgcggtagtt 780
   tatcacagtt aaattgctaa cgcagtcagt gcttctgaca caacagtctc gaacttaagc 840
   tgcagtgact ctcttaatta actccaccag tctcacttca gttccttttg cctccaccag 900
   tctcacttca gttccttttg catgaagagc tcagaatcaa aagaggaaac caacccctaa 960
   gatgagcttt ccatgtaaat ttgtagccag cttccttctg attttcaatg tttcttccaa 1020
   aggtgcagtc tccaaagaga ttacgaatgc cttggaaacc tggggtgcct tgggtcagga 1080
   catcaacttg gacattccta gttttcaaat gagtgatgat attgacgata taaaatggga 1140
   aaaaacttca gacaagaaaa agattgcaca attcagaaaa gagaaagaga ctttcaagga 1200
   aaaagataca tataagctat ttaaaaatgg aactctgaaa attaagcatc tgaagaccga 1260
   tgatcaggat atctacaagg tatcaatata tgatacaaaa ggaaaaaatg tgttggaaaa 1320
   aatatttgat ttgaagattc aagagagggt ctcaaaacca aagatctcct ggacttgtat 1380
   caacacaacc ctgacctgtg aggtaatgaa tggaactgac cccgaattaa acctgtatca 1440
   agatgggaaa catctaaaac tttctcagag ggtcatcaca cacaagtgga ccaccagcct 1500
   gagtgcaaaa ttcaagtgca cagcagggaa caaagtcagc aaggaatcca gtgtcgagcc 1560
   tgtcagctgt ccagagaaag ggatccaggt gagtagggcc cgatccttct agagtcgagc 1620
   tctcttaagg tagcaaggtt acaagacagg tttaaggaga ccaatagaaa ctgggcttgt 1680
   cgagacagag aagactcttg cgtttctgat aggcacctat tggtcttacg cggccgcgaa 1740
   ttccaagctt gagtattcta tcgtgtcacc taaataactt ggcgtaatca tggtcatatc 1800
   tgtttcctgt gtgaaattgt tatccgctca caattccaca caacatacga gccggaagca 1860
   taaagtgtaa agcctggggt gcctaatgag tgagctaact cacattaatt gcgttgcgcg 1920
   atgcttccat tttgtgaggg ttaatgcttc gagaagacat gataagatac attgatgagt 1980
   ttggacaaac cacaacaaga atgcagtgaa aaaaatgctt tatttgtgaa atttgtgatg 2040
   ctattgcttt atttgtaacc attataagct gcaataaaca agttaacaac aacaattgca 2100
   ttcattttat gtttcaggtt cagggggaga tgtgggaggt tttttaaagc aagtaaaacc 2160
   tctacaaatg tggtaaaatc cgataaggat cgattccgga gcctgaatgg cgaatggacg 2220
   cgccctgtag cggcgcatta agcgcggcgg gtgtggtggt tacgcgcacg tgaccgctac 2280
   acttgccagc gccctagcgc ccgctccttt cgctttcttc ccttcctttc tcgccacgtt 2340
   cgccggcttt ccccgtcaag ctctaaatcg ggggctccct ttagggttcc gatttagtgc 2400
   tttacggcac ctcgacccca aaaaacttga ttagggtgat ggttcacgta gtgggccatc 2460
   gccctgatag acggtttttc gccctttgac gttggagtcc acgttcttta atagtggact 2520
   cttgttccaa actggaacaa cactcaaccc tatctcggtc tattcttttg atttataagg 2580
   gattttgccg atttcggcct attggttaaa aaatgagctg atttaacaaa aatttaacgc 2640
   gaattttaac aaaatattaa cgcttacaat ttcgcctgtg taccttctga ggcggaaaga 2700
   accagctgtg gaatgtgtgt cagttagggt gtggaaagtc cccaggctcc ccagcaggca 2760
   gaagtatgca aagcatgcat ctcaattagt cagcaaccag gtgtggaaag tccccaggct 2820
   ccccagcagg cagaagtatg caaagcatgc atctcaatta gtcagcaacc atagtcccgc 2880
   ccctaactcc gcccatcccg cccctaactc cgcccagttc cgcccattct ccgccccatg 2940
   gctgactaat tttttttatt tatgcagagg ccgaggccgc ctcggcctct gagctattcc 3000
   agaagtagtg aggaggcttt tttggaggcc taggcttttg caaaaagctt gattcttctg 3060
   acacaacagt ctcgaactta aggctagagc caccatgatt gaacaagatg gattgcacgc 3120
   aggttctccg gccgcttggg tggagaggct attcggctat gactgggcac aacagacaat 3180
   cggctgctct gatgccgccg tgttccggct gtcagcgcag gggcgcccgg ttctttttgt 3240
   caagaccgac ctgtccggtg ccctgaatga actgcaggac gaggcagcgc ggctatcgtg 3300
   gctggccacg acgggcgttc cttgcgcagc tgtgctcgac gttgtcactg aagcgggaag 3360
   ggactggctg ctattgggcg aagtgccggg gcaggatctc ctgtcatctc accttgctcc 3420
   tgccgagaaa gtatccatca tggctgatgc aatgcggcgg ctgcatacgc ttgatccggc 3480
   tacctgccca ttcgaccacc aagcgaaaca tcgcatcgag cgagcacgta ctcggatgga 3540
   agccggtctt gtcgatcagg atgatctgga cgaagagcat caggggctcg cgccagccga 3600
   actgttcgcc aggctcaagg cgcgcatgcc cgacggcgag gatctcgtcg tgacccatgg 3660
   cgatgcctgc ttgccgaata tcatggtgga aaatggccgc ttttctggat tcatcgactg 3720
   tggccggctg ggtgtggcgg accgctatca ggacatagcg ttggctaccc gtgatattgc 3780
   tgaagagctt ggcggcgaat gggctgaccg cttcctcgtg ctttacggta tcgccgctcc 3840
   cgattcgcag cgcatcgcct tctatcgcct tcttgacgag ttcttctgag cgggactctg 3900
   gggttcgaaa tgaccgacca agcgacgccc aacctgccat cacgatggcc gcaataaaat 3960
   atctttattt tcattacatc tgtgtgttgg ttttttgtgt gaagatccgc gtatggtgca 4020
   ctctcagtac aatctgctct gatgccgcat agttaagcca gccccgacac ccgccaacac 4080
   ccgctgacgc gccctgacgg gcttgtctgc tcccggcatc cgcttacaga caagctgtga 4140
   ccgtctccgg gagctgcatg tgtcagaggt tttcaccgtc atcaccgaaa cgcgcgagac 4200
   gaaagggcct cgtgatacgc ctatttttat aggttaatgt catgataata atggtttctt 4260
   agacgtcagg tggcactttt cggggaaatg tgcgcggaac ccctatttgt ttatttttct 4320
   aaatacattc aaatatgtat ccgctcatga gacaataacc ctgataaatg cttcaataat 4380
   attgaaaaag gaagagtatg agtattcaac atttccgtgt cgcccttatt cccttttttg 4440
   cggcattttg ccttcctgtt tttgctcacc cagaaacgct ggtgaaagta aaagatgctg 4500
   aagatcagtt gggtgcacga gtgggttaca tcgaactgga tctcaacagc ggtaagatcc 4560
   ttgagagttt tcgccccgaa gaacgttttc caatgatgag cacttttaaa gttctgctat 4620
   gtggcgcggt attatcccgt attgacgccg ggcaagagca actcggtcgc cgcatacact 4680
   attctcagaa tgacttggtt gagtactcac cagtcacaga aaagcatctt acggatggca 4740
   tgacagtaag agaattatgc agtgctgcca taaccatgag tgataacact gcggccaact   4800
   tacttctgac aacgatcgga ggaccgaagg agctaaccgc ttttttgcac aacatggggg   4860
   atcatgtaac tcgccttgat cgttgggaac cggagctgaa tgaagccata ccaaacgacg   4920
   agcgtgacac cacgatgcct gtagcaatgg caacaacgtt gcgcaaacta ttaactggcg   4980
   aactacttac tctagcttcc cggcaacaat taatagactg gatggaggcg gataaagttg   5040
   caggaccact tctgcgctcg gcccttccgg ctggctggtt tattgctgat aaatctggag   5100
   ccggtgagcg tgggtctcgc ggtatcattg cagcactggg gccagatggt aagccctccc   5160
   gtatcgtagt tatctacacg acggggagtc aggcaactat ggatgaacga aatagacaga   5220
   tcgctgagat aggtgcctca ctgattaagc attggtaact gtcagaccaa gtttactcat   5280
   atatacttta gattgattta aaacttcatt tttaatttaa aaggatctag gtgaagatcc   5340
   tttttgataa tctcatgacc aaaatccctt aacgtgagtt ttcgttccac tgagcgtcag   5400
   accccgtaga aaagatcaaa ggatcttctt gagatccttt ttttctgcgc gtaatctgct   5460
   gcttgcaaac aaaaaaacca ccgctaccag cggtggtttg tttgccggat caagagctac   5520
   caactctttt tccgaaggta actggcttca gcagagcgca gataccaaat actgtccttc   5580
   tagtgtagcc gtagttaggc caccacttca agaactctgt agcaccgcct acatacctcg   5640
   ctctgctaat cctgttacca gtggctgctg ccagtggcga taagtcgtgt cttaccgggt   5700
   tggactcaag acgatagtta ccggataagg cgcagcggtc gggctgaacg gggggttcgt   5760
   gcacacagcc cagcttggag cgaacgacct acaccgaact gagataccta cagcgtgagc   5820
   tatgagaaag cgccacgctt cccgaaggga gaaaggcgga caggtatccg gtaagcggca   5880
   gggtcggaac aggagagcgc acgagggagc ttccaggggg aaacgcctgg tatctttata   5940
   gtcctgtcgg gtttcgccac ctctgacttg agcgtcgatt tttgtgatgc tcgtcagggg   6000
   ggcggagcct atggaaaaac gccagcaacg cggccttttt acggttcctg gccttttgct   6060
   ggccttttgc tcacatggct cgac                                           6084
<210> 8
   <211> 6085
   <212> DNA
   <213> Artificial Sequence
<220>
   <223> pRIG8R2-CD2
<400> 8
   agatcttcaa tattggccat tagccatatt attcattggt tatatagcat aaatcaatat     60
   tggctattgg ccattgcata cgttgtatct atatcataat atgtacattt atattggctc    120
   atgtccaata tgaccgccat gttggcattg attattgact agttattaat agtaatcaat    180
   tacggggtca ttagttcata gcccatatat ggagttccgc gttacataac ttacggtaaa    240
   tggcccgcct ggctgaccgc ccaacgaccc ccgcccattg acgtcaataa tgacgtatgt    300
   tcccatagta acgccaatag ggactttcca ttgacgtcaa tgggtggagt atttacggta    360
   aactgcccac ttggcagtac atcaagtgta tcatatgcca agtccgcccc ctattgacgt    420
   caatgacggt aaatggcccg cctggcatta tgcccagtac atgaccttac gggactttcc    480
   tacttggcag tacatctacg tattagtcat cgctattacc atggtgatgc ggttttggca    540
   gtacaccaat gggcgtggat agcggtttga ctcacgggga tttccaagtc tccaccccat    600
   tgacgtcaat gggagtttgt tttggcacca aaatcaacgg gactttccaa aatgtcgtaa    660
   caactgcgat cgcccgcccc gttgacgcaa atgggcggta ggcgtgtacg gtgggaggtc    720
   tatataagca gagctcgttt agtgaaccgt cagatcacta gaagctttat tgcggtagtt    780
   tatcacagtt aaattgctaa cgcagtcagt gcttctgaca caacagtctc gaacttaagc    840
   tgcagtgact ctcttaatta actccaccag tctcacttca gttccttttg cctccaccag    900
   tctcacttca gttccttttg catgaagagc tcagaatcaa aagaggaaac caacccctaa    960
   gatgagcttt ccatgtaaat ttgtagccag cttccttctg attttcaatg tttcttccaa   1020
   aggtgcagtc tccaaagaga ttacgaatgc cttggaaacc tggggtgcct tgggtcagga   1080
   catcaacttg gacattccta gttttcaaat gagtgatgat attgacgata taaaatggga   1140
   aaaaacttca gacaagaaaa agattgcaca attcagaaaa gagaaagaga ctttcaagga   1200
   aaaagataca tataagctat ttaaaaatgg aactctgaaa attaagcatc tgaagaccga   1260
   tgatcaggat atctacaagg tatcaatata tgatacaaaa ggaaaaaatg tgttggaaaa   1320
   aatatttgat ttgaagattc aagagagggt ctcaaaacca aagatctcct ggacttgtat   1380
   caacacaacc ctgacctgtg aggtaatgaa tggaactgac cccgaattaa acctgtatca   1440
   agatgggaaa catctaaaac tttctcagag ggtcatcaca cacaagtgga ccaccagcct   1500
   gagtgcaaaa ttcaagtgca cagcagggaa caaagtcagc aaggaatcca gtgtcgagcc   1560
   tgtcagctgt ccagagaaag ggatcccagg tgagtagggc ccgatccttc tagagtcgag 1620
   ctctcttaag gtagcaaggt tacaagacag gtttaaggag accaatagaa actgggcttg 1680
   tcgagacaga gaagactctt gcgtttctga taggcaccta ttggtcttac gcggccgcga 1740
   attccaagct tgagtattct atcgtgtcac ctaaataact tggcgtaatc atggtcatat 1800
   ctgtttcctg tgtgaaattg ttatccgctc acaattccac acaacatacg agccggaagc 1860
   ataaagtgta aagcctgggg tgcctaatga gtgagctaac tcacattaat tgcgttgcgc 1920
   gatgcttcca ttttgtgagg gttaatgctt cgagaagaca tgataagata cattgatgag 1980
   tttggacaaa ccacaacaag aatgcagtga aaaaaatgct ttatttgtga aatttgtgat 2040
   gctattgctt tatttgtaac cattataagc tgcaataaac aagttaacaa caacaattgc 2100
   attcatttta tgtttcaggt tcagggggag atgtgggagg ttttttaaag caagtaaaac 2160
   ctctacaaat gtggtaaaat ccgataagga tcgattccgg agcctgaatg gcgaatggac 2220
   gcgccctgta gcggcgcatt aagcgcggcg ggtgtggtgg ttacgcgcac gtgaccgcta 2280
   cacttgccag cgccctagcg cccgctcctt tcgctttctt cccttccttt ctcgccacgt 2340
   tcgccggctt tccccgtcaa gctctaaatc gggggctccc tttagggttc cgatttagtg 2400
   ctttacggca cctcgacccc aaaaaacttg attagggtga tggttcacgt agtgggccat 2460
   cgccctgata gacggttttt cgccctttga cgttggagtc cacgttcttt aatagtggac 2520
   tcttgttcca aactggaaca acactcaacc ctatctcggt ctattctttt gatttataag 2580
   ggattttgcc gatttcggcc tattggttaa aaaatgagct gatttaacaa aaatttaacg 2640
   cgaattttaa caaaatatta acgcttacaa tttcgcctgt gtaccttctg aggcggaaag 2700
   aaccagctgt ggaatgtgtg tcagttaggg tgtggaaagt ccccaggctc cccagcaggc 2760
   agaagtatgc aaagcatgca tctcaattag tcagcaacca ggtgtggaaa gtccccaggc 2820
   tccccagcag gcagaagtat gcaaagcatg catctcaatt agtcagcaac catagtcccg 2880
   cccctaactc cgcccatccc gcccctaact ccgcccagtt ccgcccattc tccgccccat 2940
   ggctgactaa ttttttttat ttatgcagag gccgaggccg cctcggcctc tgagctattc 3000
   cagaagtagt gaggaggctt ttttggaggc ctaggctttt gcaaaaagct tgattcttct 3060
   gacacaacag tctcgaactt aaggctagag ccaccatgat tgaacaagat ggattgcacg 3120
   caggttctcc ggccgcttgg gtggagaggc tattcggcta tgactgggca caacagacaa 3180
   tcggctgctc tgatgccgcc gtgttccggc tgtcagcgca ggggcgcccg gttctttttg 3240
   tcaagaccga cctgtccggt gccctgaatg aactgcagga cgaggcagcg cggctatcgt 3300
   ggctggccac gacgggcgtt ccttgcgcag ctgtgctcga cgttgtcact gaagcgggaa 3360
   gggactggct gctattgggc gaagtgccgg ggcaggatct cctgtcatct caccttgctc 3420
   ctgccgagaa agtatccatc atggctgatg caatgcggcg gctgcatacg cttgatccgg 3480
   ctacctgccc attcgaccac caagcgaaac atcgcatcga gcgagcacgt actcggatgg 3540
   aagccggtct tgtcgatcag gatgatctgg acgaagagca tcaggggctc gcgccagccg 3600
   aactgttcgc caggctcaag gcgcgcatgc ccgacggcga ggatctcgtc gtgacccatg 3660
   gcgatgcctg cttgccgaat atcatggtgg aaaatggccg cttttctgga ttcatcgact 3720
   gtggccggct gggtgtggcg gaccgctatc aggacatagc gttggctacc cgtgatattg 3780
   ctgaagagct tggcggcgaa tgggctgacc gcttcctcgt gctttacggt atcgccgctc 3840
   ccgattcgca gcgcatcgcc ttctatcgcc ttcttgacga gttcttctga gcgggactct 3900
   ggggttcgaa atgaccgacc aagcgacgcc caacctgcca tcacgatggc cgcaataaaa 3960
   tatctttatt ttcattacat ctgtgtgttg gttttttgtg tgaagatccg cgtatggtgc 4020
   actctcagta caatctgctc tgatgccgca tagttaagcc agccccgaca cccgccaaca 4080
   cccgctgacg cgccctgacg ggcttgtctg ctcccggcat ccgcttacag acaagctgtg 4140
   accgtctccg ggagctgcat gtgtcagagg ttttcaccgt catcaccgaa acgcgcgaga 4200
   cgaaagggcc tcgtgatacg cctattttta taggttaatg tcatgataat aatggtttct 4260
   tagacgtcag gtggcacttt tcggggaaat gtgcgcggaa cccctatttg tttatttttc 4320
   taaatacatt caaatatgta tccgctcatg agacaataac cctgataaat gcttcaataa 4380
   tattgaaaaa ggaagagtat gagtattcaa catttccgtg tcgcccttat tccctttttt 4440
   gcggcatttt gccttcctgt ttttgctcac ccagaaacgc tggtgaaagt aaaagatgct 4500
   gaagatcagt tgggtgcacg agtgggttac atcgaactgg atctcaacag cggtaagatc 4560
   cttgagagtt ttcgccccga agaacgtttt ccaatgatga gcacttttaa agttctgcta 4620
   tgtggcgcgg tattatcccg tattgacgcc gggcaagagc aactcggtcg ccgcatacac 4680
   tattctcaga atgacttggt tgagtactca ccagtcacag aaaagcatct tacggatggc 4740
   atgacagtaa gagaattatg cagtgctgcc ataaccatga gtgataacac tgcggccaac 4800
   ttacttctga caacgatcgg aggaccgaag gagctaaccg cttttttgca caacatgggg 4860
   gatcatgtaa ctcgccttga tcgttgggaa ccggagctga atgaagccat accaaacgac 4920
   gagcgtgaca ccacgatgcc tgtagcaatg gcaacaacgt tgcgcaaact attaactggc 4980
   gaactactta ctctagcttc ccggcaacaa ttaatagact ggatggaggc ggataaagtt 5040
   gcaggaccac ttctgcgctc ggcccttccg gctggctggt ttattgctga taaatctgga 5100
   gccggtgagc gtgggtctcg cggtatcatt gcagcactgg ggccagatgg taagccctcc 5160
   cgtatcgtag ttatctacac gacggggagt caggcaacta tggatgaacg aaatagacag 5220
   atcgctgaga taggtgcctc actgattaag cattggtaac tgtcagacca agtttactca   5280
   tatatacttt agattgattt aaaacttcat ttttaattta aaaggatcta ggtgaagatc   5340
   ctttttgata atctcatgac caaaatccct taacgtgagt tttcgttcca ctgagcgtca  5400
   gaccccgtag aaaagatcaa aggatcttct tgagatcctt tttttctgcg cgtaatctgc   5460
   tgcttgcaaa caaaaaaacc accgctacca gcggtggttt gtttgccgga tcaagagcta   5520
   ccaactcttt ttccgaaggt aactggcttc agcagagcgc agataccaaa tactgtcctt   5580
   ctagtgtagc cgtagttagg ccaccacttc aagaactctg tagcaccgcc tacatacctc   5640
   gctctgctaa tcctgttacc agtggctgct gccagtggcg ataagtcgtg tcttaccggg   5700
   ttggactcaa gacgatagtt accggataag gcgcagcggt cgggctgaac ggggggttcg   5760
   tgcacacagc ccagcttgga gcgaacgacc tacaccgaac tgagatacct acagcgtgag   5820
   ctatgagaaa gcgccacgct tcccgaaggg agaaaggcgg acaggtatcc ggtaagcggc   5880
   agggtcggaa caggagagcg cacgagggag cttccagggg gaaacgcctg gtatctttat   5940
   agtcctgtcg ggtttcgcca cctctgactt gagcgtcgat ttttgtgatg ctcgtcaggg   6000
   gggcggagcc tatggaaaaa cgccagcaac gcggcctttt tacggttcct ggccttttgc   6060
   tggccttttg ctcacatggc tcgac                                          6085
<210> 9
   <211> 6086
   <212> DNA
   <213> Artificial Sequence
<220>
   <223> pRIG8R3-CD2
<400> 9
   agatcttcaa tattggccat tagccatatt attcattggt tatatagcat aaatcaatat     60
   tggctattgg ccattgcata cgttgtatct atatcataat atgtacattt atattggctc    120
   atgtccaata tgaccgccat gttggcattg attattgact agttattaat agtaatcaat    180
   tacggggtca ttagttcata gcccatatat ggagttccgc gttacataac ttacggtaaa    240
   tggcccgcct ggctgaccgc ccaacgaccc ccgcccattg acgtcaataa tgacgtatgt    300
   tcccatagta acgccaatag ggactttcca ttgacgtcaa tgggtggagt atttacggta    360
   aactgcccac ttggcagtac atcaagtgta tcatatgcca agtccgcccc ctattgacgt    420
   caatgacggt aaatggcccg cctggcatta tgcccagtac atgaccttac gggactttcc    480
   tacttggcag tacatctacg tattagtcat cgctattacc atggtgatgc ggttttggca    540
   gtacaccaat gggcgtggat agcggtttga ctcacgggga tttccaagtc tccaccccat    600
   tgacgtcaat gggagtttgt tttggcacca aaatcaacgg gactttccaa aatgtcgtaa    660
   caactgcgat cgcccgcccc gttgacgcaa atgggcggta ggcgtgtacg gtgggaggtc    720
   tatataagca gagctcgttt agtgaaccgt cagatcacta gaagctttat tgcggtagtt    780
   tatcacagtt aaattgctaa cgcagtcagt gcttctgaca caacagtctc gaacttaagc    840
   tgcagtgact ctcttaatta actccaccag tctcacttca gttccttttg cctccaccag    900
   tctcacttca gttccttttg catgaagagc tcagaatcaa aagaggaaac caacccctaa    960
   gatgagcttt ccatgtaaat ttgtagccag cttccttctg attttcaatg tttcttccaa   1020
   aggtgcagtc tccaaagaga ttacgaatgc cttggaaacc tggggtgcct tgggtcagga   1080
   catcaacttg gacattccta gttttcaaat gagtgatgat attgacgata taaaatggga   1140
   aaaaacttca gacaagaaaa agattgcaca attcagaaaa gagaaagaga ctttcaagga   1200
   aaaagataca tataagctat ttaaaaatgg aactctgaaa attaagcatc tgaagaccga   1260
   tgatcaggat atctacaagg tatcaatata tgatacaaaa ggaaaaaatg tgttggaaaa   1320
   aatatttgat ttgaagattc aagagagggt ctcaaaacca aagatctcct ggacttgtat   1380
   caacacaacc ctgacctgtg aggtaatgaa tggaactgac cccgaattaa acctgtatca   1440
   agatgggaaa catctaaaac tttctcagag ggtcatcaca cacaagtgga ccaccagcct   1500
   gagtgcaaaa ttcaagtgca cagcagggaa caaagtcagc aaggaatcca gtgtcgagcc   1560
   tgtcagctgt ccagagaaag ggatccacag gtgagtaggg cccgatcctt ctagagtcga   1620
   gctctcttaa ggtagcaagg ttacaagaca ggtttaagga gaccaataga aactgggctt   1680
   gtcgagacag agaagactct tgcgtttctg ataggcacct attggtctta cgcggccgcg   1740
   aattccaagc ttgagtattc tatcgtgtca cctaaataac ttggcgtaat catggtcata   1800
   tctgtttcct gtgtgaaatt gttatccgct cacaattcca cacaacatac gagccggaag   1860
   cataaagtgt aaagcctggg gtgcctaatg agtgagctaa ctcacattaa ttgcgttgcg   1920
   cgatgcttcc attttgtgag ggttaatgct tcgagaagac atgataagat acattgatga   1980
   gtttggacaa accacaacaa gaatgcagtg aaaaaaatgc tttatttgtg aaatttgtga   2040
   tgctattgct ttatttgtaa ccattataag ctgcaataaa caagttaaca acaacaattg   2100
   cattcatttt atgtttcagg ttcaggggga gatgtgggag gttttttaaa gcaagtaaaa   2160
   cctctacaaa tgtggtaaaa tccgataagg atcgattccg gagcctgaat ggcgaatgga   2220
   cgcgccctgt agcggcgcat taagcgcggc gggtgtggtg gttacgcgca cgtgaccgct   2280
   acacttgcca gcgccctagc gcccgctcct ttcgctttct tcccttcctt tctcgccacg   2340
   ttcgccggct ttccccgtca agctctaaat cgggggctcc ctttagggtt ccgatttagt   2400
   gctttacggc acctcgaccc caaaaaactt gattagggtg atggttcacg tagtgggcca   2460
   tcgccctgat agacggtttt tcgccctttg acgttggagt ccacgttctt taatagtgga   2520
   ctcttgttcc aaactggaac aacactcaac cctatctcgg tctattcttt tgatttataa   2580
   gggattttgc cgatttcggc ctattggtta aaaaatgagc tgatttaaca aaaatttaac   2640
   gcgaatttta acaaaatatt aacgcttaca atttcgcctg tgtaccttct gaggcggaaa   2700
   gaaccagctg tggaatgtgt gtcagttagg gtgtggaaag tccccaggct ccccagcagg   2760
   cagaagtatg caaagcatgc atctcaatta gtcagcaacc aggtgtggaa agtccccagg   2820
   ctccccagca ggcagaagta tgcaaagcat gcatctcaat tagtcagcaa ccatagtccc   2880
   gcccctaact ccgcccatcc cgcccctaac tccgcccagt tccgcccatt ctccgcccca   2940
   tggctgacta atttttttta tttatgcaga ggccgaggcc gcctcggcct ctgagctatt   3000
   ccagaagtag tgaggaggct tttttggagg cctaggcttt tgcaaaaagc ttgattcttc   3060
   tgacacaaca gtctcgaact taaggctaga gccaccatga ttgaacaaga tggattgcac   3120
   gcaggttctc cggccgcttg ggtggagagg ctattcggct atgactgggc acaacagaca   3180
   atcggctgct ctgatgccgc cgtgttccgg ctgtcagcgc aggggcgccc ggttcttttt   3240
   gtcaagaccg acctgtccgg tgccctgaat gaactgcagg acgaggcagc gcggctatcg   3300
   tggctggcca cgacgggcgt tccttgcgca gctgtgctcg acgttgtcac tgaagcggga   3360
   agggactggc tgctattggg cgaagtgccg gggcaggatc tcctgtcatc tcaccttgct   3420
   cctgccgaga aagtatccat catggctgat gcaatgcggc ggctgcatac gcttgatccg   3480
   gctacctgcc cattcgacca ccaagcgaaa catcgcatcg agcgagcacg tactcggatg   3540
   gaagccggtc ttgtcgatca ggatgatctg gacgaagagc atcaggggct cgcgccagcc   3600
   gaactgttcg ccaggctcaa ggcgcgcatg cccgacggcg aggatctcgt cgtgacccat   3660
   ggcgatgcct gcttgccgaa tatcatggtg gaaaatggcc gcttttctgg attcatcgac   3720
   tgtggccggc tgggtgtggc ggaccgctat caggacatag cgttggctac ccgtgatatt   3780
   gctgaagagc ttggcggcga atgggctgac cgcttcctcg tgctttacgg tatcgccgct   3840
   cccgattcgc agcgcatcgc cttctatcgc cttcttgacg agttcttctg agcgggactc   3900
   tggggttcga aatgaccgac caagcgacgc ccaacctgcc atcacgatgg ccgcaataaa   3960
   atatctttat tttcattaca tctgtgtgtt ggttttttgt gtgaagatcc gcgtatggtg   4020
   cactctcagt acaatctgct ctgatgccgc atagttaagc cagccccgac acccgccaac   4080
   acccgctgac gcgccctgac gggcttgtct gctcccggca tccgcttaca gacaagctgt   4140
   gaccgtctcc gggagctgca tgtgtcagag gttttcaccg tcatcaccga aacgcgcgag   4200
   acgaaagggc ctcgtgatac gcctattttt ataggttaat gtcatgataa taatggtttc   4260
   ttagacgtca ggtggcactt ttcggggaaa tgtgcgcgga acccctattt gtttattttt   4320
   ctaaatacat tcaaatatgt atccgctcat gagacaataa ccctgataaa tgcttcaata   4380
   atattgaaaa aggaagagta tgagtattca acatttccgt gtcgccctta ttcccttttt   4440
   tgcggcattt tgccttcctg tttttgctca cccagaaacg ctggtgaaag taaaagatgc   4500
   tgaagatcag ttgggtgcac gagtgggtta catcgaactg gatctcaaca gcggtaagat   4560
   ccttgagagt tttcgccccg aagaacgttt tccaatgatg agcactttta aagttctgct   4620
   atgtggcgcg gtattatccc gtattgacgc cgggcaagag caactcggtc gccgcataca   4680
   ctattctcag aatgacttgg ttgagtactc accagtcaca gaaaagcatc ttacggatgg   4740
   catgacagta agagaattat gcagtgctgc cataaccatg agtgataaca ctgcggccaa   4800
   cttacttctg acaacgatcg gaggaccgaa ggagctaacc gcttttttgc acaacatggg   4860
   ggatcatgta actcgccttg atcgttggga accggagctg aatgaagcca taccaaacga   4920
   cgagcgtgac accacgatgc ctgtagcaat ggcaacaacg ttgcgcaaac tattaactgg   4980
   cgaactactt actctagctt cccggcaaca attaatagac tggatggagg cggataaagt   5040
   tgcaggacca cttctgcgct cggcccttcc ggctggctgg tttattgctg ataaatctgg   5100
   agccggtgag cgtgggtctc gcggtatcat tgcagcactg gggccagatg gtaagccctc   5160
   ccgtatcgta gttatctaca cgacggggag tcaggcaact atggatgaac gaaatagaca   5220
   gatcgctgag ataggtgcct cactgattaa gcattggtaa ctgtcagacc aagtttactc   5280
   atatatactt tagattgatt taaaacttca tttttaattt aaaaggatct aggtgaagat   5340
   cctttttgat aatctcatga ccaaaatccc ttaacgtgag ttttcgttcc actgagcgtc   5400
   agaccccgta gaaaagatca aaggatcttc ttgagatcct ttttttctgc gcgtaatctg   5460
   ctgcttgcaa acaaaaaaac caccgctacc agcggtggtt tgtttgccgg atcaagagct   5520
   accaactctt tttccgaagg taactggctt cagcagagcg cagataccaa atactgtcct   5580
   tctagtgtag ccgtagttag gccaccactt caagaactct gtagcaccgc ctacatacct   5640
   cgctctgcta atcctgttac cagtggctgc tgccagtggc gataagtcgt gtcttaccgg   5700
   gttggactca agacgatagt taccggataa ggcgcagcgg tcgggctgaa cggggggttc     5760
   gtgcacacag cccagcttgg agcgaacgac ctacaccgaa ctgagatacc tacagcgtga     5820
   gctatgagaa agcgccacgc ttcccgaagg gagaaaggcg gacaggtatc cggtaagcgg     5880
   cagggtcgga acaggagagc gcacgaggga gcttccaggg ggaaacgcct ggtatcttta     5940
   tagtcctgtc gggtttcgcc acctctgact tgagcgtcga tttttgtgat gctcgtcagg     6000
   ggggcggagc ctatggaaaa acgccagcaa cgcggccttt ttacggttcc tggccttttg     6060
   ctggcctttt gctcacatgg ctcgac                                          6086
<210> 10
   <211> 38
   <212> DNA
   <213> Artificial Sequence
<220>
   <223> oligonucleotide primer
<221> misc feature
   <222> (1)...(38)
   <223> n = A,T,C or G
<400> 10
   tttttttttt ttcgtcagcg gccgcatcnn nntttatt 38
<210> 11
   <211> 25
   <212> DNA
   <213> Artificial Sequence
<220>
   <223> oligonucleotide primer
<221> misc feature
   <222> (0)... (0)
   <223> The nucleotide at position 1 is biotin labeled.
<400> 11
   cagatcacta gaagctttat tgcgg 25
<210> 12
   <211> 20
   <212> DNA
   <213> Artificial Sequence
<220>
   <223> oligonucleotide primer
<400> 12
   ttttcgtcag cggccgcatc 20
<210> 13
   <211> 45
   <212> DNA
   <213> Artificial Sequence
<220>
   <223> oligonucleotide primer
<400> 13
   actcataggc catagaggcc tatcacagtt aaattgctaa cgcag 45
<210> 14
   <211> 43
   <212> DNA
   <213> Artificial Sequence
<220>
   <223> oligonucleotide primer
<221> misc_feature
   <222> (0)... (0)
   <223> the nucleotide at position 1 is biotin labeled.
<400> 14
   ctcgtttagt qcggccgctc agatcactga attctgacga cct 43
<210> 15
   <211> 41
   <212> DNA
   <213> Artificial Sequence
<220>
   <223> oligonucleotide primer
<221> misc_feature
   <222> (0)...(0)
   <223> The nucleotide at position 41 is biotin labeled.
<400> 15
   ctcgtttagt ggcgcgccag atcactgaat tctgacgacc t 41
<210> 16
   <211> 22
   <212> DNA
   <213> Artificial Sequence
<220>
   <223> oligonucleotide primer
<400> 16
   gacctactga ttaacggcca ta 22
<210> 17
   <211> 20
   <212> DNA
   <213> Artificial Sequence
<220>
   <223> oligonucleotide primer
<221> misc_feature
   <222> (0)...(0)
   <223> The nucleotide at position 20 is biotin labeled.
<400> 17
   tcgtcagaat tcagtgatct 20
<210> 18
   <211> 6836
   <212> DNA
   <213> Artificial Sequence
<220>
   <223> pRIG1 plasmid
<400> 18
   agatcttcaa tattggccat tagccatatt attcattggt tatatagcat aaatcaatat      60
   tggctattgg ccattgcata cgttgtatct atatcataat atgtacattt atattggctc     120
   atgtccaata tgaccgccat gttggcattg attattgact agttattaat agtaatcaat     180
   tacggggtca ttagttcata gcccatatat ggagttccgc gttacataac ttacggtaaa     240
   tggcccgcct ggctgaccgc ccaacgaccc ccgcccattg acgtcaataa tgacgtatgt     300
   tcccatagta acgccaatag ggactttcca ttgacgtcaa tgggtggagt atttacggta     360
   aactgcccac ttggcagtac atcaagtgta tcatatgcca agtccgcccc ctattgacgt     420
   caatgacggt aaatggcccg cctggcatta tgcccagtac atgaccttac gggactttcc     480
   tacttggcag tacatctacg tattagtcat cgctattacc atggtgatgc ggttttggca     540
   gtacaccaat gggcgtggat agcggtttga ctcacgggga tttccaagtc tccaccccat     600
   tgacgtcaat gggagtttgt tttggcacca aaatcaacgg gactttccaa aatgtcgtaa     660
   caactgcgat cgcccgcccc gttgacgcaa atgggcggta ggcgtgtacg gtgggaggtc     720
   tatataagca gagctcgttt agtgaaccgt cagatcacta gaagctttat tgcggtagtt     780
   tatcacagtt aaattgctaa cgcagtcagt gcttctgaca caacagtctc gaacttaagc     840
   tgcagtgact ctcttaaatc caccatggct acaggtgagt actcggatct agcgctatat     900
   gcgttgatgc aatttctatg cgcacccgtt ctcggagcac tgtccgaccg ctttggccgc     960
   cgcccagtcc tgctcgcttc gctacttgga gccactatcg actacgcgat catggcgacc    1020
   acacccgtcc tgtggatcct ctacgccgga cgcatcgtgg ccggcatcac cggcgccaca    1080
   ggtgcggttg ctggcgccta tatcgccgac atcaccgatg gggaagatcg ggctcgccac    1140
   ttcgggctca tgagcgcttg tttcggctct cttaaggtag cagatccttg ctagagtcga    1200
   ccaattctca tgtttgacag cttatcatcg cagatcctga gcttgtatgg tgcactctca    1260
   gtacaatctg ctctgctgcc gcatagttaa gccagtatct gctccctgct tgtgtgttgg    1320
   aggtcgctga gtagtgcgcg agcaaaattt aagctacaac aaggcaaggc ttgaccgaca    1380
   attgcatgaa gaatctgctt agggttaggc gttttgcgct gcttcgcgat gtacgggcca    1440
   gatatacgcg tatctgaggg gactagggtg tgtttaggcg cccagcgggg cttcggttgt    1500
   acgcggttag gagtcccctc aggatatagt agtttcgctt ttgcataggg agggggaaat   1560
   gtagtcttat gcaatacact tgtagtcttg caacatggta acgatgagtt agcaacatgc    1620
   cttacaagga gagaaaaagc accgtgcatg ccgattggtg gaagtaaggt ggtacgatcg    1680
   tgccttatta ggaaggcaac agacaggtct gacatggatt ggacgaacca ctgaattccg    1740
   cattgcagag ataattgtat ttaagtgcct agctcgatac aataaacgcc atttgaccat    1800
   tcaccacatt ggtgtgcacc tccaagctgg gtaccagctg ctagcctcga gacgcgtgat    1860
   ttccttcgaa gcttgtcatg gttggttcgc taaactgcat cgtcgctgtg tcccagaaca    1920
   tgggcatcgg caagaacggg gacctgccct ggccaccgct caggaatgaa ttcagatatt    1980
   tccagagaat gaccacaacc tcttcagtag aaggtaaaca gaatctggtg attatgggta    2040
   agaagacctg gttctccatt cctgagaaga atcgaccttt aaagggtaga attaatttag     2100
   ttctcagcag agaactcaag gaacctccac aaggagctca ttttctttcc agaagtctag    2160
   atgatgcctt aaaacttact gaacaaccag aattagcaaa taaagtagac atggtctgga    2220
   tagttggtgg cagttctgtt tataaggaag ccatgaatca cccaggccat cttaaactat    2280
   ttgtgacaag gatcatgcaa gactttgaaa gtgacacgtt ttttccagaa attgatttgg    2340
   agaaatataa acttctgcca gaatacccag gtgttctctc tgatgtccag gaggagaaag    2400
   gcattaagta caaatttgaa gtatatgaga agaatgatta atcgatctta agtttaatct    2460
   ttcccggggg taccgtcgac tgcggccgcg aattccaagc ttgagtattc tatcgtgtca    2520
   cctaaataac ttggcgtaat catggtcata tctgtttcct gtgtgaaatt gttatccgct    2580
   cacaattcca cacaacatac gagccggaag cataaagtgt aaagcctggg gtgcctaatg    2640
   agtgagctaa ctcacattaa ttgcgttgcg cgatgcttcc attttgtgag ggttaatgct    2700
   tcgagaagac atgataagat acattgatga gtttggacaa accacaacaa gaatgcagtg    2760
   aaaaaaatgc tttatttgtg aaatttgtga tgctattgct ttatttgtaa ccattataag    2820
   ctgcaataaa caagttaaca acaacaattg cattcatttt atgtttcagg ttcaggggga    2880
   gatgtgggag gttttttaaa gcaagtaaaa cctctacaaa tgtggtaaaa tccgataagg   2940
   atcgattccg gagcctgaat ggcgaatgga cgcgccctgt agcggcgcat taagcgcggc    3000
   gggtgtggtg gttacgcgca cgtgaccgct acacttgcca gcgccctagc gcccgctcct    3060
   ttcgctttct tcccttcctt tctcgccacg ttcgccggct ttccccgtca agctctaaat    3120
   cgggggctcc ctttagggtt ccgatttagt gctttacggc acctcgaccc caaaaaactt    3180
   gattagggtg atggttcacg tagtgggcca tcgccctgat agacggtttt tcgccctttg    3240
   acgttggagt ccacgttctt taatagtgga ctcttgttcc aaactggaac aacactcaac    3300
   cctatctcgg tctattcttt tgatttataa gggattttgc cgatttcggc ctattggtta    3360
   aaaaatgagc tgatttaaca aaaatttaac gcgaatttta acaaaatatt aacgcttaca      3420
   atttcgcctg tgtaccttct gaggcggaaa gaaccagctg tggaatgtgt gtcagttagg      3480
   gtgtggaaag tccccaggct ccccagcagg cagaagtatg caaagcatgc atctcaatta      3540
   gtcagcaacc aggtgtggaa agtccccagg ctccccagca ggcagaagta tgcaaagcat      3600
   gcatctcaat tagtcagcaa ccatagtccc gcccctaact ccgcccatcc cgcccctaac      3660
   tccgcccagt tccgcccatt ctccgcccca tggctgacta atttttttta tttatgcaga      3720
   ggccgaggcc gcctcggcct ctgagctatt ccagaagtag tgaggaggct tttttggagg      3780
   cctaggcttt tgcaaaaagc ttgattcttc tgacacaaca gtctcgaact taaggctaga      3840
   gccaccatga ttgaacaaga tggattgcac gcaggttctc cggccgcttg ggtggagagg      3900
   ctattcggct atgactgggc acaacagaca atcggctgct ctgatgccgc cgtgttccgg      3960
   ctgtcagcgc aggggcgccc ggttcttttt gtcaagaccg acctgtccgg tgccctgaat      4020
   gaactgcagg acgaggcagc gcggctatcg tggctggcca cgacgggcgt tccttgcgca      4080
   gctgtgctcg acgttgtcac tgaagcggga agggactggc tgctattggg cgaagtgccg      4140
   gggcaggatc tcctgtcatc tcaccttgct cctgccgaga aagtatccat catggctgat      4200
   gcaatgcggc ggctgcatac gcttgatccg gctacctgcc cattcgacca ccaagcgaaa      4260
   catcgcatcg agcgagcacg tactcggatg gaagccggtc ttgtcgatca ggatgatctg      4320
   gacgaagagc atcaggggct cgcgccagcc gaactgttcg ccaggctcaa ggcgcgcatg      4380
   cccgacggcg aggatctcgt cgtgacccat ggcgatgcct gcttgccgaa tatcatggtg      4440
   gaaaatggcc gcttttctgg attcatcgac tgtggccggc tgggtgtggc ggaccgctat      4500
   caggacatag cgttggctac ccgtgatatt gctgaagagc ttggcggcga atgggctgac      4560
   cgcttcctcg tgctttacgg tatcgccgct cccgattcgc agcgcatcgc cttctatcgc      4620
   cttcttgacg agttcttctg agcgggactc tggggttcga aatgaccgac caagcgacgc      4680
   ccaacctgcc atcacgatgg ccgcaataaa atatctttat tttcattaca tctgtgtgtt      4740
   ggttttttgt gtgaagatcc gcgtatggtg cactctcagt acaatctgct ctgatgccgc      4800
   atagttaagc cagccccgac acccgccaac acccgctgac gcgccctgac gggcttgtct      4860
   gctcccggca tccgcttaca gacaagctgt gaccgtctcc gggagctgca tgtgtcagag      4920
   gttttcaccg tcatcaccga aacgcgcgag acgaaagggc ctcgtgatac gcctattttt      4980
   ataggttaat gtcatgataa taatggtttc ttagacgtca ggtggcactt ttcggggaaa      5040
   tgtgcgcgga acccctattt gtttattttt ctaaatacat tcaaatatgt atccgctcat      5100
   gagacaataa ccctgataaa tgcttcaata atattgaaaa aggaagagta tgagtattca      5160
   acatttccgt gtcgccctta ttcccttttt tgcggcattt tgccttcctg tttttgctca      5220
   cccagaaacg ctggtgaaag taaaagatgc tgaagatcag ttgggtgcac gagtgggtta      5280
   catcgaactg gatctcaaca gcggtaagat ccttgagagt tttcgccccg aagaacgttt      5340
   tccaatgatg agcactttta aagttctgct atgtggcgcg gtattatccc gtattgacgc      5400
   cgggcaagag caactcggtc gccgcataca ctattctcag aatgacttgg ttgagtactc      5460
   accagtcaca gaaaagcatc ttacggatgg catgacagta agagaattat gcagtgctgc      5520
   cataaccatg agtgataaca ctgcggccaa cttacttctg acaacgatcg gaggaccgaa      5580
   ggagctaacc gcttttttgc acaacatggg ggatcatgta actcgccttg atcgttggga      5640
   accggagctg aatgaagcca taccaaacga cgagcgtgac accacgatgc ctgtagcaat      5700
   ggcaacaacg ttgcgcaaac tattaactgg cgaactactt actctagctt cccggcaaca      5760
   attaatagac tggatggagg cggataaagt tgcaggacca cttctgcgct cggcccttcc      5820
   ggctggctgg tttattgctg ataaatctgg agccggtgag cgtgggtctc gcggtatcat      5880
   tgcagcactg gggccagatg gtaagccctc ccgtatcgta gttatctaca cgacggggag      5940
   tcaggcaact atggatgaac gaaatagaca gatcgctgag ataggtgcct cactgattaa      6000
   gcattggtaa ctgtcagacc aagtttactc atatatactt tagattgatt taaaacttca      6060
   tttttaattt aaaaggatct aggtgaagat cctttttgat aatctcatga ccaaaatccc      6120
   ttaacgtgag ttttcgttcc actgagcgtc agaccccgta gaaaagatca aaggatcttc      6180
   ttgagatcct ttttttctgc gcgtaatctg ctgcttgcaa acaaaaaaac caccgctacc      6240
   agcggtggtt tgtttgccgg atcaagagct accaactctt tttccgaagg taactggctt      6300
   cagcagagcg cagataccaa atactgtcct tctagtgtag ccgtagttag gccaccactt      6360
   caagaactct gtagcaccgc ctacatacct cgctctgcta atcctgttac cagtggctgc      6420
   tgccagtggc gataagtcgt gtcttaccgg gttggactca agacgatagt taccggataa      6480
   ggcgcagcgg tcgggctgaa cggggggttc gtgcacacag cccagcttgg agcgaacgac      6540
   ctacaccgaa ctgagatacc tacagcgtga gctatgagaa agcgccacgc ttcccgaagg      6600
   gagaaaggcg gacaggtatc cggtaagcgg cagggtcgga acaggagagc gcacgaggga      6660
   gcttccaggg ggaaacgcct ggtatcttta tagtcctgtc gggtttcgcc acctctgact      6720
   tgagcgtcga tttttgtgat gctcgtcagg ggggcggagc ctatggaaaa acgccagcaa      6780
   cgcggccttt ttacggttcc tggccttttg ctggcctttt gctcacatgg ctcgac          6836
<210> 19
   <211> 4644
   <212> DNA
   <213> Artificial Sequence
<220>
   <223> pRIG21b plasmid
<400> 19
   gatcttcaat attggccatt agccatatta ttcattggtt atatagcata aatcaatatt     60
   ggctattggc cattgcatac gttgtatcta tatcataata tgtacattta tattggctca    120
   tgtccaatat gaccgccatg ttggcattga ttattgacta gttattaata gtaatcaatt    180
   acggggtcat tagttcatag cccatatatg gagttccgcg ttacataact tacggtaaat    240
   ggcccgcctg gctgaccgcc caacgacccc cgcccattga cgtcaataat gacgtatgtt    300
   cccatagtaa cgccaatagg gactttccat tgacgtcaat gggtggagta tttacggtaa    360
   actgcccact tggcagtaca tcaagtgtat catatgccaa gtccgccccc tattgacgtc    420
   aatgacggta aatggcccgc ctggcattat gcccagtaca tgaccttacg ggactttcct    480
   acttggcagt acatctacgt attagtcatc gctattacca tggtgatgcg gttttggcag    540
   tacaccaatg ggcgtggata gcggtttgac tcacggggat ttccaagtct ccaccccatt    600
   gacgtcaatg ggagtttgtt ttggcaccaa aatcaacggg actttccaaa atgtcgtaac    660
   aactgcgatc gcccgccccg ttgacgcaaa tgggcggtag gcgtgtacgg tgggaggtct    720
   atataagcag agctcgttta gtgaaccgtc agatcactga attctgacga cctactgatt    780
   aacggccata gaggcctcct gcagatcact agaagcttta ttgcggtagt ttatcacagt    840
   taaattgcta acgcagtcag tgcttctgac acaacagtct cgaacttaag ctgcagtgac    900
   tctcttaaat ccaccatggc tacaggtgag tactcgctac cttaagagag gcctatctgg    960
   ccagttagca gtcgaagaaa gaagtttaag agagccgaaa caagcgctca tgagcccgaa   1020
   gtggcgagcc cgatcttccc catcggtgat gtcggcgata taggcgccag caaccgcacc   1080
   tgtggcgccg gtgatgccgg ccacgatgcg tccggcgtag aggatccaca ggacgggtgt   1140
   ggtcgccatg atcgcgtagt cgatagtggc tccaagtagc gaagcgagca ggactgggcg   1200
   gcggccaaag cggtcggaca gtgctccgag aacgggtgcg catagaaatt gcatcaacgc   1260
   atatagcgct agatccttgc tagagtcgag atctgtcgag ccatgtgagc aaaaggccag   1320
   caaaaggcca ggaaccgtaa aaaggccgcg ttgctggcgt ttttccatag gctccgcccc   1380
   cctgacgagc atcacaaaaa tcgacgctca agtcagaggt ggcgaaaccc gacaggacta   1440
   taaagatacc aggcgtttcc ccctggaagc tccctcgtgc gctctcctgt tccgaccctg   1500
   ccgcttaccg gatacctgtc cgcctttctc ccttcgggaa gcgtggcgct ttctcatagc   1560
   tcacgctgta ggtatctcag ttcggtgtag gtcgttcgct ccaagctggg ctgtgtgcac   1620
   gaaccccccg ttcagcccga ccgctgcgcc ttatccggta actatcgtct tgagtccaac   1680
   ccggtaagac acgacttatc gccactggca gcagccactg gtaacaggat tagcagagcg   1740
   aggtatgtag gcggtgctac agagttcttg aagtggtggc ctaactacgg ctacactaga   1800
   aggacagtat ttggtatctg cgctctgctg aagccagtta ccttcggaaa aagagttggt   1860
   agctcttgat ccggcaaaca aaccaccgct ggtagcggtg gtttttttgt ttgcaagcag   1920
   cagattacgc gcagaaaaaa aggatctcaa gaagatcctt tgatcttttc tacggggtct   1980
   gacgctcagt ggaacgaaaa ctcacgttaa gggattttgg tcatgagatt atcaaaaagg   2040
   atcttcacct agatcctttt atcggtgtga aataccgcac agatgcgtaa ggagaaaata   2100
   ccgcatcagg aaattgtaag cgttaataat tcagaagaac tcgtcaagaa ggcgatagaa   2160
   ggcgatgcgc tgcgaatcgg gagcggcgat accgtaaagc acgaggaagc ggtcagccca   2220
   ttcgccgcca agctcttcag caatatcacg ggtagccaac gctatgtcct gatagcggtc   2280
   cgccacaccc agccggccac agtcgatgaa tccagaaaag cggccatttt ccaccatgat   2340
   attcggcaag caggcatcgc catgggtcac gacgagatcc tcgccgtcgg gcatgctcgc   2400
   cttgagcctg gcgaacagtt cggctggcgc gagcccctga tgctcttcgt ccagatcatc   2460
   ctgatcgaca agaccggctt ccatccgagt acgtgctcgc tcgatgcgat gtttcgcttg   2520
   gtggtcgaat gggcaggtag ccggatcaag cgtatgcagc cgccgcattg catcagccat   2580
   gatggatact ttctcggcag gagcaaggtg agatgacagg agatcctgcc ccggcacttc   2640
   gcccaatagc agccagtccc ttcccgcttc agtgacaacg tcgagcacag ctgcgcaagg   2700
   aacgcccgtc gtggccagcc acgatagccg cgctgcctcg tcttgcagtt cattcagggc   2760
   accggacagg tcggtcttga caaaaagaac cgggcgcccc tgcgctgaca gccggaacac   2820
   ggcggcatca gagcagccga ttgtctgttg tgcccagtca tagccgaata gcctctccac   2880
   ccaagcggcc ggagaacctg cgtgcaatcc atcttgttca atcatgcgaa acgatcctca   2940
   tcctgtctct tgatcagagc ttgatcccct gcgccatcag atccttggcg gcgagaaagc   3000
   catccagttt actttgcagg gcttgtcaac cttaccagat aaaagtgctc atcattggaa   3060
   aacgttcaat tctgaggcgg aaagaaccag ctgtggaatg tgtgtcagtt agggtgtgga   3120
   aagtccccag gctccccagc aggcagaagt atgcaaagca tgcatctcaa ttagtcagca     3180
   accaggtgtg gaaagtcccc aggctcccca gcaggcagaa gtatgcaaag catgcatctc     3240
   aattagtcag caaccatagt cccgccccta actccgccca tcccgcccct aactccgccc     3300
   agttccgccc attctccgcc ccatggctga ctaatttttt ttatttatgc agaggccgag     3360
   gccgcctcgg cctctgagct attccagaag tagtgaggag gcttttttgg aggcctaggc     3420
   ttttgcaaaa agcttgattc ttctgacaca acagtctcga acttaaggct agagccacca     3480
   tgattgaaca agatggattg cacgcaggtt ctccggccgc ttgggtggag aggctattcg     3540
   gctatgactg ggcacaacag acaatcggct gctctgatgc cgccgtgttc cggctgtcag     3600
   cgcaggggcg cccggttctt tttgtcaaga ccgacctgtc cggtgccctg aatgaactgc     3660
   aggacgaggc agcgcggcta tcgtggctgg ccacgacggg cgttccttgc gcagctgtgc     3720
   tcgacgttgt cactgaagcg ggaagggact ggctgctatt gggcgaagtg ccggggcagg     3780
   atctcctgtc atctcacctt gctcctgccg agaaagtatc catcatggct gatgcaatgc     3840
   ggcggctgca tacgcttgat ccggctacct gcccattcga ccaccaagcg aaacatcgca     3900
   tcgagcgagc acgtactcgg atggaagccg gtcttgtcga tcaggatgat ctggacgaag     3960
   agcatcaggg gctcgcgcca gccgaactgt tcgccaggct caaggcgcgc atgcccgacg     4020
   gcgaggatct cgtcgtgacc catggcgatg cctgcttgcc gaatatcatg gtggaaaatg     4080
   gccgcttttc tggattcatc gactgtggcc ggctgggtgt ggcggaccgc tatcaggaca     4140
   tagcgttggc tacccgtgat attgctgaag agcttggcgg cgaatgggct gaccgcttcc     4200
   tcgtgcttta cggtatcgcc gctcccgatt cgcagcgcat cgccttctat cgccttcttg     4260
   acgagccatt ctgatggagg tagcggccgc taacctggtt gctgactaat tgagatgcat     4320
   gctttgcata cttctgcctg ctggggagcc tggggacttt ccacacccta actgacacac     4380
   attccacagc tggttctttc cgcctcagaa ggtacacagg cgaaattgta agcgttaata     4440
   ttttgttaaa attcgcgtta aatttttgtt aaatcagctc attttttaac caataggccg     4500
   aaatcggcaa aatcccttat aaatcaaaag aatagaccga gatagggttg agtgttgttc     4560
   cagtttggaa caagagtcca ctattaaaga acgtggactc caacgtcaaa gggcgaaaaa     4620
   ccgtctatca gggcgatggc ccac                                            4644
<210> 20
   <211> 5247
   <212> DNA
   <213> Artificial Sequence
<220>
   <223> pRIG22b
<400> 20
   gatcttcaat attggccatt agccatatta ttcattggtt atatagcata aatcaatatt      60
   ggctattggc cattgcatac gttgtatcta tatcataata tgtacattta tattggctca     120
   tgtccaatat gaccgccatg ttggcattga ttattgacta gttattaata gtaatcaatt     180
   acggggtcat tagttcatag cccatatatg gagttccgcg ttacataact tacggtaaat     240
   ggcccgcctg gctgaccgcc caacgacccc cgcccattga cgtcaataat gacgtatgtt     300
   cccatagtaa cgccaatagg gactttccat tgacgtcaat gggtggagta tttacggtaa     360
   actgcccact tggcagtaca tcaagtgtat catatgccaa gtccgccccc tattgacgtc     420
   aatgacggta aatggcccgc ctggcattat gcccagtaca tgaccttacg ggactttcct     480
   acttggcagt acatctacgt attagtcatc gctattacca tggtgatgcg gttttggcag     540
   tacaccaatg ggcgtggata gcggtttgac tcacggggat ttccaagtct ccaccccatt     600
   gacgtcaatg ggagtttgtt ttggcaccaa aatcaacggg actttccaaa atgtcgtaac     660
   aactgcgatc gcccgccccg ttgacgcaaa tgggcggtag gcgtgtacgg tgggaggtct     720
   atataagcag agctcgttta gtgaaccgtc agatcactag aagctttatt gcggtagttt     780
   atcacagtta aattgctaac gcagtcagtg cttctgacac aacagtctcg aacttaagct     840
   gcagtgactc tcttaaatcc accatggcta caggtgagta ctcgctacct taagagaggc     900
   ctatctggcc agttagcagt cgaagaaaga agtttaagag agccgaaaca agcgctcatg     960
   agcccgaagt ggcgagcccg atcttcccca tcggtgatgt cggcgatata ggcgccagca    1020
   accgcacctg tggcgccggt gatgccggcc acgatgcgtc cggcgtagag gatccacagg    1080
   acgggtgtgg tcgccatgat cgcgtagtcg atagtggctc caagtagcga agcgagcagg    1140
   actgggcggc ggccaaagcg gtcggacagt gctccgagaa cgggtgcgca tagaaattgc    1200
   atcaacgcat atagcgctag atccttgcta gagtcgagat ctgtcgagcc atgtgagcaa    1260
   aaggccagca aaaggccagg aaccgtaaaa aggccgcgtt gctggcgttt ttccataggc    1320
   tccgcccccc tgacgagcat cacaaaaatc gacgctcaag tcagaggtgg cgaaacccga    1380
   caggactata aagataccag gcgtttcccc ctggaagctc cctcgtgcgc tctcctgttc    1440
   cgaccctgcc gcttaccgga tacctgtccg cctttctccc ttcgggaagc gtggcgcttt    1500
   ctcatagctc acgctgtagg tatctcagtt cggtgtaggt cgttcgctcc aagctgggct 1560
   gtgtgcacga accccccgtt cagcccgacc gctgcgcctt atccggtaac tatcgtcttg 1620
   agtccaaccc ggtaagacac gacttatcgc cactggcagc agccactggt aacaggatta 1680
   gcagagcgag gtatgtaggc ggtgctacag agttcttgaa gtggtggcct aactacggct 1740
   acactagaag gacagtattt ggtatctgcg ctctgctgaa gccagttacc ttcggaaaaa 1800
   gagttggtag ctcttgatcc ggcaaacaaa ccaccgctgg tagcggtggt ttttttgttt 1860
   gcaagcagca gattacgcgc agaaaaaaag gatctcaaga agatcctttg atcttttcta 1920
   cggggtctga cgctcagtgg aacgaaaact cacgttaagg gattttggtc atgagattat 1980
   caaaaaggat cttcacctag atccttttat cggtgtgaaa taccgcacag atgcgtaagg 2040
   agaaaatacc gcatcaggaa attgtaagcg ttaataattc agaagaactc gtcaagaagg 2100
   cgatagaagg cgatgcgctg cgaatcggga gcggcgatac cgtaaagcac gaggaagcgg 2160
   tcagcccatt cgccgccaag ctcttcagca atatcacggg tagccaacgc tatgtcctga 2220
   tagcggtccg ccacacccag ccggccacag tcgatgaatc cagaaaagcg gccattttcc 2280
   accatgatat tcggcaagca ggcatcgcca tgggtcacga cgagatcctc gccgtcgggc 2340
   atgctcgcct tgagcctggc gaacagttcg gctggcgcga gcccctgatg ctcttcgtcc 2400
   agatcatcct gatcgacaag accggcttcc atccgagtac gtgctcgctc gatgcgatgt 2460
   ttcgcttggt ggtcgaatgg gcaggtagcc ggatcaagcg tatgcagccg ccgcattgca 2520
   tcagccatga tggatacttt ctcggcagga gcaaggtgag atgacaggag atcctgcccc 2580
   ggcacttcgc ccaatagcag ccagtccctt cccgcttcag tgacaacgtc gagcacagct 2640
   gcgcaaggaa cgcccgtcgt ggccagccac gatagccgcg ctgcctcgtc ttgcagttca 2700
   ttcagggcac cggacaggtc ggtcttgaca aaaagaaccg ggcgcccctg cgctgacagc 2760
   cggaacacgg cggcatcaga gcagccgatt gtctgttgtg cccagtcata gccgaatagc 2820
   ctctccaccc aagcggccgg agaacctgcg tgcaatccat cttgttcaat catgcgaaac 2880
   gatcctcatc ctgtctcttg atcagagctt gatcccctgc gccatcagat ccttggcggc 2940
   gagaaagcca tccagtttac tttgcagggc ttgtcaacct taccagataa aagtgctcat 3000
   cattggaaaa cgttcaattc tgaggcggaa agaaccagct gtggaatgtg tgtcagttag 3060
   ggtgtggaaa gtccccaggc tccccagcag gcagaagtat gcaaagcatg catctcaatt 3120
   agtcagcaac caggtgtgga aagtccccag gctccccagc aggcagaagt atgcaaagca 3180
   tgcatctcaa ttagtcagca accatagtcc cgcccctaac tccgcccatc ccgcccctaa 3240
   ctccgcccag ttccgcccat tctccgcccc atggctgact aatttttttt atttatgcag 3300
   aggccgaggc cgcctcggcc tctgagctat tccagaagta gtgaggaggc ttttttggag 3360
   gcctaggctt ttgcaaaaag cttgattctt ctgacacaac agtctcgaac ttaaggctag 3420
   agccaccatg attgaacaag atggattgca cgcaggttct ccggccgctt gggtggagag 3480
   gctattcggc tatgactggg cacaacagac aatcggctgc tctgatgccg ccgtgttccg 3540
   gctgtcagcg caggggcgcc cggttctttt tgtcaagacc gacctgtccg gtgccctgaa 3600
   tgaactgcag gacgaggcag cgcggctatc gtggctggcc acgacgggcg ttccttgcgc 3660
   agctgtgctc gacgttgtca ctgaagcggg aagggactgg ctgctattgg gcgaagtgcc 3720
   ggggcaggat ctcctgtcat ctcaccttgc tcctgccgag aaagtatcca tcatggctga 3780
   tgcaatgcgg cggctgcata cgcttgatcc ggctacctgc ccattcgacc accaagcgaa 3840
   acatcgcatc gagcgagcac gtactcggat ggaagccggt cttgtcgatc aggatgatct 3900
   ggacgaagag catcaggggc tcgcgccagc cgaactgttc gccaggctca aggcgcgcat 3960
   gcccgacggc gaggatctcg tcgtgaccca tggcgatgcc tgcttgccga atatcatggt 4020
   ggaaaatggc cgcttttctg gattcatcga ctgtggccgg ctgggtgtgg cggaccgcta 4080
   tcaggacata gcgttggcta cccgtgatat tgctgaagag cttggcggcg aatgggctga 4140
   ccgcttcctc gtgctttacg gtatcgccgc tcccgattcg cagcgcatcg ccttctatcg 4200
   ccttcttgac gagccattct gctggatggc tacaggtcgc agccctggcg tcgtgattag 4260
   tgatgatgaa ccaggttatg accttgattt attttgcata cctaatcatt atgctgagga 4320
   tttggaaagg gtgtttattc ctcatggact aattatggac aggactgaac gtcttgctcg 4380
   agatgtgatg aaggagatgg gaggccatca cattgtagcc ctctgtgtgc tcaagggggg 4440
   ctataaattc tttgctgacc tgctggatta catcaaagca ctgaatagaa atagtgatag 4500
   atccattcct atgactgtag attttatcag actgaagagc tattgtaatg accagtcaac 4560
   aggggacata aaagtaattg gtggagatga tctctcaact ttaactggaa agaatgtctt 4620
   gattgtggaa gatataattg acactggcaa aacaatgcag actttgcttt ccttggtcag 4680
   gcagtataat ccaaagatgg tcaaggtcgc aagcttgctg gtgaaaagga ccccacgaag 4740
   tgttggatat aagccagact ttgttggatt tgaaattcca gacaagtttg ttgtaggata 4800
   tgcccttgac tataatgaat acttcaggga tttgaatcat gtttgtgtca ttagtgaaac 4860
   tggaaaagca aaatacaaag cctaagcggc cgctaacctg gttgctgact aattgagatg 4920
   catgctttgc atacttctgc ctgctgggga gcctggggac tttccacacc ctaactgaca 4980
   cacattccac agctggttct ttccgcctca gaaggtacac aggcgaaatt gtaagcgtta 5040
   atattttgtt aaaattcgcg ttaaattttt gttaaatcag ctcatttttt aaccaatagg 5100
   ccgaaatcgg caaaatccct tataaatcaa aagaatagac cgagataggg ttgagtgttg 5160
   ttccagtttg gaacaagagt ccactattaa agaacgtgga ctccaacgtc aaagggcgaa    5220
   aaaccgtcta tcagggcgat ggcccac                                        5247
<210> 21
   <211> 5382
   <212> DNA
   <213> Artificial Sequence
<220>
   <223> pRIG14 plasmid
<221> misc_feature
   <222> (1)...(5382)
   <223> n = A,T,C or G
<400> 21
   cacctaaatt gtaagcgtta atattttgtt aaaattcgcg ttaaattttt gttaaatcag     60
   ctcatttttt aaccaatagg ccgaaatcgg caaaatccct tataaatcaa aagaatagac    120
   cgagataggg ttgagtgttg ttccagtttg gaacaagagt ccactattaa agaacgtgga    180
   ctccaacgtc aaagggcgaa aaaccgtcta tcagggcgat ggcccactac gtgaaccatc    240
   accctaatca agttttttgg ggtcgaggtg ccgtaaagca ctaaatcgga accctaaagg    300
   gagcccccga tttagagctt gacggggaaa gccggcgaac gtggcgagaa aggaagggaa    360
   gaaagcgaaa ggagcgggcg ctagggcgct ggcaagtgta gcggtcacgc tgcgcgtaac    420
   caccacaccc gccgcgctta atgcgccgct acagggcgcg tcccattcgc cattcaggct    480
   gcgcaactgt tgggaagggc gatcggtgcg ggcctcttcg ctattacgcc agctggcgaa    540
   agggggatgt gctgcaaggc gattaagttg ggtaacgcca gggttttccc agtcacgacg    600
   ttgtaaaacg acggccagtg aattgtaata cgactcacta tagggcgaat tgggtacaat    660
   tcaattcgtc gacctcgaaa ttctaccggg taggggaggc gcttttccca aggcagtctg    720
   gagcatgcgc tttagcagcc ccgctgggca cttggcgcta cacaagtggc ctctggcctc    780
   gcacacattc cacatccacc ggtaggcgcc aaccggctcc gttctttggt ggccccttcg    840
   cgccaccttc tactcctccc ctagtcagga agttcccccc cgccccgcan ctcgcgtcgt    900
   gcaggacgtg acaaatggaa atagcacgtc tcactagtct cgtgcagatg gacaagcacc    960
   gctgagcaat ggagcgggta ggcctttggg gcagcggcca atagcagctt tgctccttcg   1020
   ctttctgggc tcagaggctg gnaaggggtg ggtccggggg cgggctcagg ggcgggctca   1080
   ggggcggggc gggcgcccga aggtcctccg gaggcccggc attctgcacg cttcaaaagc   1140
   gcacgtctgc cgcgctgttc tcctcttcct catctccggg cctttcgacc tgcatccatc   1200
   tagatctcga gcagctgaag cttaccatga ccgagtacaa gcccacggtg cgcctcgcca   1260
   cccgcgacga cgtcccccgg gccgtacgca ccctcgccgc cgcgttcgcc gactaccccg   1320
   ccacgcgcca caccgtcgac ccggaccgcc acatcgagcg ggtcaccgag ctgcaagaac   1380
   tcttcctcac gcgcgtcggg ctcgacatcg gcaaggtgtg ggtcgcggac gacggcgccg   1440
   cggtggcggt ctggaccacg ccggagagcg tcgaagcggg ggcggtgttc gccgagatcg   1500
   gcccgcgcat ggccgagttg agcggttccc ggctggccgc gcagcaacag atggaaggcc   1560
   tcctggcgcc gcaccggccc caaggagccc gcgtggttcc ttggcccacc gtcgggcgtc   1620
   ttcgcccgac caccagggca agggtctggc aagcgccgtc gtgctccccg gagtggaggc   1680
   ggccgagcgc gccggggtgc ccgccttcct ggagacctcc gcgccccgca acctcccctt   1740
   ctacgagcgg ctcggcttca ccgtcaccgc cgacgtcgag gtgcccgaag gaccgcgcac   1800
   ctggtgcatg acccgcaagc ccggtgcctg acgcccgccc cacgacccgc agcgcccgac   1860
   cgaaaggagc gcacgacccc atgcatcgat ggcactgggc aggtaagtat caaggttagc   1920
   gatcttcaat attggccatt agccatatta ttcattggtt atatagcata aatcaatatt   1980
   ggctattggc cattgcatac gttgtatcta tatcataata tgtacattta tattggctca   2040
   tgtccaatat gaccgccatg ttggcattga ttattgacta gttattaata gtaatcaatt   2100
   acggggtcat tagttcatag cccatatatg gagttccgcg ttacataact tacggtaaat   2160
   ggcccgcctg gctgaccgcc caacgacccc cgcccattga cgtcaataat gacgtatgtt   2220
   cccatagtaa cgccaatagg gactttccat tgacgtcaat gggtggagta tttacggtaa   2280
   actgcccact tggcagtaca tcaagtgtat catatgccaa gtccgccccc tattgacgtc   2340
   aatgacggta aatggcccgc ctggcattat gcccagtaca tgaccttacg ggactttcct   2400
   acttggcagt acatctacgt attagtcatc gctattacca tggtgatgcg gttttggcag   2460
   tacaccaatg ggcgtggata gcggtttgac tcacggggat ttccaagtct ccaccccatt   2520
   gacgtcaatg ggagtttgtt ttggcaccaa aatcaacggg actttccaaa atgtcgtaac   2580
   aactgcgatc gcccgccccg ttgacgcaaa tgggcggtag gcgtgtacgg tgggaggtct    2640
   atataagcag agctcgttta gtgaaccgtc agatcactag aagctttatt gcggtagttt    2700
   atcacagtta aattgctaac gcagtcagtg cttctgacac aacagtctcg aacttaagct    2760
   gcagtgactc tcttaattaa ccaccgctac aggtgagtac tcggatctgc taccttaaga    2820
   gaggcctatc tggccagtta gcagtcgaag aaagaagttt aagagagccg aaacaagcgc    2880
   tcatgagccc gaagtggcga gcccgatctt ccccatcggt gatgtcggcg atataggcgc    2940
   cagcaaccgc acctgtggcg ccggtgatgc cggccacgat gcgtccggcg tagaggatcc    3000
   acaggacggg tgtggtcgcc atgatcgcgt agtcgatagt ggctccaagt agcgaagcga    3060
   gcaggactgg gcggcggcca aagcggtcgg acagtgctcc gagaacgggt gcgcatagaa    3120
   attgcatcaa cgcatatagc gctagatcct tgctagagtc gaggccgcca ccgcggtgga    3180
   gctccagctt ttgttccctt tagtgagggt taatttcgag cttggcgtaa tcatggtcat    3240
   agctgtttcc tgtgtgaaat tgttatccgc tcacaattcc acacaacata cgagccggaa    3300
   gcataaagtg taaagcctgg ggtgcctaat gagtgagcta actcacatta attgcgttgc    3360
   gctcactgcc cgctttccag tcgggaaacc tgtcgtgcca gctgcattaa tgaatcggcc    3420
   aacgcgcggg gagaggcggt ttgcgtattg ggcgctcttc cgcttcctcg ctcactgact    3480
   cgctgcgctc ggtcgttcgg ctgcggcgag cggtatcagc tcactcaaag gcggtaatac    3540
   ggttatccac agaatcaggg gataacgcag gaaagaacat gtgagcaaaa ggccagcaaa    3600
   aggccaggaa ccgtaaaaag gccgcgttgc tggcgttttt ccataggctc cgcccccctg    3660
   acgagcatca caaaaatcga cgctcaagtc agaggtggcg aaacccgaca ggactataaa    3720
   gataccaggc gtttccccct ggaagctccc tcgtgcgctc tcctgttccg accctgccgc    3780
   ttaccggata cctgtccgcc tttctccctt cgggaagcgt ggcgctttct catagctcac    3840
   gctgtaggta tctcagttcg gtgtaggtcg ttcgctccaa gctgggctgt gtgcacgaac    3900
   cccccgttca gcccgaccgc tgcgccttat ccggtaacta tcgtcttgag tccaacccgg    3960
   taagacacga cttatcgcca ctggcagcag ccactggtaa caggattagc agagcgaggt    4020
   atgtaggcgg tgctacagag ttcttgaagt ggtggcctaa ctacggctac actagaagga    4080
   cagtatttgg tatctgcgct ctgctgaagc cagttacctt cggaaaaaga gttggtagct    4140
   cttgatccgg caaacaaacc accgctggta gcggtggttt ttttgtttgc aagcagcaga    4200
   ttacgcgcag aaaaaaagga tctcaagaag atcctttgat cttttctacg gggtctgacg    4260
   ctcagtggaa cgaaaactca cgttaaggga ttttggtcat gagattatca aaaaggatct    4320
   tcacctagat ccttttaaat taaaaatgaa gttttaaatc aatctaaagt atatatgagt    4380
   aaacttggtc tgacagttac caatgcttaa tcagtgaggc acctatctca gcgatctgtc    4440
   tatttcgttc atccatagtt gcctgactcc ccgtcgtgta gataactacg atacgggagg    4500
   gcttaccatc tggccccagt gctgcaatga taccgcgaga cccacgctca ccggctccag    4560
   atttatcagc aataaaccag ccagccggaa gggccgagcg cagaagtggt cctgcaactt    4620
   tatccgcctc catccagtct attaattgtt gccgggaagc tagagtaagt agttcgccag    4680
   ttaatagttt gcgcaacgtt gttgccattg ctacaggcat cgtggtgtca cgctcgtcgt    4740
   ttggtatggc ttcattcagc tccggttccc aacgatcaag gcgagttaca tgatccccca    4800
   tgttgtgcaa aaaagcggtt agctccttcg gtcctccgat cgttgtcaga agtaagttgg    4860
   ccgcagtgtt atcactcatg gttatggcag cactgcataa ttctcttact gtcatgccat    4920
   ccgtaagatg cttttctgtg actggtgagt actcaaccaa gtcattctga gaatagtgta    4980
   tgcggcgacc gagttgctct tgcccggcgt caatacggga taataccgcg ccacatagca    5040
   gaactttaaa agtgctcatc attggaaaac gttcttcggg gcgaaaactc tcaaggatct    5100
   taccgctgtt gagatccagt tcgatgtaac ccactcgtgc acccaactga tcttcagcat    5160
   cttttacttt caccagcgtt tctgggtgag caaaaacagg aaggcaaaat gccgcaaaaa    5220
   agggaataag ggcgacacgg aaatgttgaa tactcatact cttccttttt caatattatt    5280
   gaagcattta tcagggttat tgtctcatga gcggatacat atttgaatgt atttagaaaa    5340
   ataaacaaat aggggttccg cgcacatttc cccgaaaagt gc                       5382
<210> 22
   <211> 9737
   <212> DNA
   <213> Artificial Sequence
<220>
   <223> pRIG19 plasmid
<221> misc feature
   <222> (1)...(9737)
   <223> n = A,T,C or G
<400> 22
   gatcttcaat attggccatt agccatatta ttcattggtt atatagcata aatcaatatt 60
   ggctattggc cattgcatac gttgtatcta tatcataata tgtacattta tattggctca 120
   tgtccaatat gaccgccatg ttggcattga ttattgacta gttattaata gtaatcaatt 180
   acggggtcat tagttcatag cccatatatg gagttccgcg ttacataact tacggtaaat 240
   ggcccgcctg gctgaccgcc caacgacccc cgcccattga cgtcaataat gacgtatgtt 300
   cccatagtaa cgccaatagg gactttccat tgacgtcaat gggtggagta tttacggtaa 360
   actgcccact tggcagtaca tcaagtgtat catatgccaa gtccgccccc tattgacgtc 420
   aatgacggta aatggcccgc ctggcattat gcccagtaca tgaccttacg ggactttcct 480
   acttggcagt acatctacgt attagtcatc gctattacca tggtgatgcg gttttggcag 540
   tacaccaatg ggcgtggata gcggtttgac tcacggggat ttccaagtct ccaccccatt 600
   gacgtcaatg ggagtttgtt ttggcaccaa aatcaacggg actttccaaa atgtcgtaac 660
   aactgcgatc gcccgccccg ttgacgcaaa tgggcggtag gcgtgtacgg tgggaggtct 720
   atataagcag agctcgttta gtgaaccgtc agatcactga attctgacga cctactgatt 780
   aacggccata gaggcctcct gcagaactgt cttagtgaca actatcgatt tccacacatt 840
   atacgagccg atgttaattg tcaacagctc atgcatgacg tcccgggagc agacaagccc 900
   gaccatggct cgagtaatac gactcactat agggcgacag gtgagtactc gctaccttaa 960
   ggcctatctg gccgtttaaa cagatgtgta taagagacag ctctcttaag gtagcctgtc 1020
   tcttatacac atctagatcc ttgctagagt cgaccaattc tcatgtttga cagcttatca 1080
   tcgcagatcc tgagcttgta tggtgcactc tcagtacaat ctgctctgct gccgcatagt 1140
   taagccagta tctgctccct gcttgtgtgt tggaggtcgc tgagtagtgc gcgagcaaaa 1200
   tttaagctac aacaaggcaa ggcttgaccg acaattgcat gaagaatctg cttagggtta 1260
   ggcgttttgc gctgcttcgc gatgtacggg ccagatatac gcgtatctga ggggactagg 1320
   gtgtgtttag gcgcccagcg gggcttcggt tgtacgcggt taggagtccc ctcaggatat    1380
   agtagtttcg cttttgcata gggaggggga aatgtagtct tatgcaatac acttgtagtc    1440
   ttgcaacatg gtaacgatga gttagcaaca tgccttacaa ggagagaaaa agcaccgtgc 1500
   atgccgattg gtggaagtaa ggtggtacga tcgtgcctta ttaggaaggc aacagacagg 1560
   tctgacatgg attggacgaa ccactgaatt ccgcattgca gagataattg tatttaagtg 1620
   cctagctcga tacaataaac gccatttgac cattcaccac attggtgtgc acctccaagc 1680
   tgggtaccag ctgctagcct cgagacgcgt gatttccttc gaagcttgtc atggttggtt 1740
   cgctaaactg catcgtcgct gtgtcccaga acatgggcat cggcaagaac ggggacctgc 1800
   cctggccacc gctcaggaat gaattcagat atttccagag aatgaccaca acctcttcag 1860
   tagaaggtaa acagaatctg gtgattatgg gtaagaagac ctggttctcc attcctgaga 1920
   agaatcgacc tttaaagggt agaattaatt tagttctcag cagagaactc aaggaacctc 1980
   cacaaggagc tcattttctt tccagaagtc tagatgatgc cttaaaactt actgaacaac 2040
   cagaattagc aaataaagta gacatggtct ggatagttgg tggcagttct gtttataagg 2100
   aagccatgaa tcacccaggc catcttaaac tatttgtgac aaggatcatg caagactttg 2160
   aaagtgacac gttttttcca gaaattgatt tggagaaata taaacttctg ccagaatacc 2220
   caggtgttct ctctgatgtc caggaggaga aaggcattaa gtacaaattt gaagtatatg 2280
   agaagaatgt taattaaggg caccaataac tgccttaaaa aaattacgcc ccgccctgcc 2340
   actcatcgca gtactgttgt aattcattaa gcattctgcc gacatggaag ccatcacaga 2400
   cggcatgatg aacctgaatc gccagcggca tcagcacctt gtcgccttgc gtataatatt 2460
   tgcccatggt gaaaacgggg gcgaagaagt tgtccatatt ggccacgttt aaatcaaaac 2520
   tggtgaaact cacccaggga ttggctgaga cgaaaaacat attctcaata aaccctttag 2580
   ggaaataggc caggttttca ccgtaacacg ccacatcttg cgaatatatg tgtagaaact 2640
   gccggaaatc gtcgtggtat tcactccaga gcgatgaaaa cgtttcagtt tgctcatgga 2700
   aaacggtgta acaagggtga acactatccc atatcaccag ctcaccgtct ttcattgcca 2760
   tacggaattc cggatgagca ttcatcaggc gggcaagaat gtgaataaag gccggataaa 2820
   acttgtgctt atttttcttt acggtcttta aaaaggccgt aatatccagc tgaacggtct 2880
   ggttataggt acattgagca actgactgaa atgcctcaaa atgttcttta cgatgccatt 2940
   gggatatatc aacggtggta tatccagtga tttttttctc cattttagct tccttagctc 3000
   ctgaaaatct cgataactca aaaaatacgc ccggtagtga tcttatttca ttatggtgaa 3060
   agttggaacc tcttacgtgc cgatcaacgt ctcattttcg ccaaattaat taaggcgcgc 3120
   cgctctcctg gctaggagtc acgtagaaag gactaccgac gaaggaactt gggtcgccgg 3180
   tgtgttcgta tatggaggta gtaagacctc cctttacaac ctaaggcgag gaactgccct 3240
   tgctattcca caatgtcgtc ttacaccatt gagtcgtctc ccctttggaa tggcccctgg 3300
   acccggccca caacctggcc cgctaaggga gtccattgtc tgttatttca tggtcttttt 3360
   acaaactcat atatttgctg aggttttgaa ggatgcgatt aaggaccttg ttatgacaaa 3420
   gcccgctcct acctgcaata tcagggtgac tgtgtgcagc tttgacgatg gagtagattt 3480
   gcctccctgg tttccaccta tggtggaagg ggctgccgcg gagggtgatg acggagatga 3540
   cggagatgaa ggaggtgatg gagatgaggg tgaggaaggg caggagtgat gtaacttgtt 3600
   aggagacgcc ctcaatcgta ttaaaagccg tgtattcccc cgcactaaag aataaatccc 3660
   cagtagacat catgcgtgct gttggtgtat ttctggccat ctgtcttgtc accattttcg 3720
   tcctcccaac atggggcaat tgggcatacc catgttgtca cgtcactcag ctccgcgctc 3780
   aacaccttct cgcgttggaa aacattagcg acatttacct ggtgagcaat cagacatgcg 3840
   acggctttag cctggcctcc ttaaattcac ctaagaatgg gagcaaccag catgcaggaa 3900
   aaggacaagc agcgaaaatt cacgccccct tgggaggtgg cggcatatgc aaaggatagc 3960
   actcccactc tactactggg tatcatatgc tgactgtata tgcatgagga tagcatatgc 4020
   tacccggata cagattagga tagcatatac tacccagata tagattagga tagcatatgc 4080
   tacccagata tagattagga tagcctatgc tacccagata taaattagga tagcatatac 4140
   tacccagata tagattagga tagcatatgc tacccagata tagattagga tagcctatgc 4200
   tacccagata tagattagga tagcatatgc tacccagata tagattagga tagcatatgc 4260
   tatccagata tttgggtagt atatgctacc cagatataaa ttaggatagc atatactacc 4320
   ctaatctcta ttaggatagc atatgctacc cggatacaga ttaggatagc atatactacc 4380
   cagatataga ttaggatagc atatgctacc cagatataga ttaggatagc ctatgctacc 4440
   cagatataaa ttaggatagc atatactacc cagatataga ttaggatagc atatgctacc 4500
   cagatataga ttaggatagc ctatgctacc cagatataga ttaggatagc atatgctatc 4560
   cagatatttg ggtagtatat gctacccatg gcaacattag cccaccgtgc tctcagcgac 4620
   ctcgtgaata tgaggaccaa caaccctgtg cttggcgctc aggcgcaagt gtgtgtaatt 4680
   tgtcctccag atcgcagcaa tcgcgcccct atcttggccc gcccacctac ttatgcaggt 4740
   attccccggg gtgccattag tggttttgtg ggcaagtggt ttgaccgcag tggttagcgg 4800
   ggttacaatc agccaagtta ttacaccctt attttacagt ccaaaaccgc agggcggcgt 4860
   gtgggggctg acgcgtgccc ccactccaca atttcaaaaa aaagagtggc cacttgtctt 4920
   tgtttatggg ccccattggc gtggagcccc gtttaatttt cgggggtgtt agagacaacc 4980
   agtggagtcc gctgctgtcg gcgtccactc tctttcccct tgttacaaat agagtgtaac 5040
   aacatggttc acctgtcttg gtccctgcct gggacacatc ttaataaccc cagtatcata 5100
   ttgcactagg attatgtgtt gcccatagcc ataaattcgt gtgagatgga catccagtct 5160
   ttacggcttg tccccacccc atggatttct attgttaaag atattcagaa tgtttcattc 5220
   ctacactagt atttattgcc caaggggttt gtgagggtta tattggtgtc atagcacaat 5280
   gccaccactg aaccccccgt ccaaatttta ttctgggggc gtcacctgaa accttgtttt 5340
   cgagcacctc acatacacct tactgttcac aactcagcag ttattctatt agctaaacga 5400
   aggagaatga agaagcaggc gaagattcag gagagttcac tgcccgctcc ttgatcttca 5460
   gccactgccc ttgtgactaa aatggttcac taccctcgtg gaatcctgac cccatgtaaa 5520
   taaaaccgtg acagctcatg gggtgggaga tatcgctgtt ccttaggacc cttttactaa 5580
   ccctaattcg atagcatatg cttcccgttg ggtaacatat gctattgaat tagggttagt 5640
   ctggatagta tatactacta cccgggaagc atatgctacc cgtttagggt taacaagggg 5700
   gccttataaa cactattgct aatgccctct tgagggtccg cttatcggta gctacacagg 5760
   cccctctgat tgacgttggt gtagcctccc gtagtcttcc tgggcccctg ggaggtacat 5820
   gtcccccagc attggtgtaa gagcttcagc caagagttac acataaaggc aatgttgtgt 5880
   tgcagtccac agactgcaaa gtctgctcca ggatgaaagc cactcagtgt tggcaaatgt 5940
   gcacatccat ttataaggat gtcaactaca gtcagagaac ccctttgtgt ttggtccccc 6000
   cccgtgtcac atgtggaaca gggcccagtt ggcaagttgt accaaccaac tgaagggatt 6060
   acatgcactg ccccgaatac aaaacaaaag cgctcctcgt accagcgaag aaggggcaga 6120
   gatgccgtag tcaggtttag ttcgtccggc ggcgggcggc cgcaaggcgc gccggatcca 6180
   caggacgggt gtggtcgcca tgatcgcgta gtcgatagtg gctccaagta gcgaagcgag 6240
   caggactggg cggcggccaa agcggtcgga cagtgctccg agaacgggtg cgcatagaaa 6300
   ttgcatcaac gcatatagcg ctagatcctt gctagagtcg agatctgtcg agccatgtga 6360
   gcaaaaggcc agcaaaaggc caggaaccgt aaaaaggccg cgttgctggc gtttttccat 6420
   aggctccgcc cccctgacga gcatcacaaa aatcgacgct caagtcagag gtggcgaaac 6480
   ccgacaggac tataaagata ccaggcgttt ccccctggaa gctccctcgt gcgctctcct 6540
   gttccgaccc tgccgcttac cggatacctg tccgcctttc tcccttcggg aagcgtggcg 6600
   ctttctcata gctcacgctg taggtatctc agttcggtgt aggtcgttcg ctccaagctg 6660
   ggctgtgtgc acgaaccccc cgttcagccc gaccgctgcg ccttatccgg taactatcgt 6720
   cttgagtcca acccggtaag acacgactta tcgccactgg cagcagccac tggtaacagg 6780
   attagcagag cgaggtatgt aggcggtgct acagagttct tgaagtggtg gcctaactac 6840
   ggctacacta gaaggacagt atttggtatc tgcgctctgc tgaagccagt taccttcgga 6900
   aaaagagttg gtagctcttg atccggcaaa caaaccaccg ctggtagcgg tggttttttt 6960
   gtttgcaagc agcagattac gcgcagaaaa aaaggatctc aagaagatcc tttgatcttt 7020
   tctacggggt ctgacgctca gtggaacgaa aactcacgtt aagggatttt ggtcatgaga 7080
   ttatcaaaaa ggatcttcac ctagatcctt ttatcggtgt gaaataccgc acagatgcgt 7140
   aaggagaaaa taccgcatca ggaaattgta agcgttaata attcagaaga actcgtcaag 7200
   aaggcgatag aaggcgatgc gctgcgaatc gggagcggcg ataccgtaaa gcacgaggaa 7260
   gcggtcagcc cattcgccgc caagctcttc agcaatatca cgggtagcca acgctatgtc 7320
   ctgatagcgg tccgccacac ccagccggcc acagtcgatg aatccagaaa agcggccatt 7380
   ttccaccatg atattcggca agcaggcatc gccatgggtc acgacgagat cctcgccgtc 7440
   gggcatgctc gccttgagcc tggcgaacag ttcggctggc gcgagcccct gatgctcttc 7500
   gtccagatca tcctgatcga caagaccggc ttccatccga gtacgtgctc gctcgatgcg 7560
   atgtttcgct tggtggtcga atgggcaggt agccggatca agcgtatgca gccgccgcat 7620
   tgcatcagcc atgatggata ctttctcggc aggagcaagg tgagatgaca ggagatcctg 7680
   ccccggcact tcgcccaata gcagccagtc ccttcccgct tcagtgacaa cgtcgagcac 7740
   agctgcgcaa ggaacgcccg tcgtggccag ccacgatagc cgcgctgcct cgtcttgcag 7800
   ttcattcagg gcaccggaca ggtcggtctt gacaaaaaga accgggcgcc cctgcgctga 7860
   cagccggaac acggcggcat cagagcagcc gattgtctgt tgtgcccagt catagccgaa 7920
   tagcctctcc acccaagcgg ccggagaacc tgcgtgcaat ccatcttgtt caatcatgcg 7980
   aaacgatcct catcctgtct cttgatcaga gcttgatccc ctgcgccatc agatccttgg 8040
   cggcgagaaa gccatccagt ttactttgca gggcttgtca accttaccag ataaaagtgc 8100
   tcatcattgg aaaacattca attcgtcgac ctcgaaattc taccgggtag gggaggcgct 8160
   tttcccaagg cagtctggag catgcgcttt agcagccccg ctgggcactt ggcgctacac 8220
   aagtggcctc tggcctcgca cacattccac atccaccggt aggcgccaac cggctccgtt 8280
   ctttggtggc cccttcgcgc caccttctac tcctccccta gtcaggaagt tcccccccgc 8340
   cccgcanctc gcgtcgtgca ggacgtgaca aatggaaata gcacgtctca ctagtctcgt 8400
   gcagatggac aagcaccgct gagcaatgga gcgggtaggc ctttggggca gcggccaata 8460
   gcagctttgc tccttcgctt tctgggctca gaggctggna aggggtgggt ccgggggcgg 8520
   gctcaggggc gggctcaggg gcggggcggg cgcccgaagg tcctccggag gcccggcatt 8580
   ctgcacgctt caaaagcgca cgtctgccgc gctgttctcc tcttcctcat ctccgggcct 8640
   ttcgacctgc atccatctag atctcgagca gctgaagctt accatgaccg agtacaagcc 8700
   cacggtgcgc ctcgccaccc gcgacgacgt cccccgggcc gtacgcaccc tcgccgccgc 8760
   gttcgccgac taccccgcca cgcgccacac cgtcgacccg gaccgccaca tcgagcgggt 8820
   caccgagctg caagaactct tcctcacgcg cgtcgggctc gacatcggca aggtgtgggt 8880
   cgcggacgac ggcgccgcgg tggcggtctg gaccacgccg gagagcgtcg aagcgggggc 8940
   ggtgttcgcc gagatcggcc cgcgcatggc cgagttgagc ggttcccggc tggccgcgca 9000
   gcaacagatg gaaggcctcc tggcgccgca ccgggcccaa ggagcccgcg tggttcctgg 9060
   gcccaccgtc gggcgtcttc gcccgaccac cagggcaagg gtctggcaag cgccgtcgtg 9120
   ctccccggag tggaggcggc cgagcgcgcc ggggtgcccg ccttcctgga gacctccgcg 9180
   ccccgcaacc tccccttcta cgagcggctc ggcttcaccg tcaccgccga cgtcgaggtg 9240
   cccgaaggac cgcgcacctg gtgcatgacc cgcaagcccg gtgcctgacg cccgccccac 9300
   gacccgcagc gcccgaccga aaggagcgca cgaccccatg catcgatggc actgggcagg 9360
   taagtatcaa ggttagcggc cgctaacctg gttgctgact aattgagatg catgctttgc 9420
   atacttctgc ctgctgggga gcctggggac tttccacacc ctaactgaca cacattccac 9480
   agctggttct ttccgcctca gaaggtacac aggcgaaatt gtaagcgtta atattttgtt 9540
   aaaattcgcg ttaaattttt gttaaatcag ctcatttttt aaccaatagg ccgaaatcgg 9600
   caaaatccct tataaatcaa aagaatagac cgagataggg ttgagtgttg ttccagtttg 9660
   gaacaagagt ccactattaa agaacgtgga ctccaacgtc aaagggcgaa aaaccgtcta 9720
   tcagggcgat ggcccac 9737
<210> 23
   <211> 9737
   <212> DNA
   <213> Artificial Sequence
<220>
   <223> pRIG20
<221> misc_feature
   <222> (1)...(9737)
   <223> n = A, T, C or G
<400> 23
<210> 24
   <211> 9871
   <212> DNA
   <213> Artificial Sequence
<220>
   <223> pRIGad1 plasmid
<221> misc_feature
   <222> (1)...(9871)
   <223> n = A,T,C or G
<400> 24
   gatcttcaat attggccatt agccatatta ttcattggtt atatagcata aatcaatatt      60
   ggctattggc cattgcatac gttgtatcta tatcataata tgtacattta tattggctca     120
   tgtccaatat gaccgccatg ttggcattga ttattgacta gttattaata gtaatcaatt     180
   acggggtcat tagttcatag cccatatatg gagttccgcg ttacataact tacggtaaat     240
   ggcccgcctg gctgaccgcc caacgacccc cgcccattga cgtcaataat gacgtatgtt     300
   cccatagtaa cgccaatagg gactttccat tgacgtcaat gggtggagta tttacggtaa     360
   actgcccact tggcagtaca tcaagtgtat catatgccaa gtccgccccc tattgacgtc     420
   aatgacggta aatggcccgc ctggcattat gcccagtaca tgaccttacg ggactttcct     480
   acttggcagt acatctacgt attagtcatc gctattacca tggtgatgcg gttttggcag     540
   tacaccaatg ggcgtggata gcggtttgac tcacggggat ttccaagtct ccaccccatt     600
   gacgtcaatg ggagtttgtt ttggcaccaa aatcaacggg actttccaaa atgtcgtaac     660
   aactgcgatc gcccgccccg ttgacgcaaa tgggcggtag gcgtgtacgg tgggaggtct     720
   atataagcag agctcgttta gtgaaccgtc agatcactga attctgacga cctactgatt     780
   aaagatctaa gctagcgccg ccaccatggg ccctaaaaag aagcgtaaag tcgccccccc     840
   gaccgatgtc agcctggggg acgagctcca cttagacggc gaggacgtgg cgatggcgca     900
   tgccgacgcg ctagacgatt tcgatctgga catgttgggg gacggggatt ccccgggggc     960
   gggatttacc ccccacgact ccgcccccta cggcgctctg gatatggccg acttcgagtt    1020
   tgagcagatg tttaccgatg cccttggaat tgacgagtac ggtggggaat tcaggtgagt    1080
   actcgctacc ttaaggccta tctggccgtt taaacagatg tgtataagag acagctctct    1140
   taaggtagcc tgtctcttat acacatctag atccttgcta gagtcgacca attctcatgt    1200
   ttgacagctt atcatcgcag atcctgagct tgtatggtgc actctcagta caatctgctc    1260
   tgctgccgca tagttaagcc agtatctgct ccctgcttgt gtgttggagg tcgctgagta    1320
   gtgcgcgagc aaaatttaag ctacaacaag gcaaggcttg accgacaatt gcatgaagaa    1380
   tctgcttagg gttaggcgtt ttgcgctgct tcgcgatgta cgggccagat atacgcgtat    1440
   ctgaggggac tagggtgtgt ttaggcgccc agcggggctt cggttgtacg cggttaggag    1500
   tcccctcagg atatagtagt ttcgcttttg catagggagg gggaaatgta gtcttatgca    1560
   atacacttgt agtcttgcaa catggtaacg atgagttagc aacatgcctt acaaggagag    1620
   aaaaagcacc gtgcatgccg attggtggaa gtaaggtggt acgatcgtgc cttattagga    1680
   aggcaacaga caggtctgac atggattgga cgaaccactg aattccgcat tgcagagata    1740
   attgtattta agtgcctagc tcgatacaat aaacgccatt tgaccattca ccacattggt    1800
   gtgcacctcc aagctgggta ccagctgcta gcctcgagac gcgtgatttc cttcgaagct    1860
   tgtcatggtt ggttcgctaa actgcatcgt cgctgtgtcc cagaacatgg gcatcggcaa    1920
   gaacggggac ctgccctggc caccgctcag gaatgaattc agatatttcc agagaatgac    1980
   cacaacctct tcagtagaag gtaaacagaa tctggtgatt atgggtaaga agacctggtt    2040
   ctccattcct gagaagaatc gacctttaaa gggtagaatt aatttagttc tcagcagaga    2100
   actcaaggaa cctccacaag gagctcattt tctttccaga agtctagatg atgccttaaa    2160
   acttactgaa caaccagaat tagcaaataa agtagacatg gtctggatag ttggtggcag    2220
   ttctgtttat aaggaagcca tgaatcaccc aggccatctt aaactatttg tgacaaggat    2280
   catgcaagac tttgaaagtg acacgttttt tccagaaatt gatttggaga aatataaact    2340
   tctgccagaa tacccaggtg ttctctctga tgtccaggag gagaaaggca ttaagtacaa    2400
   atttgaagta tatgagaaga atgttaatta agggcaccaa taactgcctt aaaaaaatta    2460
   cgccccgccc tgccactcat cgcagtactg ttgtaattca ttaagcattc tgccgacatg    2520
   gaagccatca cagacggcat gatgaacctg aatcgccagc ggcatcagca ccttgtcgcc    2580
   ttgcgtataa tatttgccca tggtgaaaac gggggcgaag aagttgtcca tattggccac    2640
   gtttaaatca aaactggtga aactcaccca gggattggct gagacgaaaa acatattctc    2700
   aataaaccct ttagggaaat aggccaggtt ttcaccgtaa cacgccacat cttgcgaata    2760
   tatgtgtaga aactgccgga aatcgtcgtg gtattcactc cagagcgatg aaaacgtttc    2820
   agtttgctca tggaaaacgg tgtaacaagg gtgaacacta tcccatatca ccagctcacc    2880
   gtctttcatt gccatacgga attccggatg agcattcatc aggcgggcaa gaatgtgaat    2940
   aaaggccgga taaaacttgt gcttattttt ctttacggtc tttaaaaagg ccgtaatatc    3000
   cagctgaacg gtctggttat aggtacattg agcaactgac tgaaatgcct caaaatgttc    3060
   tttacgatgc cattgggata tatcaacggt ggtatatcca gtgatttttt tctccatttt    3120
   agcttcctta gctcctgaaa atctcgataa ctcaaaaaat acgcccggta gtgatcttat    3180
   ttcattatgg tgaaagttgg aacctcttac gtgccgatca acgtctcatt ttcgccaaat    3240
   taattaaggc gcgccgctct cctggctagg agtcacgtag aaaggactac cgacgaagga    3300
   acttgggtcg ccggtgtgtt cgtatatgga ggtagtaaga cctcccttta caacctaagg   3360
   cgaggaactg cccttgctat tccacaatgt cgtcttacac cattgagtcg tctccccttt   3420
   ggaatggccc ctggacccgg cccacaacct ggcccgctaa gggagtccat tgtctgttat    3480
   ttcatggtct ttttacaaac tcatatattt gctgaggttt tgaaggatgc gattaaggac    3540
   cttgttatga caaagcccgc tcctacctgc aatatcaggg tgactgtgtg cagctttgac    3600
   gatggagtag atttgcctcc ctggtttcca cctatggtgg aaggggctgc cgcggagggt    3660
   gatgacggag atgacggaga tgaaggaggt gatggagatg agggtgagga agggcaggag    3720
   tgatgtaact tgttaggaga cgccctcaat cgtattaaaa gccgtgtatt cccccgcact    3780
   aaagaataaa tccccagtag acatcatgcg tgctgttggt gtatttctgg ccatctgtct    3840
   tgtcaccatt ttcgtcctcc caacatgggg caattgggca tacccatgtt gtcacgtcac    3900
   tcagctccgc gctcaacacc ttctcgcgtt ggaaaacatt agcgacattt acctggtgag    3960
   caatcagaca tgcgacggct ttagcctggc ctccttaaat tcacctaaga atgggagcaa    4020
   ccagcatgca ggaaaaggac aagcagcgaa aattcacgcc cccttgggag gtggcggcat    4080
   atgcaaagga tagcactccc actctactac tgggtatcat atgctgactg tatatgcatg    4140
   aggatagcat atgctacccg gatacagatt aggatagcat atactaccca gatatagatt    4200
   aggatagcat atgctaccca gatatagatt aggatagcct atgctaccca gatataaatt    4260
   aggatagcat atactaccca gatatagatt aggatagcat atgctaccca gatatagatt    4320
   aggatagcct atgctaccca gatatagatt aggatagcat atgctaccca gatatagatt    4380
   aggatagcat atgctatcca gatatttggg tagtatatgc tacccagata taaattagga    4440
   tagcatatac taccctaatc tctattagga tagcatatgc tacccggata cagattagga    4500
   tagcatatac tacccagata tagattagga tagcatatgc tacccagata tagattagga    4560
   tagcctatgc tacccagata taaattagga tagcatatac tacccagata tagattagga    4620
   tagcatatgc tacccagata tagattagga tagcctatgc tacccagata tagattagga    4680
   tagcatatgc tatccagata tttgggtagt atatgctacc catggcaaca ttagcccacc    4740
   gtgctctcag cgacctcgtg aatatgagga ccaacaaccc tgtgcttggc gctcaggcgc    4800
   aagtgtgtgt aatttgtcct ccagatcgca gcaatcgcgc ccctatcttg gcccgcccac    4860
   ctacttatgc aggtattccc cggggtgcca ttagtggttt tgtgggcaag tggtttgacc    4920
   gcagtggtta gcggggttac aatcagccaa gttattacac ccttatttta cagtccaaaa    4980
   ccgcagggcg gcgtgtgggg gctgacgcgt gcccccactc cacaatttca aaaaaaagag    5040
   tggccacttg tctttgttta tgggccccat tggcgtggag ccccgtttaa ttttcggggg    5100
   tgttagagac aaccagtgga gtccgctgct gtcggcgtcc actctctttc cccttgttac    5160
   aaatagagtg taacaacatg gttcacctgt cttggtccct gcctgggaca catcttaata    5220
   accccagtat catattgcac taggattatg tgttgcccat agccataaat tcgtgtgaga    5280
   tggacatcca gtctttacgg cttgtcccca ccccatggat ttctattgtt aaagatattc    5340
   agaatgtttc attcctacac tagtatttat tgcccaaggg gtttgtgagg gttatattgg    5400
   tgtcatagca caatgccacc actgaacccc ccgtccaaat tttattctgg gggcgtcacc    5460
   tgaaaccttg ttttcgagca cctcacatac accttactgt tcacaactca gcagttattc    5520
   tattagctaa acgaaggaga atgaagaagc aggcgaagat tcaggagagt tcactgcccg    5580
   ctccttgatc ttcagccact gcccttgtga ctaaaatggt tcactaccct cgtggaatcc    5640
   tgaccccatg taaataaaac cgtgacagct catggggtgg gagatatcgc tgttccttag    5700
   gaccctttta ctaaccctaa ttcgatagca tatgcttccc gttgggtaac atatgctatt    5760
   gaattagggt tagtctggat agtatatact actacccggg aagcatatgc tacccgttta    5820
   gggttaacaa gggggcctta taaacactat tgctaatgcc ctcttgaggg tccgcttatc    5880
   ggtagctaca caggcccctc tgattgacgt tggtgtagcc tcccgtagtc ttcctgggcc    5940
   cctgggaggt acatgtcccc cagcattggt gtaagagctt cagccaagag ttacacataa    6000
   aggcaatgtt gtgttgcagt ccacagactg caaagtctgc tccaggatga aagccactca 6060
   gtgttggcaa atgtgcacat ccatttataa ggatgtcaac tacagtcaga gaaccccttt 6120
   gtgtttggtc cccccccgtg tcacatgtgg aacagggccc agttggcaag ttgtaccaac    6180
   caactgaagg gattacatgc actgccccga atacaaaaca aaagcgctcc tcgtaccagc    6240
   gaagaagggg cagagatgcc gtagtcaggt ttagttcgtc cggcggcggg cggccgcaag    6300
   gcgcgccgga tccacaggac gggtgtggtc gccatgatcg cgtagtcgat agtggctcca    6360
   agtagcgaag cgagcaggac tgggcggcgg ccaaagcggt cggacagtgc tccgagaacg    6420
   ggtgcgcata gaaattgcat caacgcatat agcgctagat ccttgctaga gtcgagatct    6480
   gtcgagccat gtgagcaaaa ggccagcaaa aggccaggaa ccgtaaaaag gccgcgttgc    6540
   tggcgttttt ccataggctc cgcccccctg acgagcatca caaaaatcga cgctcaagtc    6600
   agaggtggcg aaacccgaca ggactataaa gataccaggc gtttccccct ggaagctccc    6660
   tcgtgcgctc tcctgttccg accctgccgc ttaccggata cctgtccgcc tttctccctt    6720
   cgggaagcgt ggcgctttct catagctcac gctgtaggta tctcagttcg gtgtaggtcg    6780
   ttcgctccaa gctgggctgt gtgcacgaac cccccgttca gcccgaccgc tgcgccttat    6840
   ccggtaacta tcgtcttgag tccaacccgg taagacacga cttatcgcca ctggcagcag    6900
   ccactggtaa caggattagc agagcgaggt atgtaggcgg tgctacagag ttcttgaagt    6960
   ggtggcctaa ctacggctac actagaagga cagtatttgg tatctgcgct ctgctgaagc    7020
   cagttacctt cggaaaaaga gttggtagct cttgatccgg caaacaaacc accgctggta    7080
   gcggtggttt ttttgtttgc aagcagcaga ttacgcgcag aaaaaaagga tctcaagaag    7140
   atcctttgat cttttctacg gggtctgacg ctcagtggaa cgaaaactca cgttaaggga    7200
   ttttggtcat gagattatca aaaaggatct tcacctagat ccttttatcg gtgtgaaata    7260
   ccgcacagat gcgtaaggag aaaataccgc atcaggaaat tgtaagcgtt aataattcag    7320
   aagaactcgt caagaaggcg atagaaggcg atgcgctgcg aatcgggagc ggcgataccg    7380
   taaagcacga ggaagcggtc agcccattcg ccgccaagct cttcagcaat atcacgggta    7440
   gccaacgcta tgtcctgata gcggtccgcc acacccagcc ggccacagtc gatgaatcca    7500
   gaaaagcggc cattttccac catgatattc ggcaagcagg catcgccatg ggtcacgacg    7560
   agatcctcgc cgtcgggcat gctcgccttg agcctggcga acagttcggc tggcgcgagc    7620
   ccctgatgct cttcgtccag atcatcctga tcgacaagac cggcttccat ccgagtacgt    7680
   gctcgctcga tgcgatgttt cgcttggtgg tcgaatgggc aggtagccgg atcaagcgta    7740
   tgcagccgcc gcattgcatc agccatgatg gatactttct cggcaggagc aaggtgagat    7800
   gacaggagat cctgccccgg cacttcgccc aatagcagcc agtcccttcc cgcttcagtg    7860
   acaacgtcga gcacagctgc gcaaggaacg cccgtcgtgg ccagccacga tagccgcgct    7920
   gcctcgtctt gcagttcatt cagggcaccg gacaggtcgg tcttgacaaa aagaaccggg    7980
   cgcccctgcg ctgacagccg gaacacggcg gcatcagagc agccgattgt ctgttgtgcc    8040
   cagtcatagc cgaatagcct ctccacccaa gcggccggag aacctgcgtg caatccatct    8100
   tgttcaatca tgcgaaacga tcctcatcct gtctcttgat cagagcttga tcccctgcgc    8160
   catcagatcc ttggcggcga gaaagccatc cagtttactt tgcagggctt gtcaacctta    8220
   ccagataaaa gtgctcatca ttggaaaaca ttcaattcgt cgacctcgaa attctaccgg    8280
   gtaggggagg cgcttttccc aaggcagtct ggagcatgcg ctttagcagc cccgctgggc    8340
   acttggcgct acacaagtgg cctctggcct cgcacacatt ccacatccac cggtaggcgc    8400
   caaccggctc cgttctttgg tggccccttc gcgccacctt ctactcctcc cctagtcagg    8460
   aagttccccc ccgccccgca nctcgcgtcg tgcaggacgt gacaaatgga aatagcacgt    8520
   ctcactagtc tcgtgcagat ggacaagcac cgctgagcaa tggagcgggt aggcctttgg    8580
   ggcagcggcc aatagcagct ttgctccttc gctttctggg ctcagaggct ggnaaggggt    8640
   gggtccgggg gcgggctcag gggcgggctc aggggcgggg cgggcgcccg aaggtcctcc    8700
   ggaggcccgg cattctgcac gcttcaaaag cgcacgtctg ccgcgctgtt ctcctcttcc    8760
   tcatctccgg gcctttcgac ctgcatccat ctagatctcg agcagctgaa gcttaccatg    8820
   accgagtaca agcccacggt gcgcctcgcc acccgcgacg acgtcccccg ggccgtacgc    8880
   accctcgccg ccgcgttcgc cgactacccc gccacgcgcc acaccgtcga cccggaccgc    8940
   cacatcgagc gggtcaccga gctgcaagaa ctcttcctca cgcgcgtcgg gctcgacatc    9000
   ggcaaggtgt gggtcgcgga cgacggcgcc gcggtggcgg tctggaccac gccggagagc    9060
   gtcgaagcgg gggcggtgtt cgccgagatc ggcccgcgca tggccgagtt gagcggttcc    9120
   cggctggccg cgcagcaaca gatggaaggc ctcctggcgc cgcaccgggc ccaaggagcc    9180
   cgcgtggttc cttggcccac cgtcgggcgt cttcgcccga ccaccagggc aagggtctgg    9240
   caagcgccgt cgtgctcccc ggagtggagg cggccgagcg cgccggggtg cccgccttcc    9300
   tggagacctc cgcgccccgc aacctcccct tctacgagcg gctcggcttc accgtcaccg    9360
   ccgacgtcga ggtgcccgaa ggaccgcgca cctggtgcat gacccgcaag cccggtgcct    9420
   gacgcccgcc ccacgacccg cagcgcccga ccgaaaggag cgcacgaccc catgcatcga    9480
   tggcactggg caggtaagta tcaaggttag cggccgctaa cctggttgct gactaattga    9540
   gatgcatgct ttgcatactt ctgcctgctg gggagcctgg ggactttcca caccctaact    9600
   gacacacatt ccacagctgg ttctttccgc ctcagaaggt acacaggcga aattgtaagc    9660
   gttaatattt tgttaaaatt cgcgttaaat ttttgttaaa tcagctcatt ttttaaccaa    9720
   taggccgaaa tcggcaaaat cccttataaa tcaaaagaat agaccgagat agggttgagt    9780
   gttgttccag tttggaacaa gagtccacta ttaaagaacg tggactccaa cgtcaaaggg    9840
   cgaaaaaccg tctatcaggg cgatggccca c                                  9871
<210> 25
   <211> 10060
   <212> DNA
   <213> Artificial Sequence
<220>
   <223> pRIGbd1 plasmid
<221> misc_feature
   <222> (1)...(10060)
   <223> n = A,T,C or G
<400> 25
   gatcttcaat attggccatt agccatatta ttcattggtt atatagcata aatcaatatt      60
   ggctattggc cattgcatac gttgtatcta tatcataata tgtacattta tattggctca     120
   tgtccaatat gaccgccatg ttggcattga ttattgacta gttattaata gtaatcaatt     180
   acggggtcat tagttcatag cccatatatg gagttccgcg ttacataact tacggtaaat     240
   ggcccgcctg gctgaccgcc caacgacccc cgcccattga cgtcaataat gacgtatgtt     300
   cccatagtaa cgccaatagg gactttccat tgacgtcaat gggtggagta tttacggtaa     360
   actgcccact tggcagtaca tcaagtgtat catatgccaa gtccgccccc tattgacgtc     420
   aatgacggta aatggcccgc ctggcattat gcccagtaca tgaccttacg ggactttcct     480
   acttggcagt acatctacgt attagtcatc gctattacca tggtgatgcg gttttggcag     540
   tacaccaatg ggcgtggata gcggtttgac tcacggggat ttccaagtct ccaccccatt     600
   gacgtcaatg ggagtttgtt ttggcaccaa aatcaacggg actttccaaa atgtcgtaac     660
   aactgcgatc gcccgccccg ttgacgcaaa tgggcggtag gcgtgtacgg tgggaggtct     720
   atataagcag agctcgttta gtgaaccgtc agatcactga attctgacga cctactgatt     780
   aacggccaga tctaagctag cttcctgaaa gatgaagcta ctgtcttcta tcgaacaagc     840
   atgcgatatt tgccgactta aaaagctcaa gtgctccaaa gaaaaaccga agtgcgccaa     900
   gtgtctgaag aacaactggg agtgtcgcta ctctcccaaa accaaaaggt ctccgctgac     960
   tagggcacat ctgacagaag tggaatcaag gctagaaaga ctggaacagc tatttctact    1020
   gatttttcct cgagaagacc ttgacatgat tttgaaaatg gattctttac aggatataaa    1080
   agcattgtta acaggattat ttgtacaaga taatgtgaat aaagatgccg tcacagatag    1140
   attggcttca gtggagactg atatgcctct aacattgaga cagcatagaa taagtgcgac    1200
   atcatcatcg gaagagagta gtaacaaagg tcaaagacag ttgactgtat cgccggaatt    1260
   caggtgagta ctcgctacct taaggcctat ctggccgttt aaacagatgt gtataagaga    1320
   cagctctctt aaggtagcct gtctcttata cacatctaga tccttgctag agtcgaccaa    1380
   ttctcatgtt tgacagctta tcatcgcaga tcctgagctt gtatggtgca ctctcagtac    1440
   aatctgctct gctgccgcat agttaagcca gtatctgctc cctgcttgtg tgttggaggt    1500
   cgctgagtag tgcgcgagca aaatttaagc tacaacaagg caaggcttga ccgacaattg    1560
   catgaagaat ctgcttaggg ttaggcgttt tgcgctgctt cgcgatgtac gggccagata    1620
   tacgcgtatc tgaggggact agggtgtgtt taggcgccca gcggggcttc ggttgtacgc    1680
   ggttaggagt cccctcagga tatagtagtt tcgcttttgc atagggaggg ggaaatgtag    1740
   tcttatgcaa tacacttgta gtcttgcaac atggtaacga tgagttagca acatgcctta    1800
   caaggagaga aaaagcaccg tgcatgccga ttggtggaag taaggtggta cgatcgtgcc    1860
   ttattaggaa ggcaacagac aggtctgaca tggattggac gaaccactga attccgcatt    1920
   gcagagataa ttgtatttaa gtgcctagct cgatacaata aacgccattt gaccattcac    1980
   cacattggtg tgcacctcca agctgggtac cagctgctag cctcgagacg cgtgatttcc    2040
   ttcgaagctt gtcatggttg gttcgctaaa ctgcatcgtc gctgtgtccc agaacatggg    2100
   catcggcaag aacggggacc tgccctggcc accgctcagg aatgaattca gatatttcca    2160
   gagaatgacc acaacctctt cagtagaagg taaacagaat ctggtgatta tgggtaagaa    2220
   gacctggttc tccattcctg agaagaatcg acctttaaag ggtagaatta atttagttct    2280
   cagcagagaa ctcaaggaac ctccacaagg agctcatttt ctttccagaa gtctagatga    2340
   tgccttaaaa cttactgaac aaccagaatt agcaaataaa gtagacatgg tctggatagt    2400
   tggtggcagt tctgtttata aggaagccat gaatcaccca ggccatctta aactatttgt    2460
   gacaaggatc atgcaagact ttgaaagtga cacgtttttt ccagaaattg atttggagaa    2520
   atataaactt ctgccagaat acccaggtgt tctctctgat gtccaggagg agaaaggcat    2580
   taagtacaaa tttgaagtat atgagaagaa tgttaattaa gggcaccaat aactgcctta    2640
   aaaaaattac gccccgccct gccactcatc gcagtactgt tgtaattcat taagcattct    2700
   gccgacatgg aagccatcac agacggcatg atgaacctga atcgccagcg gcatcagcac    2760
   cttgtcgcct tgcgtataat atttgcccat ggtgaaaacg ggggcgaaga agttgtccat    2820
   attggccacg tttaaatcaa aactggtgaa actcacccag ggattggctg agacgaaaaa    2880
   catattctca ataaaccctt tagggaaata ggccaggttt tcaccgtaac acgccacatc    2940
   ttgcgaatat atgtgtagaa actgccggaa atcgtcgtgg tattcactcc agagcgatga    3000
   aaacgtttca gtttgctcat ggaaaacggt gtaacaaggg tgaacactat cccatatcac    3060
   cagctcaccg tctttcattg ccatacggaa ttccggatga gcattcatca ggcgggcaag    3120
   aatgtgaata aaggccggat aaaacttgtg cttatttttc tttacggtct ttaaaaaggc    3180
   cgtaatatcc agctgaacgg tctggttata ggtacattga gcaactgact gaaatgcctc    3240
   aaaatgttct ttacgatgcc attgggatat atcaacggtg gtatatccag tgattttttt    3300
   ctccatttta gcttccttag ctcctgaaaa tctcgataac tcaaaaaata cgcccggtag    3360
   tgatcttatt tcattatggt gaaagttgga acctcttacg tgccgatcaa cgtctcattt    3420
   tcgccaaatt aattaaggcg cgccgctctc ctggctagga gtcacgtaga aaggactacc    3480
   gacgaaggaa cttgggtcgc cggtgtgttc gtatatggag gtagtaagac ctccctttac    3540
   aacctaaggc gaggaactgc ccttgctatt ccacaatgtc gtcttacacc attgagtcgt    3600
   ctcccctttg gaatggcccc tggacccggc ccacaacctg gcccgctaag ggagtccatt    3660
   gtctgttatt tcatggtctt tttacaaact catatatttg ctgaggtttt gaaggatgcg    3720
   attaaggacc ttgttatgac aaagcccgct cctacctgca atatcagggt gactgtgtgc    3780
   agctttgacg atggagtaga tttgcctccc tggtttccac ctatggtgga aggggctgcc    3840
   gcggagggtg atgacggaga tgacggagat gaaggaggtg atggagatga gggtgaggaa    3900
   gggcaggagt gatgtaactt gttaggagac gccctcaatc gtattaaaag ccgtgtattc    3960
   ccccgcacta aagaataaat ccccagtaga catcatgcgt gctgttggtg tatttctggc    4020
   catctgtctt gtcaccattt tcgtcctccc aacatggggc aattgggcat acccatgttg    4080
   tcacgtcact cagctccgcg ctcaacacct tctcgcgttg gaaaacatta gcgacattta    4140
   cctggtgagc aatcagacat gcgacggctt tagcctggcc tccttaaatt cacctaagaa    4200
   tgggagcaac cagcatgcag gaaaaggaca agcagcgaaa attcacgccc ccttgggagg    4260
   tggcggcata tgcaaaggat agcactccca ctctactact gggtatcata tgctgactgt    4320
   atatgcatga ggatagcata tgctacccgg atacagatta ggatagcata tactacccag    4380
   atatagatta ggatagcata tgctacccag atatagatta ggatagccta tgctacccag    4440
   atataaatta ggatagcata tactacccag atatagatta ggatagcata tgctacccag    4500
   atatagatta ggatagccta tgctacccag atatagatta ggatagcata tgctacccag    4560
   atatagatta ggatagcata tgctatccag atatttgggt agtatatgct acccagatat    4620
   aaattaggat agcatatact accctaatct ctattaggat agcatatgct acccggatac    4680
   agattaggat agcatatact acccagatat agattaggat agcatatgct acccagatat    4740
   agattaggat agcctatgct acccagatat aaattaggat agcatatact acccagatat    4800
   agattaggat agcatatgct acccagatat agattaggat agcctatgct acccagatat    4860
   agattaggat agcatatgct atccagatat ttgggtagta tatgctaccc atggcaacat    4920
   tagcccaccg tgctctcagc gacctcgtga atatgaggac caacaaccct gtgcttggcg    4980
   ctcaggcgca agtgtgtgta atttgtcctc cagatcgcag caatcgcgcc cctatcttgg    5040
   cccgcccacc tacttatgca ggtattcccc ggggtgccat tagtggtttt gtgggcaagt    5100
   ggtttgaccg cagtggttag cggggttaca atcagccaag ttattacacc cttattttac    5160
   agtccaaaac cgcagggcgg cgtgtggggg ctgacgcgtg cccccactcc acaatttcaa    5220
   aaaaaagagt ggccacttgt ctttgtttat gggccccatt ggcgtggagc cccgtttaat    5280
   tttcgggggt gttagagaca accagtggag tccgctgctg tcggcgtcca ctctctttcc    5340
   ccttgttaca aatagagtgt aacaacatgg ttcacctgtc ttggtccctg cctgggacac    5400
   atcttaataa ccccagtatc atattgcact aggattatgt gttgcccata gccataaatt    5460
   cgtgtgagat ggacatccag tctttacggc ttgtccccac cccatggatt tctattgtta    5520
   aagatattca gaatgtttca ttcctacact agtatttatt gcccaagggg tttgtgaggg    5580
   ttatattggt gtcatagcac aatgccacca ctgaaccccc cgtccaaatt ttattctggg    5640
   ggcgtcacct gaaaccttgt tttcgagcac ctcacataca ccttactgtt cacaactcag    5700
   cagttattct attagctaaa cgaaggagaa tgaagaagca ggcgaagatt caggagagtt    5760
   cactgcccgc tccttgatct tcagccactg cccttgtgac taaaatggtt cactaccctc    5820
   gtggaatcct gaccccatgt aaataaaacc gtgacagctc atggggtggg agatatcgct    5880
   gttccttagg acccttttac taaccctaat tcgatagcat atgcttcccg ttgggtaaca    5940
   tatgctattg aattagggtt agtctggata gtatatacta ctacccggga agcatatgct    6000
   acccgtttag ggttaacaag ggggccttat aaacactatt gctaatgccc tcttgagggt    6060
   ccgcttatcg gtagctacac aggcccctct gattgacgtt ggtgtagcct cccgtagtct    6120
   tcctgggccc ctgggaggta catgtccccc agcattggtg taagagcttc agccaagagt    6180
   tacacataaa ggcaatgttg tgttgcagtc cacagactgc aaagtctgct ccaggatgaa    6240
   agccactcag tgttggcaaa tgtgcacatc catttataag gatgtcaact acagtcagag    6300
   aacccctttg tgtttggtcc ccccccgtgt cacatgtgga acagggccca gttggcaagt    6360
   tgtaccaacc aactgaaggg attacatgca ctgccccgaa tacaaaacaa aagcgctcct    6420
   cgtaccagcg aagaaggggc agagatgccg tagtcaggtt tagttcgtcc ggcggcgggc    6480
   ggccgcaagg cgcgccggat ccacaggacg ggtgtggtcg ccatgatcgc gtagtcgata    6540
   gtggctccaa gtagcgaagc gagcaggact gggcggcggc caaagcggtc ggacagtgct    6600
   ccgagaacgg gtgcgcatag aaattgcatc aacgcatata gcgctagatc cttgctagag    6660
   tcgagatctg tcgagccatg tgagcaaaag gccagcaaaa ggccaggaac cgtaaaaagg    6720
   ccgcgttgct ggcgtttttc cataggctcc gcccccctga cgagcatcac aaaaatcgac    6780
   gctcaagtca gaggtggcga aacccgacag gactataaag ataccaggcg tttccccctg    6840
   gaagctccct cgtgcgctct cctgttccga ccctgccgct taccggatac ctgtccgcct    6900
   ttctcccttc gggaagcgtg gcgctttctc atagctcacg ctgtaggtat ctcagttcgg    6960
   tgtaggtcgt tcgctccaag ctgggctgtg tgcacgaacc ccccgttcag cccgaccgct    7020
   gcgccttatc cggtaactat cgtcttgagt ccaacccggt aagacacgac ttatcgccac    7080
   tggcagcagc cactggtaac aggattagca gagcgaggta tgtaggcggt gctacagagt    7140
   tcttgaagtg gtggcctaac tacggctaca ctagaaggac agtatttggt atctgcgctc    7200
   tgctgaagcc agttaccttc ggaaaaagag ttggtagctc ttgatccggc aaacaaacca    7260
   ccgctggtag cggtggtttt tttgtttgca agcagcagat tacgcgcaga aaaaaaggat    7320
   ctcaagaaga tcctttgatc ttttctacgg ggtctgacgc tcagtggaac gaaaactcac    7380
   gttaagggat tttggtcatg agattatcaa aaaggatctt cacctagatc cttttatcgg    7440
   tgtgaaatac cgcacagatg cgtaaggaga aaataccgca tcaggaaatt gtaagcgtta    7500
   ataattcaga agaactcgtc aagaaggcga tagaaggcga tgcgctgcga atcgggagcg    7560
   gcgataccgt aaagcacgag gaagcggtca gcccattcgc cgccaagctc ttcagcaata    7620
   tcacgggtag ccaacgctat gtcctgatag cggtccgcca cacccagccg gccacagtcg    7680
   atgaatccag aaaagcggcc attttccacc atgatattcg gcaagcaggc atcgccatgg    7740
   gtcacgacga gatcctcgcc gtcgggcatg ctcgccttga gcctggcgaa cagttcggct    7800
   ggcgcgagcc cctgatgctc ttcgtccaga tcatcctgat cgacaagacc ggcttccatc    7860
   cgagtacgtg ctcgctcgat gcgatgtttc gcttggtggt cgaatgggca ggtagccgga    7920
   tcaagcgtat gcagccgccg cattgcatca gccatgatgg atactttctc ggcaggagca    7980
   aggtgagatg acaggagatc ctgccccggc acttcgccca atagcagcca gtcccttccc    8040
   gcttcagtga caacgtcgag cacagctgcg caaggaacgc ccgtcgtggc cagccacgat    8100
   agccgcgctg cctcgtcttg cagttcattc agggcaccgg acaggtcggt cttgacaaaa    8160
   agaaccgggc gcccctgcgc tgacagccgg aacacggcgg catcagagca gccgattgtc    8220
   tgttgtgccc agtcatagcc gaatagcctc tccacccaag cggccggaga acctgcgtgc    8280
   aatccatctt gttcaatcat gcgaaacgat cctcatcctg tctcttgatc agagcttgat    8340
   cccctgcgcc atcagatcct tggcggcgag aaagccatcc agtttacttt gcagggcttg    8400
   tcaaccttac cagataaaag tgctcatcat tggaaaacat tcaattcgtc gacctcgaaa    8460
   ttctaccggg taggggaggc gcttttccca aggcagtctg gagcatgcgc tttagcagcc    8520
   ccgctgggca cttggcgcta cacaagtggc ctctggcctc gcacacattc cacatccacc    8580
   ggtaggcgcc aaccggctcc gttctttggt ggccccttcg cgccaccttc tactcctccc    8640
   ctagtcagga agttcccccc cgccccgcan ctcgcgtcgt gcaggacgtg acaaatggaa    8700
   atagcacgtc tcactagtct cgtgcagatg gacaagcacc gctgagcaat ggagcgggta    8760
   ggcctttggg gcagcggcca atagcagctt tgctccttcg ctttctgggc tcagaggctg    8820
   gnaaggggtg ggtccggggg cgggctcagg ggcgggctca ggggcggggc gggcgcccga    8880
   aggtcctccg gaggcccggc attctgcacg cttcaaaagc gcacgtctgc cgcgctgttc    8940
   tcctcttcct catctccggg cctttcgacc tgcatccatc tagatctcga gcagctgaag    9000
   cttaccatga ccgagtacaa gcccacggtg cgcctcgcca cccgcgacga cgtcccccgg    9060
   gccgtacgca ccctcgccgc cgcgttcgcc gactaccccg ccacgcgcca caccgtcgac    9120
   ccggaccgcc acatcgagcg ggtcaccgag ctgcaagaac tcttcctcac gcgcgtcggg    9180
   ctcgacatcg gcaaggtgtg ggtcgcggac gacggcgccg cggtggcggt ctggaccacg    9240
   ccggagagcg tcgaagcggg ggcggtgttc gccgagatcg gcccgcgcat ggccgagttg    9300
   agcggttccc ggctggccgc gcagcaacag atggaaggcc tcctggcgcc gcaccgggcc    9360
   caaggagccc gcgtggttcc ttggcccacc gtcgggcgtc ttcgcccgac caccagggca    9420
   agggtctggc aagcgccgtc gtgctccccg gagtggaggc ggccgagcgc gccggggtgc    9480
   ccgccttcct ggagacctcc gcgccccgca acctcccctt ctacgagcgg ctcggcttca    9540
   ccgtcaccgc cgacgtcgag gtgcccgaag gaccgcgcac ctggtgcatg acccgcaagc    9600
   ccggtgcctg acgcccgccc cacgacccgc agcgcccgac cgaaaggagc gcacgacccc    9660
   atgcatcgat ggcactgggc aggtaagtat caaggttagc ggccgctaac ctggttgctg    9720
   actaattgag atgcatgctt tgcatacttc tgcctgctgg ggagcctggg gactttccac    9780
   accctaactg acacacattc cacagctggt tctttccgcc tcagaaggta cacaggcgaa    9840
   attgtaagcg ttaatatttt gttaaaattc gcgttaaatt tttgttaaat cagctcattt    9900
   tttaaccaat aggccgaaat cggcaaaatc ccttataaat caaaagaata gaccgagata    9960
   gggttgagtg ttgttccagt ttggaacaag agtccactat taaagaacgt ggactccaac   10020
   gtcaaagggc gaaaaaccgt ctatcagggc gatggcccac                         10060
<210> 26
   <211> 7714
   <212> DNA
   <213> Artificial Sequence
<220>
   <223> pUniBAC
<400> 26
   tcaacgacag gagcacgatc atgcgcaccc gtggccagga cccaacgctg cccgagatgc      60
   gccgcgtgcg gctgctggag atggcggacg cgatggatat gttctgccaa gggttggttt     120
   gcgcattcac agttctccgc aagaattgat tggctccaat tcttggagtg gtgaatccgt     180
   tagcgaggtg ccgccggctt ccattcaggt cgaggtggcc cggctccatg caccgcgacg     240
   caacgcgggg aggcagacaa ggtatagggc ggcgcctaca atccatgcca acccgttcca     300
   tgtgctcgcc gaggcggcat aaatcgccgt gacgatcagc ggtccagtga tcgaagttag     360
   gctggtaaga gccgcgagcg atccttgaag ctgtccctga tggtcgtcat ctacctgcct     420
   ggacagcatg gcctgcaacg cgggcatccc gatgccgccg gaagcgagaa gaatcataat     480
   ggggaaggcc atccagcctc gcgtcgcgaa cgccagcaag acgtagccca gcgcgtcggc     540
   cgccatgccg gcgataatgg cctgcttctc gccgaaacgt ttggtggcgg gaccagtgac     600
   gaaggcttga gcgagggcgt gcaagattcc gaataccgca agcgacaggc cgatcatcgt     660
   cgcgctccag cgaaagcggt cctcgccgaa aatgacccag agcgctgccg gcacctgtcc    720
   tacgagttgc atgataaaga agacagtcat aagtgcggcg acgatagtca tgccccgcgc    780
   ccaccggaag gagctgactg ggttgaaggc tctcaagggc atcggtcgac gctctccctt    840
   atgcgactcc tgcattagga agcagcccag tagtaggttg aggccgttga gcaccgccgc    900
   cgcaaggaat ggtgcatgca aggagatggc gcccaacagt cccccggcca cggggcctgc    960
   caccataccc acgccgaaac aagcgctcat gagcccgaag tggcgagccc gatcttcccc   1020
   atcggtgatg tcggcgatat aggcgccagc aaccgcacct gtggcgccgg tgatgccggc   1080
   cacgatgcgt ccggcgtaga ggatccacag gacgggtgtg gtcgccatga tcgcgtagtc   1140
   gatagtggct ccaagtagcg aagcgagcag gactgggcgg cggccaaagc ggtcggacag   1200
   tgctccgaga acgggtgcgc atagaaattg catcaacgca tatagcgcta gcagcacgcc   1260
   atagtgactg gcgatgctgt cggaatggac gatatcccgc aagaggcccg gcagtaccgg   1320
   cataaccaag cctatgccta cagcatccag ggtgacggtg ccgaggatga cgatgagcgc   1380
   attgttagat ttcatacacg gtgcctgact gcgttagcaa tttaactgtg ataaactacc   1440
   gcattaaagc ttatcgattt ccacacatta tacgagccga tgttaattgt caacagctca   1500
   tgcatgacgt cccgggagca gacaagcccg tcagggcgcg tcagcgggtg ttggcgggtg   1560
   tcggggctgg cttaactatg cggcatcaga gcagattgta ctgagagtgc accatatgcg   1620
   gtgtgaaata ccgcacagat gcgtaaggag aaaataccgc atcaggcgcc attcgccatt   1680
   caggctgcgc aactgttggg aagggcgatc ggtgcgggcc tcttcgctat tacgccagct   1740
   ggcgaaaggg ggatgtgctg caaggcgatt aagttgggta acgccagggt tttcccagtc   1800
   acgacgttgt aaaacgacgg ccagtgaatt cgagctcata cttcgaatag ggataacagg   1860
   gtaatgcgat agcggccgca atcgctctct taaggtagcc cgtgctggca aacagctatt   1920
   atgggtatta tgggtgggcc ctagaaagct tggcgtaatc atggtcatag ctgtttcctg   1980
   tgtgaaattg ttatccgctc acaattccac acaacatacg agccggaagc ataaagtgta   2040
   aagcctgggg tgcctaatga gtgagctaac tcacattaat tgcgttgcgc tcactgcccg   2100
   ctttccagtc gggaaacctg tcgtgccagc tgcattaatg acccgcgagg tcgccgcccc   2160
   gtaaccccct accgctgaaa gttctgcaaa gcctgatggg acataagtcc atcagttcaa   2220
   cggaagtcta cacgaaggtt tttgcgctgg atgtggctgc ccggcaccgg gtgcagtttg   2280
   cgatgccgga gtctgatgcg gttgcgatgc tgaaacaatt atcctgagaa taaatgcctt   2340
   ggcctttata tggaaatgtg gaactgagtg gatatgctgt ttttgtctgt taaacagaga   2400
   agctggctgt tatccactga gaagcgaacg aaacagtcgg gaaaatctcc cattatcgta   2460
   gagatccgca ttattaatct caggagcctg tgtagcgttt ataggaagta gtgttctgtc   2520
   atgatgcctg caagcggtaa cgaaaacgat ttgaatatgc cttcaggaac aatagaaatc   2580
   ttcgtgcggt gttacgttga agtggagcgg attatgtcag caatggacag aacaacctaa   2640
   tgaacacaga accatgatgt ggtctgtcct tttacagcca gtagtgctcg ccgcagtcga   2700
   gcgacagggc gaagccctcg agtgagcgag gaagcaccag ggaacagcac ttatatattc   2760
   tgcttacaca cgatgcctga aaaaacttcc cttggggtta tccacttatc cacggggata   2820
   tttttataat tatttttttt atagttttta gatcttcttt tttagagcgc cttgtaggcc   2880
   tttatccatg ctggttctag agaaggtgtt gtgacaaatt gccctttcag tgtgacaaat   2940
   caccctcaaa tgacagtcct gtctgtgaca aattgccctt aaccctgtga caaattgccc   3000
   tcagaagaag ctgttttttc acaaagttat ccctgcttat tgactctttt ttatttagtg   3060
   tgacaatcta aaaacttgtc acacttcaca tggatctgtc atggcggaaa cagcggttat   3120
   caatcacaag aaacgtaaaa atagcccgcg aatcgtccag tcaaacgacc tcactgaggc   3180
   ggcatatagt ctctcccggg atcaaaaacg tatgctgtat ctgttcgttg accagatcag   3240
   aaaatctgat ggcaccctac aggaacatga cggtatctgc gagatccatg ttgctaaata   3300
   tgctgaaata ttcggattga cctctgcgga agccagtaag gatatacggc aggcattgaa   3360
   gagtttcgcg gggaaggaag tggtttttta tcgccctgaa gaggatgccg gcgatgaaaa   3420
   aggctatgaa tcttttcctt ggtttatcaa acgtgcgcac agtccatcca gagggcttta   3480
   cagtgtacat atcaacccat atctcattcc cttctttatc gggttacaga accggtttac   3540
   gcagtttcgg cttagtgaaa caaaagaaat caccaatccg tatgccatgc gtttatacga   3600
   atccctgtgt cagtatcgta agccggatgg ctcaggcatc gtctctctga aaatcgactg   3660
   gatcatagag cgttaccagc tgcctcaaag ttaccagcgt atgcctgact tccgccgccg   3720
   cttcctgcag gtctgtgtta atgagatcaa cagcagaact ccaatgcgcc tctcatacat   3780
   tgagaaaaag aaaggccgcc agacgactca tatcgtattt tccttccgcg atatcacttc   3840
   catgacgaca ggatagtctg agggttatct gtcacagatt tgagggtggt tcgtcacatt   3900
   tgttctgacc tactgagggt aatttgtcac agttttgctg tttccttcag cctgcatgga   3960
   ttttctcata ctttttgaac tgtaattttt aaggaagcca aatttgaggg cagtttgtca   4020
   cagttgattt ccttctcttt cccttcgtca tgtgacctga tatcgggggt tagttcgtca   4080
   tcattgatga gggttgatta tcacagttta ttactctgaa ttggctatcc gcgtgtgtac   4140
   ctctacctgg agtttttccc acggtggata tttcttcttg cgctgagcgt aagagctatc   4200
   tgacagaaca gttcttcttt gcttcctcgc cagttcgctc gctatgctcg gttacacggc   4260
   tgcggcgagc gctagtgata ataagtgact gaggtatgtg ctcttcttat ctccttttgt   4320
   agtgttgctc ttattttaaa caactttgcg gttttttgat gactttgcga ttttgttgtt 4380
   gctttgcagt aaattgcaag atttaataaa aaaacgcaaa gcaatgatta aaggatgttc 4440
   agaatgaaac tcatggaaac acttaaccag tgcataaacg ctggtcatga aatgacgaag 4500
   gctatcgcca ttgcacagtt taatgatgac agcccggaag cgaggaaaat aacccggcgc 4560
   tggagaatag gtgaagcagc ggatttagtt ggggtttctt ctcaggctat cagagatgcc 4620
   gagaaagcag ggcgactacc gcacccggat atggaaattc gaggacgggt tgagcaacgt 4680
   gttggttata caattgaaca aattaatcat atgcgtgatg tgtttggtac gcgattgcga 4740
   cgtgctgaag acgtatttcc accggtgatc ggggttgctg cccataaagg tggcgtttac 4800
   aaaacctcag tttctgttca tcttgctcag gatctggctc tgaaggggct acgtgttttg 4860
   ctcgtggaag gtaacgaccc ccagggaaca gcctcaatgt atcacggatg ggtaccagat 4920
   cttcatattc atgcagaaga cactctcctg cctttctatc ttggggaaaa ggacgatgtc 4980
   acttatgcaa taaagcccac ttgctggccg gggcttgaca ttattccttc ctgtctggct 5040
   ctgcaccgta ttgaaactga gttaatgggc aaatttgatg aaggtaaact gcccaccgat 5100
   ccacacctga tgctccgact ggccattgaa actgttgctc atgactatga tgtcatagtt 5160
   attgacagcg cgcctaacct gggtatcggc acgattaatg tcgtatgtgc tgctgatgtg 5220
   ctgattgttc ccacgcctgc tgagttgttt gactacacct ccgcactgca gtttttcgat 5280
   atgcttcgtg atctgctcaa gaacgttgat cttaaagggt tcgagcctga tgtacgtatt 5340
   ttgcttacca aatacagcaa tagtaatggc tctcagtccc cgtggatgga ggagcaaatt 5400
   cgggatgcct ggggaagcat ggttctaaaa aatgttgtac gtgaaacgga tgaagttggt 5460
   aaaggtcaga tccggatgag aactgttttt gaacaggcca ttgatcaacg ctcttcaact 5520
   ggtgcctgga gaaatgctct ttctatttgg gaacctgtct gcaatgaaat tttcgatcgt 5580
   ctgattaaac cacgctggga gattagataa tgaagcgtgc gcctgttatt ccaaaacata 5640
   cgctcaatac tcaaccggtt gaagatactt cgttatcgac accagctgcc ccgatggtgg 5700
   attcgttaat tgcgcgcgta ggagtaatgg ctcgcggtaa tgccattact ttgcctgtat 5760
   gtggtcggga tgtgaagttt actcttgaag tgctccgggg tgatagtgtt gagaagacct 5820
   ctcgggtatg gtcaggtaat gaacgtgacc aggagctgct tactgaggac gcactggatg 5880
   atctcatccc ttcttttcta ctgactggtc aacagacacc ggcgttcggt cgaagagtat 5940
   ctggtgtcat agaaattgcc gatgggagtc gccgtcgtaa agctgctgca cttaccgaaa 6000
   gtgattatcg tgttctggtt ggcgagctgg atgatgagca gatggctgca ttatccagat 6060
   tgggtaacga ttatcgccca acaagtgctt atgaacgtgg tcagcgttat gcaagccgat 6120
   tgcagaatga atttgctgga aatatttctg cgctggctga tgcggaaaat atttcacgta 6180
   agattattac ccgctgtatc aacaccgcca aattgcctaa atcagttgtt gctctttttt 6240
   ctcaccccgg tgaactatct gcccggtcag gtgatgcact tcaaaaagcc tttacagata 6300
   aagaggaatt acttaagcag caggcatcta accttcatga gcagaaaaaa gctggggtga 6360
   tatttgaagc tgaagaagtt atcactcttt taacttctgt gcttaaaacg tcatctgcat 6420
   caagaactag tttaagctca cgacatcagt ttgctcctgg agcgacagta ttgtataagg 6480
   gcgataaaat ggtgcttaac ctggacaggt ctcgtgttcc aactgagtgt atagagaaaa 6540
   ttgaggccat tcttaaggaa cttgaaaagc cagcaccctg atgcgaccac gttttagtct 6600
   acgtttatct gtctttactt aatgtccttt gttacaggcc agaaagcata actggcctga 6660
   atattctctc tgggccagaa gcttggccca ctgttccact tgtatcgtcg gtctgataat 6720
   cagactggga ccacggtccc actcgtatcg tcggtctgat tattagtctg ggaccacggt 6780
   cccactcgta tcgtcggtct gattattagt ctgggaccac ggtcccactc gtatcgtcgg 6840
   tctgataatc agactgggac cacggtccca ctcgtatcgt cggtctgatt attagtctgg 6900
   gaccatggtc ccactcgtat cgtcggtctg attattagtc tgggaccacg gtcccactcg 6960
   tatcgtcggt ctgattatta gtctggaacc acggtcccac tcgtatcgtc ggtctgatta 7020
   ttagtctggg accacggtcc cactcgtatc gtcggtctga ttattagtct gggaccacga 7080
   tcccactcgt gttgtcggtc tgattatcgg tctgggacca cggtcccact tgtattgtcg 7140
   atcagactat cagcgtgaga ctacgattcc atcaatgcct gtcaagggca agtattgaca 7200
   tgtcgtcgta acctgtagaa cggagtaacc tcggtgtgcg gttgtatgcc tgctgtggat 7260
   tgctgctgtg tcctgcttat ccacaacatt ttgcgcacgg ttatgtggac aaaatacctg 7320
   cgctagagaa aagagtttgt agaaacgcaa aaaggccatc cgtcaggatg gccttctgct 7380
   taatttgatg cctggcagtt tatggcgggc gtcctgcccg ccaccctccg ggccgttgct 7440
   tcgcaacgtt caaatccgct cccggcggat ttgtcctact caggagagcg ttcaccgaca 7500
   aacaacagat aaaacgaaag gcccagtctt tcgactgagc ctttcgtttt atttgatgcc 7560
   tggcagttcc ctactctcgc atggggagac cccacactac catcggcgct acggcgtttc 7620
   acttctgagt tcggcatggg gtcaggtggg accaccgcgc tactgccgcc aggcaaattc 7680
   tgttttatca gaccgcttct gcgttctggg ccgc 7714
<210> 27
   <211> 5314
   <212> DNA
   <213> Artificial Sequence
<220>
   <223> pRIG22 plasmid
<400> 27
   gatcttcaat attggccatt agccatatta ttcattggtt atatagcata aatcaatatt      60
   ggctattggc cattgcatac gttgtatcta tatcataata tgtacattta tattggctca     120
   tgtccaatat gaccgccatg ttggcattga ttattgacta gttattaata gtaatcaatt     180
   acggggtcat tagttcatag cccatatatg gagttccgcg ttacataact tacggtaaat     240
   ggcccgcctg gctgaccgcc caacgacccc cgcccattga cgtcaataat gacgtatgtt     300
   cccatagtaa cgccaatagg gactttccat tgacgtcaat gggtggagta tttacggtaa     360
   actgcccact tggcagtaca tcaagtgtat catatgccaa gtccgccccc tattgacgtc     420
   aatgacggta aatggcccgc ctggcattat gcccagtaca tgaccttacg ggactttcct     480
   acttggcagt acatctacgt attagtcatc gctattacca tggtgatgcg gttttggcag     540
   tacaccaatg ggcgtggata gcggtttgac tcacggggat ttccaagtct ccaccccatt     600
   gacgtcaatg ggagtttgtt ttggcaccaa aatcaacggg actttccaaa atgtcgtaac     660
   aactgcgatc gcccgccccg ttgacgcaaa tgggcggtag gcgtgtacgg tgggaggtct     720
   atataagcag agctcgttta gtgaaccgtc agatcactga attctgacga cctactgatt     780
   aacggccata gaggcctcct gcagaactgt cttagtgaca actatcgatt tccacacatt     840
   atacgagccg atgttaattg tcaacagctc atgcatgacg tcccgggagc agacaagccc     900
   gaccatggct cgagtaatac gactcactat agggcgacag gtgagtactc gctaccttaa     960
   gagaggccta tctggccagt tagcagtcga agaaagaagt ttaagagagc cgaaacaagc    1020
   gctcatgagc ccgaagtggc gagcccgatc ttccccatcg gtgatgtcgg cgatataggc    1080
   gccagcaacc gcacctgtgg cgccggtgat gccggccacg atgcgtccgg cgtagaggat    1140
   ccacaggacg ggtgtggtcg ccatgatcgc gtagtcgata gtggctccaa gtagcgaagc    1200
   gagcaggact gggcggcggc caaagcggtc ggacagtgct ccgagaacgg gtgcgcatag    1260
   aaattgcatc aacgcatata gcgctagatc cttgctagag tcgagatctg tcgagccatg    1320
   tgagcaaaag gccagcaaaa ggccaggaac cgtaaaaagg ccgcgttgct ggcgtttttc    1380
   cataggctcc gcccccctga cgagcatcac aaaaatcgac gctcaagtca gaggtggcga    1440
   aacccgacag gactataaag ataccaggcg tttccccctg gaagctccct cgtgcgctct    1500
   cctgttccga ccctgccgct taccggatac ctgtccgcct ttctcccttc gggaagcgtg    1560
   gcgctttctc atagctcacg ctgtaggtat ctcagttcgg tgtaggtcgt tcgctccaag    1620
   ctgggctgtg tgcacgaacc ccccgttcag cccgaccgct gcgccttatc cggtaactat    1680
   cgtcttgagt ccaacccggt aagacacgac ttatcgccac tggcagcagc cactggtaac    1740
   aggattagca gagcgaggta tgtaggcggt gctacagagt tcttgaagtg gtggcctaac    1800
   tacggctaca ctagaaggac agtatttggt atctgcgctc tgctgaagcc agttaccttc    1860
   ggaaaaagag ttggtagctc ttgatccggc aaacaaacca ccgctggtag cggtggtttt    1920
   tttgtttgca agcagcagat tacgcgcaga aaaaaaggat ctcaagaaga tcctttgatc    1980
   ttttctacgg ggtctgacgc tcagtggaac gaaaactcac gttaagggat tttggtcatg    2040
   agattatcaa aaaggatctt cacctagatc cttttatcgg tgtgaaatac cgcacagatg    2100
   cgtaaggaga aaataccgca tcaggaaatt gtaagcgtta ataattcaga agaactcgtc    2160
   aagaaggcga tagaaggcga tgcgctgcga atcgggagcg gcgataccgt aaagcacgag    2220
   gaagcggtca gcccattcgc cgccaagctc ttcagcaata tcacgggtag ccaacgctat    2280
   gtcctgatag cggtccgcca cacccagccg gccacagtcg atgaatccag aaaagcggcc    2340
   attttccacc atgatattcg gcaagcaggc atcgccatgg gtcacgacga gatcctcgcc    2400
   gtcgggcatg ctcgccttga gcctggcgaa cagttcggct ggcgcgagcc cctgatgctc    2460
   ttcgtccaga tcatcctgat cgacaagacc ggcttccatc cgagtacgtg ctcgctcgat    2520
   gcgatgtttc gcttggtggt cgaatgggca ggtagccgga tcaagcgtat gcagccgccg    2580
   cattgcatca gccatgatgg atactttctc ggcaggagca aggtgagatg acaggagatc    2640
   ctgccccggc acttcgccca atagcagcca gtcccttccc gcttcagtga caacgtcgag    2700
   cacagctgcg caaggaacgc ccgtcgtggc cagccacgat agccgcgctg cctcgtcttg    2760
   cagttcattc agggcaccgg acaggtcggt cttgacaaaa agaaccgggc gcccctgcgc    2820
   tgacagccgg aacacggcgg catcagagca gccgattgtc tgttgtgccc agtcatagcc    2880
   gaatagcctc tccacccaag cggccggaga acctgcgtgc aatccatctt gttcaatcat    2940
   gcgaaacgat cctcatcctg tctcttgatc agagcttgat cccctgcgcc atcagatcct    3000
   tggcggcgag aaagccatcc agtttacttt gcagggcttg tcaaccttac cagataaaag    3060
   tgctcatcat tggaaaacgt tcaattctga ggcggaaaga accagctgtg gaatgtgtgt    3120
   cagttagggt gtggaaagtc cccaggctcc ccagcaggca gaagtatgca aagcatgcat    3180
   ctcaattagt cagcaaccag gtgtggaaag tccccaggct ccccagcagg cagaagtatg 3240
   caaagcatgc atctcaatta gtcagcaacc atagtcccgc ccctaactcc gcccatcccg 3300
   cccctaactc cgcccagttc cgcccattct ccgccccatg gctgactaat tttttttatt 3360
   tatgcagagg ccgaggccgc ctcggcctct gagctattcc agaagtagtg aggaggcttt 3420
   tttggaggcc taggcttttg caaaaagctt gattcttctg acacaacagt ctcgaactta 3480
   aggctagagc caccatgatt gaacaagatg gattgcacgc aggttctccg gccgcttggg 3540
   tggagaggct attcggctat gactgggcac aacagacaat cggctgctct gatgccgccg 3600
   tgttccggct gtcagcgcag gggcgcccgg ttctttttgt caagaccgac ctgtccggtg 3660
   ccctgaatga actgcaggac gaggcagcgc ggctatcgtg gctggccacg acgggcgttc 3720
   cttgcgcagc tgtgctcgac gttgtcactg aagcgggaag ggactggctg ctattgggcg 3780
   aagtgccggg gcaggatctc ctgtcatctc accttgctcc tgccgagaaa gtatccatca 3840
   tggctgatgc aatgcggcgg ctgcatacgc ttgatccggc tacctgccca ttcgaccacc 3900
   aagcgaaaca tcgcatcgag cgagcacgta ctcggatgga agccggtctt gtcgatcagg 3960
   atgatctgga cgaagagcat caggggctcg cgccagccga actgttcgcc aggctcaagg 4020
   cgcgcatgcc cgacggcgag gatctcgtcg tgacccatgg cgatgcctgc ttgccgaata 4080
   tcatggtgga aaatggccgc ttttctggat tcatcgactg tggccggctg ggtgtggcgg 4140
   accgctatca ggacatagcg ttggctaccc gtgatattgc tgaagagctt ggcggcgaat 4200
   gggctgaccg cttcctcgtg ctttacggta tcgccgctcc cgattcgcag cgcatcgcct 4260
   tctatcgcct tcttgacgag ccattctgct ggatggctac aggtcgcagc cctggcgtcg 4320
   tgattagtga tgatgaacca ggttatgacc ttgatttatt ttgcatacct aatcattatg 4380
   ctgaggattt ggaaagggtg tttattcctc atggactaat tatggacagg actgaacgtc 4440
   ttgctcgaga tgtgatgaag gagatgggag gccatcacat tgtagccctc tgtgtgctca 4500
   aggggggcta taaattcttt gctgacctgc tggattacat caaagcactg aatagaaata 4560
   gtgatagatc cattcctatg actgtagatt ttatcagact gaagagctat tgtaatgacc 4620
   agtcaacagg ggacataaaa gtaattggtg gagatgatct ctcaacttta actggaaaga 4680
   atgtcttgat tgtggaagat ataattgaca ctggcaaaac aatgcagact ttgctttcct 4740
   tggtcaggca gtataatcca aagatggtca aggtcgcaag cttgctggtg aaaaggaccc 4800
   cacgaagtgt tggatataag ccagactttg ttggatttga aattccagac aagtttgttg 4860
   taggatatgc ccttgactat aatgaatact tcagggattt gaatcatgtt tgtgtcatta 4920
   gtgaaactgg aaaagcaaaa tacaaagcct aagcggccgc taacctggtt gctgactaat 4980
   tgagatgcat gctttgcata cttctgcctg ctggggagcc tggggacttt ccacacccta 5040
   actgacacac attccacagc tggttctttc cgcctcagaa ggtacacagg cgaaattgta 5100
   agcgttaata ttttgttaaa attcgcgtta aatttttgtt aaatcagctc attttttaac 5160
   caataggccg aaatcggcaa aatcccttat aaatcaaaag aatagaccga gatagggttg 5220
   agtgttgttc cagtttggaa caagagtcca ctattaaaga acgtggactc caacgtcaaa 5280
   gggcgaaaaa ccgtctatca gggcgatggc ccac 5314
<210> 28
   <211> 9737
   <212> DNA
   <213> Artificial Sequence
<220>
   <223> pRIG-T
<221> misc_feature
   <222> (1)...(9737)
   <223> n = A,T,C or G
<400> 28
   gatcttcaat attggccatt agccatatta ttcattggtt atatagcata aatcaatatt      60
   ggctattggc cattgcatac gttgtatcta tatcataata tgtacattta tattggctca     120
   tgtccaatat gaccgccatg ttggcattga ttattgacta gttattaata gtaatcaatt     180
   acggggtcat tagttcatag cccatatatg gagttccgcg ttacataact tacggtaaat     240
   ggcccgcctg gctgaccgcc caacgacccc cgcccattga cgtcaataat gacgtatgtt     300
   cccatagtaa cgccaatagg gactttccat tgacgtcaat gggtggagta tttacggtaa     360
   actgcccact tggcagtaca tcaagtgtat catatgccaa gtccgccccc tattgacgtc     420
   aatgacggta aatggcccgc ctggcattat gcccagtaca tgaccttacg ggactttcct     480
   acttggcagt acatctacgt attagtcatc gctattacca tggtgatgcg gttttggcag     540
   tacaccaatg ggcgtggata gcggtttgac tcacggggat ttccaagtct ccaccccatt     600
   gacgtcaatg ggagtttgtt ttggcaccaa aatcaacggg actttccaaa atgtcgtaac     660
   aactgcgatc gcccgccccg ttgacgcaaa tgggcggtag gcgtgtacgg tgggaggtct     720
   atataagcag agctcgttta gtgaaccgtc agatcactga attctgacga cctactgatt     780
   aacggccata gaggcctcct gcagaactgt cttagtgaca actatcgatt tccacacatt     840
   atacgagccg atgttaattg tcaacagctc atgcatgacg tcccgggagc agacaagccc     900
   gaccatggct cgagtaatac gactcactat agggcgacag gtgagtactc gctaccttaa     960
   ggcctatctg gccgtttaaa cagatgtgta taagagacag ctctcttaag gtagcctgtc    1020
   tcttatacac atctagatcc ttgctagagt cgaccaattc tcatgtttga cagcttatca    1080
   tcgcagatcc tgagcttgta tggtgcactc tcagtacaat ctgctctgct gccgcatagt    1140
   taagccagta tctgctccct gcttgtgtgt tggaggtcgc tgagtagtgc gcgagcaaaa    1200
   tttaagctac aacaaggcaa ggcttgaccg acaattgcat gaagaatctg cttagggtta    1260
   ggcgttttgc gctgcttcgc gatgtacggg ccagatatac gcgtatctga ggggactagg    1320
   gtgtgtttag gcgcccagcg gggcttcggt tgtacgcggt taggagtccc ctcaggatat     1380
   agtagtttcg cttttgcata gggaggggga aatgtagtct tatgcaatac acttgtagtc     1440
   ttgcaacatg gtaacgatga gttagcaaca tgccttacaa ggagagaaaa agcaccgtgc    1500
   atgccgattg gtggaagtaa ggtggtacga tcgtgcctta ttaggaaggc aacagacagg    1560
   tctgacatgg attggacgaa ccactgaatt ccgcattgca gagataattg tatttaagtg    1620
   cctagctcga tacaataaac gccatttgac cattcaccac attggtgtgc acctccaagc    1680
   tgggtaccag ctgctagcct cgagacgcgt gatttccttc gaagcttgtc atggttggtt    1740
   cgctaaactg catcgtcgct gtgtcccaga acatgggcat cggcaagaac ggggacctgc    1800
   cctggccacc gctcaggaat gaattcagat atttccagag aatgaccaca acctcttcag    1860
   tagaaggtaa acagaatctg gtgattatgg gtaagaagac ctggttctcc attcctgaga    1920
   agaatcgacc tttaaagggt agaattaatt tagttctcag cagagaactc aaggaacctc    1980
   cacaaggagc tcattttctt tccagaagtc tagatgatgc cttaaaactt actgaacaac    2040
   cagaattagc aaataaagta gacatggtct ggatagttgg tggcagttct gtttataagg    2100
   aagccatgaa tcacccaggc catcttaaac tatttgtgac aaggatcatg caagactttg    2160
   aaagtgacac gttttttcca gaaattgatt tggagaaata taaacttctg ccagaatacc   2220
   caggtgttct ctctgatgtc caggaggaga aaggcattaa gtacaaattt gaagtatatg    2280
   agaagaatgt taattaaggg caccaataac tgccttaaaa aaattacgcc ccgccctgcc    2340
   actcatcgca gtactgttgt aattcattaa gcattctgcc gacatggaag ccatcacaga    2400
   cggcatgatg aacctgaatc gccagcggca tcagcacctt gtcgccttgc gtataatatt    2460
   tgcccatggt gaaaacgggg gcgaagaagt tgtccatatt ggccacgttt aaatcaaaac    2520
   tggtgaaact cacccaggga ttggctgaga cgaaaaacat attctcaata aaccctttag    2580
   ggaaataggc caggttttca ccgtaacacg ccacatcttg cgaatatatg tgtagaaact    2640
   gccggaaatc gtcgtggtat tcactccaga gcgatgaaaa cgtttcagtt tgctcatgga    2700
   aaacggtgta acaagggtga acactatccc atatcaccag ctcaccgtct ttcattgcca    2760
   tacggaattc cggatgagca ttcatcaggc gggcaagaat gtgaataaag gccggataaa    2820
   acttgtgctt atttttcttt acggtcttta aaaaggccgt aatatccagc tgaacggtct    2880
   ggttataggt acattgagca actgactgaa atgcctcaaa atgttcttta cgatgccatt    2940
   gggatatatc aacggtggta tatccagtga tttttttctc cattttagct tccttagctc    3000
   ctgaaaatct cgataactca aaaaatacgc ccggtagtga tcttatttca ttatggtgaa    3060
   agttggaacc tcttacgtgc cgatcaacgt ctcattttcg ccaaattaat taaggcgcgc    3120
   cgctctcctg gctaggagtc acgtagaaag gactaccgac gaaggaactt gggtcgccgg    3180
   tgtgttcgta tatggaggta gtaagacctc cctttacaac ctaaggcgag gaactgccct    3240
   tgctattcca caatgtcgtc ttacaccatt gagtcgtctc ccctttggaa tggcccctgg    3300
   acccggccca caacctggcc cgctaaggga gtccattgtc tgttatttca tggtcttttt    3360
   acaaactcat atatttgctg aggttttgaa ggatgcgatt aaggaccttg ttatgacaaa    3420
   gcccgctcct acctgcaata tcagggtgac tgtgtgcagc tttgacgatg gagtagattt    3480
   gcctccctgg tttccaccta tggtggaagg ggctgccgcg gagggtgatg acggagatga    3540
   cggagatgaa ggaggtgatg gagatgaggg tgaggaaggg caggagtgat gtaacttgtt    3600
   aggagacgcc ctcaatcgta ttaaaagccg tgtattcccc cgcactaaag aataaatccc    3660
   cagtagacat catgcgtgct gttggtgtat ttctggccat ctgtcttgtc accattttcg    3720
   tcctcccaac atggggcaat tgggcatacc catgttgtca cgtcactcag ctccgcgctc    3780
   aacaccttct cgcgttggaa aacattagcg acatttacct ggtgagcaat cagacatgcg    3840
   acggctttag cctggcctcc ttaaattcac ctaagaatgg gagcaaccag catgcaggaa    3900
   aaggacaagc agcgaaaatt cacgccccct tgggaggtgg cggcatatgc aaaggatagc   3960
   actcccactc tactactggg tatcatatgc tgactgtata tgcatgagga tagcatatgc    4020
   tacccggata cagattagga tagcatatac tacccagata tagattagga tagcatatgc    4080
   tacccagata tagattagga tagcctatgc tacccagata taaattagga tagcatatac    4140
   tacccagata tagattagga tagcatatgc tacccagata tagattagga tagcctatgc    4200
   tacccagata tagattagga tagcatatgc tacccagata tagattagga tagcatatgc 4260
   tatccagata tttgggtagt atatgctacc cagatataaa ttaggatagc atatactacc 4320
   ctaatctcta ttaggatagc atatgctacc cggatacaga ttaggatagc atatactacc 4380
   cagatataga ttaggatagc atatgctacc cagatataga ttaggatagc ctatgctacc 4440
   cagatataaa ttaggatagc atatactacc cagatataga ttaggatagc atatgctacc 4500
   cagatataga ttaggatagc ctatgctacc cagatataga ttaggatagc atatgctatc 4560
   cagatatttg ggtagtatat gctacccatg gcaacattag cccaccgtgc tctcagcgac 4620
   ctcgtgaata tgaggaccaa caaccctgtg cttggcgctc aggcgcaagt gtgtgtaatt 4680
   tgtcctccag atcgcagcaa tcgcgcccct atcttggccc gcccacctac ttatgcaggt 4740
   attccccggg gtgccattag tggttttgtg ggcaagtggt ttgaccgcag tggttagcgg 4800
   ggttacaatc agccaagtta ttacaccctt attttacagt ccaaaaccgc agggcggcgt 4860
   gtgggggctg acgcgtgccc ccactccaca atttcaaaaa aaagagtggc cacttgtctt 4920
   tgtttatggg ccccattggc gtggagcccc gtttaatttt cgggggtgtt agagacaacc 4980
   agtggagtcc gctgctgtcg gcgtccactc tctttcccct tgttacaaat agagtgtaac 5040
   aacatggttc acctgtcttg gtccctgcct gggacacatc ttaataaccc cagtatcata 5100
   ttgcactagg attatgtgtt gcccatagcc ataaattcgt gtgagatgga catccagtct 5160
   ttacggcttg tccccacccc atggatttct attgttaaag atattcagaa tgtttcattc 5220
   ctacactagt atttattgcc caaggggttt gtgagggtta tattggtgtc atagcacaat 5280
   gccaccactg aaccccccgt ccaaatttta ttctgggggc gtcacctgaa accttgtttt 5340
   cgagcacctc acatacacct tactgttcac aactcagcag ttattctatt agctaaacga 5400
   aggagaatga agaagcaggc gaagattcag gagagttcac tgcccgctcc ttgatcttca 5460
   gccactgccc ttgtgactaa aatggttcac taccctcgtg gaatcctgac cccatgtaaa 5520
   taaaaccgtg acagctcatg gggtgggaga tatcgctgtt ccttaggacc cttttactaa 5580
   ccctaattcg atagcatatg cttcccgttg ggtaacatat gctattgaat tagggttagt 5640
   ctggatagta tatactacta cccgggaagc atatgctacc cgtttagggt taacaagggg 5700
   gccttataaa cactattgct aatgccctct tgagggtccg cttatcggta gctacacagg 5760
   cccctctgat tgacgttggt gtagcctccc gtagtcttcc tgggcccctg ggaggtacat 5820
   gtcccccagc attggtgtaa gagcttcagc caagagttac acataaaggc aatgttgtgt 5880
   tgcagtccac agactgcaaa gtctgctcca ggatgaaagc cactcagtgt tggcaaatgt 5940
   gcacatccat ttataaggat gtcaactaca gtcagagaac ccctttgtgt ttggtccccc 6000
   cccgtgtcac atgtggaaca gggcccagtt ggcaagttgt accaaccaac tgaagggatt 6060
   acatgcactg ccccgaatac aaaacaaaag cgctcctcgt accagcgaag aaggggcaga 6120
   gatgccgtag tcaggtttag ttcgtccggc ggcgggcggc cgcaaggcgc gccggatcca 6180
   caggacgggt gtggtcgcca tgatcgcgta gtcgatagtg gctccaagta gcgaagcgag 6240
   caggactggg cggcggccaa agcggtcgga cagtgctccg agaacgggtg cgcatagaaa 6300
   ttgcatcaac gcatatagcg ctagatcctt gctagagtcg agatctgtcg agccatgtga 6360
   gcaaaaggcc agcaaaaggc caggaaccgt aaaaaggccg cgttgctggc gtttttccat 6420
   aggctccgcc cccctgacga gcatcacaaa aatcgacgct caagtcagag gtggcgaaac 6480
   ccgacaggac tataaagata ccaggcgttt ccccctggaa gctccctcgt gcgctctcct 6540
   gttccgaccc tgccgcttac cggatacctg tccgcctttc tcccttcggg aagcgtggcg 6600
   ctttctcata gctcacgctg taggtatctc agttcggtgt aggtcgttcg ctccaagctg 6660
   ggctgtgtgc acgaaccccc cgttcagccc gaccgctgcg ccttatccgg taactatcgt 6720
   cttgagtcca acccggtaag acacgactta tcgccactgg cagcagccac tggtaacagg 6780
   attagcagag cgaggtatgt aggcggtgct acagagttct tgaagtggtg gcctaactac 6840
   ggctacacta gaaggacagt atttggtatc tgcgctctgc tgaagccagt taccttcgga 6900
   aaaagagttg gtagctcttg atccggcaaa caaaccaccg ctggtagcgg tggttttttt 6960
   gtttgcaagc agcagattac gcgcagaaaa aaaggatctc aagaagatcc tttgatcttt 7020
   tctacggggt ctgacgctca gtggaacgaa aactcacgtt aagggatttt ggtcatgaga 7080
   ttatcaaaaa ggatcttcac ctagatcctt ttatcggtgt gaaataccgc acagatgcgt 7140
   aaggagaaaa taccgcatca ggaaattgta agcgttaata attcagaaga actcgtcaag 7200
   aaggcgatag aaggcgatgc gctgcgaatc gggagcggcg ataccgtaaa gcacgaggaa 7260
   gcggtcagcc cattcgccgc caagctcttc agcaatatca cgggtagcca acgctatgtc 7320
   ctgatagcgg tccgccacac ccagccggcc acagtcgatg aatccagaaa agcggccatt 7380
   ttccaccatg atattcggca agcaggcatc gccatgggtc acgacgagat cctcgccgtc 7440
   gggcatgctc gccttgagcc tggcgaacag ttcggctggc gcgagcccct gatgctcttc 7500
   gtccagatca tcctgatcga caagaccggc ttccatccga gtacgtgctc gctcgatgcg 7560
   atgtttcgct tggtggtcga atgggcaggt agccggatca agcgtatgca gccgccgcat 7620
   tgcatcagcc atgatggata ctttctcggc aggagcaagg tgagatgaca ggagatcctg 7680
   ccccggcact tcgcccaata gcagccagtc ccttcccgct tcagtgacaa cgtcgagcac 7740
   agctgcgcaa ggaacgcccg tcgtggccag ccacgatagc cgcgctgcct cgtcttgcag 7800
   ttcattcagg gcaccggaca ggtcggtctt gacaaaaaga accgggcgcc cctgcgctga 7860
   cagccggaac acggcggcat cagagcagcc gattgtctgt tgtgcccagt catagccgaa 7920
   tagcctctcc acccaagcgg ccggagaacc tgcgtgcaat ccatcttgtt caatcatgcg 7980
   aaacgatcct catcctgtct cttgatcaga gcttgatccc ctgcgccatc agatccttgg 8040
   cggcgagaaa gccatccagt ttactttgca gggcttgtca accttaccag ataaaagtgc 8100
   tcatcattgg aaaacattca attcgtcgac ctcgaaattc taccgggtag gggaggcgct 8160
   tttcccaagg cagtctggag catgcgcttt agcagccccg ctgggcactt ggcgctacac 8220
   aagtggcctc tggcctcgca cacattccac atccaccggt aggcgccaac cggctccgtt 8280
   ctttggtggc cccttcgcgc caccttctac tcctccccta gtcaggaagt tcccccccgc 8340
   cccgcanctc gcgtcgtgca ggacgtgaca aatggaaata gcacgtctca ctagtctcgt 8400
   gcagatggac aagcaccgct gagcaatgga gcgggtaggc ctttggggca gcggccaata 8460
   gcagctttgc tccttcgctt tctgggctca gaggctggna aggggtgggt ccgggggcgg 8520
   gctcaggggc gggctcaggg gcggggcggg cgcccgaagg tcctccggag gcccggcatt 8580
   ctgcacgctt caaaagcgca cgtctgccgc gctgttctcc tcttcctcat ctccgggcct 8640
   ttcgacctgc atccatctag atctcgagca gctgaagctt accatgaccg agtacaagcc 8700
   cacggtgcgc ctcgccaccc gcgacgacgt cccccgggcc gtacgcaccc tcgccgccgc 8760
   gttcgccgac taccccgcca cgcgccacac cgtcgacccg gaccgccaca tcgagcgggt 8820
   caccgagctg caagaactct tcctcacgcg cgtcgggctc gacatcggca aggtgtgggt 8880
   cgcggacgac ggcgccgcgg tggcggtctg gaccacgccg gagagcgtcg aagcgggggc 8940
   ggtgttcgcc gagatcggcc cgcgcatggc cgagttgagc ggttcccggc tggccgcgca 9000
   gcaacagatg gaaggcctcc tggcgccgca ccgggcccaa ggagcccgcg tggttccttg 9060
   gcccaccgtc gggcgtcttc gcccgaccac cagggcaagg gtctggcaag cgccgtcgtg 9120
   ctccccggag tggaggcggc cgagcgcgcc ggggtgcccg ccttcctgga gacctccgcg 9180
   ccccgcaacc tccccttcta cgagcggctc ggcttcaccg tcaccgccga cgtcgaggtg 9240
   cccgaaggac cgcgcacctg gtgcatgacc cgcaagcccg gtgcctgacg cccgccccac 9300
   gacccgcagc gcccgaccga aaggagcgca cgaccccatg catcgatggc actgggcagg 9360
   taagtatcaa ggttagcggc cgctaacctg gttgctgact aattgagatg catgctttgc 9420
   atacttctgc ctgctgggga gcctggggac tttccacacc ctaactgaca cacattccac 9480
   agctggttct ttccgcctca gaaggtacac aggcgaaatt gtaagcgtta atattttgtt 9540
   aaaattcgcg ttaaattttt gttaaatcag ctcatttttt aaccaatagg ccgaaatcgg 9600
   caaaatccct tataaatcaa aagaatagac cgagataggg ttgagtgttg ttccagtttg 9660
   gaacaagagt ccactattaa agaacgtgga ctccaacgtc aaagggcgaa aaaccgtcta 9720
   tcagggcgat ggcccac                                                9737
<210> 29
   <211> 9
   <212> DNA
   <213> Artificial Sequence
<220>
   <223> 5' splice site consensus sequence
<400> 29
   aagguragu 9
<210> 30
   <211> 6
   <212> DNA
   <213> Artificial Sequence
<220>
   <223> 5' splice site
<400> 30
   guragu 6

## Claims

1. A eukaryotic host cell which is *in vitro,* the cell comprising a vector non-homologously integrated into the genome of the cell, the vector comprising:
(a) a first promoter operably linked to an exon and an unpaired splice donor site; and
(b) a second promoter operably linked to a selectable marker lacking a polyadenylation signal,
where said first and second promoters are present in said vector in the same orientation, both the first and second promoters function in the eukaryotic cell, the vector is non-homologously integrated into the genome of the cell so that the unpaired splice donor site of the vector is paired with a splice acceptor site from an endogenous gene and where the endogenous gene is activated or over-expressed to produce protein.

2. The cell of claim 1, wherein the selectable marker is located 3' to said first promoter.

3. The cell of claim 1, wherein the said second promoter is located 5' to the unpaired splice donor site.

4. The cell of any one of the preceding claims, wherein said exon comprises a translation start codon and a signal secretion sequence.

5. The cell of any one of the preceding claims said vector further comprising one or more transposition signals.

6. The cell of any one of the preceding claims said vector further comprising one or more amplifiable markers.

7. The ceil of claim 6, wherein said amplifiable marker is selected from the group consisting of dihydrofolate reductase, adenosine deaminase, aspartate transcarbarnylase, dihydro-orotase, and carbamyl phosphate synthase.

8. The cell of any one of the preceding claims, said vector further comprising one or more viral origins of replication.

9. The cell of claim 8, wherein said viral origin of replication is selected from the group consisting of Epstein Barr virus ori P and SV40 ori.

10. The cell of any one of the preceding claims, said vector further comprising one or more viral replication factor genes.

11. The cell of any one of the preceding claims, said vector further comprising genomic DNA.

12. The cell of any one of the preceding claims, wherein the first and/or second promoter is/are selected from a CMV immediate early gene promoter, an SV40T antigen promoter, a tetracycline-inducible promoter, and a β-actin promoter.

13. A cell of any one of the preceding claims, wherein said selectable marker is selected from a neomycin gene, a hypoxanthine phosphribosyl transferase gene, a puromycin gene, a dihydrooratase gene, a glutamine synthetase gene, a histidine D gene, a carbamyl phosphate synthase gene, a dihydrofolate reductase gene, a multidrug resistance 1 gene, an aspartate transcarbamylase gene, a xanthine-guanine phosphoribosyl transferase gene, an adenosine deaminase gene, or a thymidine kinase gene.

14. The cell of any one of the preceding claims, wherein said host cell is an isolated and cloned cell.

15. A library of cells comprising cells according to any one of the preceding claims.

16. An *in vitro* method for activating or over-expressing an endogenous gene to produce protein in a cell comprising:
(a) transfecting a genome-containing cell with a vector where the vector comprises:(i) a first promoter operably linked to an exon and an unpaired splice donor site; and (ii) a second promoter operably linked to a selectable marker lacking a polyadenylation signal, where said first and second promoters are present in said vector in the same orientation and both the first and second promoters function in the eukaryotic cell; and
(b) allowing the vector to integrate non-homologously into the genome of the cell so that the unpaired donor site of the vector is paired with a splice acceptor site of the endogenous gene.

17. A method according to claim 16, wherein the vector is as defined in any one of claims 2 to 14.

## Patentansprüche

1. Eukaryotische Wirtszelle, die in vitro vorliegt, wobei die Zelle einen Vektor umfasst, der nicht-homolog in das Genom der Zelle integriert ist, wobei der Vektor umfasst:
(a) einen ersten Promotor, der funktionell mit einem Exon und einer ungepaarten Spleißdonorstelle verknüpft ist, und
(b) einen zweiten Promotor, der funktionell mit einem selektierbaren Marker verknüpft ist, der kein Polyadenylierungssignal aufweist,
wobei der erste und der zweite Promotor in dem Vektor in der gleichen Orientierung vorliegen, wobei sowohl der erste als auch der zweite Promotor in der eukaryotischen Zelle eine Funktion ausüben, der Vektor derart nicht-homolog in das Genom der Zelle integriert ist, so dass die ungepaarte Spleißdonorstelle des Vektors mit einer Spleißakzeptorstelle von einem endogenen Gen gepaart ist, und wobei das endogene Gen aktiviert oder überexprimiert ist, um Protein zu erzeugen.

2. Zelle nach Anspruch 1, bei welcher der selektierbare Marker 3' zu dem ersten Promotor angeordnet ist.

3. Zelle nach Anspruch 1, bei welcher der zweite Promotor 5' zu der ungepaarten Spleißdonorstelle angeordnet ist.

4. Zelle nach einem der vorstehenden Ansprüche, bei der das Exon ein Translationsstartkodon und eine Signalabgabesequenz umfasst.

5. Zelle nach einem der vorstehenden Ansprüche, bei welcher der Vektor ferner ein oder mehrere Transpositionssignal(e) umfasst.

6. Zelle nach einem der vorstehenden Ansprüche, bei welcher der Vektor ferner einen oder mehrere amplifizierbare(n) Marker umfasst.

7. Zelle nach Anspruch 6, bei welcher der amplifizierbare Marker aus der Gruppe, bestehend aus Dihydrofolatreduktase, Adenosindeaminase, Aspartattranscarbamylase, Dihydroorotase und Carbamylphosphatsynthase, ausgewählt ist.

8. Zelle nach einem der vorstehenden Ansprüche, bei welcher der Vektor ferner einen viralen Replikationsursprung oder mehrere virale Replikationsursprünge umfasst.

9. Zelle nach Anspruch 8, bei welcher der virale Replikationsursprung aus der Gruppe, bestehend aus Epstein-Barr-Virus ori P und SV40 ori, ausgewählt ist.

10. Zelle nach einem der vorstehenden Ansprüche, bei welcher der Vektor ferner ein oder mehrere virale Replikationsfaktorgen(e) umfasst.

11. Zelle nach einem der vorstehenden Ansprüche, bei welcher der Vektor ferner genomische DNA umfasst.

12. Zelle nach einem der vorstehenden Ansprüche, bei welcher der erste und/oder der zweite Promotor aus einem CMV-unmittelbar frühes Gen-Promotor, einem SV40T-Antigenpromotor, einem Tetracyclin-induzierbaren Promotor und einem β-Actinpromotor ausgewählt ist bzw. sind.

13. Zelle nach einem der vorstehenden Ansprüche, bei welcher der selektierbare Marker aus einem Neomycin-Gen, einem Hypoxanthinphosphoribosyltransferase-Gen, einem Puromycin-Gen, einem Dihydrooratase-Gen, einem Glutaminsynthetase-Gen, einem Histidin D-Gen, einem Carbamylphosphatsynthase-Gen, einem Dihydrofolatreduktase-Gen, einem Mehrfacharzneistoffresistenz 1-Gen, einem Aspartattranscarbamylase-Gen, einem Xanthin-Guanin-Phosphoribosyltransferase-Gen, einem Adenosindeaminase-Gen oder einem Thymidinkinase-Gen ausgewählt ist.

14. Zelle nach einem der vorstehenden Ansprüche, wobei die Wirtszelle eine isolierte und klonierte Zelle ist.

15. Bibliothek von Zellen, die Zellen gemäß einem der vorstehenden Ansprüche umfasst.

16. In vitro-Verfahren zur Aktivierung oder Überexprimierung eines endogenen Gens zur Erzeugung von Protein in einer Zelle, umfassend:
(a) Transfizieren einer Genom-enthaltenden Zelle mit einem Vektor, wobei der Vektor umfasst: (i) einen ersten Promotor, der funktionell mit einem Exon und einer ungepaarten Spleißdonorstelle verknüpft ist, und (ii) einen zweiten Promotor, der funktionell mit einem selektierbaren Marker verknüpft ist, der kein Polyadenylierungssignal aufweist, wobei der erste und der zweite Promotor in dem Vektor in der gleichen Orientierung vorliegen und sowohl der erste als auch der zweite Promotor in der eukaryotischen Zelle eine Funktion ausüben, und
(b) nicht-homologes Integrierenlassen des Vektors in das Genom der Zelle, so dass die ungepaarte Donorstelle des Vektors mit einer Spleißakzeptorstelle des endogenen Gens gepaart wird.

17. Verfahren nach Anspruch 16, bei dem der Vektor gemäß einem der Ansprüche 2 bis 14 definiert ist.

## Revendications

1. Cellule hôte eucaryotique qui est, in *vitro,* la cellule comprenant un vecteur intégré de manière non homologue dans le génome de la cellule, le vecteur comprenant :
(a) un premier promoteur opérationnellement lié à un exon et à un site donneur d'épissage non apparié ; et
(b) un second promoteur opérationnellement lié à un marqueur de sélection dépourvu de signal de polyadénylation,
où lesdits premier et second promoteurs sont présents dans ledit vecteur dans la même orientation, les premier et second promoteurs fonctionnent tous deux dans la cellule eucaryotique, le vecteur est intégré de manière non homologue dans le génome de la cellule de telle sorte que le site donneur d'épissage non apparié du vecteur est apparié à un site accepteur d'épissage d'un gène endogène et où le gène endogène est activé ou surexprimé afin de produire une protéine.

2. Cellule selon la revendication 1, dans laquelle le marqueur de sélection est situé en 3' dudit premier promoteur.

3. Cellule selon la revendication 1, dans laquelle ledit second promoteur est situé en 5' du site donneur d'épissage non apparié.

4. Cellule selon l'une quelconque des revendications précédentes, dans laquelle ledit exon comprend un codon d'initiation de la traduction et une séquence signal de sécrétion.

5. Cellule selon l'une quelconque des revendications précédentes, ledit vecteur comprenant en outre un ou plusieurs signaux de transposition.

6. Cellule selon l'une quelconque des revendications précédentes, ledit vecteur comprenant en outre un ou plusieurs marqueurs amplifiables.

7. Cellule selon la revendication 6, dans laquelle ledit marqueur amplifiable est choisi dans le groupe constitué par la dihydrofolate-réductase, l'adénosine-désaminase, l'aspartate-transcarbamylase, la dihydroorotase et la carbamyl-phosphate-synthase.

8. Cellule selon l'une quelconque des revendications précédentes, ledit vecteur comprenant en outre une ou plusieurs origines virales de réplication.

9. Cellule selon la revendication 8, dans laquelle ladite origine virale de réplication est choisie dans le groupe constitué par l'ori P du virus Epstein Barr et l'ori du SV40.

10. Cellule selon l'une quelconque des revendications précédentes, ledit vecteur comprenant en outre un ou plusieurs gènes de facteurs de réplication virale.

11. Cellule selon l'une quelconque des revendications précédentes, ledit vecteur comprenant en outre de l'ADN génomique.

12. Cellule selon l'une quelconque des revendications précédentes, dans laquelle le premier et/ou le second promoteur(s) est/sont choisi(s) parmi un promoteur du gène précoce immédiat du CMV, un promoteur de l'antigène T du SV40, un promoteur inductible par la tétracycline et un promoteur β-actine.

13. Cellule selon l'une quelconque des revendications précédentes, dans laquelle ledit marqueur de sélection est choisi parmi un gène néomycine, un gène hypoxanthine-phosphoribosyl-transférase, un gène puromycine, un gène dihydrooratase, un gène glutamine-synthétase, un gène histidine D, un gène carbamyl-phosphate-synthase, un gène dihydrofolate réductase, un gène de résistance multiple aux médicaments 1, un gène aspartate-transcarbamylase, un gène xanthine-guanine phosphoribosyl-transférase, un gène adénosine-désaminase ou un gène thymidine-kinase.

14. Cellule selon l'une quelconque des revendications précédentes, dans laquelle ladite cellule hôte est une cellule isolée et clonée.

15. Banque de cellules comprenant des cellules selon l'une quelconque des revendications précédentes.

16. Procédé *in vitro* pour activer ou surexprimer un gène endogène afin de produire une protéine dans une cellule, comprenant :
(a) la transfection d'une cellule contenant un génome avec un vecteur, où le vecteur comprend : (i) un premier promoteur opérationnellement lié à un exon et à un site donneur d'épissage non apparié ; et (ii) un second promoteur opérationnellement lié à un marqueur de sélection dépourvu de signal de polyadénylation, où les premier et second promoteurs sont présents dans ledit vecteur dans la même orientation et le premier et le second promoteurs fonctionnent tous deux dans la cellule eucaryotique ; et
(b) la permission au vecteur de s'intégrer de manière non homologue dans le génome de la cellule de sorte que le site donneur non apparié du vecteur est apparié avec un site accepteur d'épissage du gène endogène.

17. Procédé selon la revendication 16, dans lequel le vecteur est comme défini selon l'une quelconque des revendications 2 à 14.
